(19) Europäisches Patentamt
European Patent Office
Office européen des brevets

(11) **EP 4 623 937 A1**

(12) **EUROPEAN PATENT APPLICATION**
published in accordance with Art. 153(4) EPC

(43) Date of publication:
**01.10.2025 Bulletin 2025/40**

(21) Application number: **23894038.1**

(22) Date of filing: **26.11.2023**

(51) International Patent Classification (IPC):
***A61K 47/68*** *(2017.01)* ***C07D 491/22*** *(2006.01)*
***C07K 16/28*** *(2006.01)* ***A61K 39/395*** *(2006.01)*
***A61P 35/00*** *(2006.01)*

(52) Cooperative Patent Classification (CPC):
**A61K 39/395; A61K 47/68; A61P 35/00; C07D 491/22; C07K 16/28**

(86) International application number:
**PCT/CN2023/134190**

(87) International publication number:
**WO 2024/109949 (30.05.2024 Gazette 2024/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.11.2022 CN 202211494434**
**11.07.2023 CN 202310846979**

(71) Applicants:
- **Minghui Pharmaceutical (Hangzhou) Limited**
  **Hangzhou, Zhejiang 310018 (CN)**
- **Minghui Pharmaceutical (Shanghai) Limited**
  **Pilot Free Trade Zone**
  **Pudong New Area Shanghai 201203 (CN)**

(72) Inventors:
- **LI, Ao**
  **Hangzhou, Zhejiang 310018 (CN)**
- **YAO, Zhili**
  **Hangzhou, Zhejiang 310018 (CN)**
- **CHEN, Yile**
  **Hangzhou, Zhejiang 310018 (CN)**
- **ZHU, Liyuan**
  **Hangzhou, Zhejiang 310018 (CN)**
- **CAO, Guoqing**
  **Hangzhou, Zhejiang 310018 (CN)**

(74) Representative: **Lavoix**
**Bayerstraße 83**
**80335 München (DE)**

# (54) ANTI-TUMOR COMPOUND AND USE THEREOF

(57) The present invention provides an anti-tumor compound and its application. Specifically, the present invention provides a ligand-drug conjugate, or its tautomer, mesomer, racemate, enantiomer, diastereomer, or pharmaceutically acceptable salt or hydrate, wherein the ligand-drug conjugate comprises a structure of formula (I). The ligand-drug conjugate can be used as an anti-tumor drug.

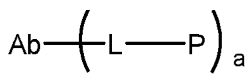
(I)

EP 4 623 937 A1

## Description

### Technical field

This application relates to the field of biomedicine, specifically to an anti-tumor compound and its application.

### Background

Antibody-drug conjugate (ADC) connects monoclonal antibodies or antibody fragments to biologically active cytotoxic toxins via stable chemical linker compounds. They make full use of the specificity of antibodies for binding to surface antigens on normal and tumor cells, as well as the high efficiency of cytotoxic substances to have an anti-tumor effect. The application of the camptothecin derivative Exatecan in antibody-drug conjugate (ADC) has been reported in the literature, but the field still needs to further develop ADC drugs with better efficacy and safety. However, currently marketed ADC drugs still have problems such as poor plasma stability and off-target toxic side effects, which affect the efficacy and safety of the products. There is still a need to further develop new, stable linkers and/or linker-drug molecules to solve these problems.

### Summary of the present invention

The present invention provides a ligand-drug conjugate with high stability, good therapeutic effect, and high safety, as well as its ligand-drug conjugate precursor, linker, linker precursor, etc.

The first aspect of the present invention provides a ligand-drug conjugate, or its tautomer, mesomer, racemate, enantiomer, diastereomer, or pharmaceutically acceptable salt or hydrate, wherein the ligand-drug conjugate comprises the structure shown in formula (I):

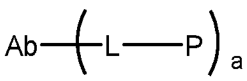

(I) ,

Wherein, L is an optionally substituted linker that is linked to any O atom, S atom or N atom in the P structure;

Ab is a ligand, "a" is a number greater than 0, and "a" is a decimal or integer;
P is a toxin, and the P has the structure shown in the following formula (II):

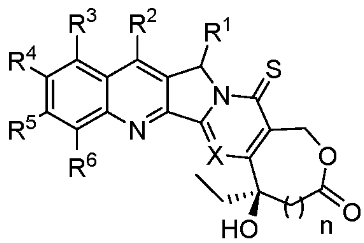

(II)

Wherein, n is 0 or 1;

X is N and $CR^0$;

$R^0$ is selected from the group consisting of H, D, halogen, $C_1$-$C_8$ alkyl, $C_1$-$C_8$ alkoxy, OH, $NH_2$, $N_3$ and $NO_2$;

$R^1$ is selected from the group consisting of hydrogen atom, deuterium atom, halogen, $C_1$-$C_8$ alkyl, $C_1$-$C_8$ alkoxy, $C_1$-$C_8$ haloalkyl, $C_1$-$C_8$ haloalkoxy, $N_3$, $NO_2$, $NH_2$, NH-OH, - NR'R", -COOR', -CONR'R", and -NHR'''NR'R"; wherein, R', R" and R''' are each independently selected from the group consisting of hydrogen, deuterium, alkyl, aryl, arylalkyl, acyl, alkoxycarbonyl, and aryloxycarbonyl;

$R^2$, $R^3$, $R^4$, $R^5$ and $R^6$ are each independently selected from the group consisting of hydrogen atom, deuterium atom, halogen, hydroxyl, cyano, $NH_2$, $NO_2$, substituted or unsubstituted $C_1$-$C_8$ alkyl, substituted or unsubstituted $C_1$-$C_8$ alkoxy, substituted or unsubstituted $C_1$-$C_8$ alkylthio, substituted or unsubstituted $C_1$-$C_8$ deuterated alkyl, $-(CH_2)_m$-

tri-($C_1$-$C_4$ alkyl)silyl, -$(CH_2)_m$($C_3$-$C_8$ cycloalkyl), -$(CH_2)_m$(3-12 membered heterocyclic group), -$(CH_2)_mN(R^7)_2$, -$(CH_2)_mS(CH_2)_pR^7$, -$(CH_2)_mS(O)(CH_2)_pR^7$, -$(CH_2)_mS(O)_2(CH_2)_pR^7$, -$(CH_2)_mNH(CH_2)_pR^7$, -$(CH_2)_mNHC(O)(CH_2)$ $pR^7$, -$(CH_2)_mOC(O)(CH_2)_pR^7$, -$(CH_2)_mC(O)(CH_2)_pR^7$ and -CH=N(OtBu); wherein, m and p are each independently 0, 1, 2, 3 or 4;

or $R^2$ and $R^3$ together with the carbon atoms to which they are attached form a substituted or unsubstituted $C_5$-$C_8$ carbocycle or a substituted or unsubstituted 5-12 membered heterocycle;

or $R^3$ and $R^4$, or $R^4$ and $R^5$, together with the carbon atoms to which they are attached form a structure selected from the group consisting of a saturated or unsaturated 5-12 membered carbocycle that is unsubstituted or substituted by one or more $R^e$, and a saturated or unsaturated 5-12 membered heterocycle that is unsubstituted or substituted by one or more $R^e$; wherein $R^e$ is a substituted or unsubstituted substituent selected from the group consisting of hydrogen, deuterium, halogen, cyano, nitro, hydroxyl, amino, $C_1$-$C_6$ alkyl-NH-, ($C_1$-$C_6$ alkyl)$_2$N-, $C_1$-$C_6$ alkyl, $C_2$-$C_6$ alkenyl, $C_2$-$C_6$ alkynyl, $C_1$-$C_6$ alkoxy, allyl, benzyl, $C_6$-$C_{12}$ aryl, $C_1$-$C_6$ alkoxy-$C_1$-$C_6$ alkyl, $C_1$-$C_6$ alkoxycarbonyl, phenoxycarbonyl, $C_2$-$C_6$ alkynylcarbonyl, $C_2$-$C_6$ alkenylcarbonyl, $C_3$-$C_6$ cycloalkylcarbonyl, $C_1$-$C_6$ alkylsulfonyl, phenyl, 5-7 membered heteroaryl, $C_3$-$C_8$ cycloalkyl, 3-12 membered heterocyclic group, - $(CH_2)_mN(R^7)_2$, -$(CH_2)_mS(CH_2)_pR^7$, -$(CH_2)_mS(O)(CH_2)_pR^7$, -$(CH_2)_mS(O)_2(CH_2)_pR^7$, - $(CH_2)_mNH(CH_2)_pR^7$, -$(CH_2)_mNHC(O)(CH_2)_pR^7$, -$(CH_2)_mOC(O)$ $(CH_2)_pR^7$, and - $(CH_2)_mC(O)(CH_2)_pR^7$; wherein, m and p are each independently 0, 1, 2, 3 or 4, preferably 0, 1 or 2;

$R^7$ is each independently selected from the group consisting of hydrogen atom, deuterium atom, halogen, substituted or unsubstituted $C_1$-$C_8$ alkyl, $C_1$-$C_8$ haloalkyl, $C_1$-$C_8$ deuterated alkyl, substituted or unsubstituted $C_1$-$C_8$ alkoxy, hydroxyl, amino, cyano, nitro, thiol, substituted or unsubstituted $C_1$-$C_8$ alkylene-OH, substituted or unsubstituted $C_1$-$C_8$ alkylene-$NH_2$, $SO_2Me$, -OC(O)(substituted or unsubstituted $C_1$-$C_4$ alkyl), -C(O)(substituted or unsubstituted $C_1$-$C_4$ alkyl), substituted or unsubstituted phenyl, substituted or unsubstituted 5-7 membered heteroaryl, substituted or unsubstituted $C_3$-$C_8$ cycloalkyl, and substituted or unsubstituted 3-12 membered heterocyclic group;

unless otherwise specified, each "substituted" refers to that one or more hydrogen atoms on the group are substituted by substituents selected from the group consisting of hydrogen, deuterium, halogen, cyano, nitro, hydroxyl, amino, $C_1$-$C_6$ alkyl-NH-, ($C_1$-$C_6$ alkyl)$_2$N-, $C_1$-$C_6$ alkyl, $C_2$-$C_6$ alkenyl, $C_2$-$C_6$ alkynyl, $C_1$-$C_6$ alkoxy, $C_1$-$C_6$ haloalkyl, $C_2$-$C_6$ haloalkenyl, $C_2$-$C_6$ haloalkynyl, $C_1$-$C_6$ haloalkoxy, allyl, benzyl, $C_6$-$C_{12}$ aryl, $C_1$-$C_6$ alkoxy-$C_1$-$C_6$ alkyl, $C_1$-$C_6$ alkoxycarbonyl, phenoxycarbonyl, $C_2$-$C_6$ alkynylcarbonyl, $C_2$-$C_6$ alkenylcarbonyl, $C_3$-$C_6$ cycloalkylcarbonyl, $C_1$-$C_6$ alkylsulfonyl, phenyl, 5-7 membered heteroaryl, $C_3$-$C_8$ cycloalkyl, 3-12 membered heterocyclic group.

In another preferred embodiment, the L has the structure shown in the following formula:

$$-L_1-L_2-L_3-L_4-L_5-;$$

Wherein, $L_1$ is an optionally substituted

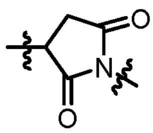 , 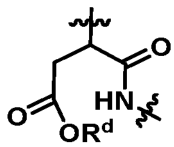 , or 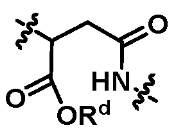 ,

and $R^d$ is H, $C_1$-$C_6$ alkyl, $C_1$-$C_6$ deuterated alkyl, $C_3$-$C_8$ cycloalkyl, or $C_3$-$C_8$ deuterated cycloalkyl;

The $L_2$ is a group selected from the group consisting of optionally substituted -$(CHR)_{m1}$-$X_1$-$(CH_2CH_2O)_{n3}$-$(CHR)_{m2}$-C(O)-, optionally substituted -$(CHR)_{m1}$-$X_1$-$X_2$-$(CH_2CH_2O)_{n3}$- $(CHR)_{m2}$-C(O)-, optionally substituted -$X_1$-$(CHROCHR)_{m2}$-C (O)-, optionally substituted-$(CHR)_{p1}$-C(O)-, optionally substituted -$(CHR)_{m1}$-$X_1$-$(CHR)_{m2}$-C(O)-, optionally substituted -$(CHR)_{m1}$-$X_1$-$(CHR)_{n3}$-$X_2$-$(CHR)_{m2}$-C(O)-, optionally substituted -$X_1$-$(CHR)_{m1}$-$X_2$-$(CHR)_{m2}$-C (O)-, and optionally substituted -( $CH_2CH_2O)_{n3}$-C(O)-;

$X_1$ and $X_2$ are each independently selected from the group consisting of -O-, -C(O)-, -C(O)-NR-, optionally substituted $C_6$-$C_{10}$ aryl, optionally substituted 5-9 membered heteroaryl, optionally substituted 3-8 membered heteroalicyclic group, and an optionally substituted $C_3$-$C_6$ alicyclic group;

wherein each R is independently selected from the group consisting of H, D, $(CH_2)_{n4}OH$, $(CH_2)_{n4}NH_2$, $(CH_2O)_{n4}$ $(CH_2CH_2O)_{n5}H$, $(CH_2O)_{n4}(CH_2CH_2O)_{n5}CH_3$, $(CH_2)_{n4}OCH_3$, $(CH_2CH_2O)_{n5}CH_3$, $CH_2C(O)NH(CH_2O)_{n4}$ $(CH_2CH_2O)_{n5}H$, and $CH_2C(O)NH(CH_2O)_{n4}(CH_2CH_2O)_{n5}CH_3$;

wherein m1, m2, n3, n4, and n5 are each independently 0, 1, 2, 3, 4, 5, 6, 7, 8, 9, 10, 11, or 12; and p1 is 0, 1, 2, 3, 4, 5, 6, 7, or 8;

The $L_3$ is a peptide residue; and the $L_3$ can be substituted by one or more substituents selected from the group consisting of

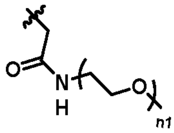,
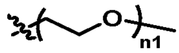,

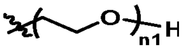

and $CH_2C(O)R^c$; the $R^c$ is selected from the group consisting of

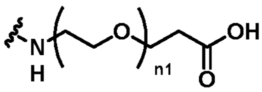, and 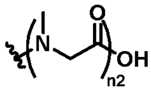;

wherein n1 and n2 are each independently 0, 1, 2, 3, 4, 5, 6, 7, 8, 9, 10, 11 or 12;

The $L_4$ is optionally substituted $-L_{4a}-(NR^b)_{n6}-R^{12}-L_{4b}-$, wherein $L_{4a}$ is absent or optionally substituted bond, $CH_2$, or $CD_2$;

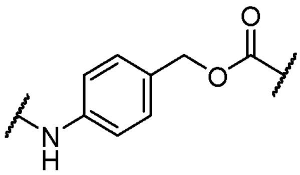;

wherein n6 is 0 or 1; and $R^{12}$ is chemical

$L_{4b}$ is absent or optionally substituted

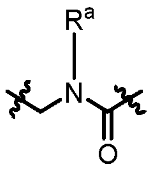,

wherein $R^a$ and $R^b$ are each independently selected from the group consisting of hydrogen, optionally substituted $C_1$-$C_4$ alkyl, and optionally substituted $C_1$-$C_4$ deuterated alkyl;

The $L_5$ is absent or optionally substituted

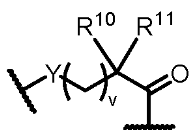,

wherein Y is selected from the group consisting of O, S and NH; and v is 0, 1, 2, 3, 4, 5, 6, 7, 8, 9 or 10; $R^{10}$ and $R^{11}$ are each independently selected from the group consisting of hydrogen, deuterium, optionally substituted $C_1$-$C_4$ alkyl, optionally substituted $C_1$-$C_4$ haloalkyl, optionally substituted $C_3$-$C_6$ cycloalkyl, and optionally substituted $C_4$-$C_8$ cycloalkylalkyl, or $R^{10}$ and $R^{11}$ together with the atom to which they are attached form an optionally substituted

3-6 membered cycloalkyl, wherein $R^{10}$ and $R^{11}$ are each independently selected from the group consisting of hydrogen, deuterium, halogen, optionally substituted $C_1$-$C_8$ alkyl, optionally substituted $C_1$-$C_8$ haloalkyl, and optionally substituted $C_1$-$C_8$ deuterated alkyl.

In another preferred embodiment, the $L_1$ is

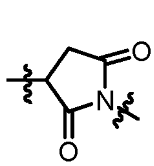
,
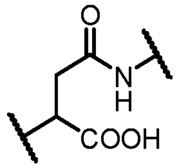
,
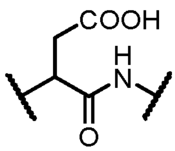
,
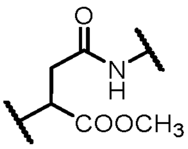
,
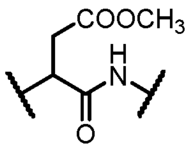
,
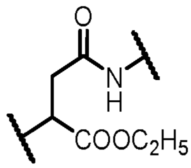
, or
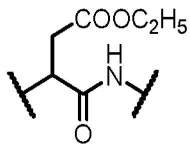
.

In another preferred embodiment, $X_1$ and $X_2$ are each independently selected from the group consisting of -O-, -C(O)-, -C(O)-NR-, optionally substituted phenyl, optionally substituted pyridyl, optionally substituted $C_3$-$C_6$ cycloalkyl, optionally substituted

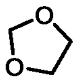
,

and optionally substituted

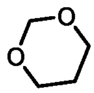
.

In another preferred embodiment, the $L_2$ is selected from the group consisting of optionally substituted $-(CH_2)_{m1}-X_1$ $-(CH_2CH_2O)_{n3}-(CH_2)_{m2}-C(O)-$; wherein, $X_1$ is -C(O)-NH-; Preferably, m1 and m2 are each independently 1, 2, or 3; n3 is 0, 1, 2, 3, 4, 5, 6, 7, 8, 9, 10, 11, or 12.

In another preferred embodiment, $L_2$ is a group selected from the group consisting of optionally substituted $-(CHR)_{m1}-X_1-X_2-(CH_2CH_2O)_{n3}-(CHR)_{m2}-C(O)-$; wherein, $X_1$ is an optionally substituted

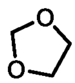

or an optionally substituted

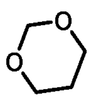
;

$X_2$ is-C(O)-NR-; preferably, m1 and m2 are each independently 0, 1 or 2; n3 is 1, 2, 3, 4, 5, 6, 7, 8, 9, 10, 11 or 12.

In another preferred embodiment, the $L_2$ is a group selected from the group consisting of optionally substituted $-X_1-(CHROCHR)_{m2}-C(O)-$; wherein $X_1$ is optionally substituted aryl or optionally substituted heteroaryl; preferably, m2 is 0, 1, 2 or 3.

In another preferred embodiment, $L_2$ is a group selected from the group consisting of optionally substituted $(CHR)_{p1}-C(O)-$; p1 is selected from 0, 1, or 2; R is selected from the group consisting of H, $(CH_2)_{n4}OH$, and $(CH_2O)_{n4}(CH_2CH_2O)_{n5}H$; preferably, n4 and n5 are each independently 0, 1, 2, or 3.

In another preferred embodiment, the $L_2$ is a group selected from the group consisting of optionally substitu-

ted-$(CH_2)_{m1}$-$X_1$-$(CH_2CH_2O)_{n3}$-$(CHR)_{m2}$-C(O)-; wherein $X_1$ is -C(O)-; preferably, m1 and m2 are each independently 0, 1, 2, or 3; and n3 is 0, 1, or 2.

In another preferred embodiment, the $L_2$ is a group selected from the group consisting of optionally substituted -$X_1$-$(CH_2)_{m1}$-$X_2$-$(CHR)_{m2}$-C(O)-; wherein $X_1$ is optionally substituted aryl or optionally substituted heteroaryl; $X_2$ is -C(O)-; preferably, m1, m2 are each independently 0, 1, or 2.

In another preferred embodiment, the $L_2$ is a group selected from the group consisting of -$(CHR)_{m1}$-$X_1$-$(CHR)_{m2}$-C(O)-; wherein $X_1$ is an optionally substituted 3-8 membered heteroalicyclic group or optionally substituted $C_3$-$C_6$ alicyclic group; preferably, m1 is 0, 1, or 2, m2 is 0.

In another preferred embodiment, the $L_2$ is a group selected from the group consisting of an optionally substituted -$(CHR)_{m1}$-$X_1$-$(CH_2CH_2O)_{n3}$-$(CHR)_{m2}$-C(O)-; wherein $X_1$ is O; preferably, m1, n3 and m2 are each independently 0, 1 or 2.

In another preferred embodiment, the $L_2$ is a group selected from the group consisting of optionally substituted -$(CHR)_{m1}$-$X_1$-$(CHR)_{n3}$-$X_2$-$(CHR)_{m2}$-C(O)-; wherein $X_1$ is optionally substituted -C(O)-NR-, $X_2$ is O; preferably, m1, n3 and m2 are each independently 1, 2 or 3; R is defined as above.

In another preferred embodiment, the $L_2$ is an optionally substituted structure selected from the group consisting of

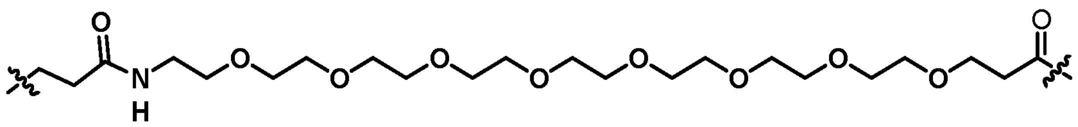

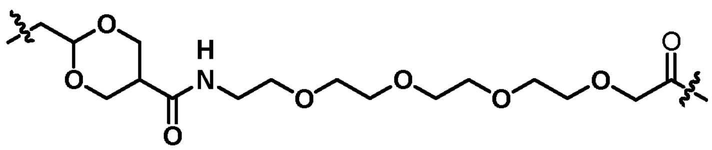,
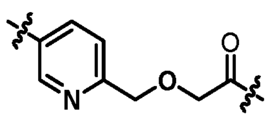,
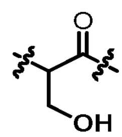,

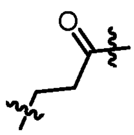,
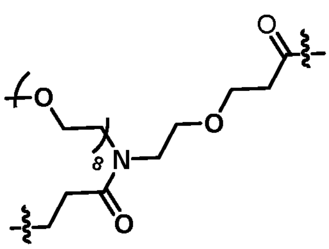,
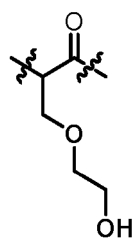,
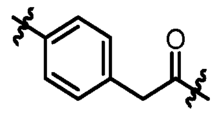,
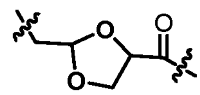,

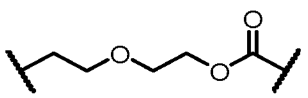,
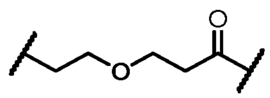,
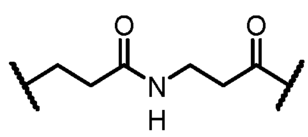,
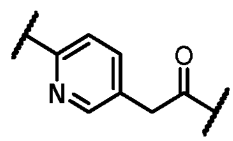,

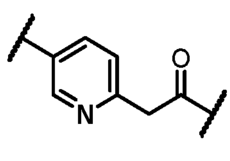,
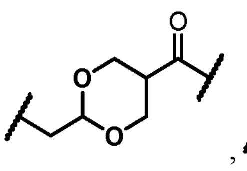,
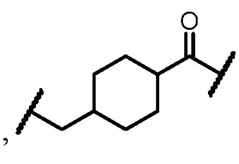 and
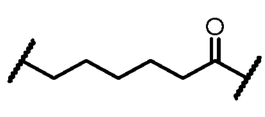.

In another preferred embodiment, selecting the $L_2$ structure defined above enable the maleimide group of the resulting ligand-drug conjugate to form an ring-opened structure, thereby inhibiting the Retro-Michael reaction, improving the stability of the ligand-drug conjugate, reducing the disengagement of the small-molecule moiety, and thus improving the safety of the ligand-drug conjugate.

In another preferred embodiment, the $L_3$ is an unsubstituted or $CH_2C(O)R^c$-substituted peptide residue composed of amino acids selected from the group consisting of phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine.

In another preferred embodiment, the $L_3$ is an unsubstituted or $CH_2C(O)R^c$-substituted peptide residue composed of amino acids selected from the group consisting of glycine, alanine, lysine, phenylalanine, valine and citrulline.

In another preferred embodiment, the $L_3$ is an unsubstituted or $CH_2C(O)R^c$-substituted peptide residue selected from the group consisting of -Glycine-Phenylalanine-Glycine-(-Gly-Phe-Gly-), -Glycine-Glycine-Phenylalanine-Glycine-(-Gly-Gly-Phe-Gly-), -Valine-Citrulline-(-Val-Cit-), -Citrulline-Valine-(-Cit-Val-), -Citrulline- Alanine -(-Cit-Vla-), -Valine-Alanine-(-Val-Ala-), -Valine-Arginine-(-Val-Arg-), -Valine-Lysine-(-Val-Lys-), -Valine-Lysine (Ac)-(-Val-Lys(Ac)-), -Lysine-Valine-(-Lys-Val-), -Leucine-Citrulline-(-Leu-Cit-), -Isoleucine-Citrulline-(-Ile-Cit-), -Tryptophan-Citrulline-(-Trp-Cit-), -Phenylalanine-Lysine-(-Phe-Lys-), - Phenylalanine-Lysine(Ac)-(-Phe-Lys(Ac)-), -Phenylalanine-Citrulline-(-Phe-Cit), -Phenylalanine-Alanine-(-Phe-Ala-), -Phenylalanine-Arginine-(-Phe-Arg-), -Alanine-Lysine-(-Ala-Lys-), -Alanine-Alanine-(-Ala-Ala-), -Alanine-Alanine-Alanine-(-Ala-Ala-Ala-), -Alanine-Alanine-Asparagine-(-Ala-Ala-Asn-), -Alanine-Alanine-Aspartic Acid-(-Ala-Ala-Asp-), - Lysine-Alanine-Alanine-Asparagine-(-Lys-Ala-Ala-Asn-), -Lysine-Alanine-Alanine-Aspartic acid-(-Lys-Ala-Ala-Asp-), -(D)-Valine-Leucine-Lysine-(-(D)-Val-Leu-Lys-), -Glycine-Glycine-Arginine-(-Gly-Gly-Arg-), -Glycine-Glycine-Asparagine- (-Gly-Gly-Asn-), -Glycine-Glycine-Phenylalanine-(-Gly-Gly-Phe-), -Valine-Lysine-Glycine-(-Val-Lys-Gly-), -Glutamic Acid-Alanine-Alanine-(Glu-Ala-Ala-), -Aspartate-Alanine-Alanine-(Asp-Ala-Ala)-, -Valine-Lysine-Glycine-Glycine-(-Val-Lys-Gly-Gly-), and -Lysine-Alanine-Asparagine-(-Lys-Ala-Asn-).

In another preferred embodiment, the $L_3$ is an unsubstituted or $CH_2C(O)R^c$- substituted structure selected from the group consisting of

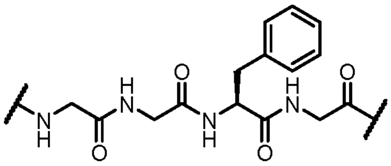 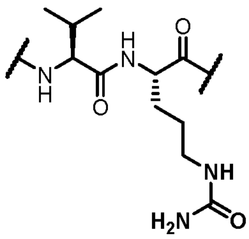 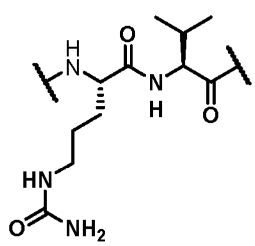 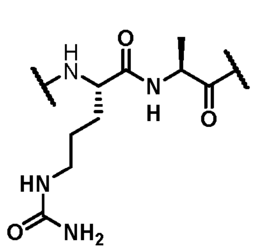

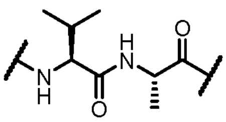 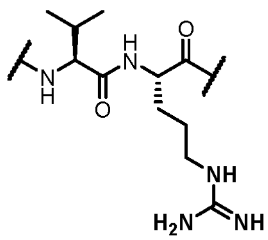 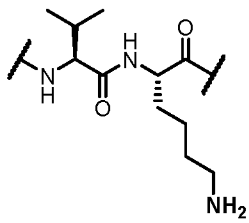 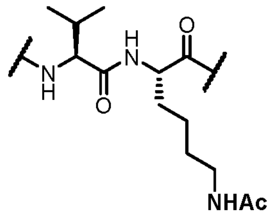

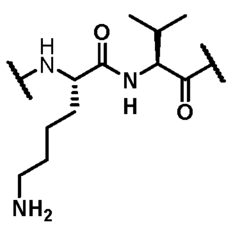 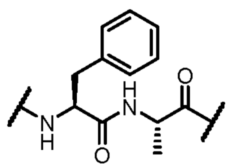 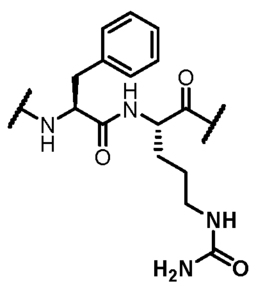 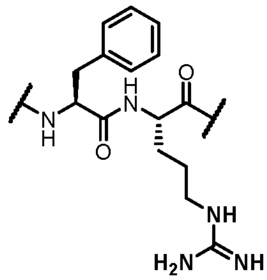

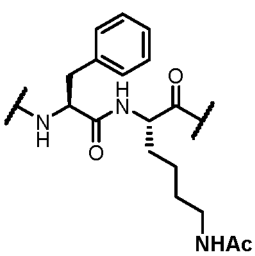 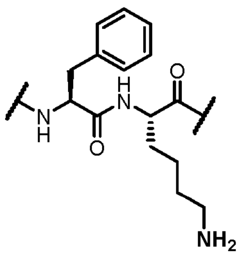 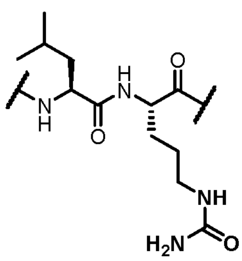 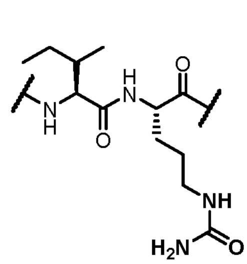

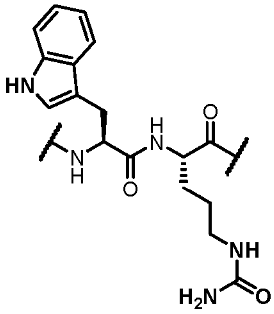
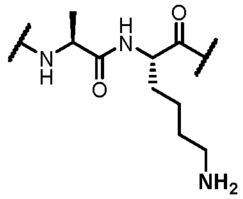
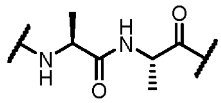
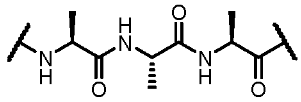

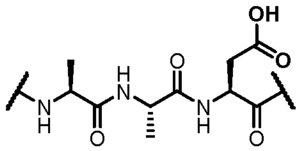
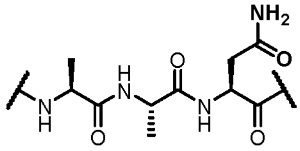
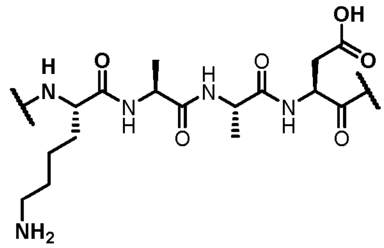

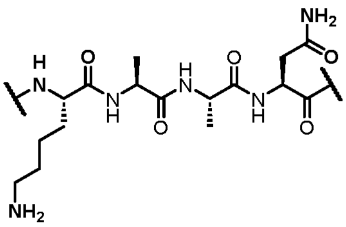
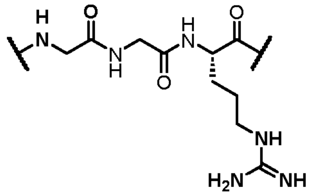
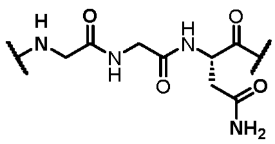

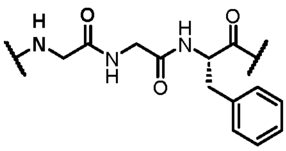
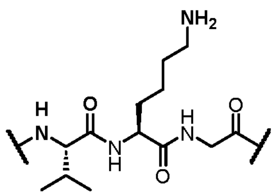
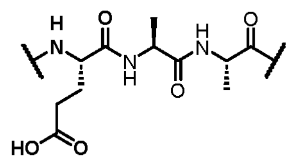

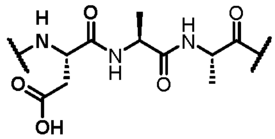
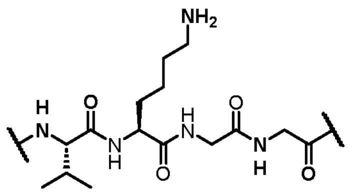
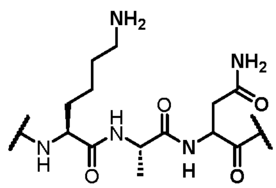

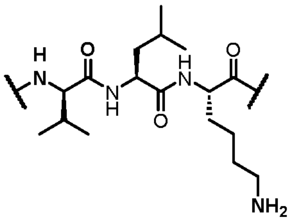
and
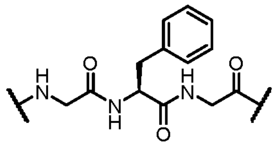

In another preferred embodiment, the $L_4$ is a chemical bond or an optionally substituted group selected from the group consisting of

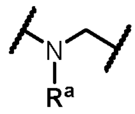, 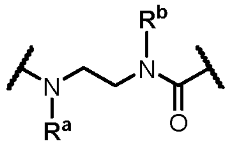,

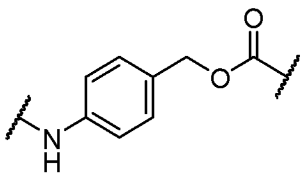, 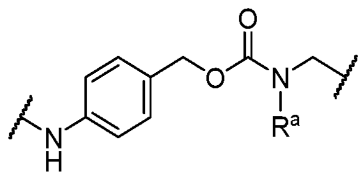 and 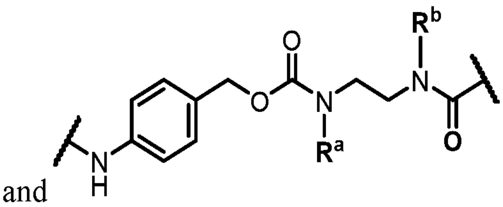;

wherein $R^a$ and $R^b$ are each independently selected from the group consisting of hydrogen, optionally substituted $C_1$-$C_4$ alkyl, and optionally substituted $C_1$-$C_4$ deuterated alkyl.

In another preferred embodiment, the $L_4$ is a chemical bond or is an optionally substituted structure selected from the group consisting of

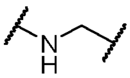, 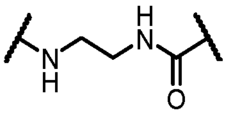, 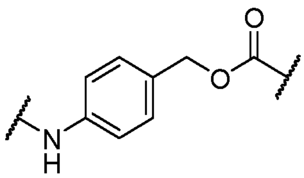, 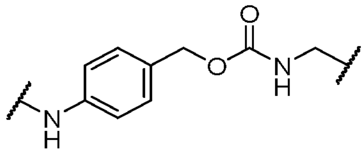,

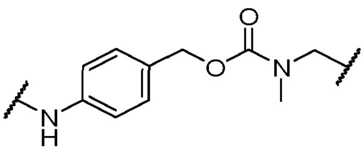 and 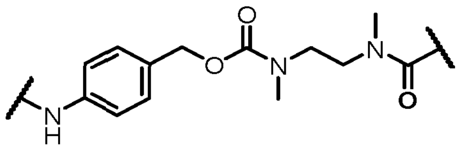.

In another preferred embodiment, the $L_5$ is a chemical bond or a structure selected from the group consisting of optionally substituted

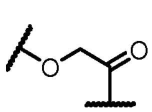, 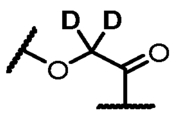,

optionally substituted

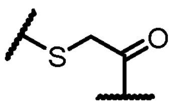,

optionally substituted

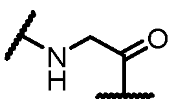,

optionally substituted

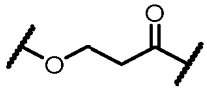
,

optionally substituted

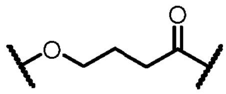
,

optionally substituted

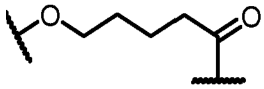
,

optionally substituted

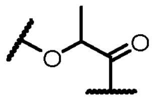
,

optionally substituted

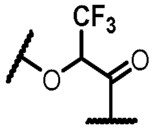
,

optionally substituted

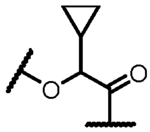
,

optionally substituted

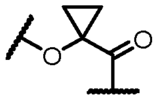
,

optionally substituted

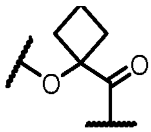
,

optionally substituted

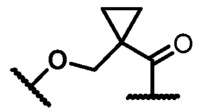

and optionally substituted

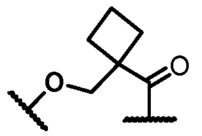

.

In another preferred embodiment, the formula (II) was selected from the group consisting of

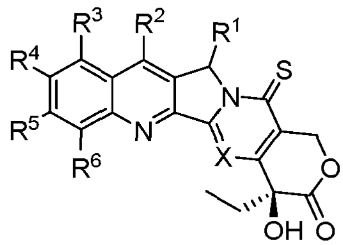

IIA

and

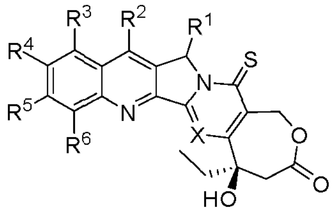

IIB.

In another preferred embodiment, the formula (II) was selected from the group consisting of

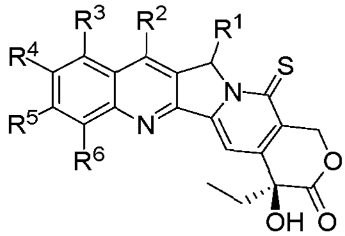

IV

and

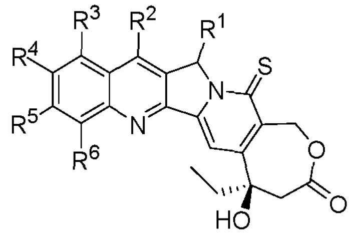

V.

In another preferred embodiment, the formula (II) was selected from the group consisting of

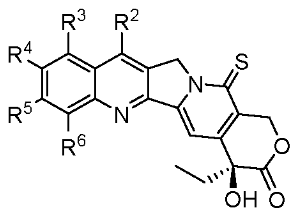

VI

and

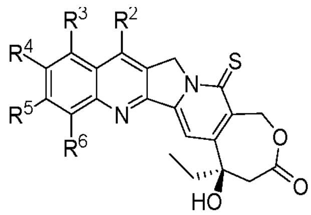

VII.

In another preferred embodiment, the $R^4$ is selected from the group consisting of hydrogen atom, deuterium atom, halogen, hydroxyl, cyano, $NH_2$, $NO_2$, substituted or unsubstituted $C_1$-$C_8$ alkyl, substituted or unsubstituted $C_1$-$C_8$ alkoxy, substituted or unsubstituted $C_1$-$C_8$ alkylthio, substituted or unsubstituted $C_1$-$C_8$ deuterated alkyl, - $(CH_2)_m(C_3$-$C_8$ cycloalkyl), $-(CH_2)_m$(3-12 membered heterocyclic group), $-(CH_2)_mN(R^7)_2$, - $(CH_2)_mS(O)(CH_2)_pR^7$, $-(CH_2)_mS(O)_2(CH_2)_pR^7$, and $-(CH_2)_mNH(CH_2)_pR^7$; wherein m and p are each independently 0, 1 or 2, $R^7$ is defined as above;

$R^5$ is selected from the group consisting of hydrogen atom, deuterium atom, halogen, $NH_2$, OH, substituted or unsubstituted $C_1$-$C_8$ alkyl, and substituted or unsubstituted $C_1$-$C_8$ alkoxy;

or $R^4$ and $R^5$ together with the carbon atoms to which they are attached form a structure selected from the group consisting of unsaturated or saturated 5-6 membered carbocycle which is unsubstituted or substituted by one or more

$R^e$, and unsaturated or saturated 5-6 membered heterocycle which is unsubstituted or substituted by one or more $R^e$; wherein $R^e$ is defined as above.

In another preferred embodiment, $R^2$ and $R^3$ are each independently selected from the group consisting of hydrogen atom, deuterium atom, halogen, $NH_2$, substituted or unsubstituted $C_1$-$C_8$ alkyl, substituted or unsubstituted $C_1$-$C_8$ deuterated alkyl, $-(CH_2)_m(C_3$-$C_6$ cycloalkyl), $-(CH_2)_m$(3-6 membered heterocyclic group), and $-(CH_2)_mN(R^7)_2$, $-(CH_2)_mOC(O)R^7$; wherein m is 0, 1, 2, 3 or 4, $R^7$ is defined as above;
or $R^2$ and $R^3$ together with the carbon atoms to which they are attached form a structure selected from the group consisting of 5-6 membered saturated or unsaturated ring which is unsubstituted or substituted by one or more $R^e$ groups, and 5-6 membered saturated or unsaturated heterocycle which is unsubstituted or substituted by one or more $R^e$ groups; wherein $R^e$ is defined as above.

In another preferred embodiment, $R^1$ and $R^6$ are each independently hydrogen atom.

In another preferred embodiment, the $R^4$ and $R^5$ are each independently selected from the group consisting of hydrogen atom, deuterium atom, halogen, hydroxyl, $NH_2$ and substituted or unsubstituted $C_1$-$C_4$ alkyl;
or $R^4$ and $R^5$ together with the carbon atoms to which they are attached form an oxa-5-6 membered heterocycle which is unsubstituted or substituted by one or more $R^e$; wherein $R^e$ is defined as above.

In another preferred embodiment, $R^2$ is selected from the group consisting of a deuterium atom, halogen, $NH_2$, substituted or unsubstituted $C_1$-$C_8$ alkyl, substituted or unsubstituted $C_1$-$C_8$ deuterated alkyl, $-(CH_2)_m(C_3$-$C_6$ cycloalkyl), $-(CH_2)_m$(3-6 membered heterocyclic group), $-(CH_2)_mN(R^7)_2$, and $-(CH_2)_mOC(O)R^7$; wherein m is 0, 1 , 2, 3, or 4;

$R^3$ is each selected from the group consisting of hydrogen atom, deuterium atom, halogen, substituted or unsubstituted $C_1$-$C_8$ alkyl, substituted or unsubstituted $C_1$-$C_8$ deuterated alkyl, $-(CH_2)_m(C_3$-$C_6$ cycloalkyl), $-(CH_2)_m$(3-6 membered heterocyclic group), $-(CH_2)_mN(R^7)_2$, and $-(CH_2)_mOC(O)R^7$; wherein m is 0, 1, 2, 3 or 4;
or $R^2$ and $R^3$ together with the carbon atoms to which they are attached form a structure selected from the group consisting of unsaturated or saturated 5-6 membered ring which is unsubstituted or substituted by one or more $R^e$, and unsaturated or saturated 5-6 membered heterocycle which is unsubstituted or substituted by one or more $R^e$;
$R^4$ is selected from the group consisting of hydrogen atom, deuterium atom, halogen, hydroxyl, cyano, $NH_2$, $NO_2$, substituted or unsubstituted $C_1$-$C_8$ alkyl, and substituted or unsubstituted $C_1$-$C_8$ alkoxy;
$R^5$ is selected from the group consisting of hydrogen atom, deuterium atom, halogen, and substituted or unsubstituted $C_1$-$C_8$ alkyl;
or $R^4$ and $R^5$ together with the carbon atoms to which they are attached form a unsubstituted or one or more $R^e$ substituted group selected from the group consisting of $-OCH_2O-$, and $-O(CH_2)_2O-$;
$R^7$ is selected from the group consisting of hydrogen atom, deuterium atom, halogen, substituted or unsubstituted $C_1$-$C_8$ alkyl, hydroxy, amino, cyano, nitro, and thiol;
wherein $R^e$ is defined as above.

In another preferred embodiment, the compounds of formula (II) are selected from the group consisting of

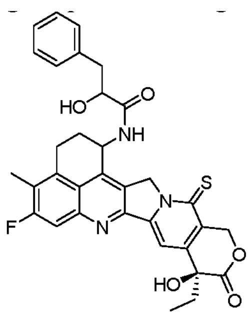 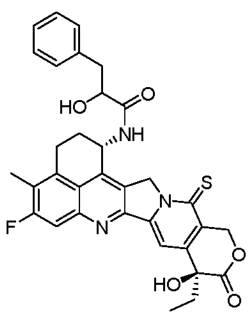 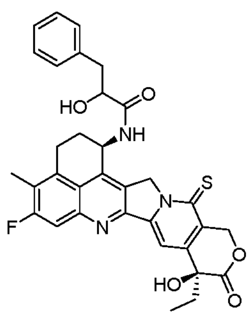 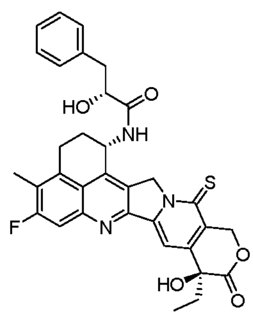

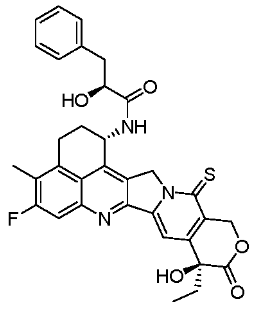
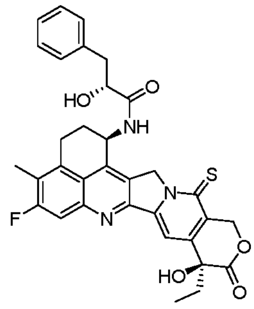
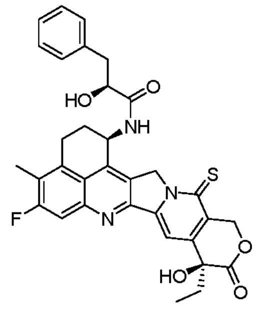
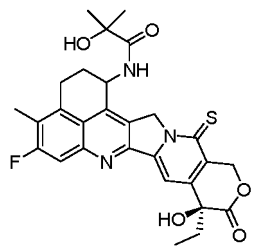
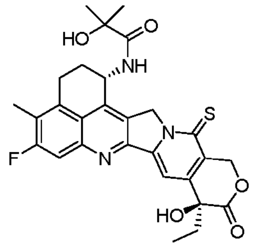
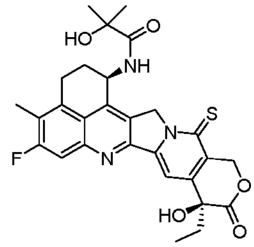
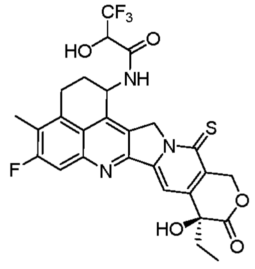
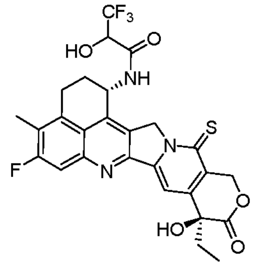
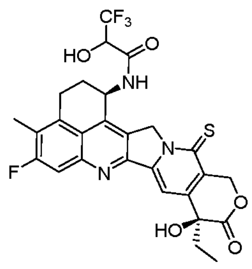
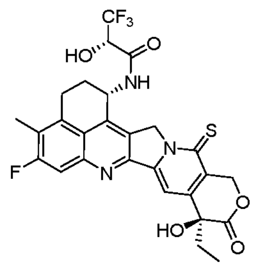
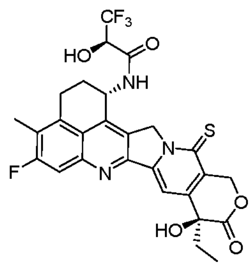
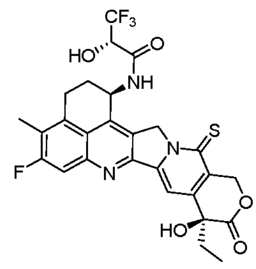
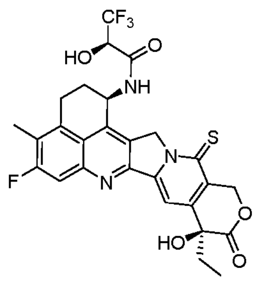
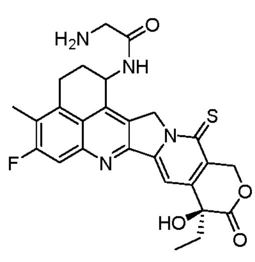
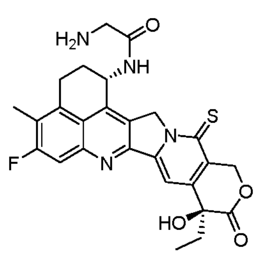
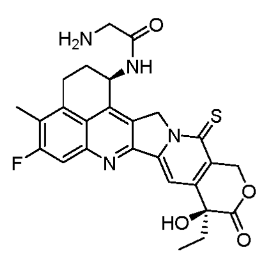
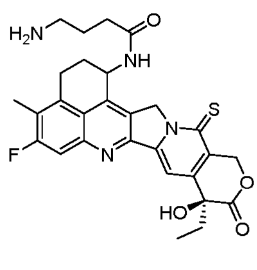
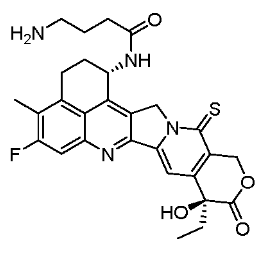
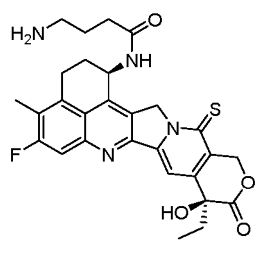
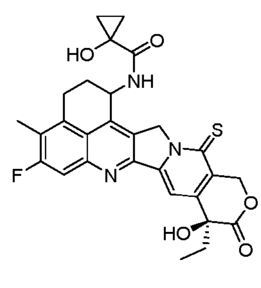

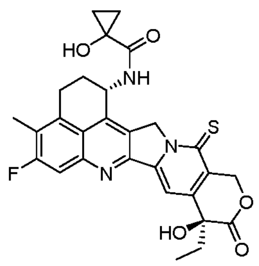
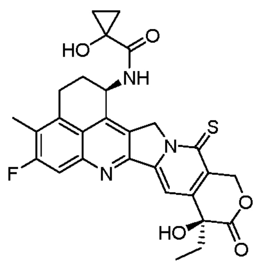
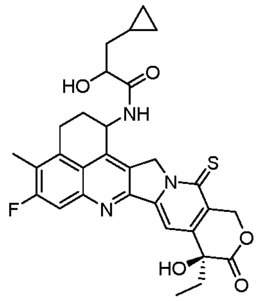
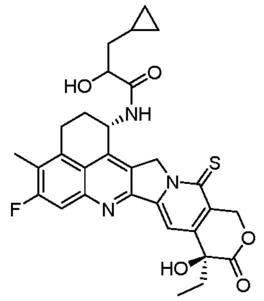

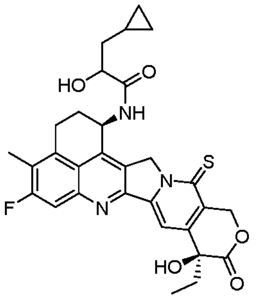
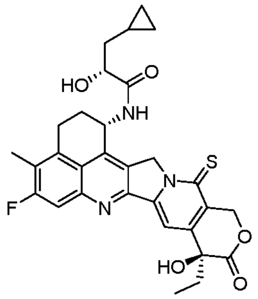
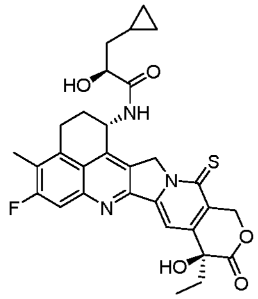
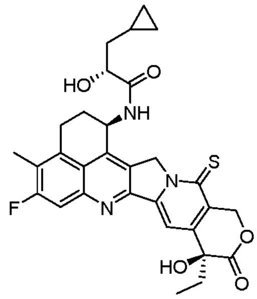

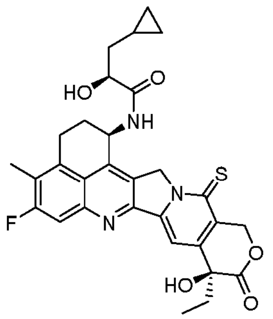
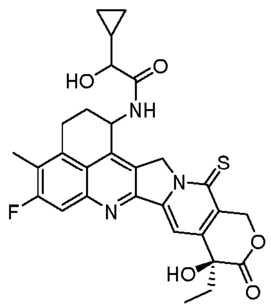
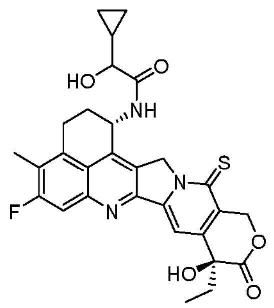
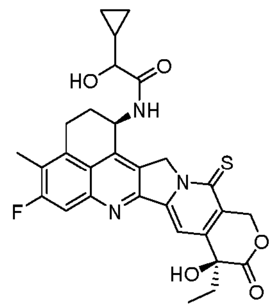

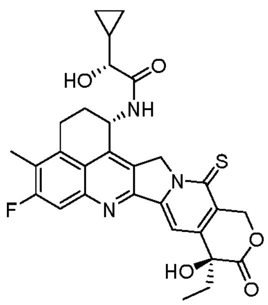
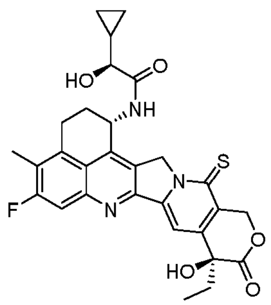
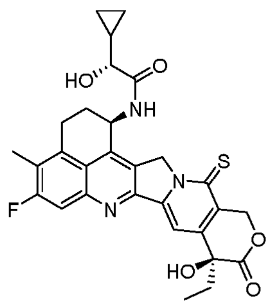
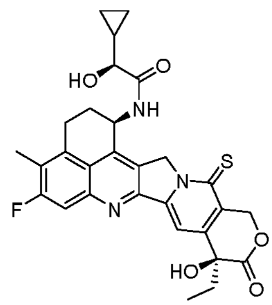

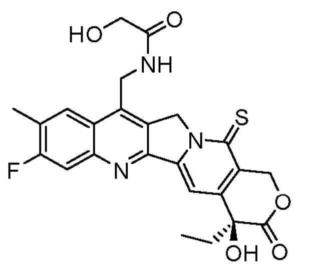
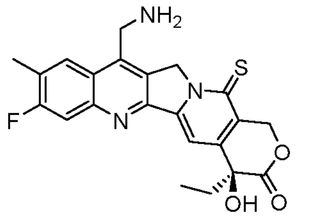
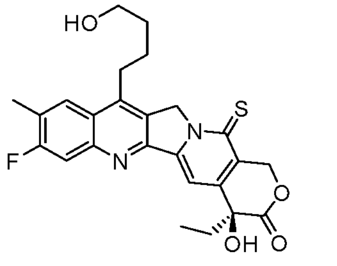
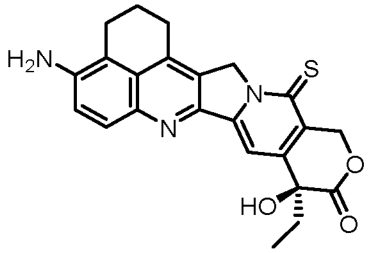
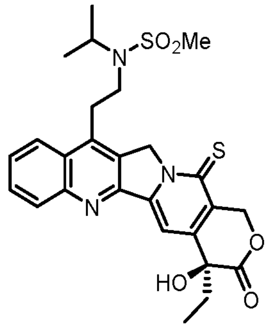
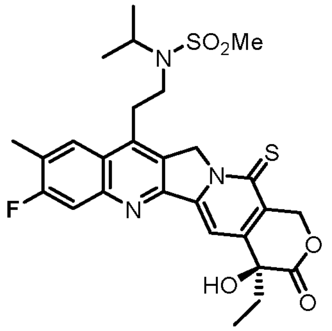
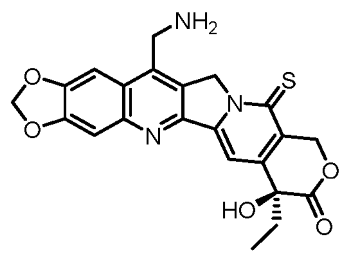
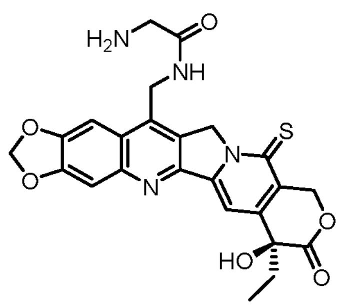
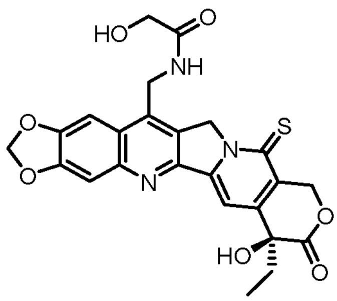
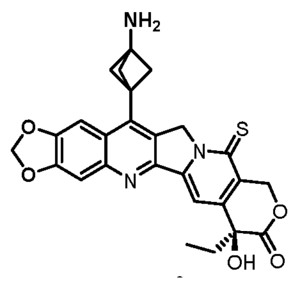
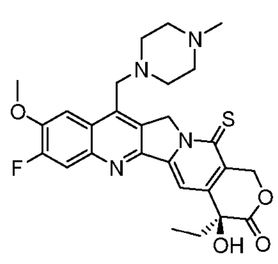
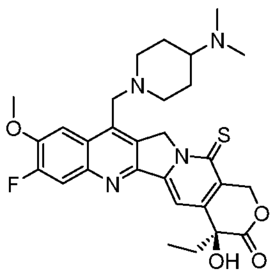
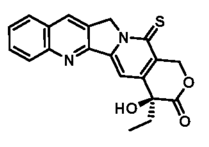
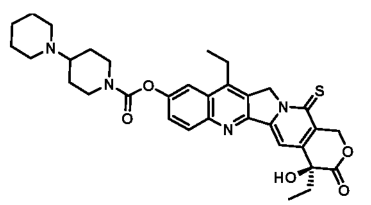
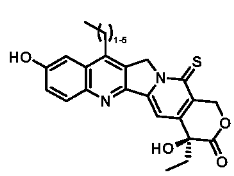
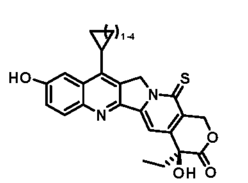
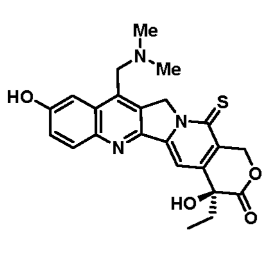
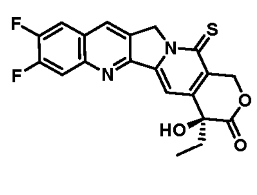
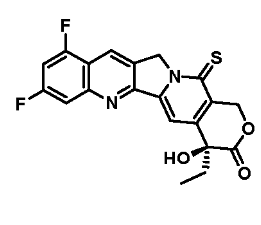
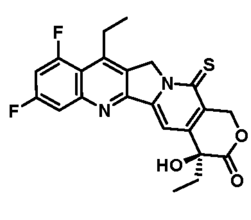

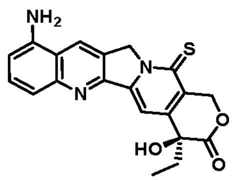
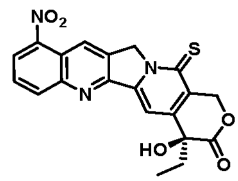
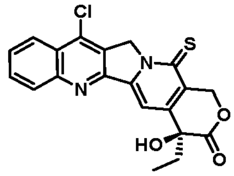
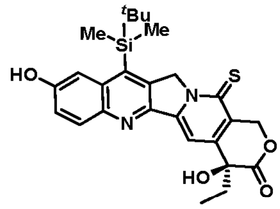

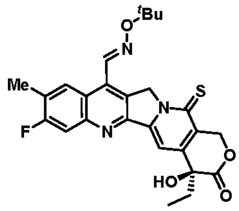
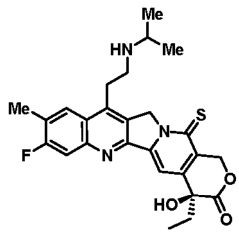
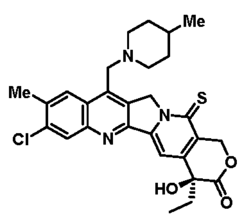
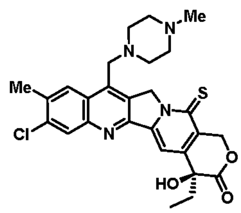

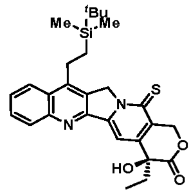
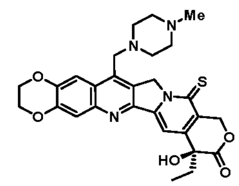
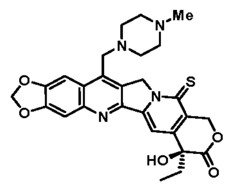

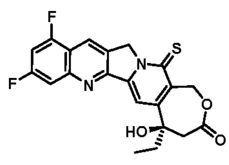
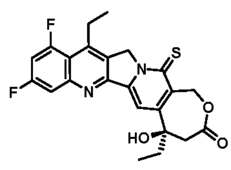

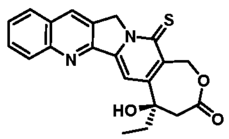
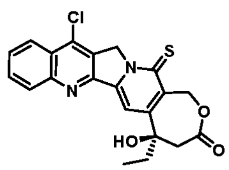
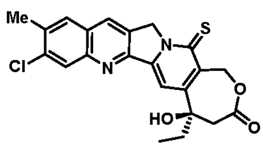
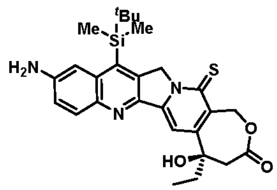

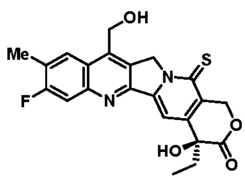
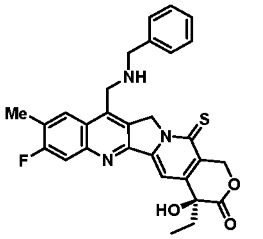
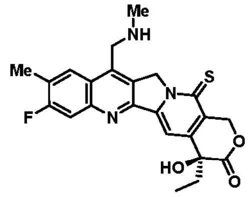
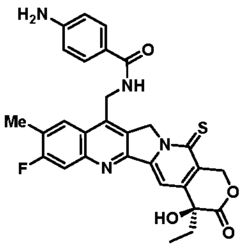

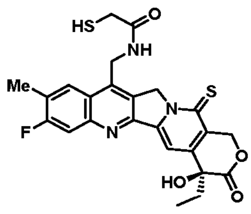
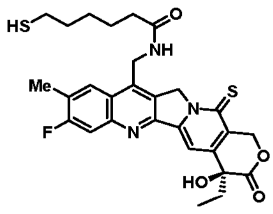
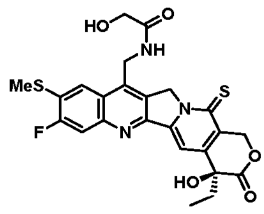
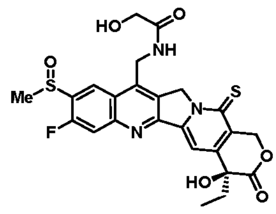

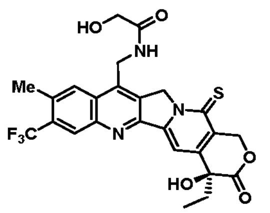
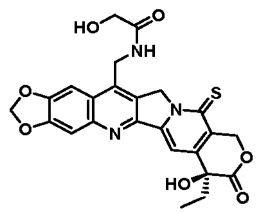
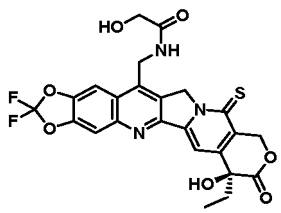
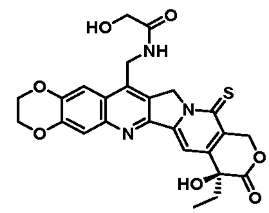

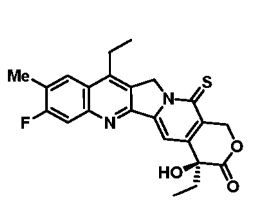
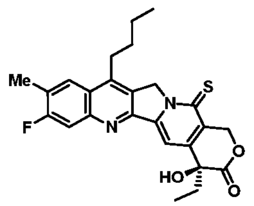
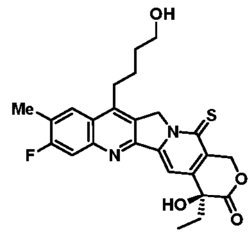
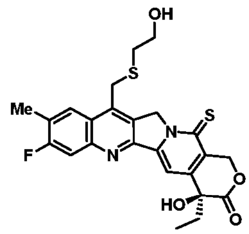

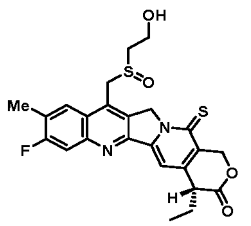
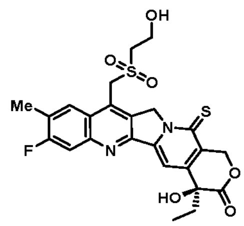
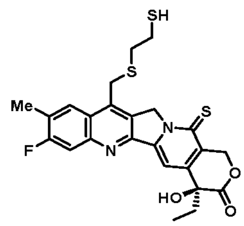
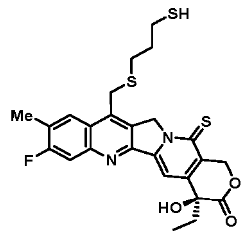

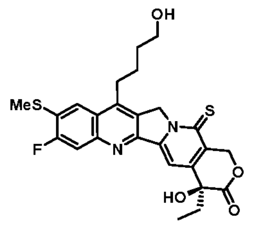
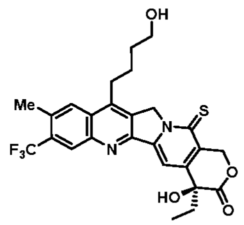
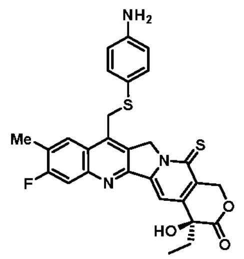

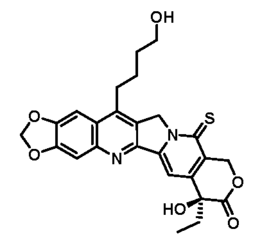
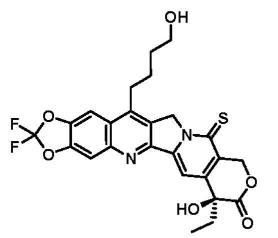
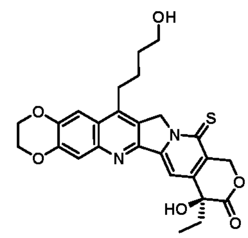

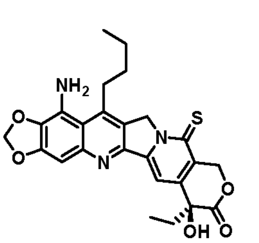
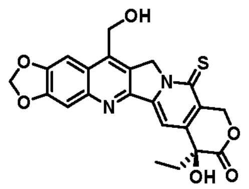
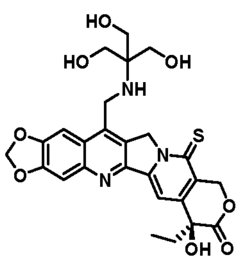

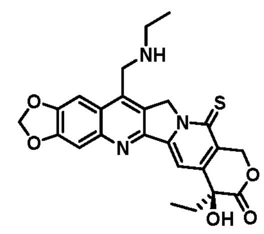
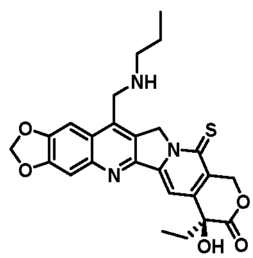
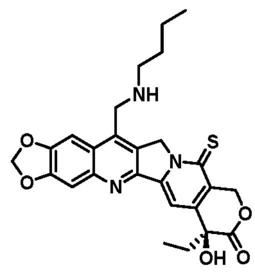
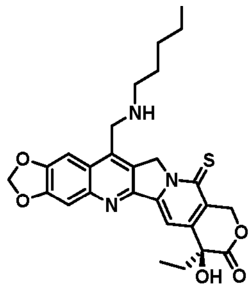

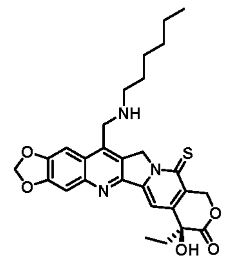
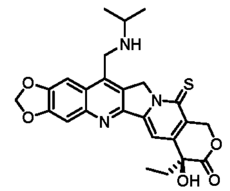
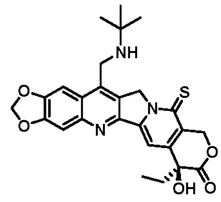

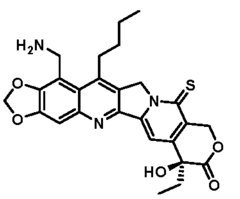
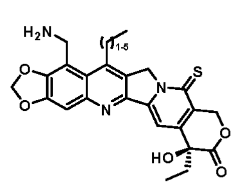
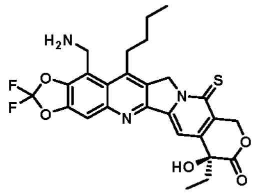
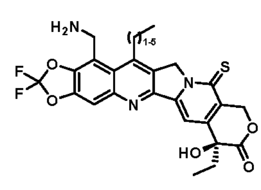

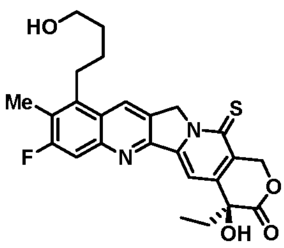
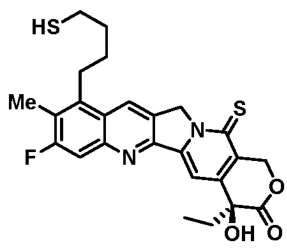
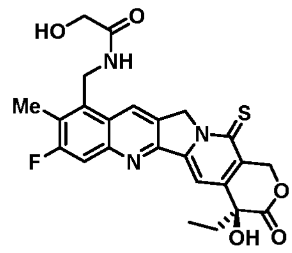

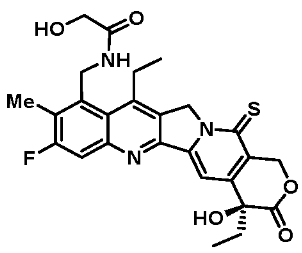
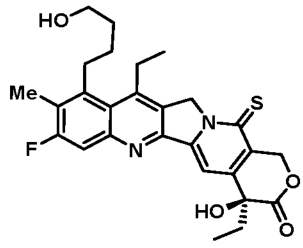
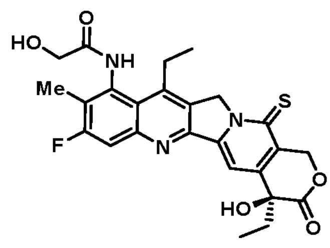

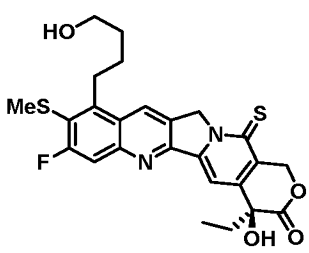
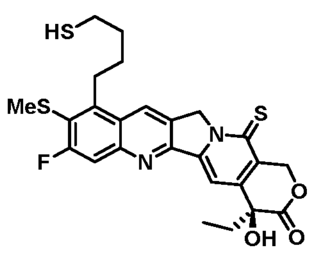
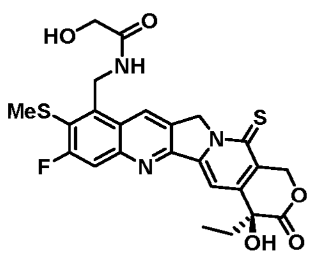

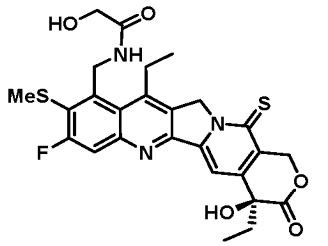
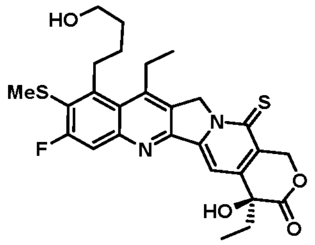
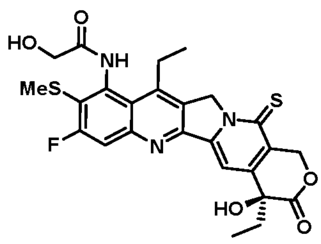

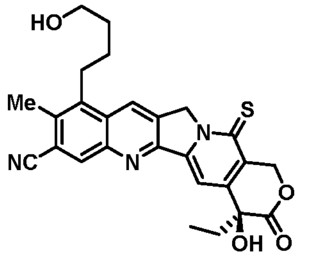
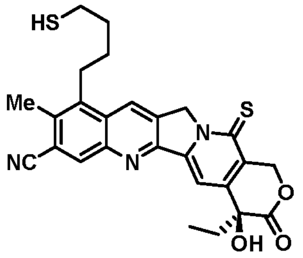
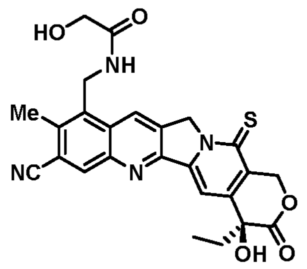

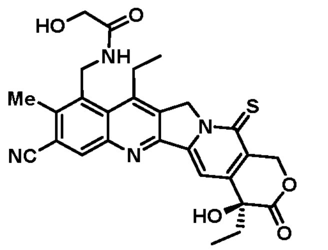
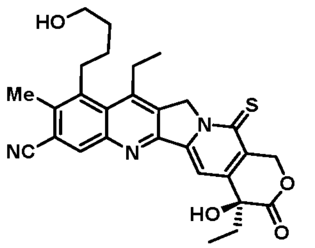
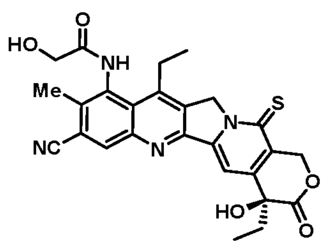

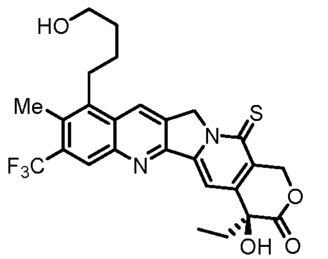
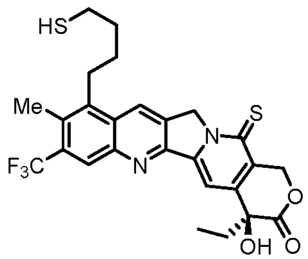
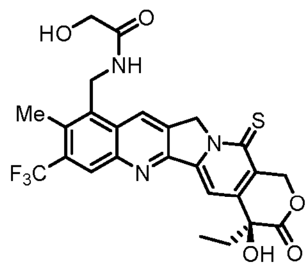

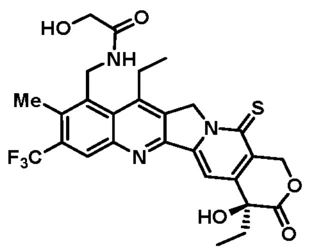
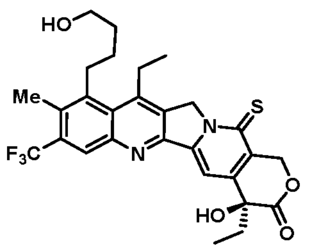
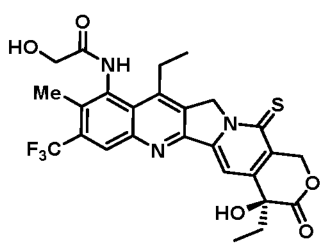

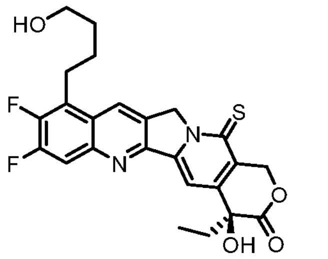
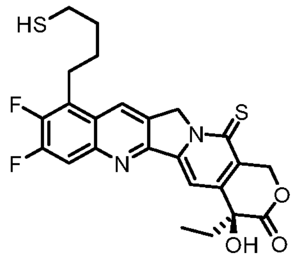
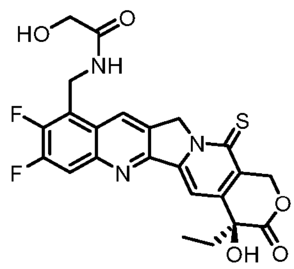

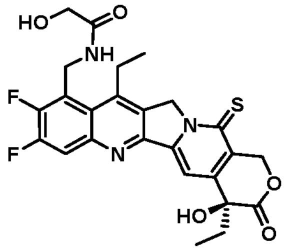
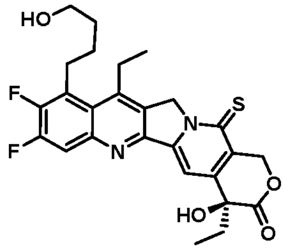
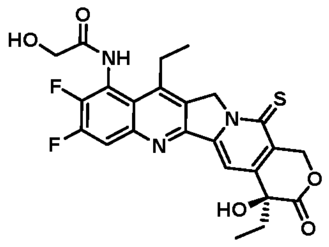

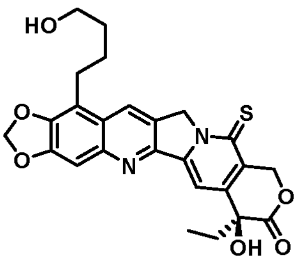
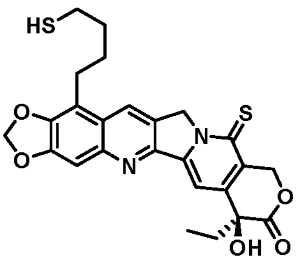
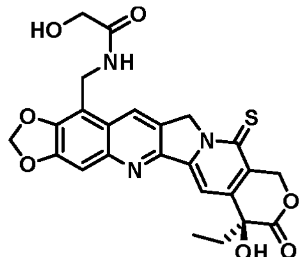

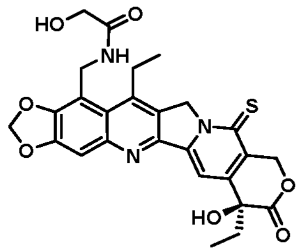
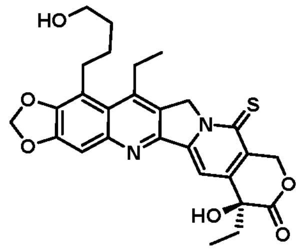
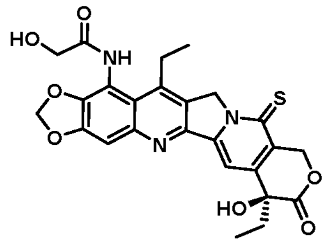

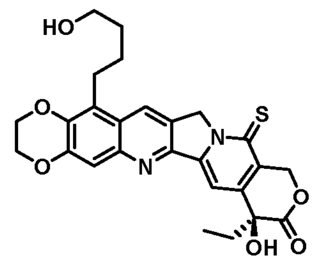
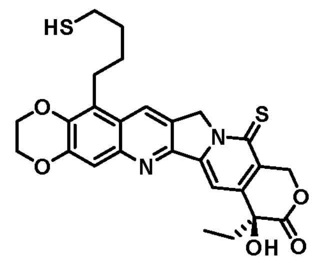
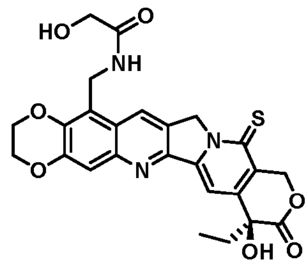

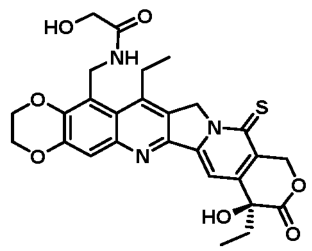
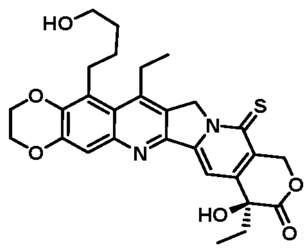
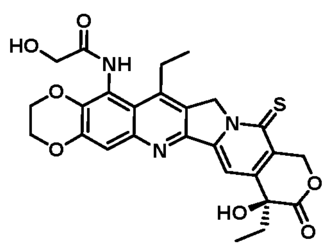

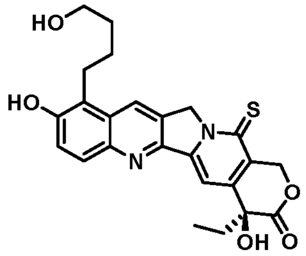
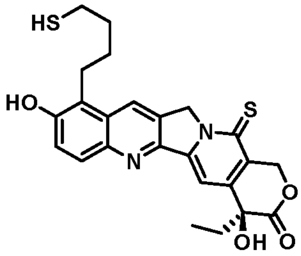
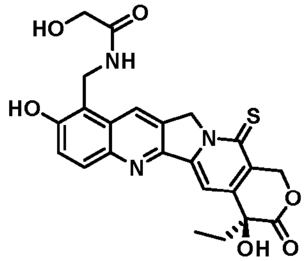

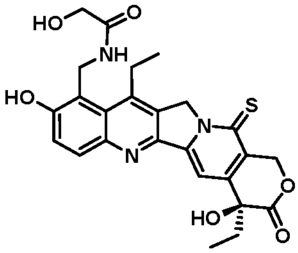

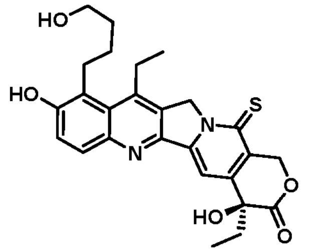

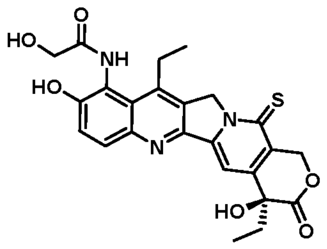

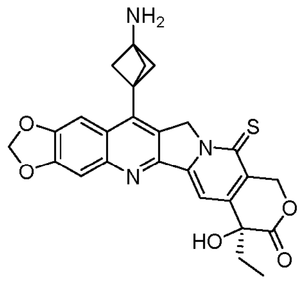

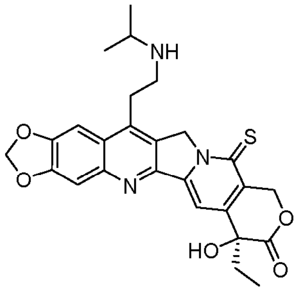

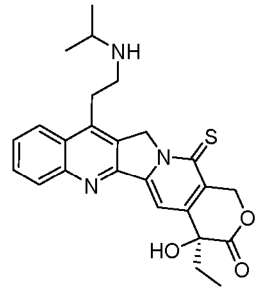

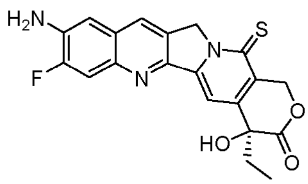

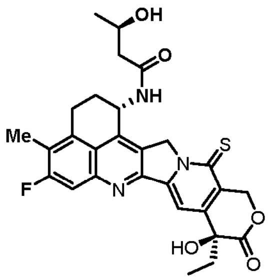

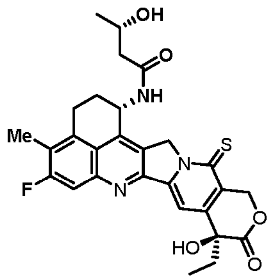

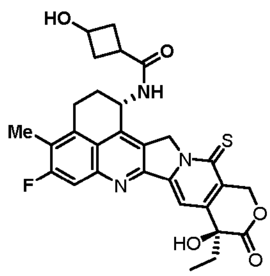

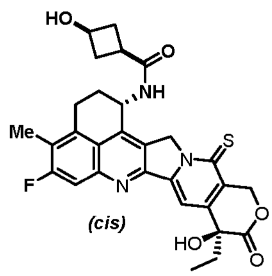

(cis)

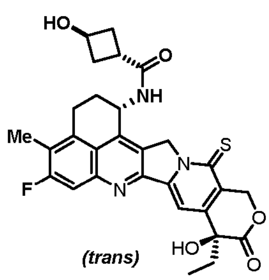

(trans)

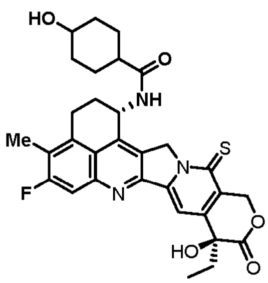

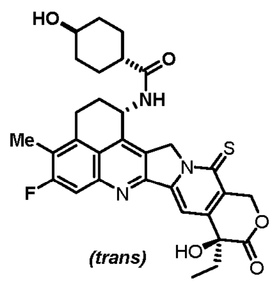

(trans)

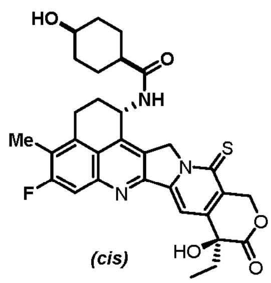

(cis)

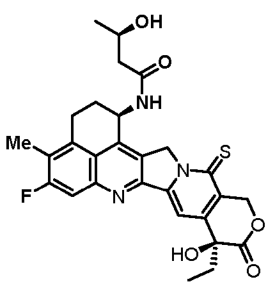

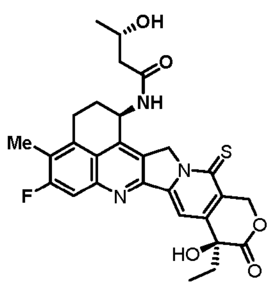

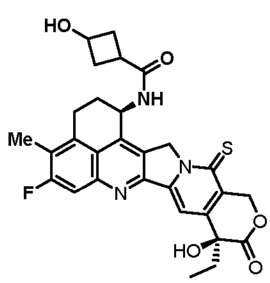

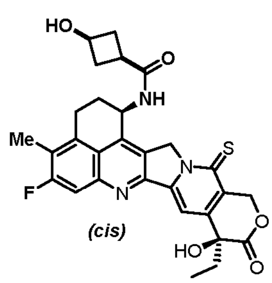

(cis)

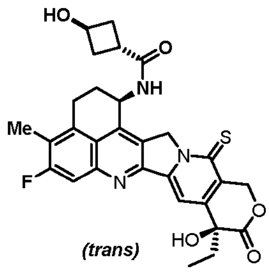

(trans)

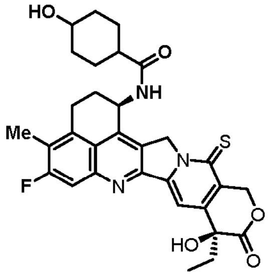

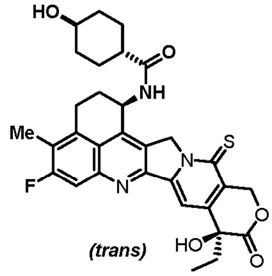

(trans)

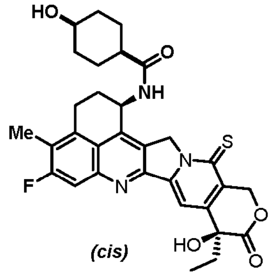

(cis)

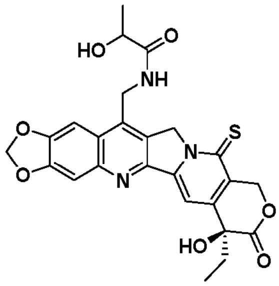

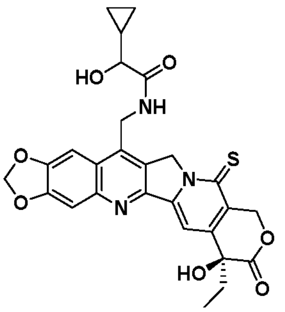

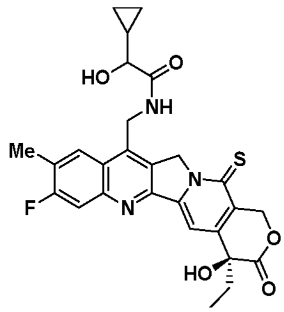

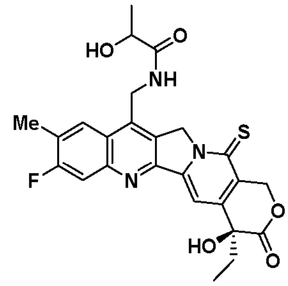

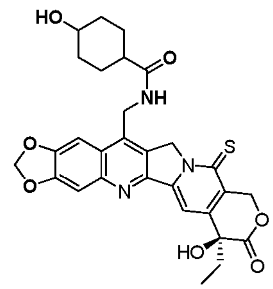

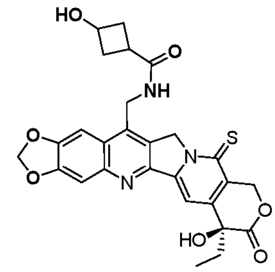

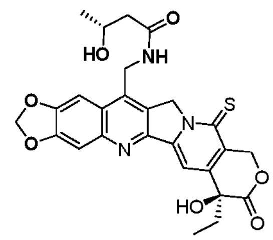

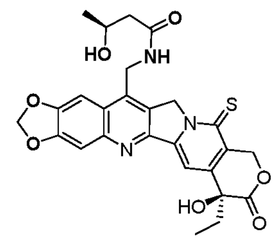

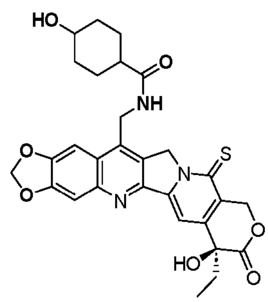

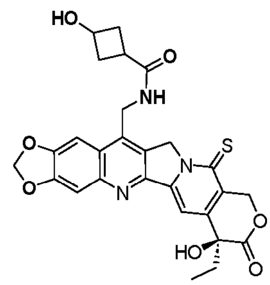

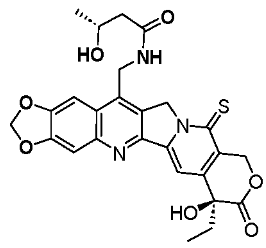

and

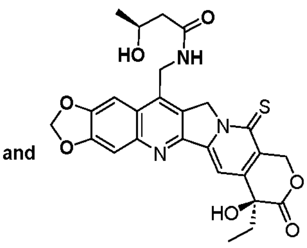

In another preferred embodiment, the ligand-drug conjugate is selected from the group consisting of

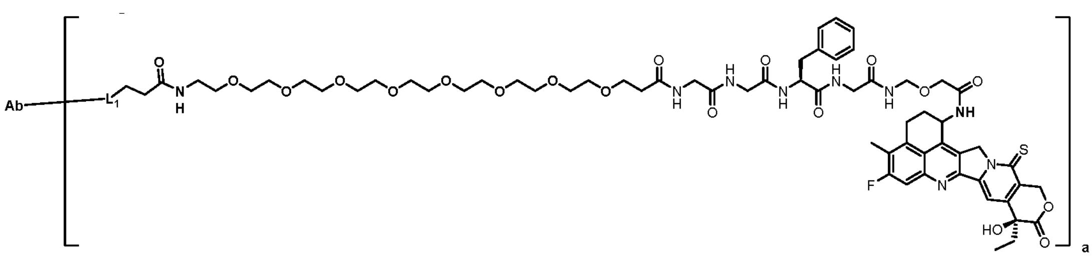

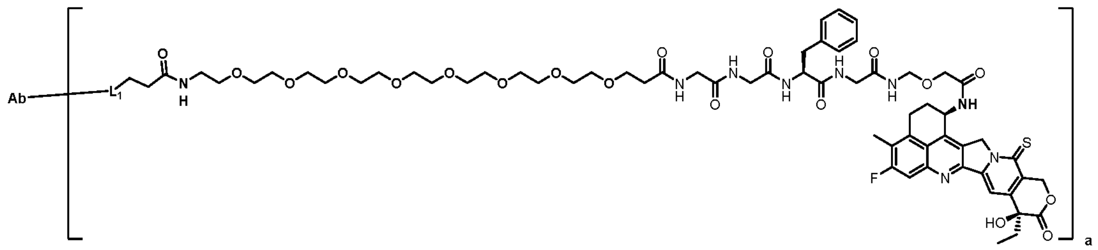

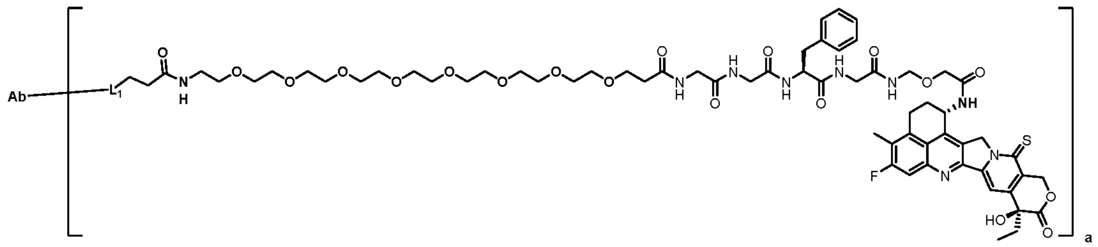

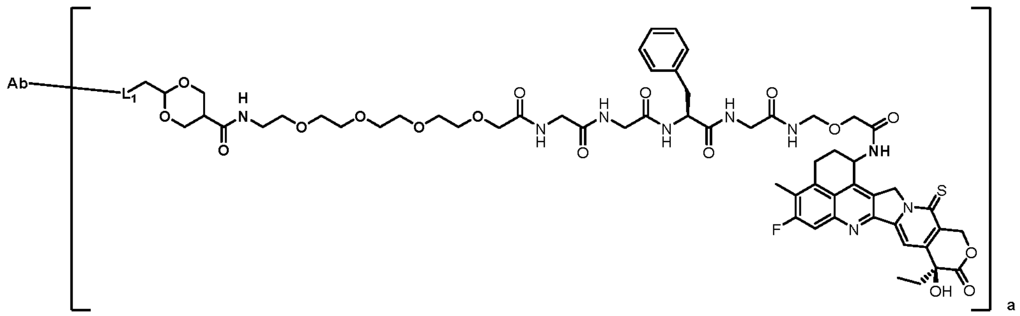

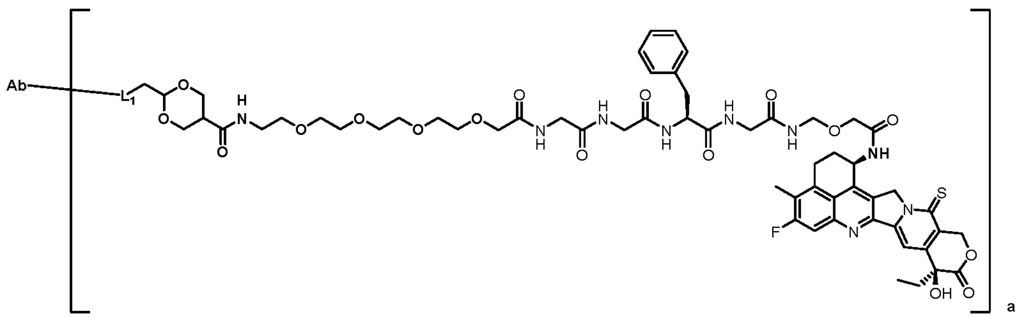

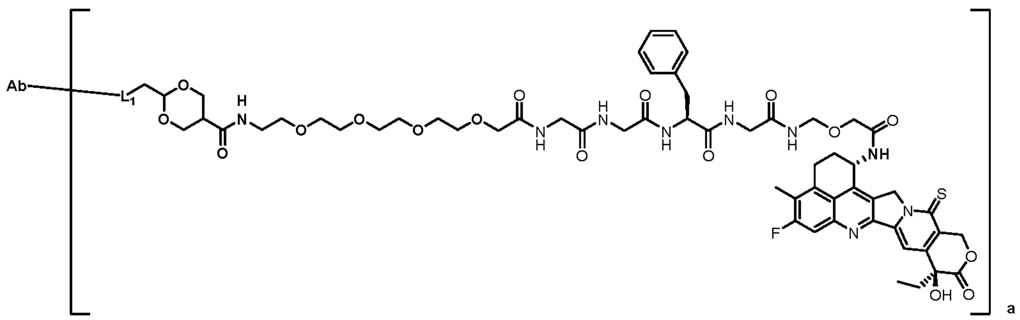

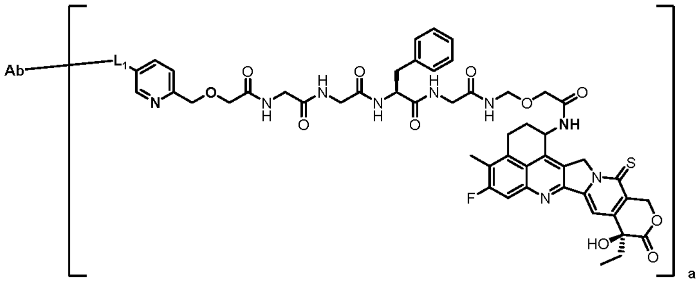

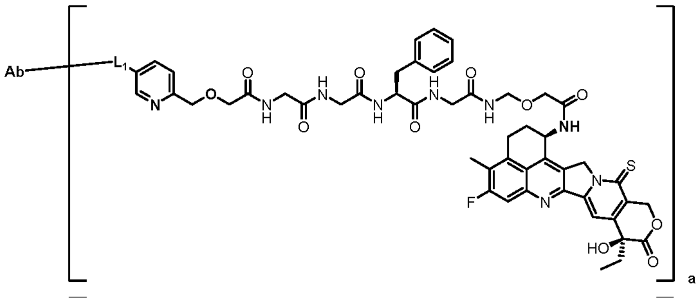

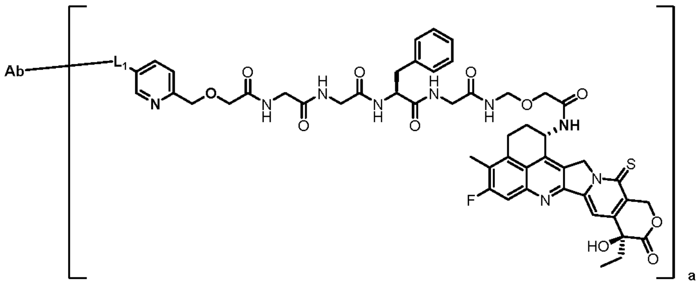

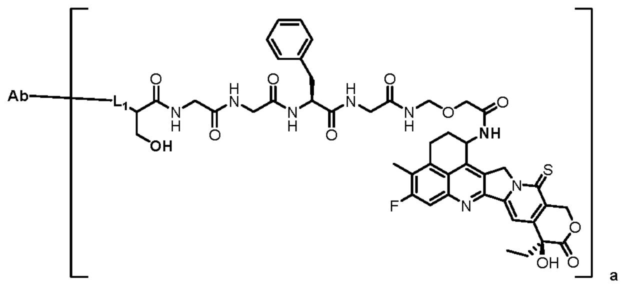

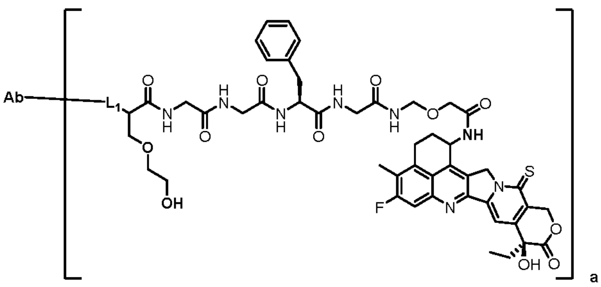

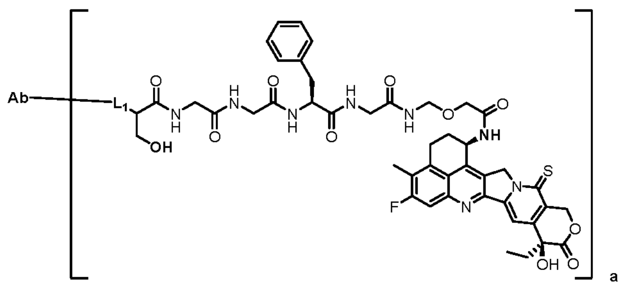

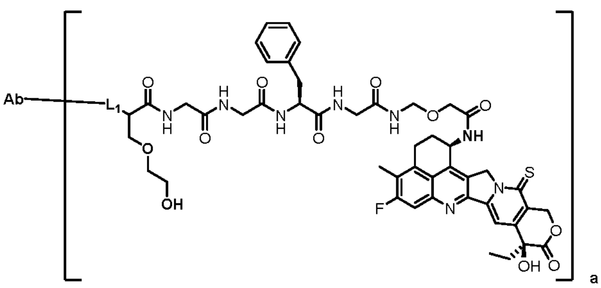

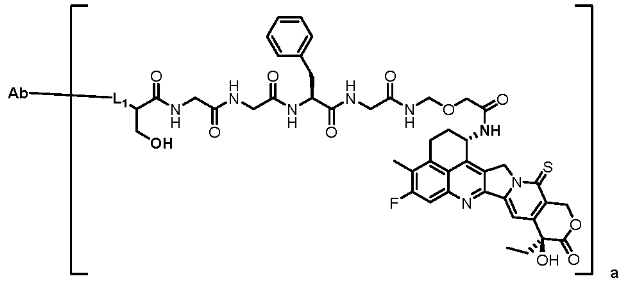
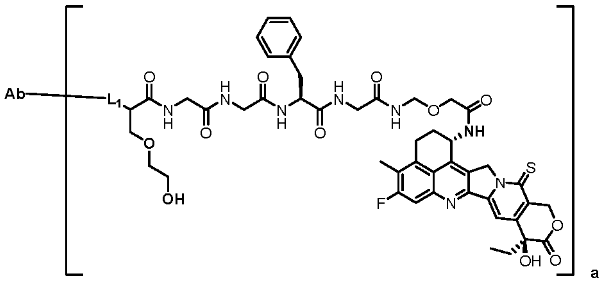

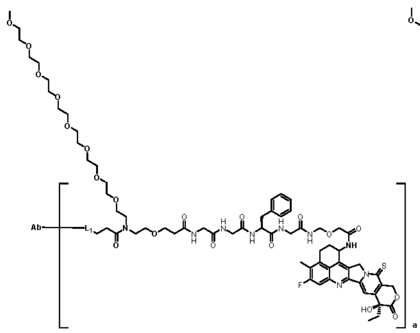
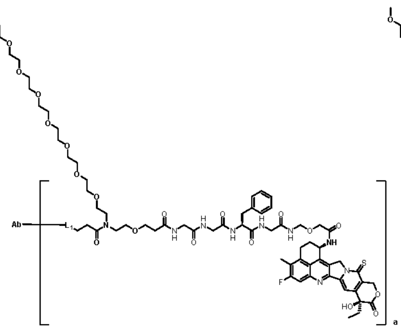
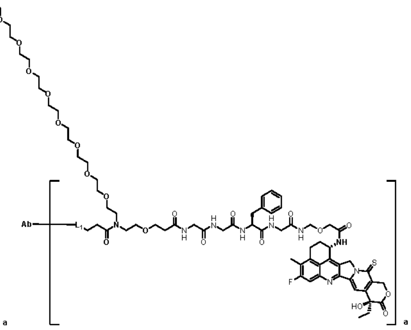

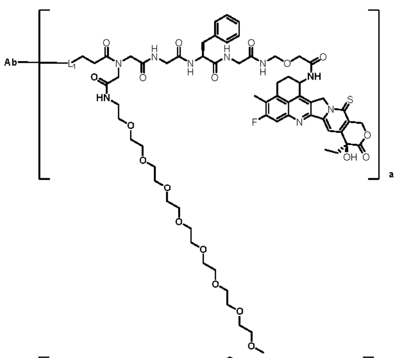
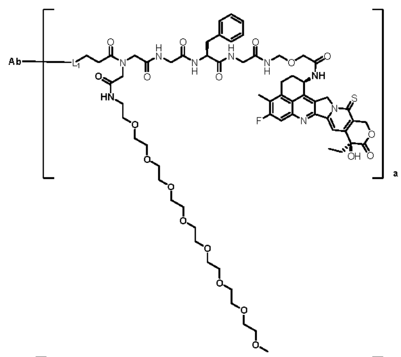
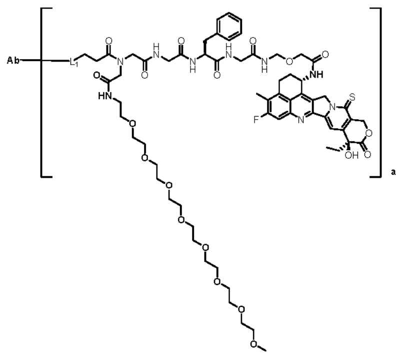

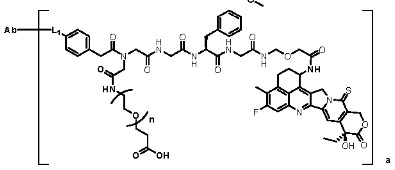
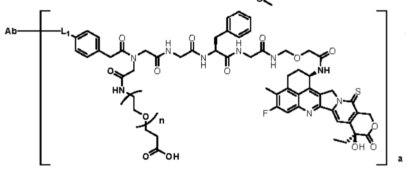
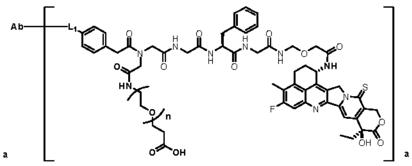

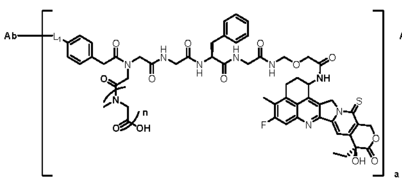
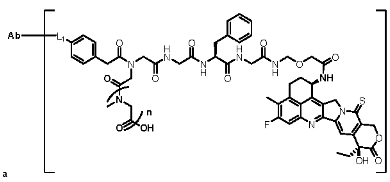
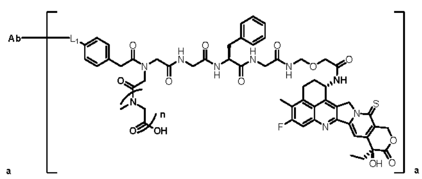

n = 1 ~ 24

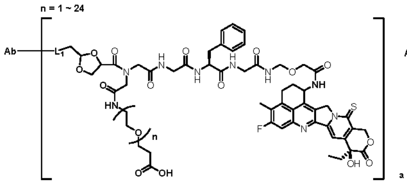
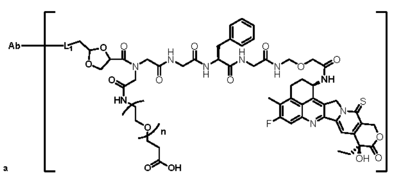
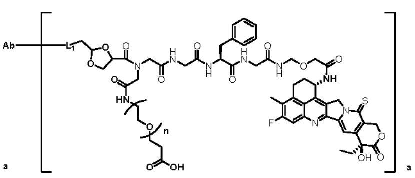

n = 1 ~ 24

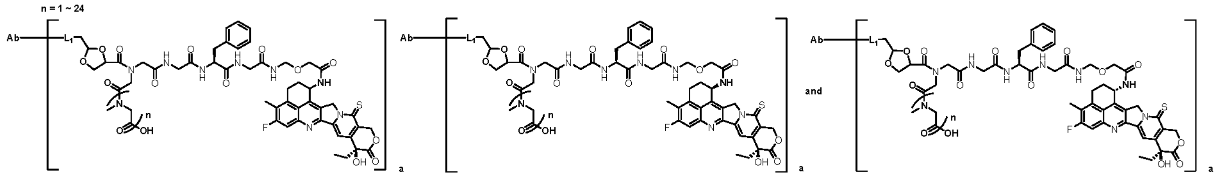

and

n = 1 ~ 24

.

In the above structure, "-$L_1$-" is selected from the following structures:

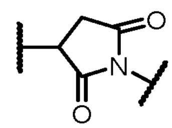

,

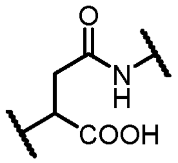

,

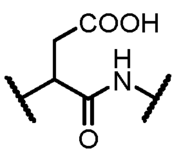

,

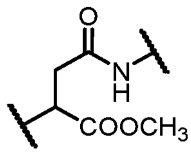

,

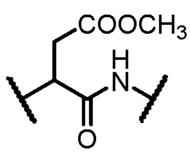

,

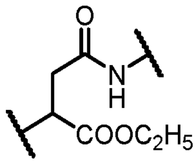

,

and

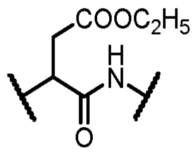

.

In one preferred embodiment, the ligand-drug conjugate is selected from the group consisting of

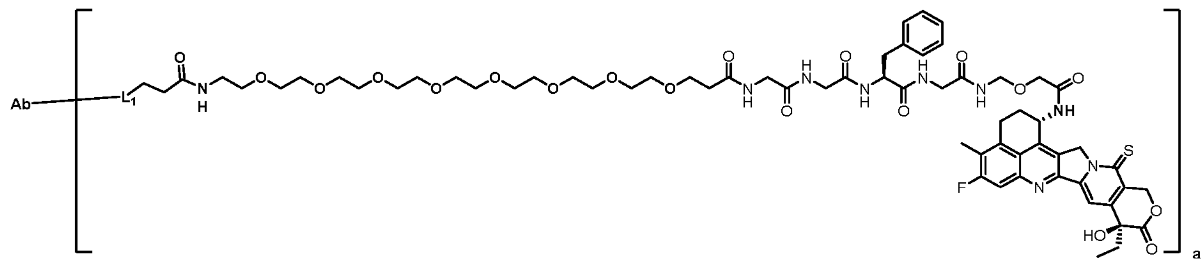

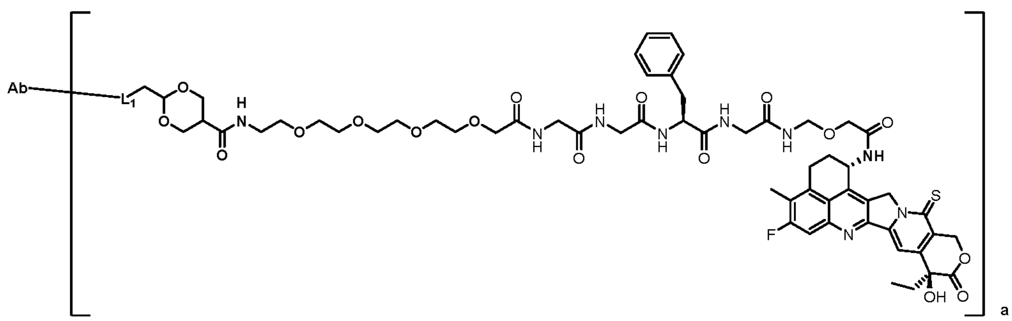

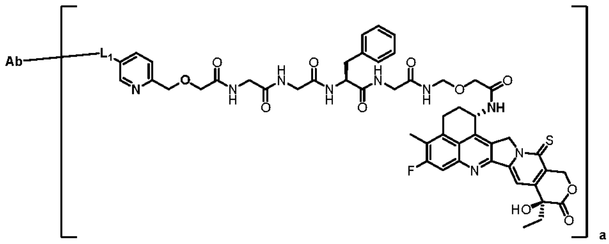

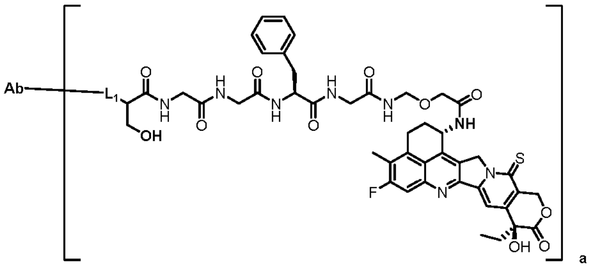

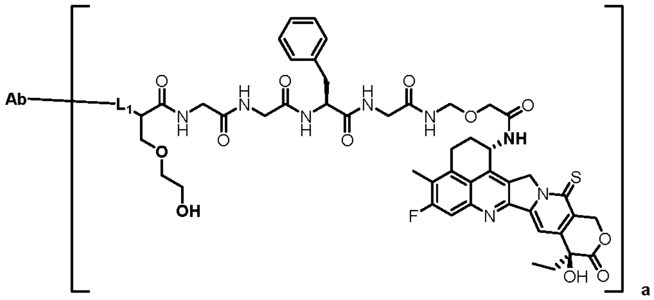

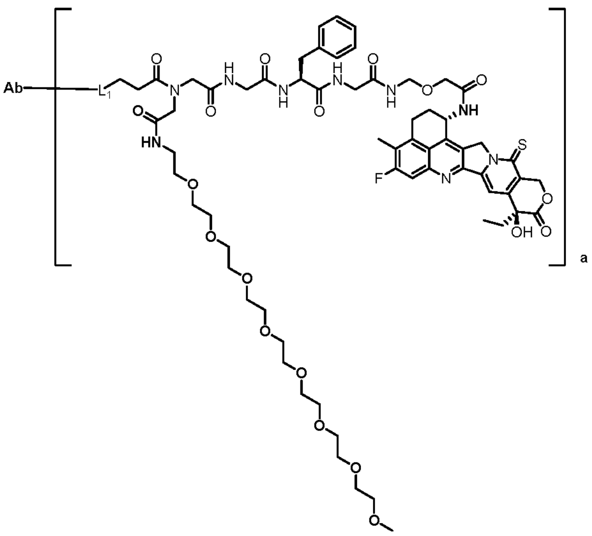

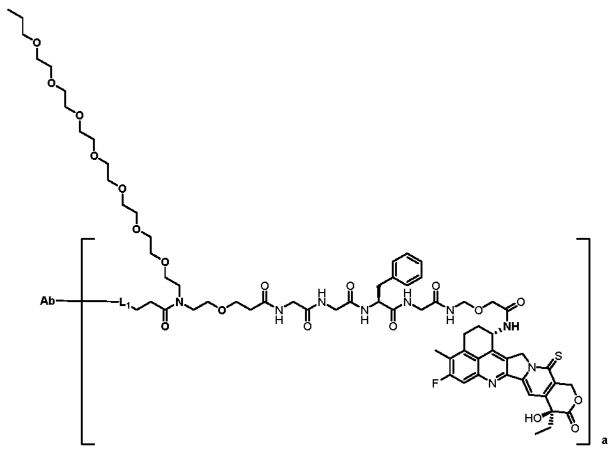

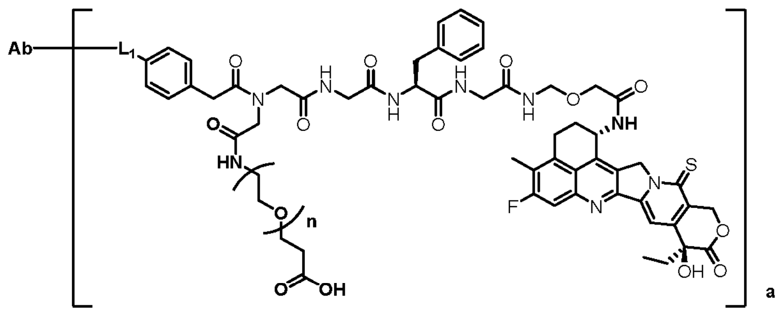

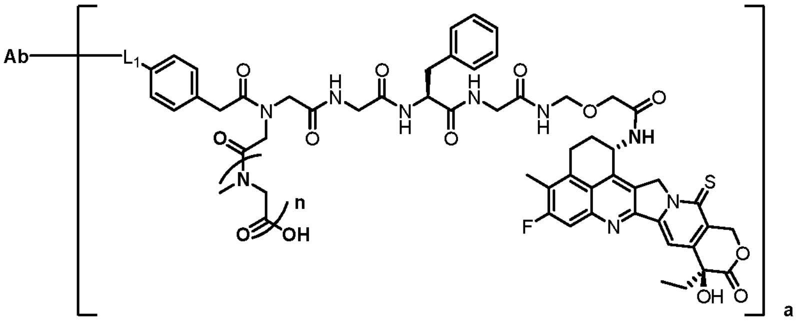

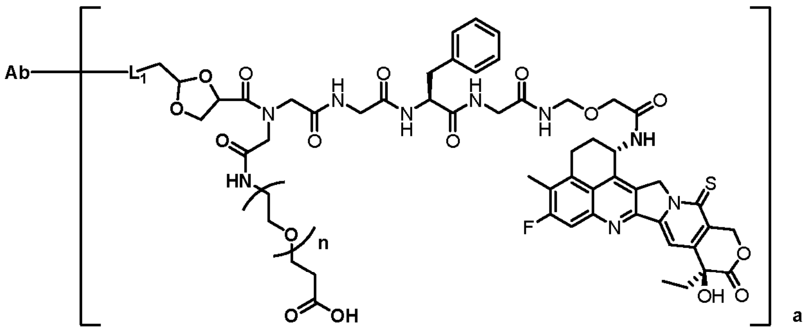

and

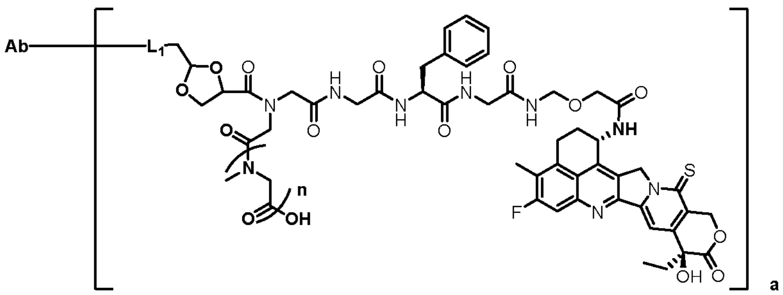.

In the above structure, "$-L_1-$" is selected from the following structures:

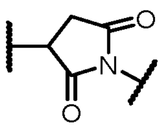,

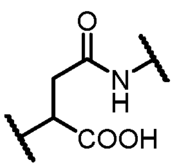, 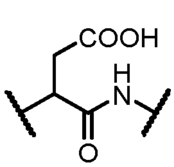, 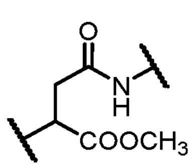, 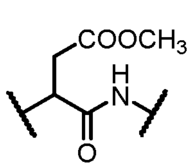, 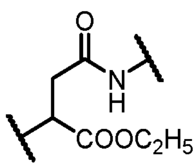,

and

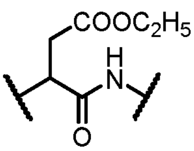;

, Preferably

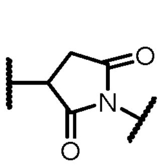 , 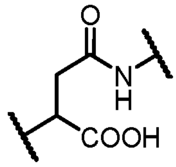 , 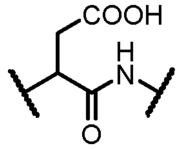 ,

more preferably

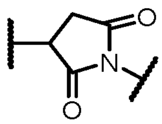 .

In another preferred embodiment, the ligand-drug conjugate comprises a structure as shown in formula (I-a):

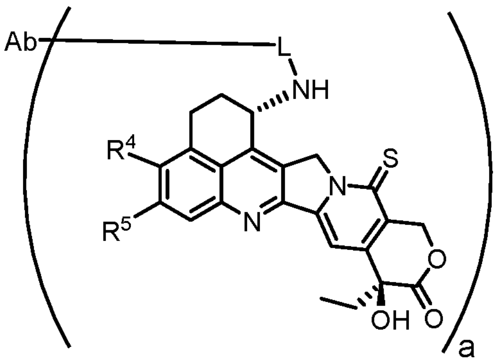 (I-a),

wherein $R^4$, $R^5$ and L are as defined in the first aspect of the present invention, "a" is a number greater than 0, and "a" is a decimal or integer.

In another preferred embodiment, the ligand-drug conjugate is a structure selected from the group consisting of

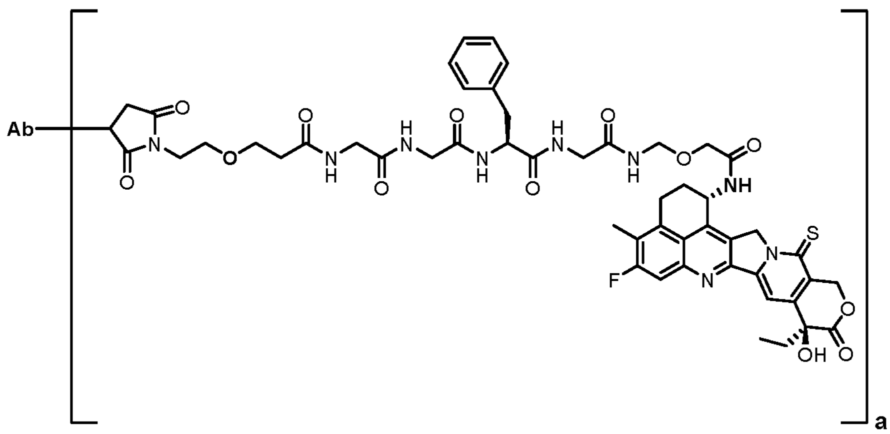

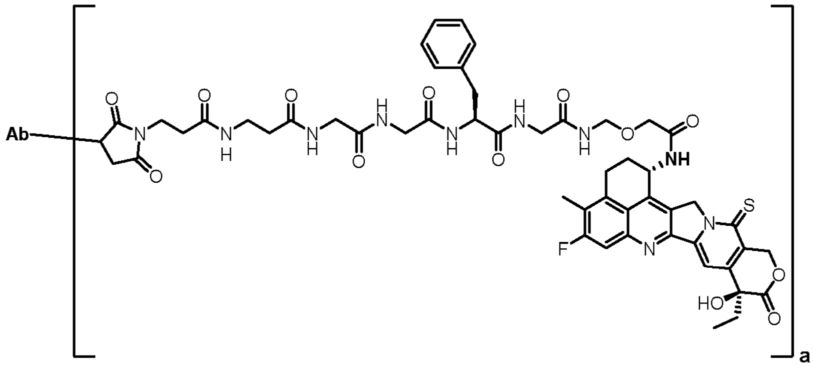

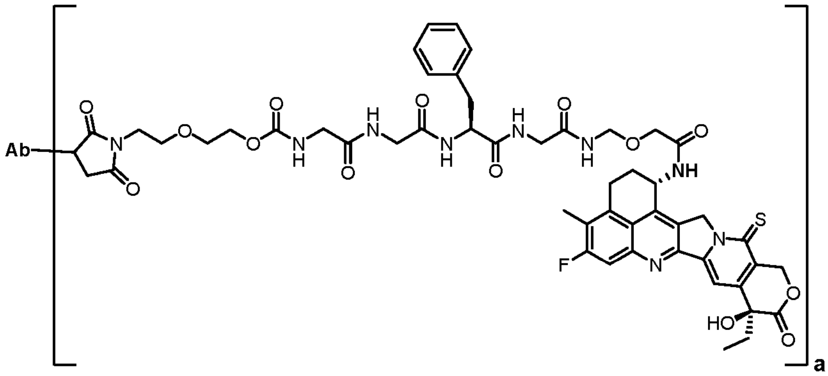

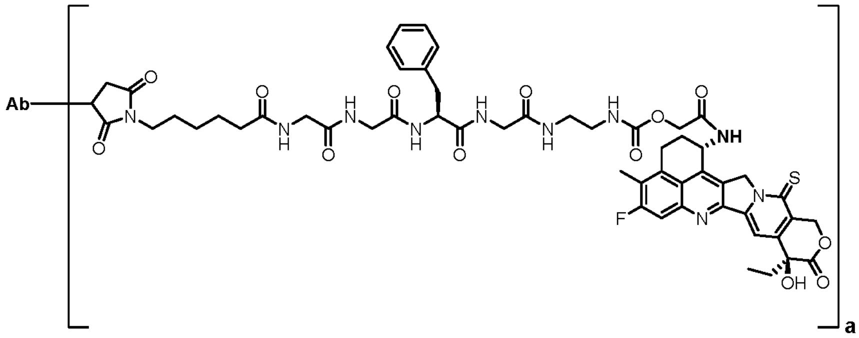

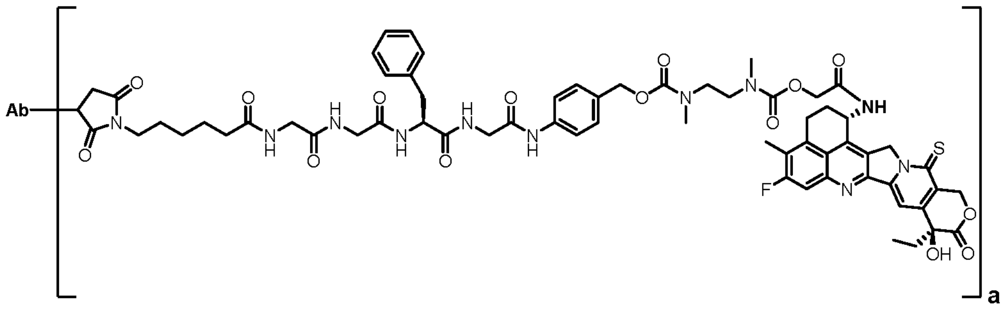

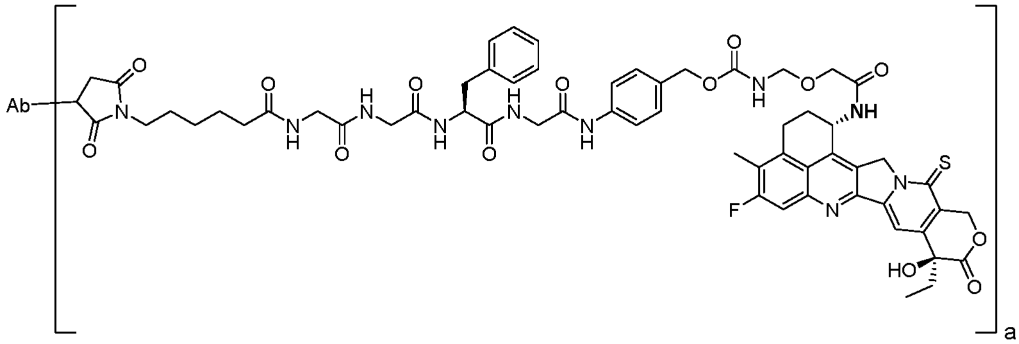

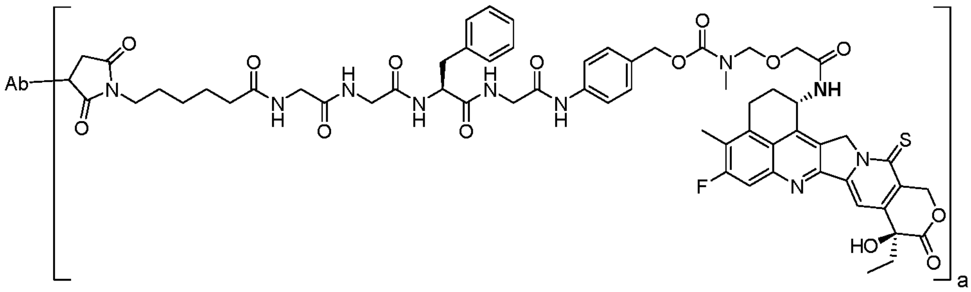

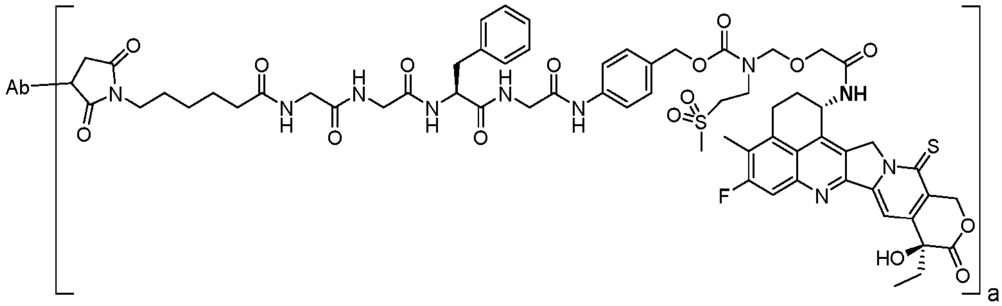

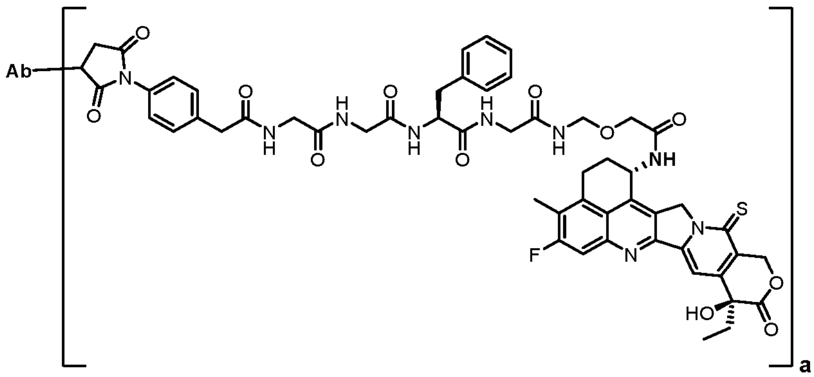

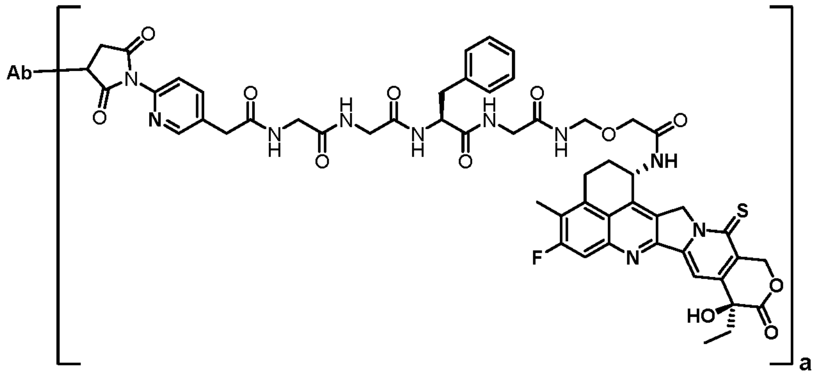

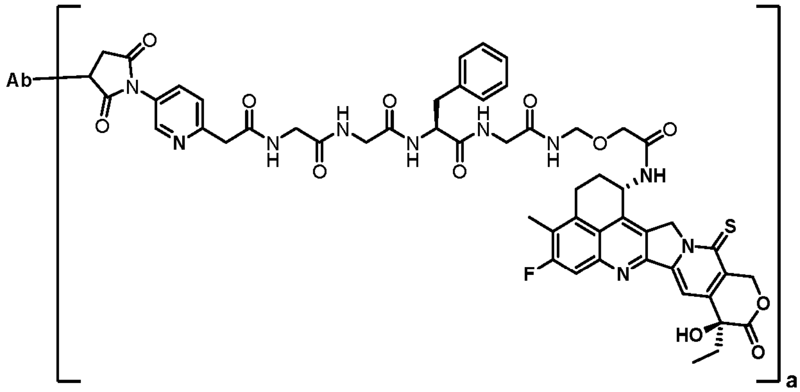

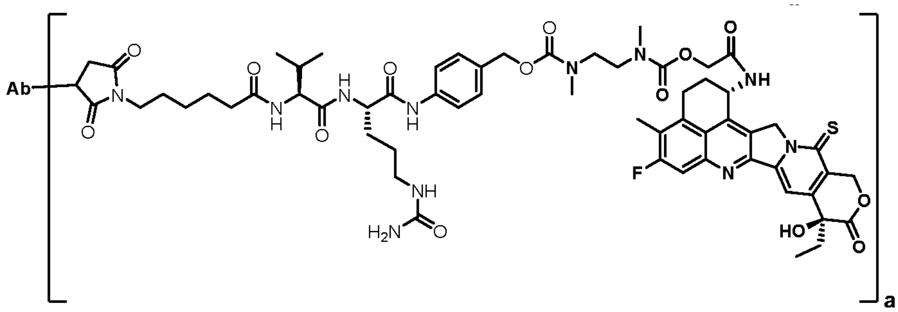

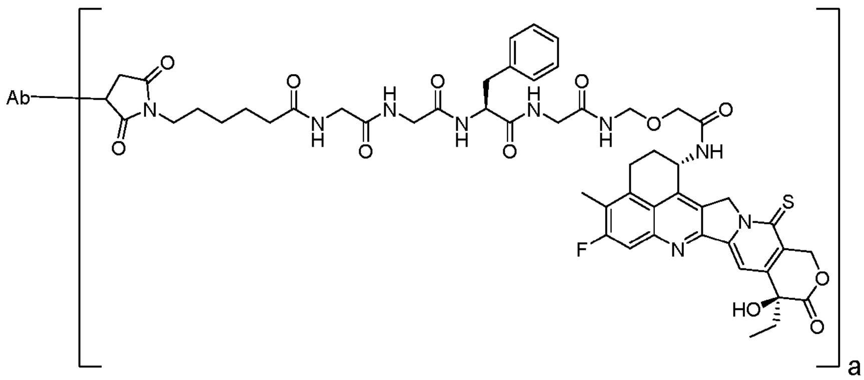

and

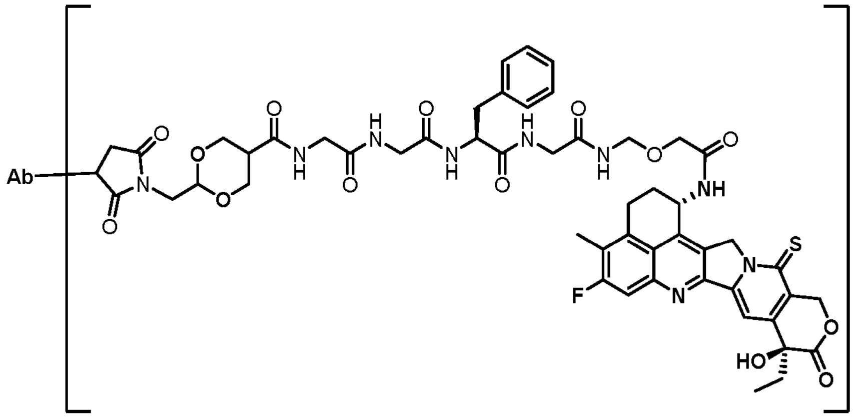

a,

wherein "a" is a number greater than 0, and "a" is a decimal or integer.

**In** another preferred embodiment, "a" is a non-zero integer or decimal from 0 to 8, preferably an integer or decimal from 1 to 8; more preferably from 2 to 8, which may be an integer or a decimal; most preferably from 3 to 8, which may be an integer, e.g. 4.0, or a decimal, e.g. 3.9.

**In** another preferred embodiment, the Ab is an antibody or its antigen binding fragment.

**In** another preferred embodiment, the antibody is selected from the group consisting of murine-derived antibody, chimeric antibody, humanized antibody, and fully human antibody.

**In** another preferred embodiment, the antibody is a monoclonal antibody.

In another preferred embodiment, the antigen-binding fragment is selected from the group consisting of Fab, Fab', Fv fragment, $F(ab')_2$, $F(ab)_2$, scFv, di-scFv, VHH, and dAb.

In another preferred embodiment, the antibody is selected from the group consisting of Her-2-specific antibodies (e.g., Trastuzumab), Trop2-specific antibodies (e.g., Humanized RS7 or Sacituzumab in US7238785B2), PSMA-specific antibodies (e.g., PSMA-specific monoclonal antibody AB-PG1-XG1-006 in WO2003034903A2), FR-$\alpha$-specific antibodies (e.g., the humanized antibody LK26 (Farletuzumab, MORAb-003) in US5952484), B7H3 antibodies (e.g., the antibody P7-C05-H4L3 in WO2021244590A1), and IGF-1R-specific antibodies.

In another preferred embodiment, the antibody is IGF-1R-specific antibody.

In another preferred embodiment, the IGF-1R comprises IGF-1R derived from a primate.

In another preferred embodiment, the antibody is IgG1 or its mutant.

In another preferred embodiment, the antibody comprises HCDR3, and the HCDR3 comprises the amino acid sequence shown in SEQ ID NO: 3.

In another preferred embodiment, the antibody comprises HCDR2, and the HCDR2 comprises the amino acid sequence shown in SEQ ID NO: 2.

In another preferred embodiment, the antibody comprises HCDR1, and the HCDR1 comprises the amino acid sequence shown in SEQ ID NO: 1.

In another preferred embodiment, the antibody comprises a heavy chain variable region (VH), the VH comprises the HCDR1, HCDR2 and HCDR3, the HCDR3 comprises the amino acid sequence shown in SEQ ID NO: 3; the HCDR2 comprises the amino acid sequence shown in SEQ ID NO: 2; and the HCDR1 comprises the amino acid sequence shown in SEQ ID NO: 1.

In another preferred embodiment, the antibody comprises a heavy chain variable region (VH), and the VH comprises the amino acid sequence shown in SEQ ID NO: 4.

In another preferred embodiment, the antibody has a full-length sequence as shown in SEQ ID NO: 5.

In another preferred embodiment, the antibody is a variant of any of the above antibodies, and the variant comprises the CDR regions.

In another preferred embodiment, the variant is a sequence formed by substituting, deleting and/or adding one or more amino acids (e.g., 1-30, 1-20 or 1-10, and e.g., 1, 2, 3, 4, 5, 6, 7, 8, or 9 amino acids substituted, deleted and/or inserted) in the amino acid sequence of the antibody.

In another preferred embodiment, the variant is a homologue of the amino acid sequence of the antibody, which homologue can be an amino acid sequence with at least about 85% (e.g., at least about 85%, about 90%, about 91%, about 92%, about 93%, about 94%, about 95%, about 96%, about 97%, about 98%, about 99% or higher) sequence identity with the amino acid sequence of the CDR.

In another preferred embodiment, the CDR is determined by the Kabat numbering scheme.

The second aspect of the present invention provides a ligand-drug conjugate precursor, or its tautomer, mesomer, racemate, enantiomer, diastereomer, or pharmaceutically acceptable salt or hydrate,

wherein the ligand-drug conjugate precursor comprises a structure of formula (IA):

$L_A$-P(IA),

wherein $L_A$ is $L_{1A}$-$L_2$-$L_3$-$L_4$-$L_5$-; wherein $L_{1A}$ is

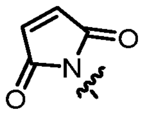 or 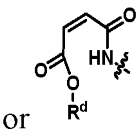 ;

wherein $R^d$, $L_2$, $L_3$, $L_4$, $L_5$, and P are as defined in the first aspect of the present invention.

In another preferred embodiment, the ligand-drug conjugate precursor is selected from the following structures:

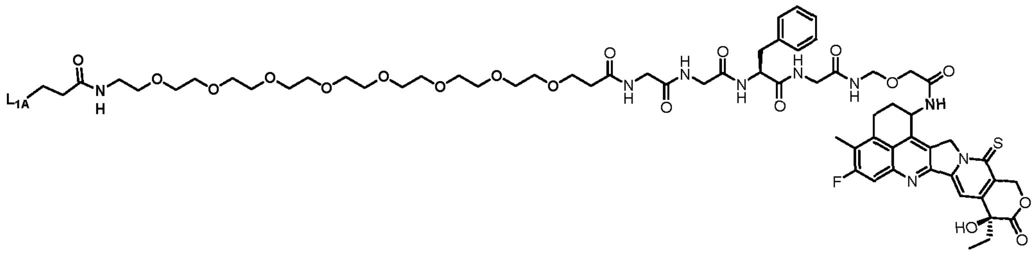

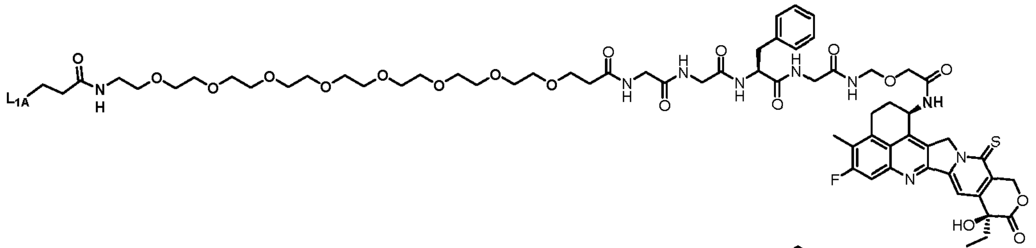

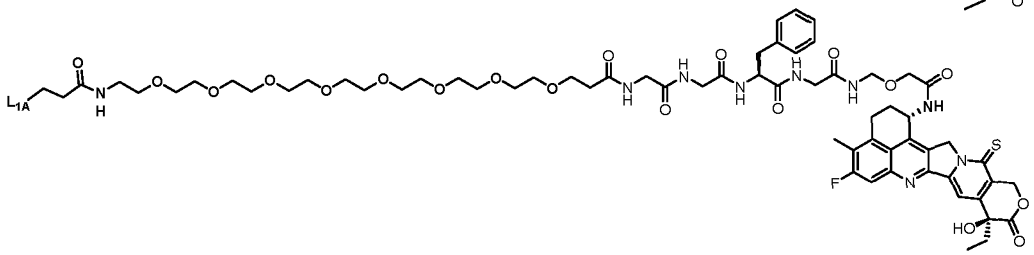

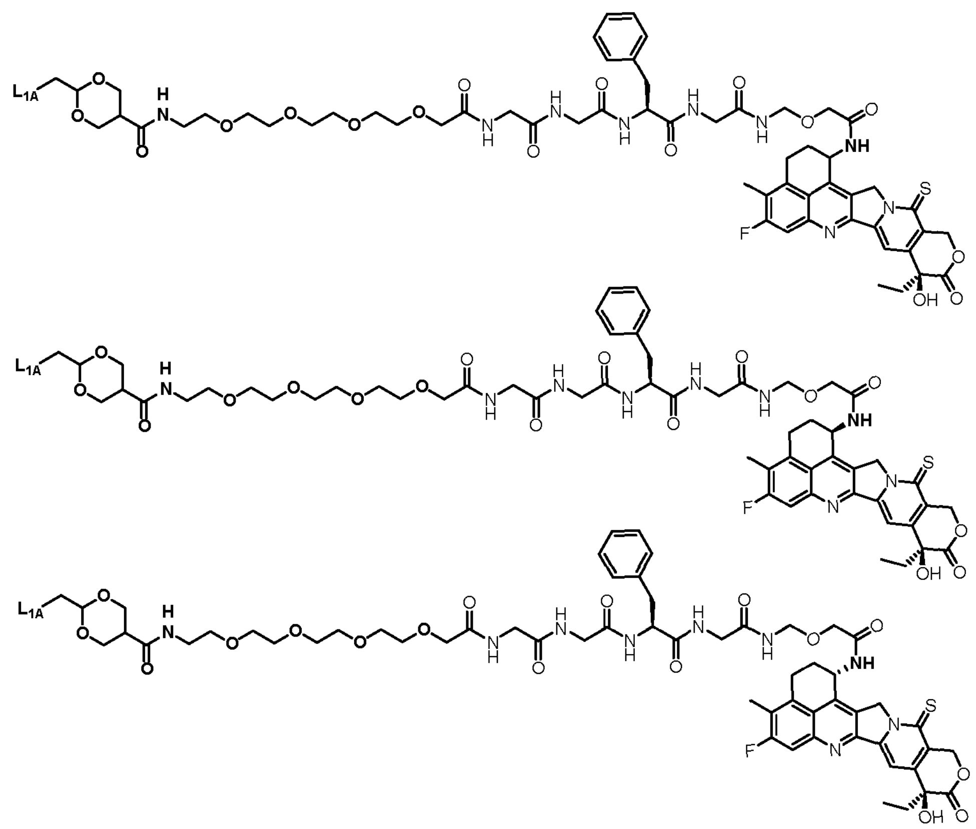

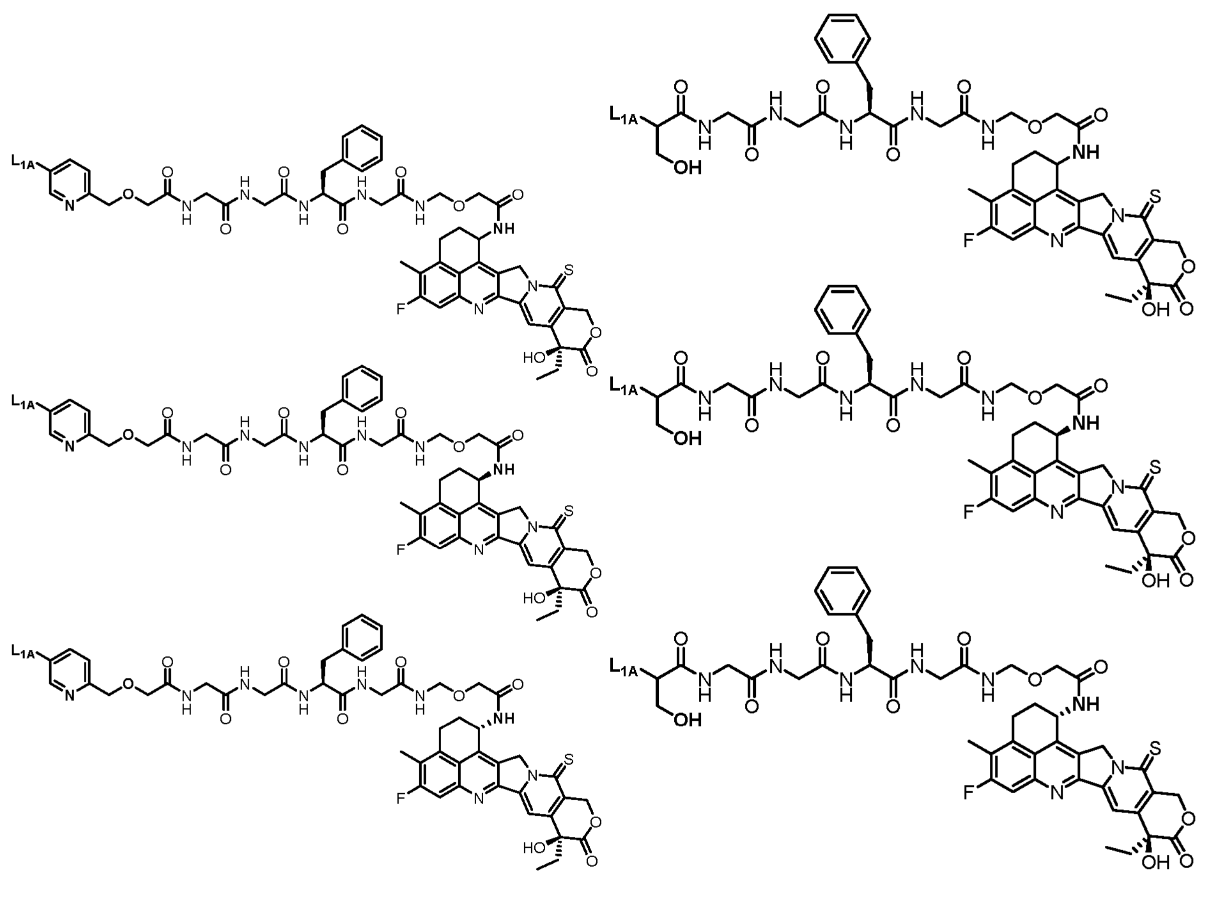

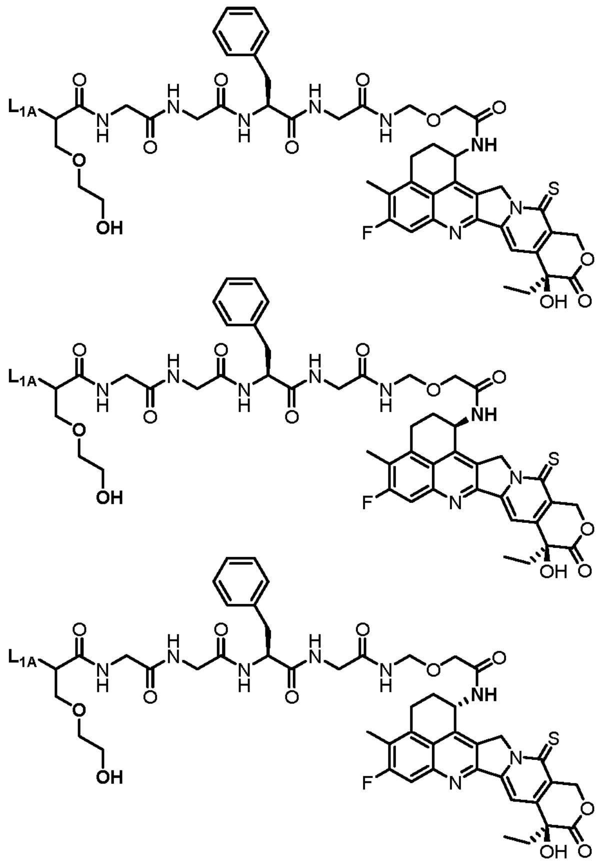

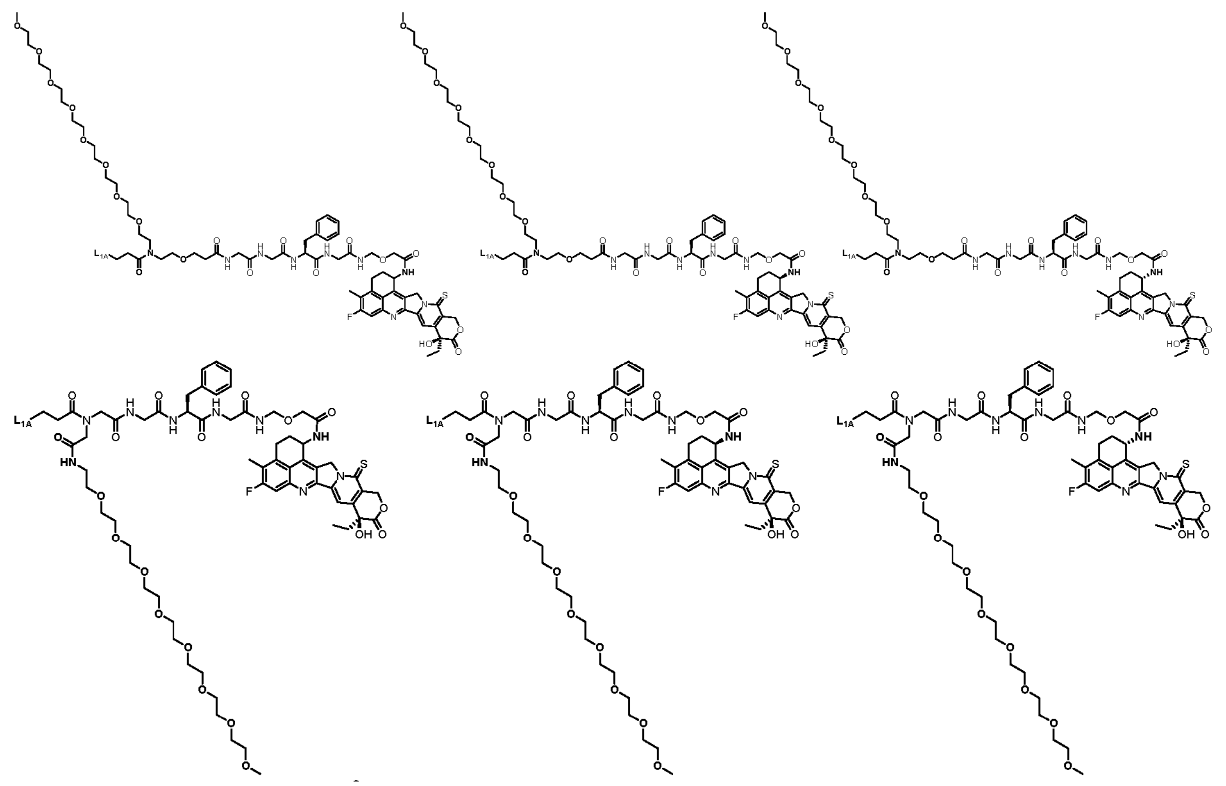

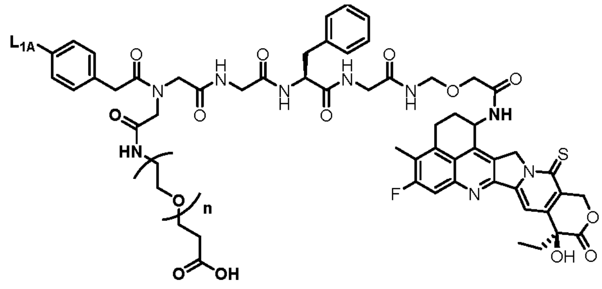

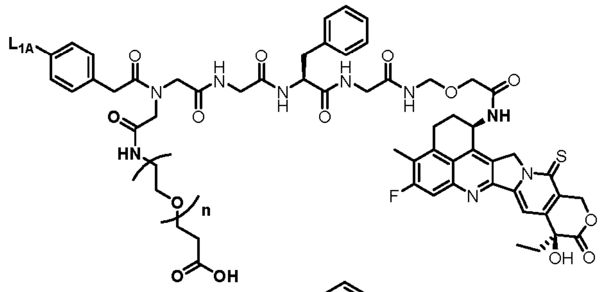

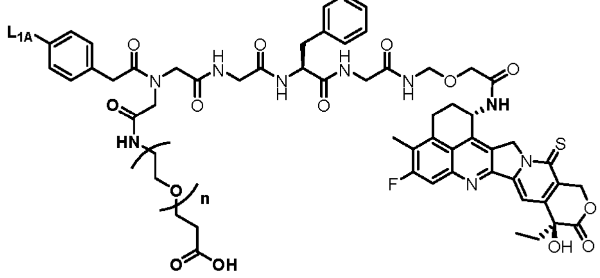

n = 1 ~ 24

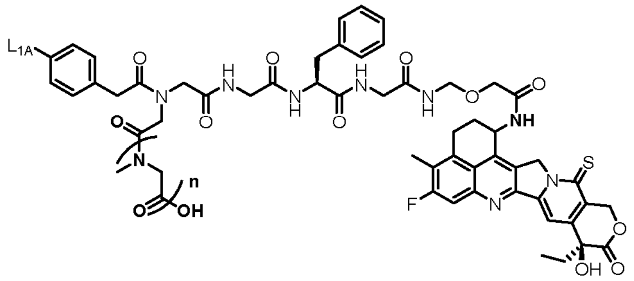

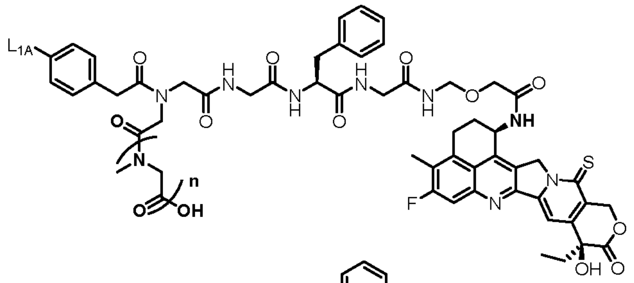

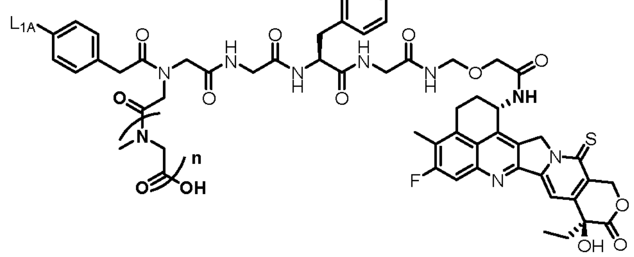

n = 1 ~ 24

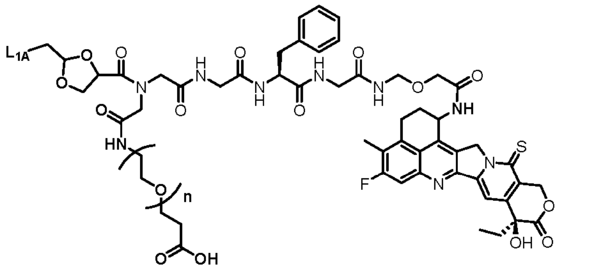

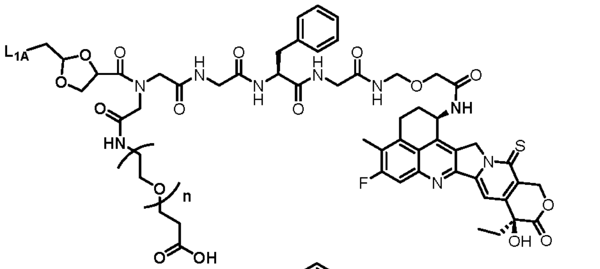

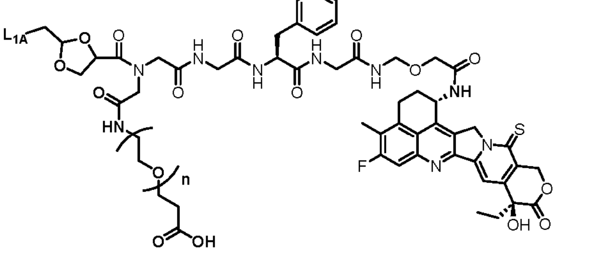

n = 1 ~ 24

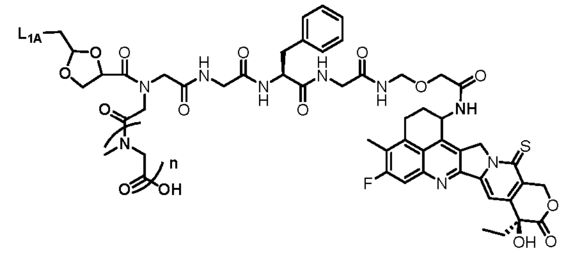

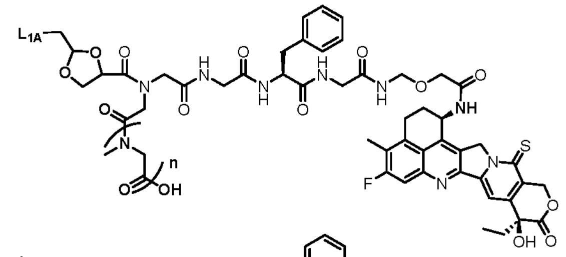

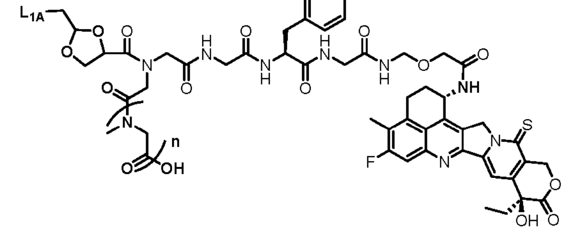

and

n = 1 ~ 24

.

In the structure shown above, $L_{1A}$ is selected from

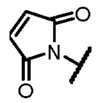 , 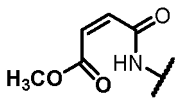 ,

and

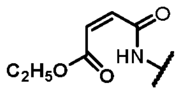 .

The third aspect of the present invention provides a linker as shown in formula (L), which connects a drug unit to a ligand to form a ligand-drug conjugate:

$$L_1\text{-}L_2\text{-}L_3\text{-}L_4\text{-}L_5 \text{ (L)},$$

wherein, the $L_1$, $L_2$, $L_3$, $L_4$, $L_5$ are defined as described in the first aspect of the present invention.

In another preferred embodiment, the linker is selected from the group consisting of

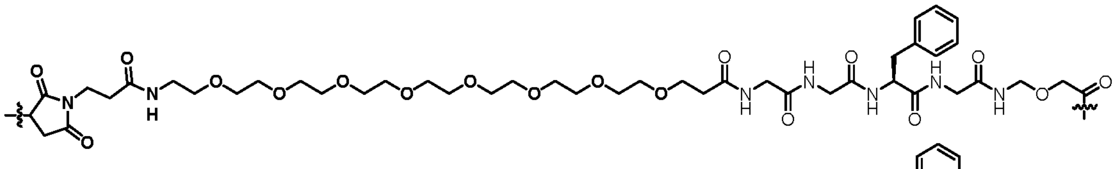

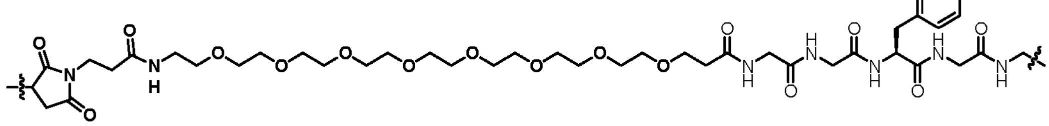

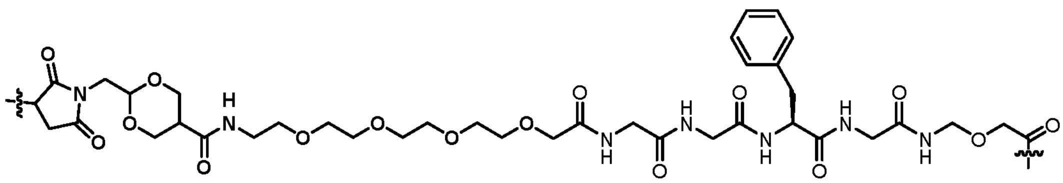

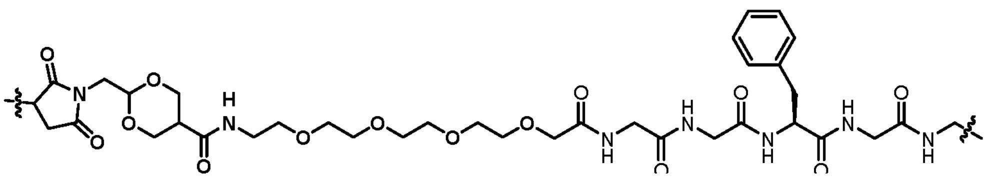

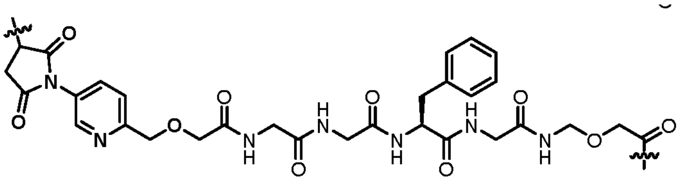

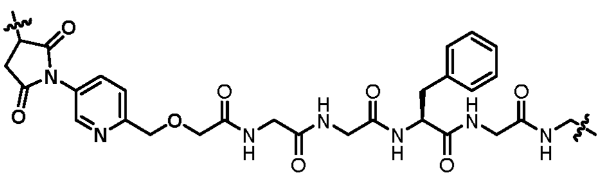

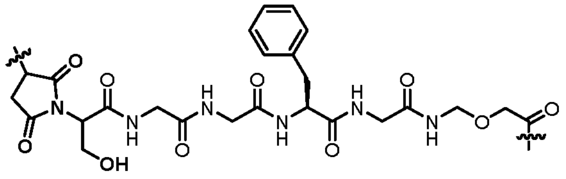

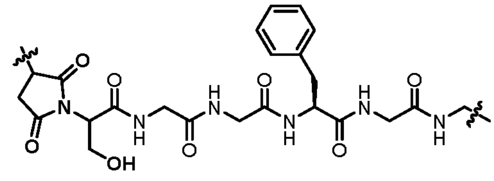

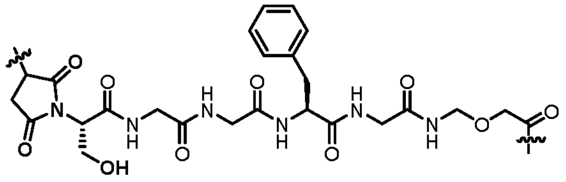

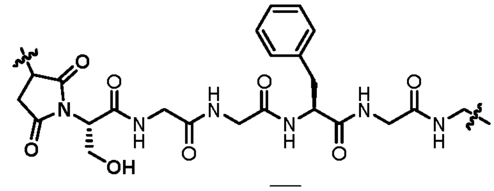

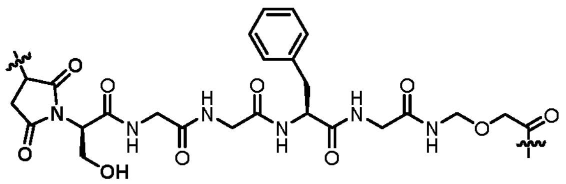

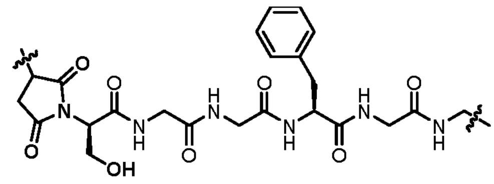

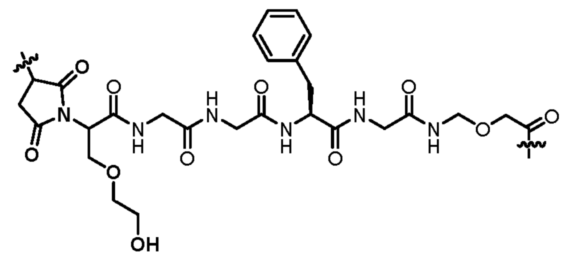

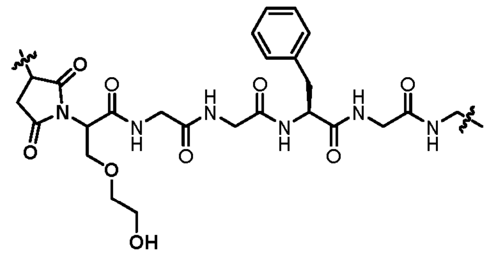

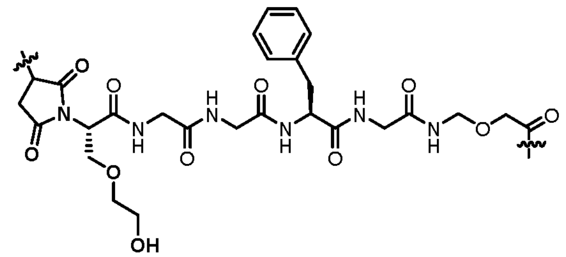

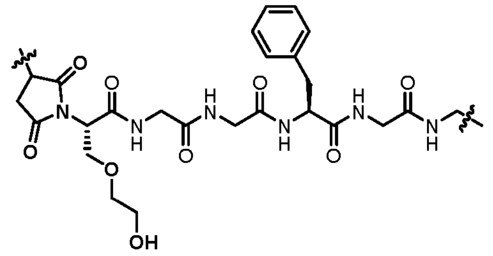

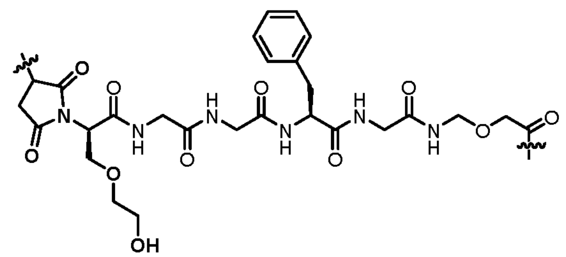

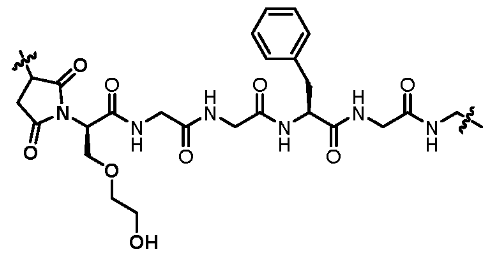

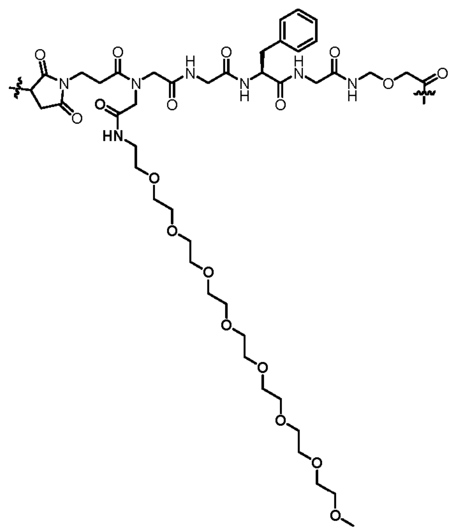

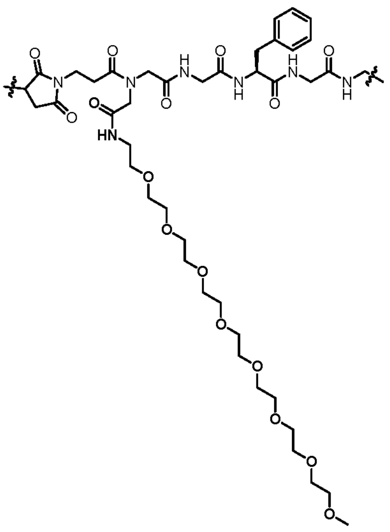

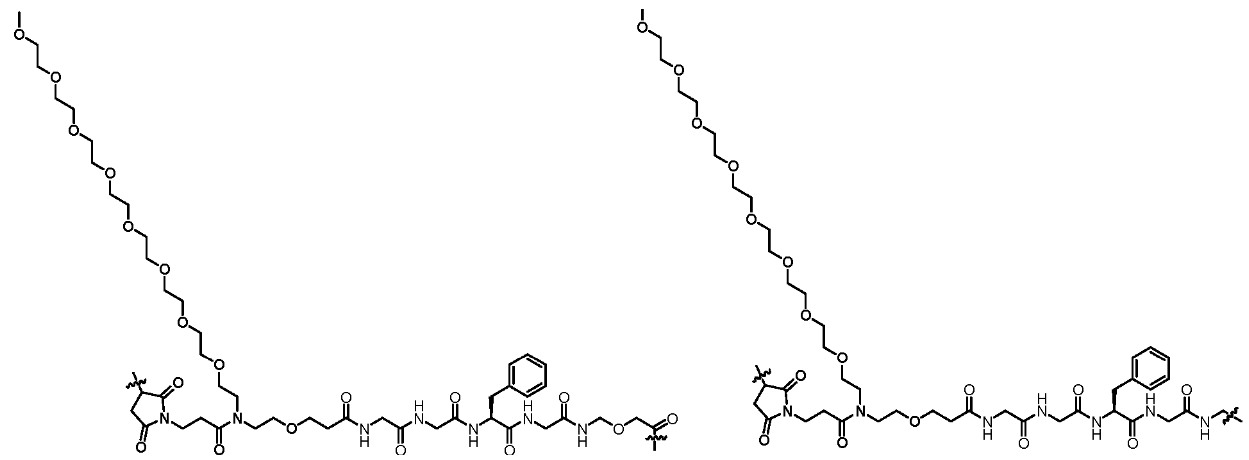

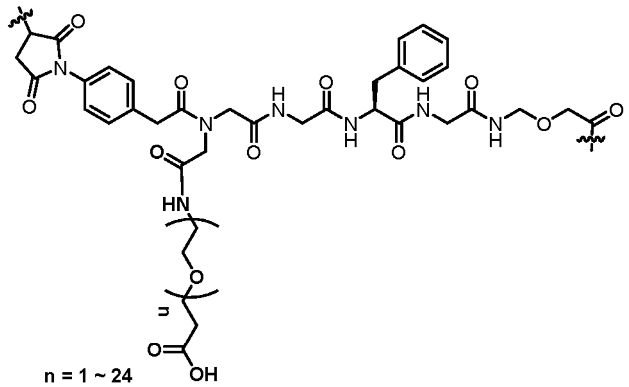

n = 1 ~ 24

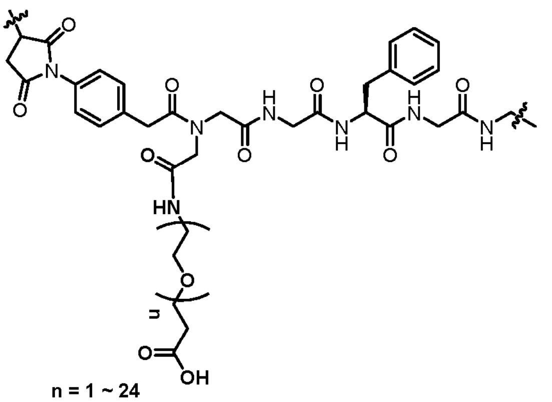

n = 1 ~ 24

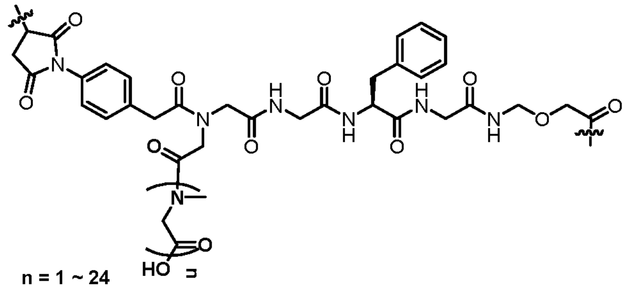

n = 1 ~ 24

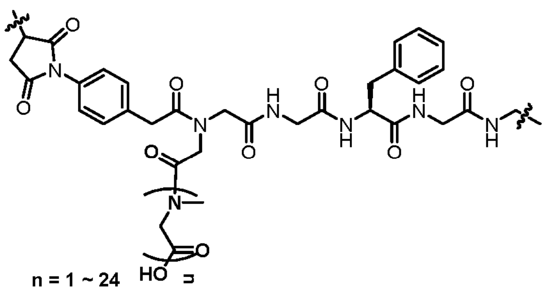

n = 1 ~ 24

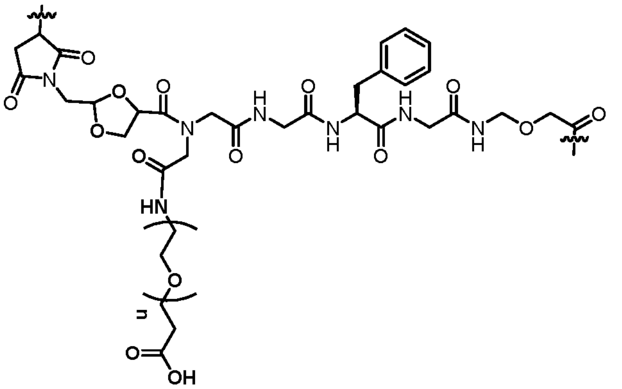

n = 1 ~ 24

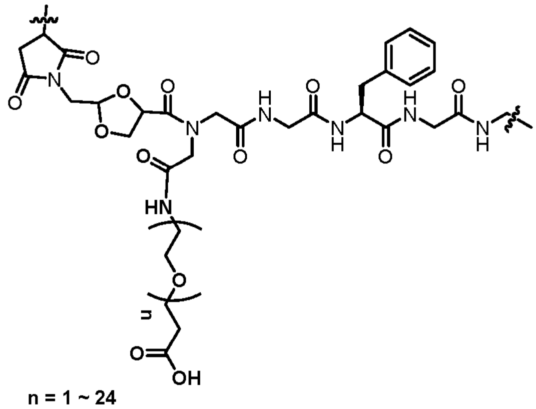

n = 1 ~ 24

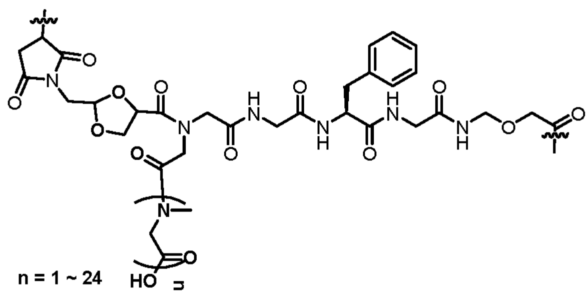

n = 1 ~ 24

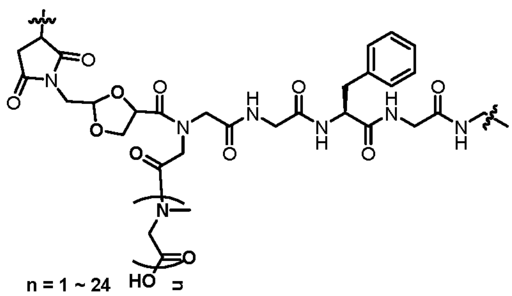

n = 1 ~ 24

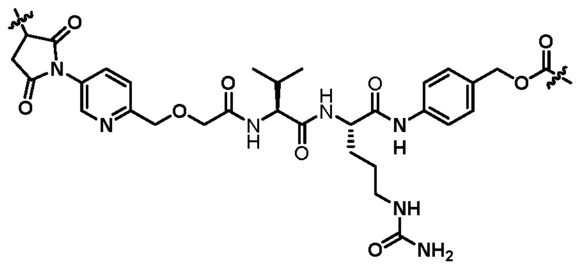

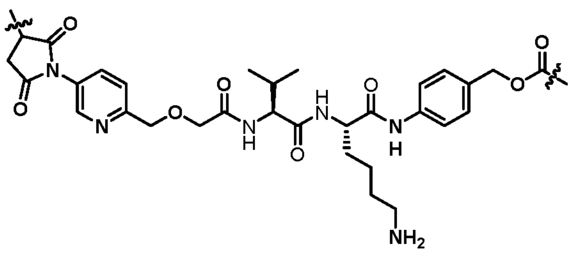

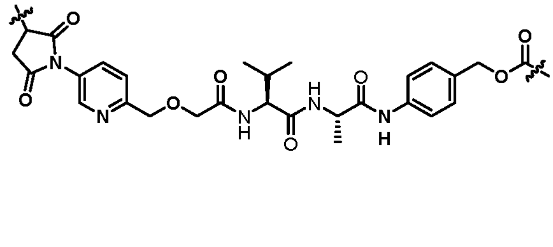

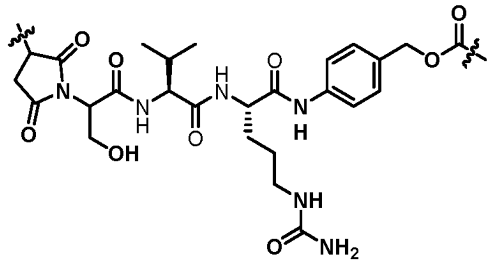

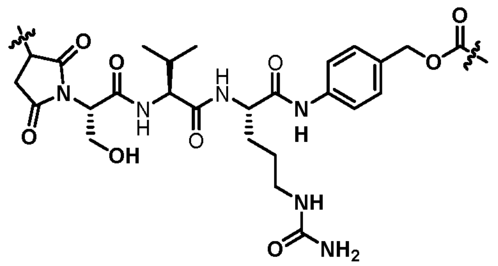

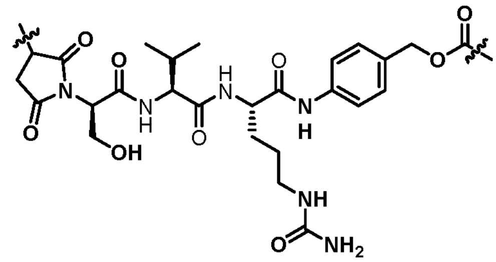

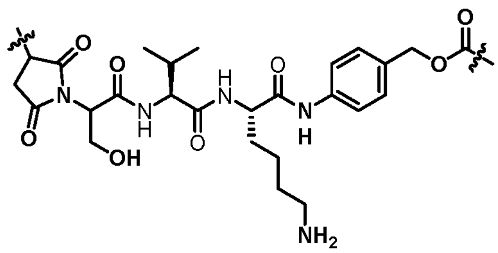

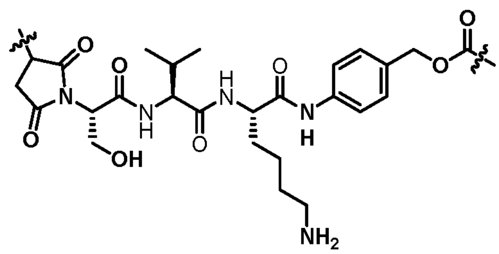

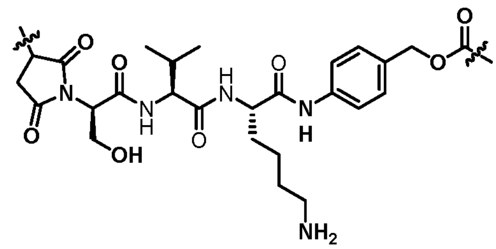

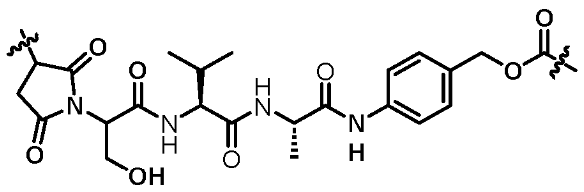

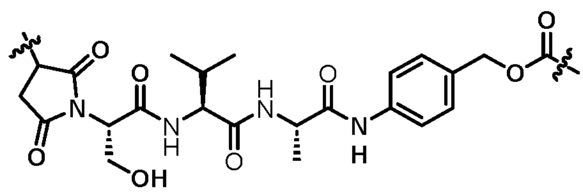

and

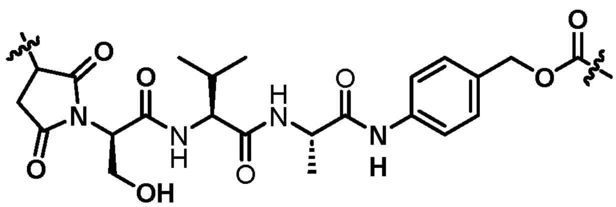

.

In another preferred embodiment, the linker is connected to the ligand via the $L_1$ segment and to P1 via the $L_5$ segment to form a ligand-drug conjugate; wherein the P1 is selected from the group consisting of Glycopeptide antibiotics,

such as bleomycin or pingyangmycin; DNA topoisomerase inhibitors, including topoisomerase I inhibitors (e.g., camptothecin, hydroxycamptothecin, 9-aminocamptothecin, SN-38, irinotecan, exatecan, topotecan, belotecan, rubitecan, or DXd) and topoisomerase II inhibitors (e.g., actinomycin D, doxorubicin, daunorubicin, mitoxantrone, podophyllotoxin, or etoposide); DNA synthesis inhibitors, such as methotrexate, 5-fluorouracil, cytarabine, gemcitabine, mercaptopurine, pentostatin, fludarabine, cladribine, or nelarabine; Structural proteintargeting agents, such as tubulin inhibitors (e.g., vinca alkaloids, vincristine, vinblastine, paclitaxel, docetaxel, or cabazitaxel); Tumor signaling pathway inhibitors, including serine/threonine kinase inhibitors, tyrosine kinase inhibitors, aspartate kinase inhibitors, or histidine kinase inhibitors; Proteasome inhibitors; Histone deacetylase inhibitors; Tumor angiogenesis inhibitors; Cyclin inhibitors; Maytansinoid derivatives (e.g., DM1, DM4); Calicheamicin derivatives; Auristatin derivatives (e.g., monomethyl auristatin E (MMAE), monomethyl auristatin F (MMAF), auristatin E, auristatin F); Pyrrolobenzodiazepine dimers (PBD) derivatives; Amatoxin derivatives (e.g., α-Amanitin); Anthracyclines; Duocarmycins; Eribulin; Melphalan; Mitomycin C; Chlorambucil; TLR agonists; STING agonists; Glucocorticoids; and Dehydro derivatives of other active substances that inhibit tumor cell growth or promote tumor cell apoptosis/necrosis.

The fourth aspect of the present invention provides a linker precursor as shown in formula (L-1), which is used to obtain a ligand-drug conjugate formed by connecting a drug unit to a ligand:

$L_A$-$R^g$ (L-1),

wherein $R^g$ is H, OH, O($C_1$-$C_6$ alkyl); and wherein $L_A$ is defined as described in the third aspect of the present invention.

In another preferred embodiment, the linker precursor is selected from the group consisting of

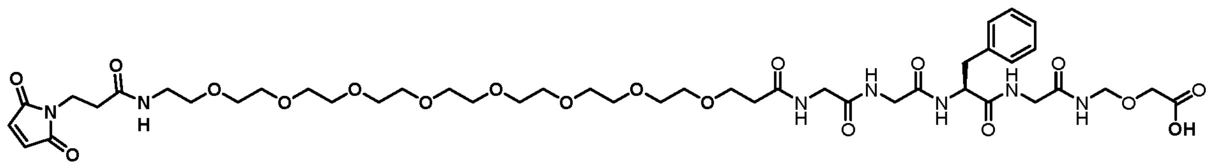

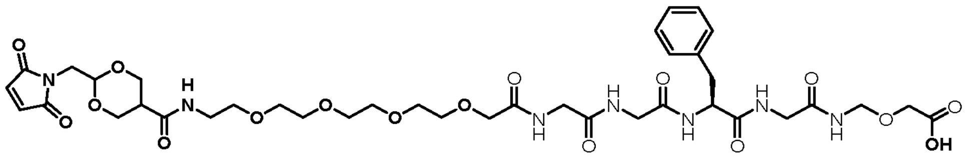

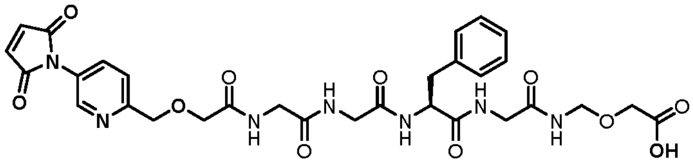

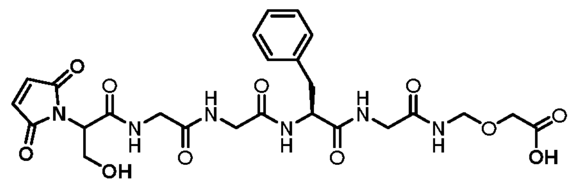

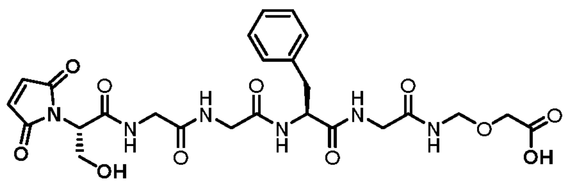

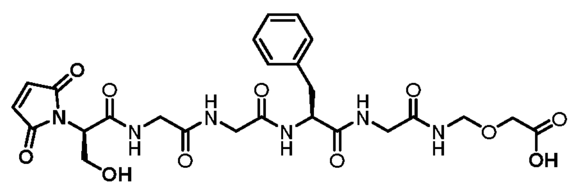

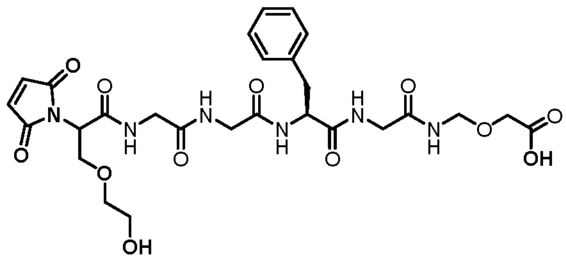

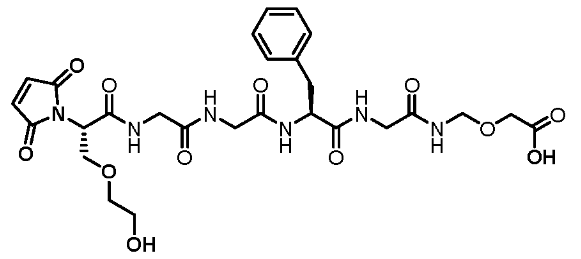

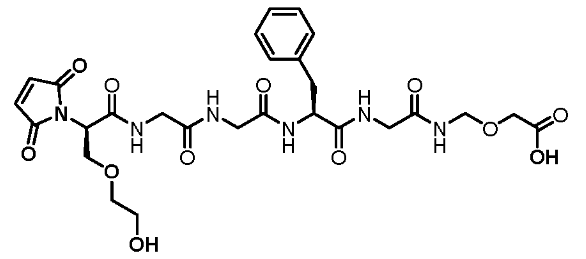

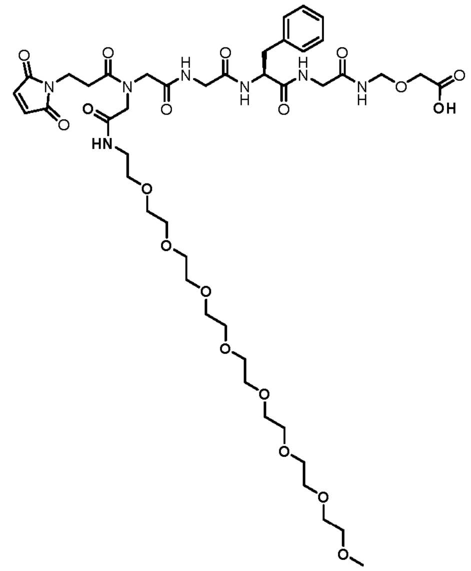

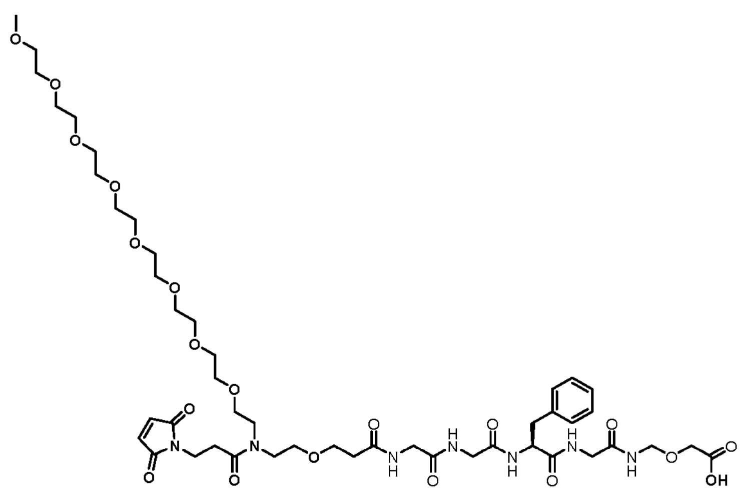

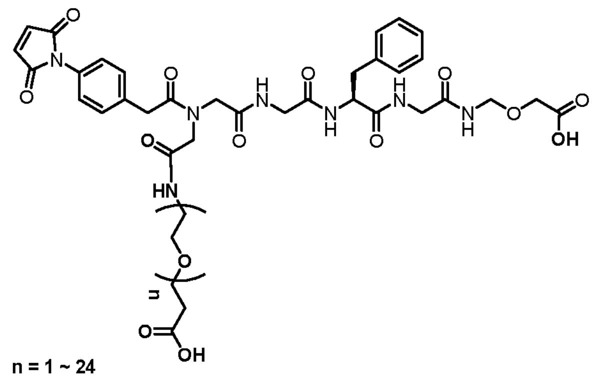

n = 1 ~ 24

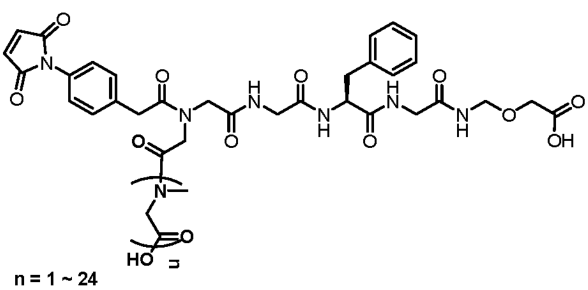

n = 1 ~ 24

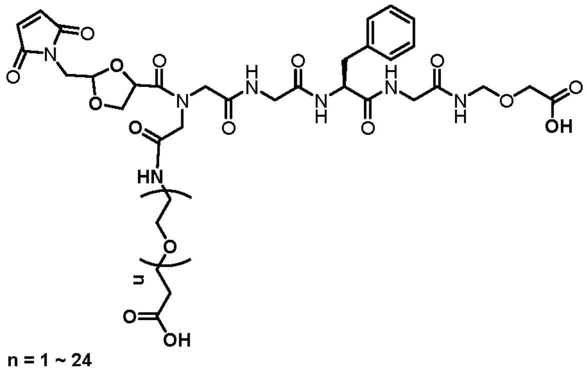

n = 1 ~ 24

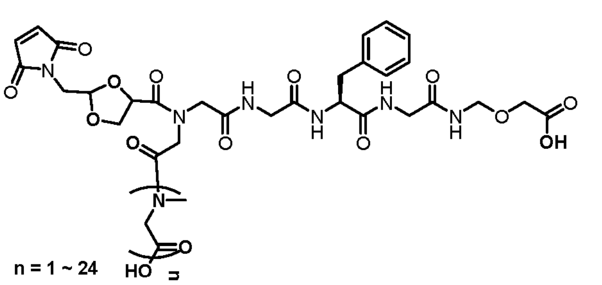

n = 1 ~ 24

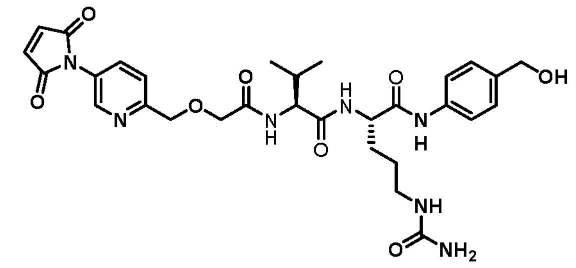

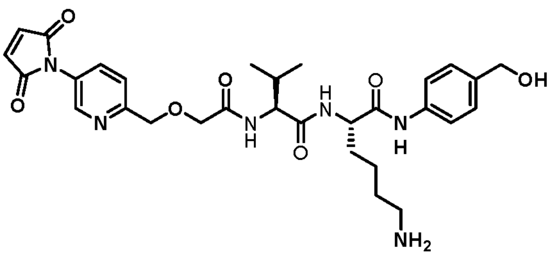

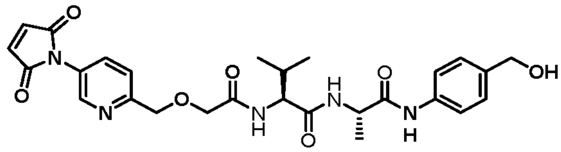

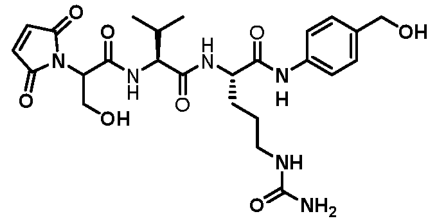

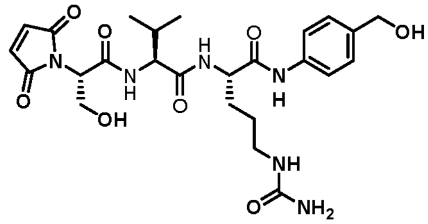

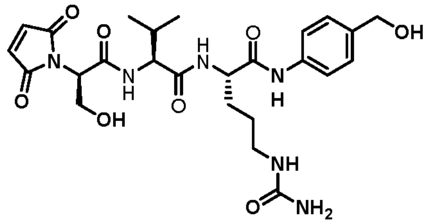

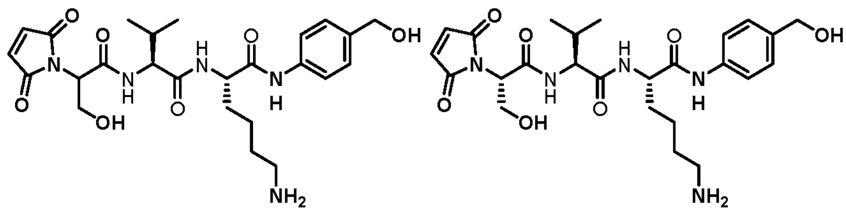

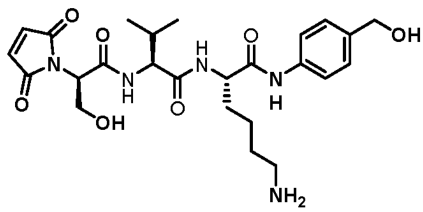

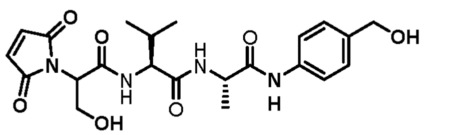

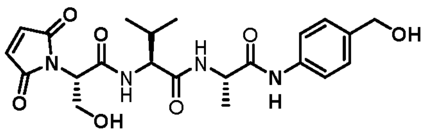 and 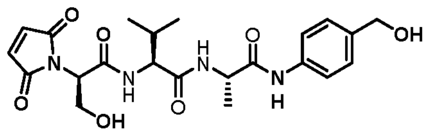 .

Also, the present invention provides a synthetic method for another preferred example of (L-1), described below:

Method 1:

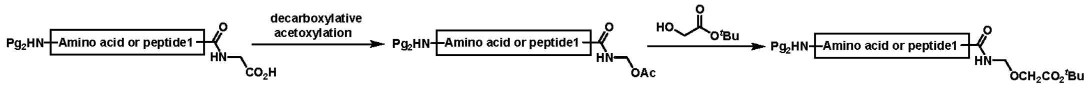

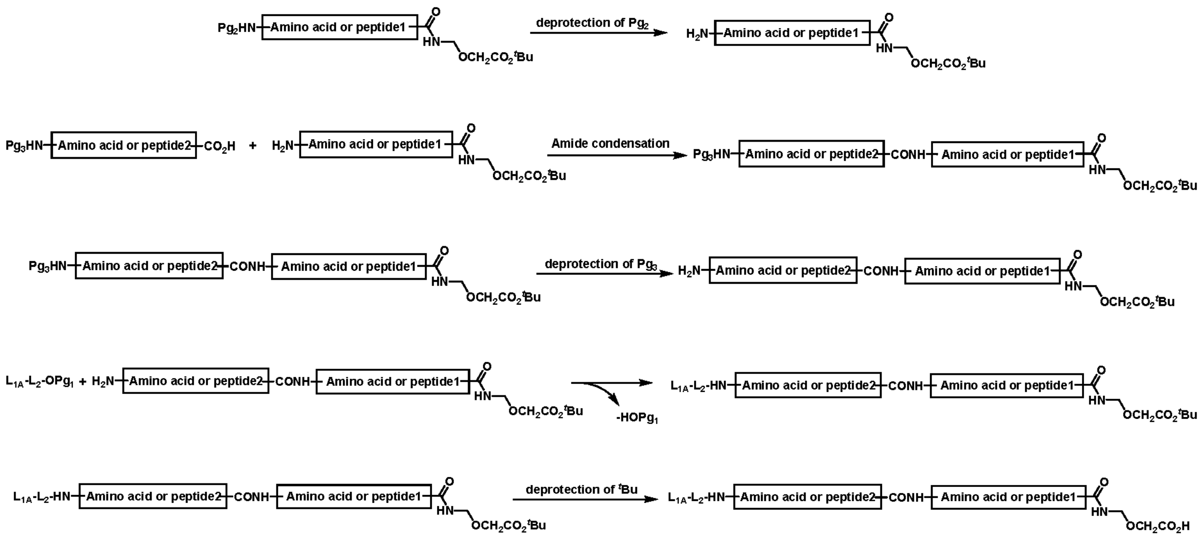


$Pg_1$ is selected from etc;

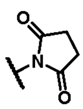, 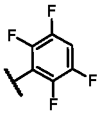, 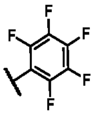, 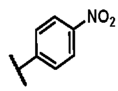, 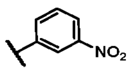, and 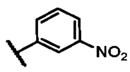,

$Pg_2$ is selected from Boc, Fmoc, and Cbz, etc;
$Pg_3$ is selected from Boc, Fmoc, and Cbz, etc;

Method 2:

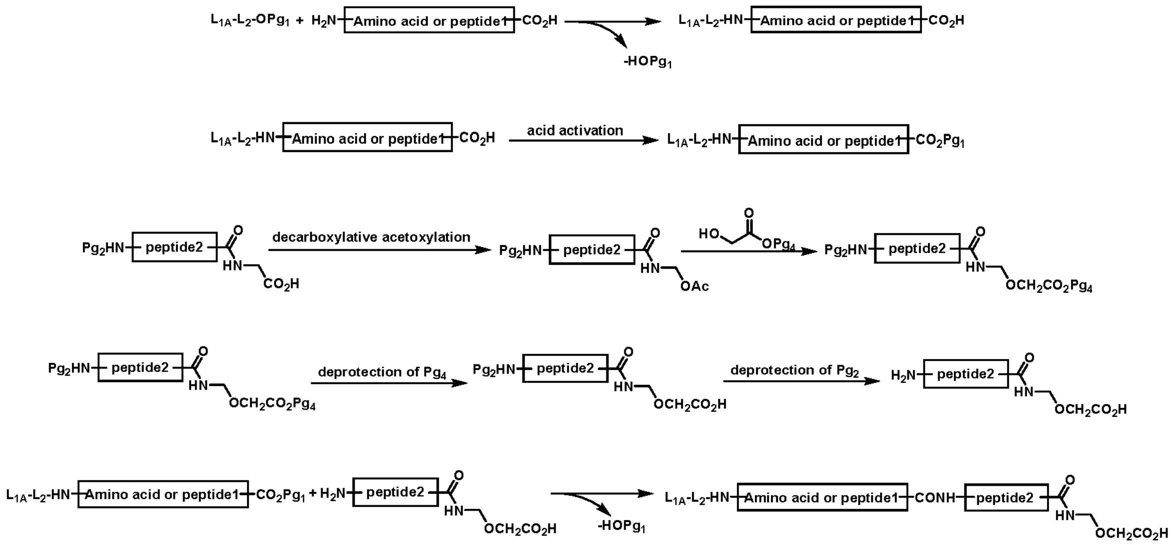


$Pg_1$ is selected from

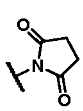, 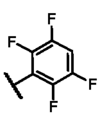, 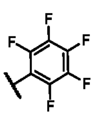, 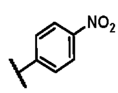, 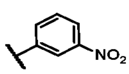, and 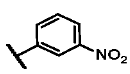,

etc;
$Pg_2$ is selected from Boc, Fmoc, and Cbz, etc;

$Pg_4$ is selected from Me, Et, $^{i}Pr$, Allyl, $^{t}Bu$, Bn, 4-Methybenzyl, 4-Methoxybenzyl, 2,4-Dimethoxybenzyl, 2,6-Dimethoxybenzyl, Trimethylsilyl, tert-Butyldimethylsilyl, and Pentafluorophenyl, etc.

The fifth aspect of the present invention provides a pharmaceutical composition comprises a ligand-drug conjugate as described in the first aspect of the present invention, or its tautomer, mesomer, racemate, enantiomer, diastereomer, or a mixture thereof, or a pharmaceutically acceptable salt, prodrug, or solvate thereof, and optionally pharmaceutically acceptable carrier.

A sixth aspect of the present invention provides a ligand-drug conjugate as described in the first aspect of the present invention, or its tautomer, mesomer, racemate, enantiomer, diastereomer, or mixture thereof, or pharmaceutically acceptable salts, prodrug, or solvate thereof, and/or the use of the pharmaceutical composition according to the fifth aspect of the present invention in the preparation of a drug for the treatment and/or prevention of diseases or disorders associated with the expression and/or abnormal expression of the target of the ligand.

In another preferred embodiment, the diseases or disorders associated with the expression and/or abnormal expression of the target of the ligand are tumors/cancers, autoimmune diseases or infectious diseases; preferably, the tumors/cancers are tumors/cancers with high, medium or low expression of the target of the ligand.

In another preferred embodiment, the tumors are selected from tumors associated with target expression in the group consisting of Her-2, Trop2, PSMA, FR-$\alpha$, B7H3, and IGF-1R.

In another preferred embodiment, the tumors comprise solid tumors and/or hematological tumors.

In another preferred embodiment, the tumors are tumors associated with target expression in the IGF-1R.

In another preferred embodiment, the tumor is selected from the group consisting of breast cancer, ovarian cancer, cervical cancer, endometrial cancer, urothelial cancer, lung cancer, prostate cancer, colorectal cancer, gastric cancer, esophageal cancer, bladder cancer, kidney cancer, pancreatic cancer, thyroid cancer, and head and neck cancer. Astrocytoma, basal or squamous cell carcinoma, brain cancer, neuroblastoma, glioblastoma, liposarcoma, bladder cancer, colorectal cancer, colon cancer, chondrosarcoma, kidney cancer, choriocarcinoma, leukemia, multiple myeloma, Ewing's sarcoma, gastrointestinal cancer, head and neck cancer, liver cancer, glioma, hepatocellular carcinoma, leiomyoma, melanoma, non-small cell lung cancer, nervous system cancer, pancreatic cancer, renal cell carcinoma, rhabdomyosarcoma, small cell lung cancer, thymoma, thyroid cancer, testicular cancer and osteosarcoma.

Another aspect of the present invention provides a method for preparing a linker precursor as shown in formula (L-1), characterized in that it comprises the steps:

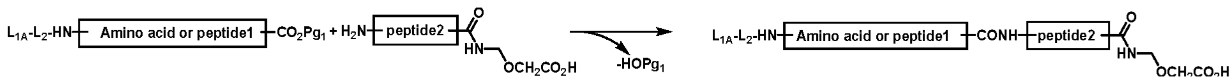


Reacting

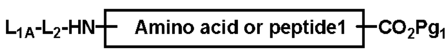 with 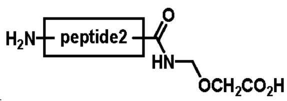

to give

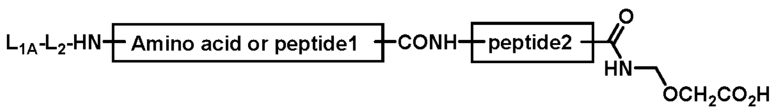;

wherein $L_{1A}$ is

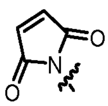 or 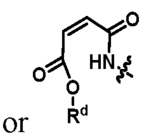;

preferably, $L_{1A}$ is

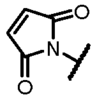, 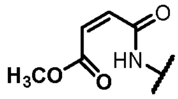,

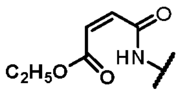;

more preferably, $L_{1A}$ is

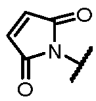.

$L_2$ is as described in any of the foregoing; preferably, $L_2$ is selected from the group consisting of

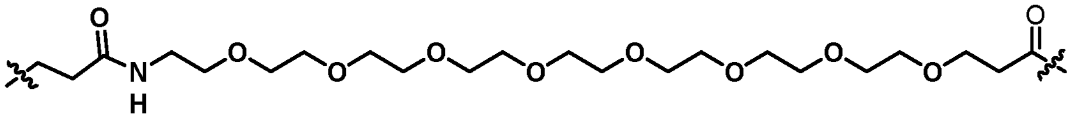

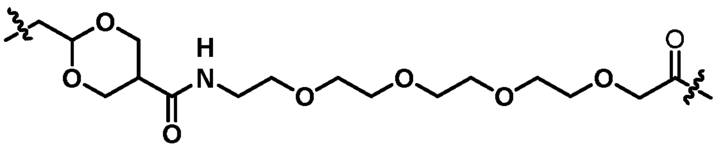, 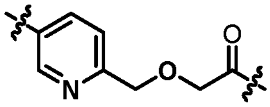, 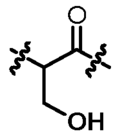,

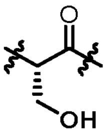, 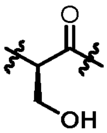, 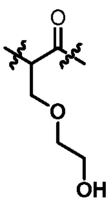, 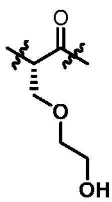, 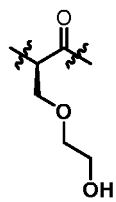, 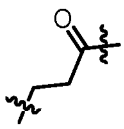, 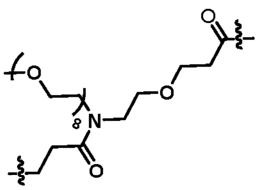,

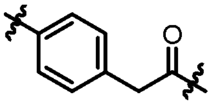, 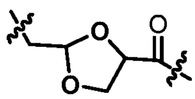, 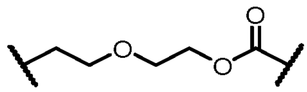, 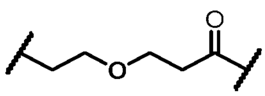,

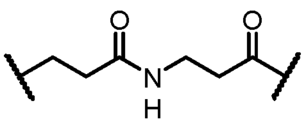, 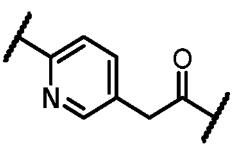, 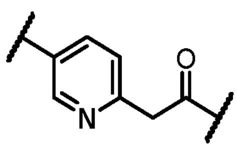, 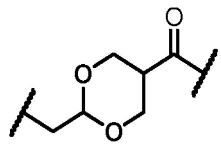,

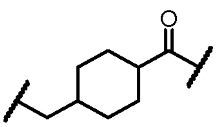, and 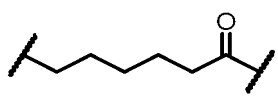;

more preferably, $L_2$ is selected from

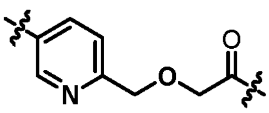, 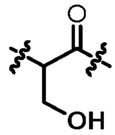, 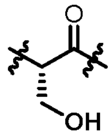, 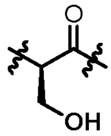,

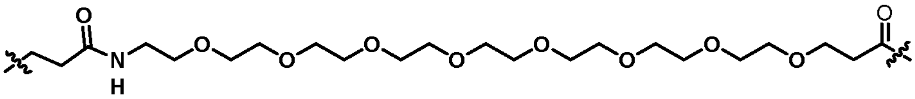,

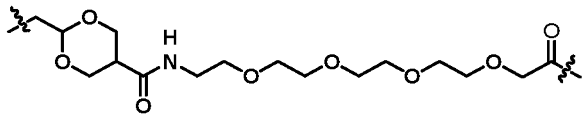, and 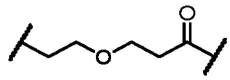;

more preferably, $L_2$ is selected from

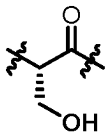 and 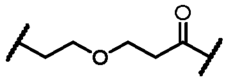.

Preferably,

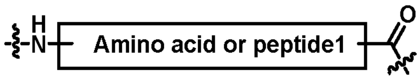

is selected from amino acids or dipeptide fragments; Preferably,

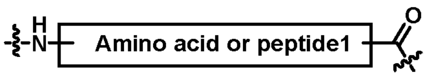

is glycine

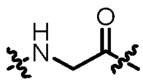

or glycine-glycine fragment

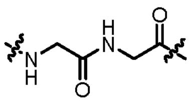;

Preferably,

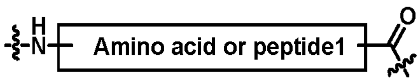

is a glycine-glycine fragment

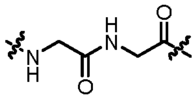

;

Preferably,

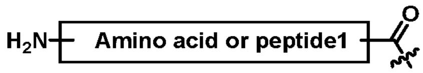

is selected from amino acids or dipeptide fragments; Preferably,

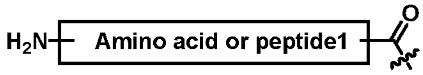

is glycine

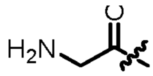

or glycine-glycine fragment

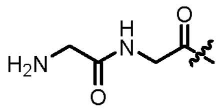

;

Preferably,

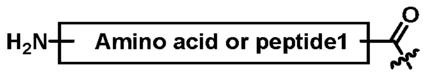

is a glycine-glycine fragment

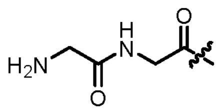

;

preferably,

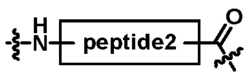

is dipeptide or tripeptide fragment; preferably,

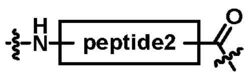

is glycine-phenylalanine-glycine fragment

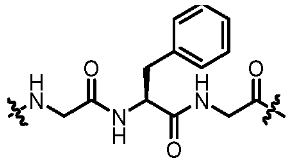,

phenylalanine-glycine fragment

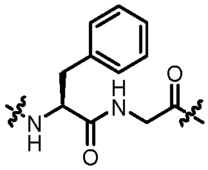;

preferably,

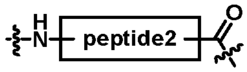

is phenylalanine-glycine fragment

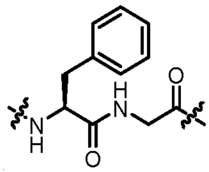;

preferably,

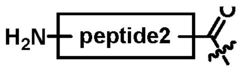

is dipeptide or tripeptide fragment; preferably,

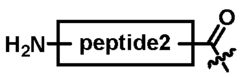

is glycine-phenylalanine-glycine fragment

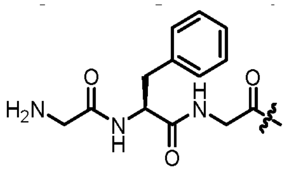,

phenylalanine-glycine fragment

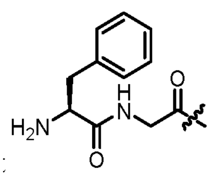;

preferably,

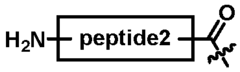

is phenylalanine-glycine fragment

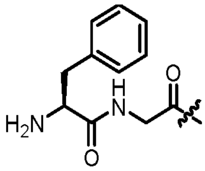

;

$Pg_1$ is selected from the group consisting of

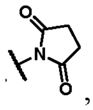

,

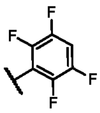

,

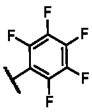

,

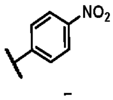

,

and

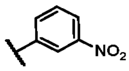

;

preferably, the $Pg_1$ is selected from the group consisting of

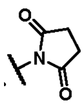

,

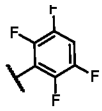

,

and

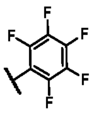

;

preferably, the $Pg_1$ is

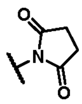

;

preferably, the $Pg_1$ is

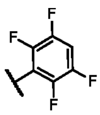

;

preferably, the $Pg_1$ is

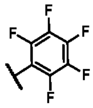
;

preferably, the

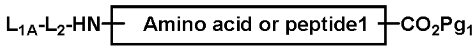

is prepared by the following method:

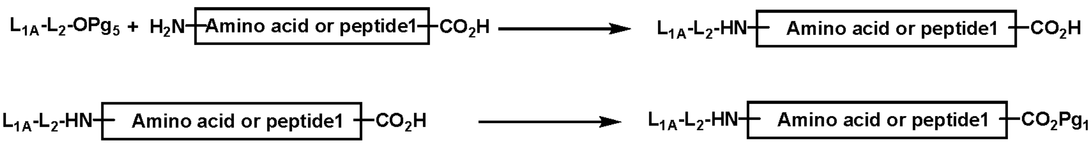

(1) Reacting $L_{1A}$-$L_2$-$OPg_5$ with

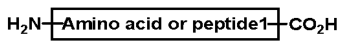

to obtain

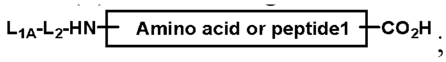
;

preferably, the reaction is conducted in the absence of a base or in the presence of a base; more preferably, the reaction is conducted in the absence of a base; more preferably, the base is selected from the group consisting of triethylamine, N,N-diisopropylethylamine, 2,4,6-collidine, DMAP, DBU, N-methylmorpholine, and $NaHCO_3$; even more preferably, the base is $NaHCO_3$;

$Pg_5$ is selected from the group consisting of

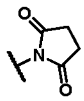
,
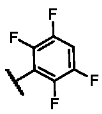
,
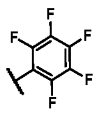
,
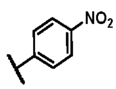
,

and

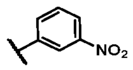
;

more preferably, $Pg_5$ is selected from the group consisting of

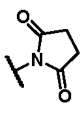
,
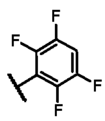
,

and

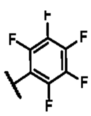
;

even more preferably, $Pg_5$ is

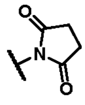;

alternatively, $Pg_5$ is

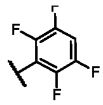;

or alternatively, $Pg_5$ is

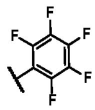;

(2) Reacting $Pg_1OH$ with

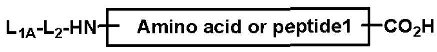

to obtain

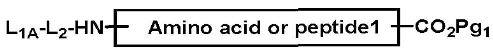;

preferably, $Pg_1OH$ is selected from the group consisting of

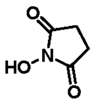, 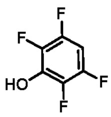, 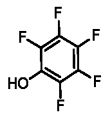,

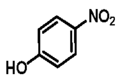, and 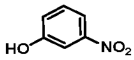;

more preferably, $Pg_1OH$ is selected from the group consisting of

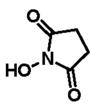, 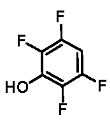, and 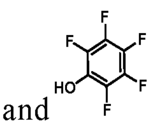;

even more preferably, $Pg_1OH$ is

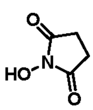;

alternatively, $Pg_1OH$ is

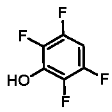
;

or alternatively, $Pg_1OH$ is

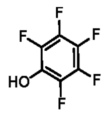
;

Preferably, the

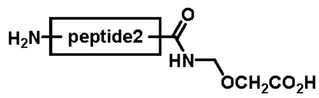

is prepared by the following method:

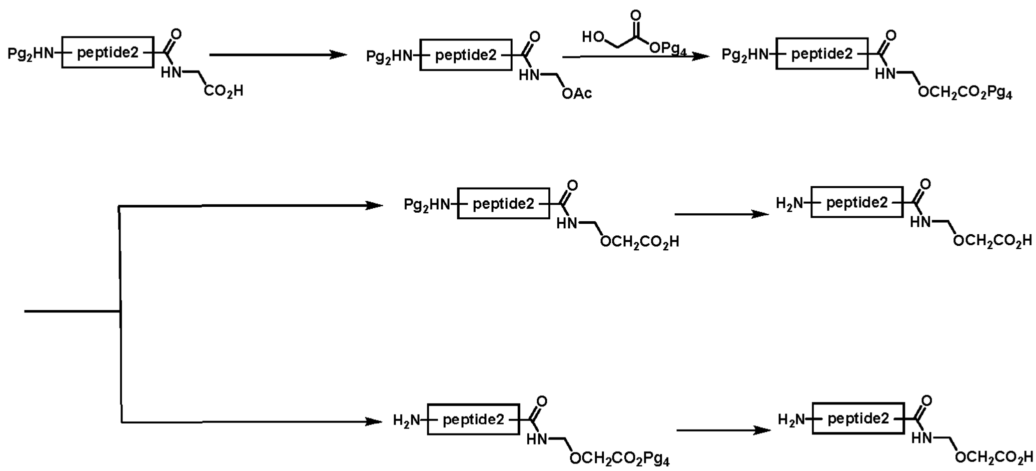

(i) reacting formula

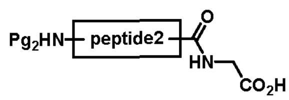

to obtain

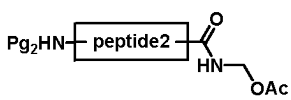
;

(ii) reacting

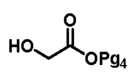

with

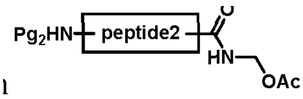

to obtain

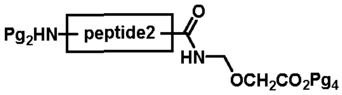;

and the method further comprises: obtaining

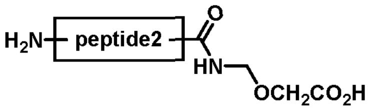

through the following method (iii) or (iv):

(iii) first deprotection of $Pg_4$ on

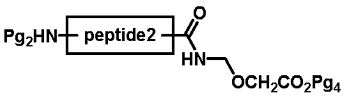

to obtain

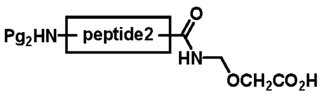,

followed by deprotection of $Pg_2$ on

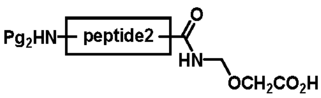

to obtain

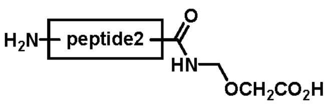;

preferably, the deprotection is performed in the presence of a base; preferably, the base is an organic base; more preferably, the base is DBU; preferably, the deprotection is performed in the presence of a reducing agent; preferably, the reducing agent is $H_2$; preferably, the deprotection is performed in the presence of a catalyst; preferably, the catalyst is Pd/C;

(iv) first deprotection of $Pg_2$ on

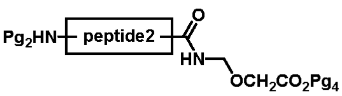 to obtain

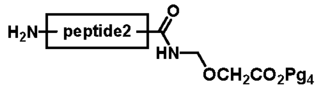
,

followed by deprotection of $Pg_4$ on

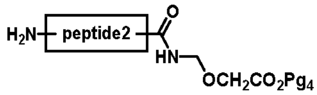

to obtain

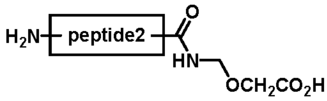
;

preferably, the deprotection is performed in the presence of a base; preferably, the base is an organic base; more preferably, the base is DBU; preferably, the deprotection is performed in the presence of a reducing agent; preferably, the reducing agent is $H_2$; preferably, the deprotection is performed in the presence of a catalyst; preferably, the catalyst is Pd/C;

wherein $Pg_2$ is selected from Boc, Fmoc, and Cbz;

$Pg_4$ is selected from Me, Et, $^i$Pr, Allyl, $^t$Bu, Bn, 4-methylbenzyl, 4-methoxybenzyl, 2,4-dimethoxybenzyl, 2,6-dimethoxybenzyl, trimethylsilyl, tert-butyldimethylsilyl, and pentafluorophenyl.

In another preferred embodiment, the linker precursor is

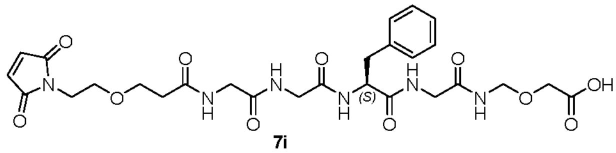
**7i**

or

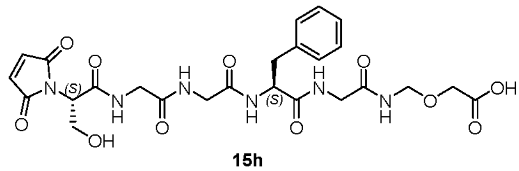
**15h**

.

Another aspect of the present invention provides a method for preparing a compound of formula 4 as follows:

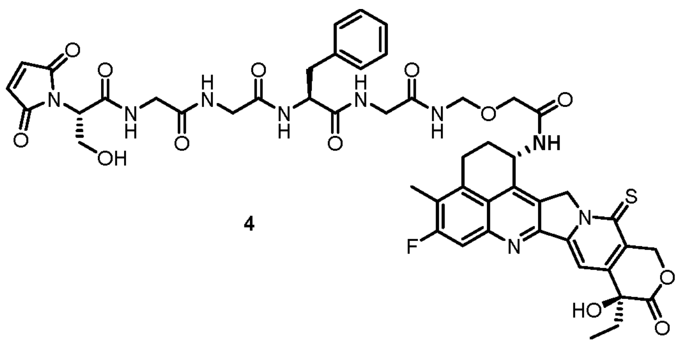
**4**

characterized in that the method comprises the step of:

conducting a condensation reaction between a compound of formula 15h and a compound of formula 1d in an inert solvent to obtain the compound of formula 4:

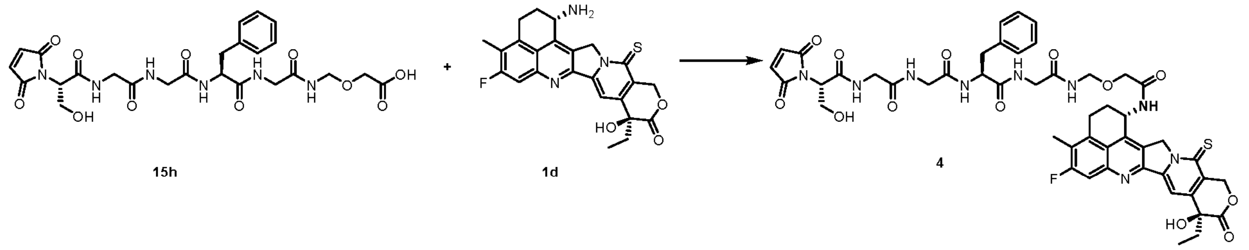

15h 1d 4

In another preferred embodiment, the inert solvent is selected from the group consisting of dichloromethane, tetrahydrofuran, N,N-dimethylformamide, N,N-dimethylacetamide, and HMPA, or a combination thereof; preferably N,N-dimethylformamide;

In another preferred embodiment, the reaction is conducted in the presence of a condensing agent; more preferably, the condensing agent is selected from the group consisting of HATU, HBTU, TBTU, EDCI, HOAt, HOBt, CDI, TCFH, TFFH, DCC, DIC, BOP, AOP, PyAOP, BrOP, PyClOP, PyBrOP, and DMTMM, or a combination thereof; more preferably, the condensing agent is HATU;

In another preferred embodiment, the reaction is conducted in the presence of a base; more preferably, the base is selected from the group consisting of TEA, DIPEA, DBU, DMAP, pyridine, 2,6-lutidine, 2,4,6-collidine, imidazole, and N-methylimidazole, or a combination thereof; more preferably, the base is 2,4,6-collidine.

Another aspect of the present invention provides a method for preparing a compound of formula 15h below,

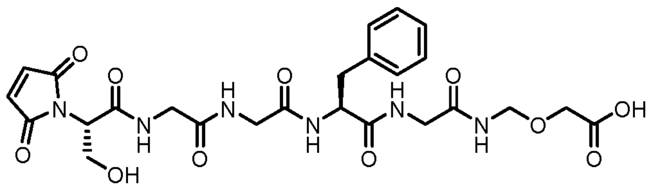

**15h**

characterized in that the method comprises the steps of:

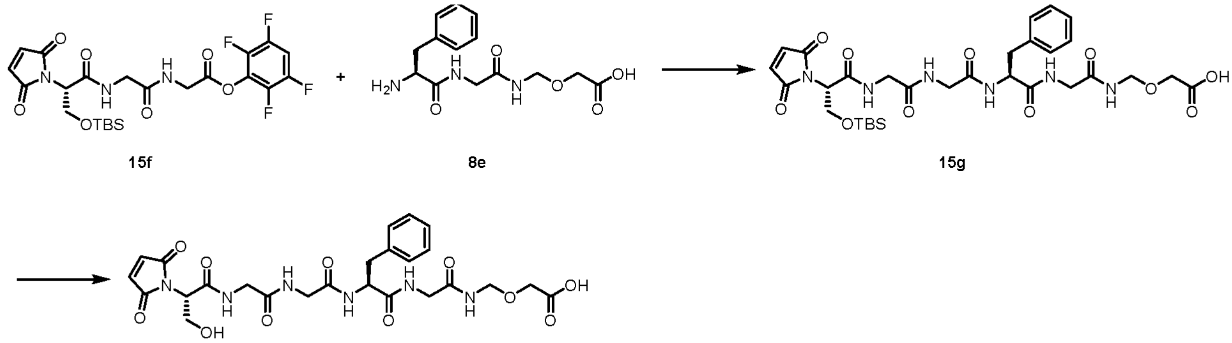

15f 8e 15g

15h

(1) reacting a compound of formula 15f with a compound of formula 8e in an inert solvent to obtain a compound of formula 15g;

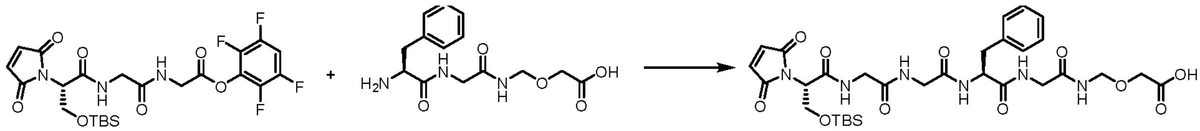

15f 8e 15g

In another preferred embodiment, the inert solvent is selected from the group consisting of acetonitrile, water, tetrahydrofuran, 2-methyltetrahydrofuran, and dichloromethane, or a combination thereof; further preferably acetonitrile, and water, or a combination thereof; further preferably a combination of acetonitrile and water in a volume ratio of 10:1 to 1:10; more preferably, the inert solvent is a combination of acetonitrile and water in a

volume ratio of 2:1 to 4:1; even more preferably, the inert solvent is a combination of acetonitrile and water in a volume ratio of 3:1;

In another preferred embodiment, the reaction may be performed in the presence of a base; preferably, the base is selected from TEA, DIPEA, NMM, sodium bicarbonate, and DBU, or a combination thereof; more preferably, the base is DIPEA;

(2) In an inert solvent, the silyl protecting group of the compound of formula **15g** is deprotected using a desilylation reagent to give the compound of formula **15h;**

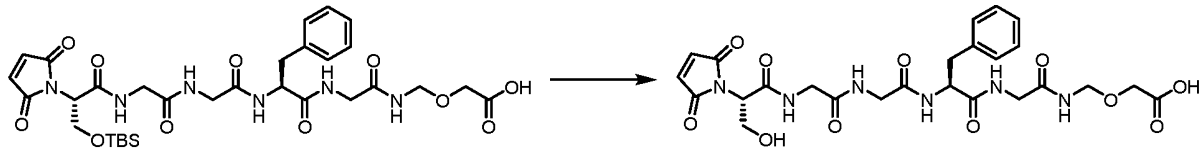

15g 15h

In another preferred embodiment, the desilylation reagent is selected from the group consisting of acids and fluorinating reagents (fluorine-containing reagents). Preferably, the desilylation reagent is selected from hydrogen fluoride, an aqueous solution of hydrogen fluoride, triethylamine trihydrofluoride, pyridine hydrofluoride, formic acid, acetic acid, trifluoroacetic acid, dichloroacetic acid, methanesulfonic acid, and trifluoromethanesulfonic acid; preferably, the desilylation reagent is formic acid;

In another preferred embodiment, the inert solvent is selected from the group consisting of tetrahydrofuran, N,N-dimethylformamide, acetonitrile, and water, or a combination thereof; further preferably acetonitrile, water, or a combination thereof; further preferably a combination of acetonitrile and water in a volume ratio of 10: 1 to 1: 10, further preferably a combination of acetonitrile and water in a volume ratio of 1:4 to 4:1, further preferably a combination of acetonitrile and water in a volume ratio of 1:1.5.

Another aspect of the present invention also provides a preparation method for a compound of formula **8e** as described below,

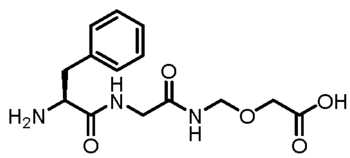

8e

characterized in that the method comprises the steps of:

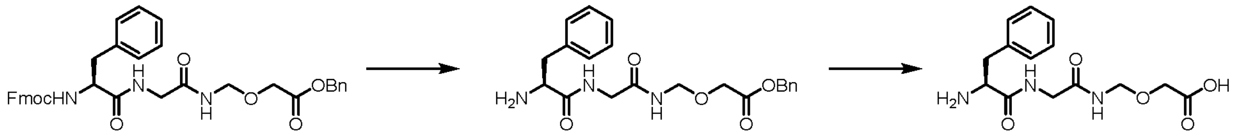

8c 15b 8e

(1) In an inert solvent, removing the Fmoc protecting group of a compound of formula 8c as described below under basic conditions to obtain a compound of formula 15b;

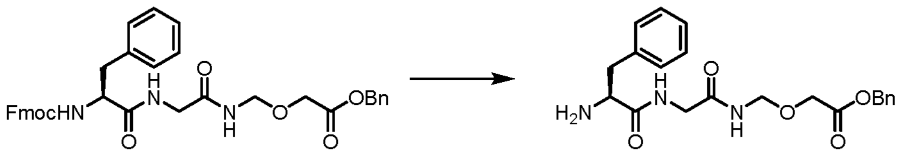

8c 15b

In another preferred embodiment, the inert solvent is selected from dichloromethane, tetrahydrofuran, and 2-methyltetrahydrofuran, or a combination thereof; more preferably, the inert solvent is dichloromethane;

In another preferred embodiment, the base is an organic base; more preferably, the base is dimethylamine, diethylamine, DBU, or piperidine; still more preferably, the base is DBU.

(2) In an inert solvent, removing the benzyl protecting group of a compound of formula **15b** as described below to obtain the compound of formula **8e;**

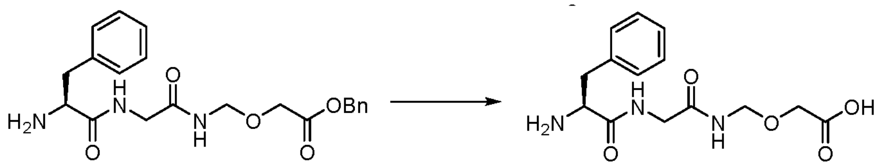

**15b** **8e**

In another preferred embodiment, the inert solvent is selected from tetrahydrofuran, and water, or a combination thereof; more preferably, the inert solvent is water; still more preferably, the inert solvent is a combination of tetrahydrofuran and water in a volume ratio of 1:5 to 5:1.
In another preferred embodiment, the deprotection is performed in the presence of a base; preferably, the base is an organic base; more preferably, the base is DBU;
In another preferred embodiment, the deprotection is performed in the presence of a reducing agent; more preferably, the reducing agent is $H_2$; more preferably, the deprotection is performed in the presence of a catalyst; more preferably, the catalyst is Pd/C;

Another aspect of the present invention also provides a preparation method for a compound of formula **8c** as described below:

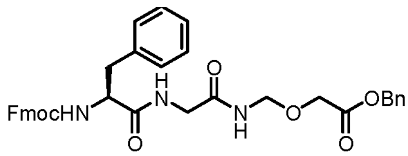

**8c**

characterized in that the method comprises the step of:

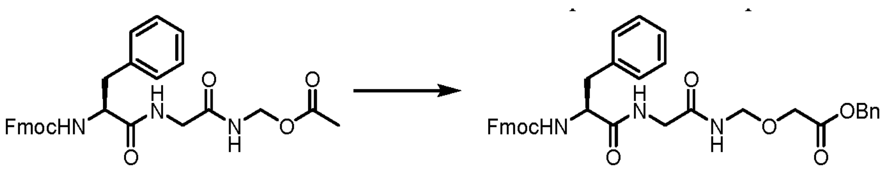

**8b** **8c**

The compound of formula **8b** is reacted with benzyl glycolate in an inert solvent to give the compound of formula **8c;**
In another preferred embodiment, the inert solvent is selected from dichloromethane, tetrahydrofuran, 2-methyl tetrahydrofuran, and dioxane, or combination thereof; more preferably, the inert solvent is tetrahydrofuran.

In another preferred embodiment, the reaction is conducted in the presence of an acid or base; more preferably, the acid is *p*-toluenesulfonic acid or *p*-toluenesulfonic acid monohydrate; more preferably, the base is lithium hydroxide, lithium hydroxide monohydrate, potassium tert-butoxide, sodium tert-butoxide, or sodium hydroxide; more preferably, the base is lithium hydroxide; more preferably, the base is lithium hydroxide monohydrate;
Another aspect of the present invention also provides a method for preparing a compound of the formula **8b** as follows:

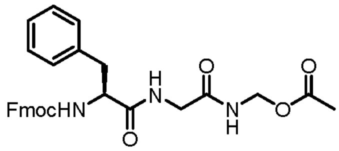

8b

Characterized in that the method comprises the steps of:

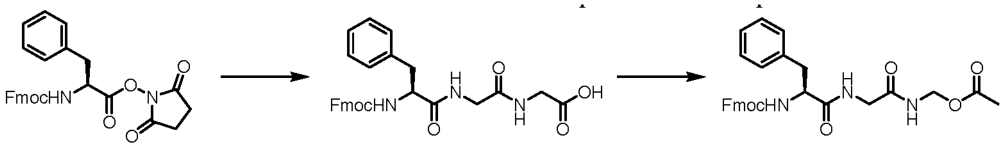

15a 8a 8b

(1) reacting the compound of the formula 15a with glycylglycine in an inert solvent to obtain the compound of the formula 8a;

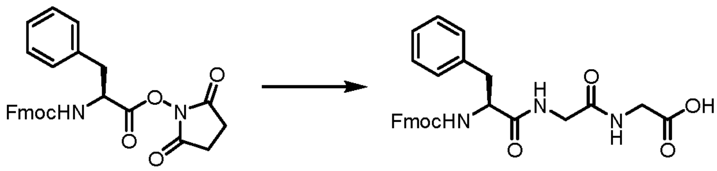

15a 8a

In another preferred embodiment, the reaction is carried out in the presence of a base; more preferably, the base is sodium bicarbonate;
In another preferred embodiment, the inert solvent is selected from 1,2-dimethoxyethane, and water, or a combination thereof; more preferably, the inert solvent is a combination of 1,2-dimethoxyethane and water; more preferably, the inert solvent is a combination of 1,2-dimethoxyethane and water in a volume ratio of 1:5 to 5:1; more preferably, the inert solvent is a combination of 1,2-dimethoxyethane and water in a volume ratio of 1:2 to 2:1;

(2) the compound of formula **8a** is reacted in the inert solvent to obtain the compound of formula **8b;**

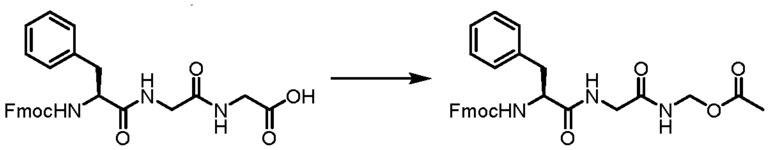

8a 8b

In another preferred embodiment, the inert solvent is selected from N,N-dimethylformamide, N,N-dimethylacetamide, and HMPA; more preferably, the inert solvent is N,N-dimethylformamide.

In another preferred embodiment, the reaction is carried out in the presence of lead tetraacetate;
In another preferred embodiment, the reaction is carried out in the presence of copper acetate;
In another preferred embodiment, the reaction is carried out in the presence of acetic acid;
In another preferred embodiment, the reaction is carried out in the presence of acetic acid, lead tetraacetate and copper acetate;
In another preferred embodiment, the reaction is carried out in the presence of acetic acid and lead tetraacetate;
Another aspect of the present invention also provides a preparation method for a compound of formula 15f as described below:

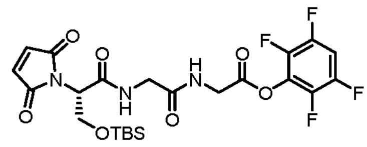

15f

characterized in that the method comprises the steps of:

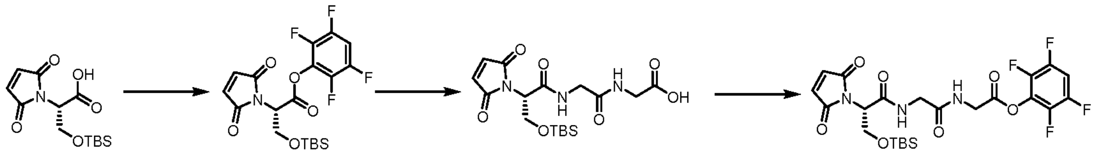

15c 15d 15e 15f

(1) In an inert solvent, performing a condensation reaction of a compound of formula **15c** as described below with tetrafluorophenol to obtain a compound of formula **15d;**

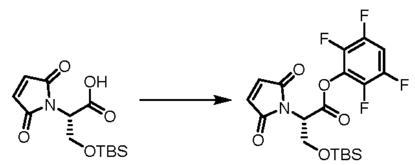

15c 15d

In another preferred embodiment, the inert solvent is selected from dichloromethane, tetrahydrofuran, and 2-methyl tetrahydrofuran; more preferably, the inert solvent is dichloromethane;

In another preferred embodiment, the reaction is performed in the presence of a condensing agent; more preferably, the condensing agent is selected from the group consisting of HATU, HBTU, TBTU, EDCI, HOAt, HOBt, CDI, TCFH, TFFH, DCC, DIC, BOP, AOP, PyAOP, BrOP, PyClOP, PyBrOP, and DMTMM, or combination thereof; more preferably, the condensing agent is DCC.

(2) In an inert solvent, reacting a compound of formula **15d** as described below with glycylglycine to obtain a compound of formula **15e;**

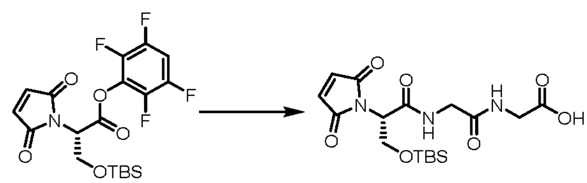

15d 15e

In another preferred embodiment, the inert solvent is selected from 1,2-dimethoxyethane, and water, or combination thereof; more preferably, the inert solvent is a combination of 1,2-dimethoxyethane and water; more preferably, the inert solvent is a combination of 1,2-dimethoxyethane and water in a volume ratio of 1:5 to 5:1; more preferably, the inert solvent is a combination of 1,2-dimethoxyethane and water in a volume ratio of 1:2 to 2:1;

In another preferred embodiment, the reaction is conducted in the absence or presence of a base; more preferably, the base is selected from triethylamine, N,N-diisopropylethylamine, 2,4,6-Collidine, DMAP, DBU, NMM, and $NaHCO_3$; more preferably, the base is $NaHCO_3$;

(3) In an inert solvent, performing a condensation reaction of a compound of formula **15e** as described below with tetrafluorophenol to obtain a compound of formula **15f;**

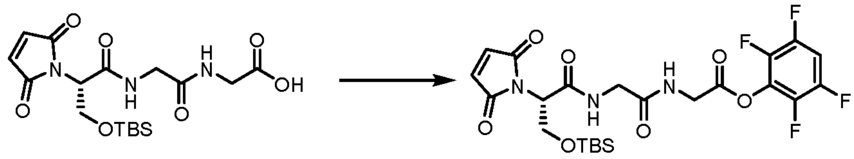

15e 15f

In another preferred embodiment, the inert solvent is selected from dichloromethane, tetrahydrofuran, and 2-methyltetrahydrofuran; more preferably, the inert solvent is dichloromethane;
In another preferred embodiment, the reaction is performed in the presence of a condensing agent; more preferably, the condensing agent is selected from the group consisting of HATU, HBTU, TBTU, EDCI, HOAt, HOBt, CDI, TCFH, TFFH, DCC, DIC, BOP, AOP, PyAOP, BrOP, PyClOP, PyBrOP, and DMTMM, or combination thereof; more preferably, the condensing agent is DCC.

In another preferred embodiment, the reaction is carried out in the presence or absence of a base; more preferably, the reaction is carried out in the absence of a base.
Another aspect of the present invention also provides a method for preparing a compound of formula **15c:**

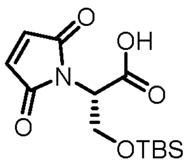

**15c**

characterized in that the method comprises the steps of:

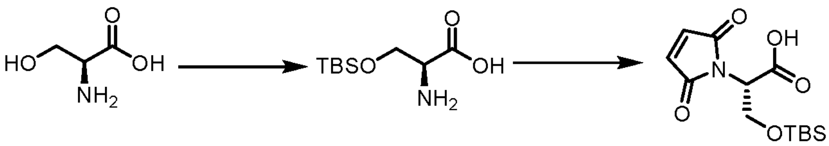

**4a** **4b** **15c**

(1) reacting the compound of formula **4a** with a silylation reagent in an inert solvent to obtain the compound of formula **4b;**

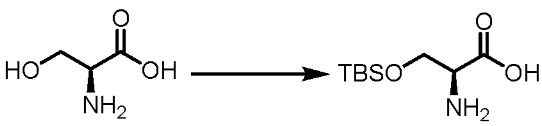

**4a** **4b**

In another preferred embodiment, the inert solvent is selected from the group consisting of acetonitrile, DMF, DMA, THF, and dichloromethane; more preferably, the inert solvent is acetonitrile;
In another preferred embodiment, the reaction is carried out in the presence of a base; more preferably, the base is selected from DBU, imidazole, DIPEA, and TEA; more preferably, the base is DBU;
In another preferred embodiment, the silylation reagent is TBSCl or TBSOTf; more preferably, the silylation reagent is TBSCl;

(2) In an inert solvent, the compound of formula **4b** is reacted with N-methoxycarbonylmaleimide in the presence of a base to give the compound of formula **15c:**

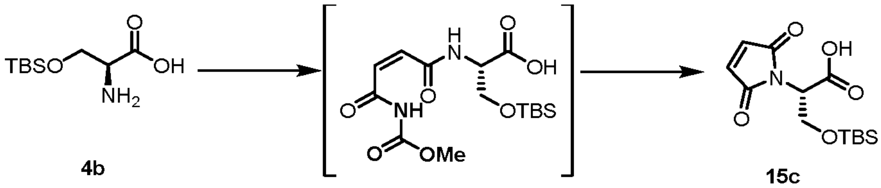

4b 15c

In another preferred embodiment, the inert solvent is selected from the group consisting of water, THF, and DMF, or a combination thereof; more preferably, the inert tetrahydrofuran and water are in a volume ratio of 1:1;
in another preferred embodiment, the base is selected from the group consisting of sodium bicarbonate, sodium carbonate, triethylamine, and diisopropylethylamine; more preferably, the base is sodium bicarbonate.

Another aspect of the present invention provides a method for preparing a compound of the formula **1d** as follows,

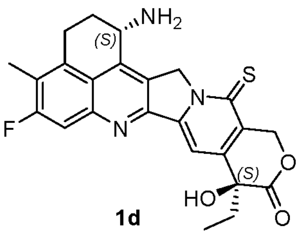

**1d**

characterized in that the method comprises the steps of:

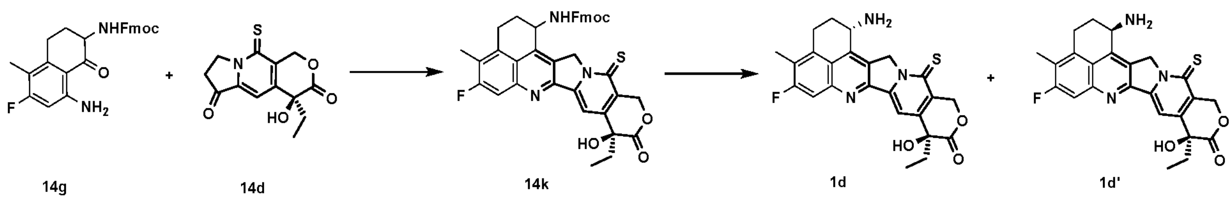

14g 14d 14k 1d 1d'

(1) in an inert solvent, a condensation cyclisation reaction is carried out between the compound of formula **14g** and the compound of formula **14d** in the presence of an additive to give the compound of formula **14k;**

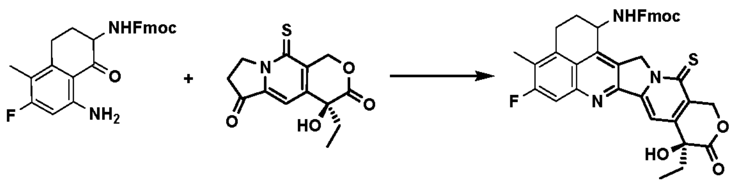

14g 14d 14k

In another preferred embodiment, the inert solvent is preferably selected from toluene, acetic acid, and chlorobenzene, or a combination thereof; more preferably, the inert solvent is a combination of toluene and acetic acid; more preferably, the inert solvent is a combination of toluene and acetic acid in a volume ratio of 1:5 to 5:1; more preferably, the inert solvent is a combination of toluene and acetic acid in a volume ratio of 1:2 to 2:1; more preferably, the inert solvent is a combination of toluene and acetic acid in a volume ratio of 1:1;
In another preferred embodiment, the reaction can be carried out in the presence of an additive; more preferably, the additive can be selected from the group consisting of *p*-toluenesulfonic acid, *p*-toluenesulfonic acid monohydrate, and pyridinium *p*-toluenesulfonate; more preferably, the additive is pyridinium*p*-toluenesulfonate.

(2) In an inert solvent, the Fmoc protecting group of the compound of formula **14k** is deprotected with organic base conditions to give a mixture compound of formula **1d** and formula **1d'**;

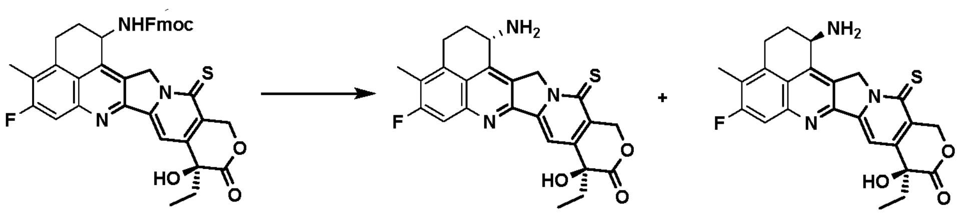

14k 1d 1d'

In another preferred embodiment, the organic base is selected from secondary amines, and tertiary amines; more preferably, the organic base is selected from piperidine, dimethylamine, and diethylamine; more preferably, the organic base is piperidine;

In another preferred embodiment, the inert solvent is selected from tetrahydrofuran, and 2-methyltetrahydrofuran; more preferably, the inert solvent is tetrahydrofuran.

(3) The mixture of obtained **1d** and **1d'** are subjected to chiral resolution to obtain **1d**;

Another aspect of the present invention provides a method for preparing a compound of formula **14g,**

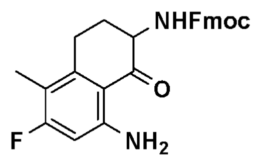

**14g**

characterized in that the method comprises the steps of:

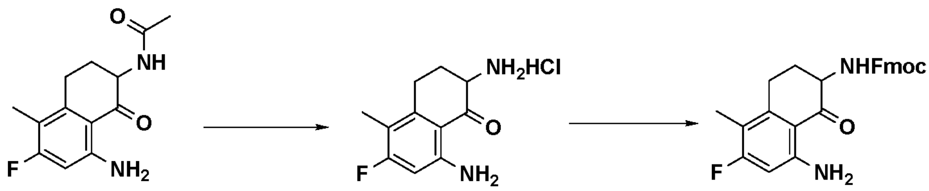

14e 14f 14g

(1) in an inert solvent, deacetylation is carried out with a compound of formula **14e** to obtain a compound of formula **14f.**

In another preferred embodiment, the inert solvent is selected from the group consisting of solvent-free, water, and tetrahydrofuran, or a combination thereof; more preferably, the inert solvent is water; more preferably, the inert solvent is solvent-free;

In another preferred embodiment, the reaction is carried out in the presence of an acid; more preferably, the acid is hydrochloric acid; more preferably, the concentration of the hydrochloric acid is 3-9 N; more preferably, the concentration of the hydrochloric acid is 6 N;

(2) in the presence of a base, reacting the compound of formula **14f** with **FmocCl** to obtain the compound of formula **14g;**

In another preferred embodiment, the base is selected from organic bases, and inorganic bases; more preferably, the base is selected from triethylamine, diisopropylethylamine, DBU, sodium carbonate, potassium carbonate, sodium bicarbonate, and potassium bicarbonate; more preferably, the base is potassium carbonate;

In another preferred embodiment, the inert solvent is selected from acetonitrile, DMF, DMA, dichloromethane, water, and tetrahydrofuran, or a combination thereof; more preferably, the inert solvent is a combination of water and tetrahydrofuran in a volume ratio of 1:5 to 5:1; more preferably, the inert solvent is a combination of water and tetrahydrofuran in a volume ratio of 3:4;

Another aspect of the present invention also provides a method for the preparation of a compound of formula **14b** as follows,

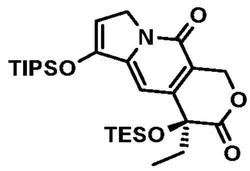

14b

characterized in that the method comprises the step of:

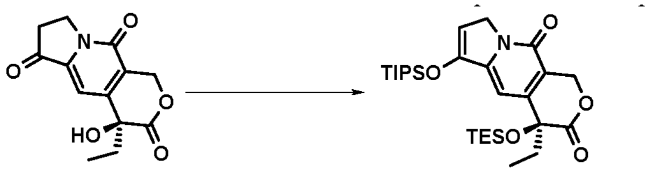

14a 14b

reacting a compound of formula **14a** in an inert solvent in the presence of a base with 2 different silylation reagents for the silyl protection, to obtain the compound of formula **14b;**
In another preferred embodiment, the silylation reagent is TESCl, TESOTf, TIPSCl, or TIPSOTf; more preferably, the silylation reagent is TESCl, or TIPSCl;
In another preferred embodiment, the inert solvent is selected from N,N-dimethylformamide, N,N-dimethylacetamide, HPMA, tetrahydrofuran, and dichloromethane; more preferably, the inert solvent is selected from N,N-dimethylformamide;
In another preferred embodiment, the base is selected from preferably TEA, DIPEA, NMM, pyridine, 2,6-dimethylpyridine, 2,4,6-Collidine, imidazole, and DMAP; more preferably, the base is imidazole.

Another aspect of the present also provides a method for the preparation of a compound of formula **14c** as follows,

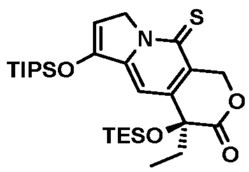

14c

characterized in that the method comprises the step of:

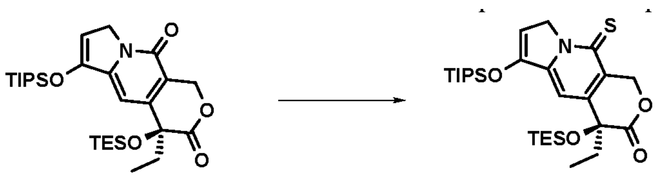

14b 14c

reacting a compound of formula **14b** with Lawesson's reagent in an inert solvent to give a compound of formula **14c;**
In another preferred embodiment, the inert solvent is selected from tetrahydrofuran, dioxane, 2-methyltetrahydrofuran, and toluene; more preferably, the inert solvent is toluene;
In another preferred embodiment, the reaction can be carried out in the presence of a base or in the absence of a base; the base is preferably selected from triethylamine, DIPEA, NMM, pyridine, 2,6-dimethylpyridine, 2,4,6-Collidine, DMAP, sodium carbonate, potassium carbonate, sodium bicarbonate, potassium bicarbonate, potassium tert-butoxide, and sodium tert-butoxide; more preferably, the base is selected from sodium carbonate, potassium carbonate, sodium bicarbonate, and potassium bicarbonate; more preferably, the base is sodium carbonate.

Another aspect of the present invention also provides a method for preparing a compound of formula **14d** as

described below,

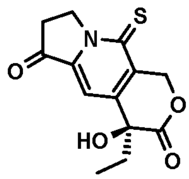

14d

characterized in that the method comprises the step of:

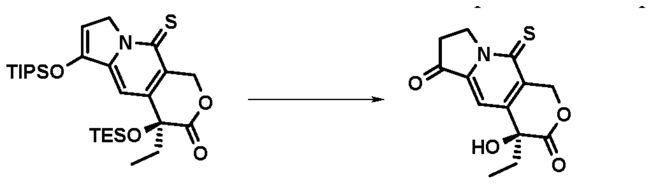

14c 14d

Removing TIPS and TES silyl protecting groups of a compound of formula **14c** with desilylation reagent in an inert solvent to obtain the compound of formula **14d.**

In another preferred embodiment, the desilylation reagent is selected from the group consisting of acid, and fluorinating reagent (fluorine-containing reagent); more preferably, the desilylation reagent is selected from formic acid, acetic acid, trifluoroacetic acid, methanesulfonic acid, trifluoromethanesulfonic acid, dichloroacetic acid, hydrogen fluoride, aqueous hydrogen fluoride solution, triethylamine trihydrofluoride, and pyridine hydrofluoride; more preferably, the desilylation reagent is aqueous hydrogen fluoride solution.

In another preferred embodiment, the inert solvent is selected from tetrahydrofuran, 2-methyl tetrahydrofuran, and toluene; more preferably, the inert solvent is tetrahydrofuran.

Another aspect of the present invention provides a method for preparing a molecule of formula 7 as follows:

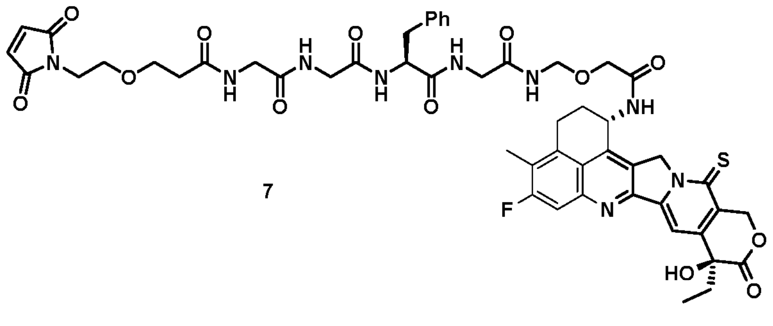

7

characterized in that the method comprises the steps of:

Conducting a condensation reaction between compound of formula **7i** and a compound of formula **1d** in an inert solvent to give the compound of formula 7:

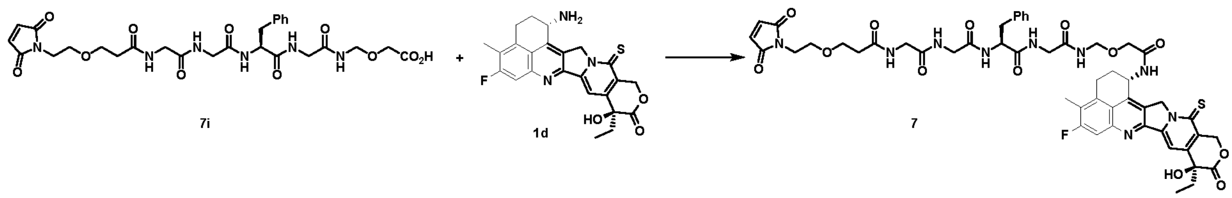

7i 1d 7

In another preferred embodiment, the inert solvent is selected from the group consisting of dichloromethane, tetrahydrofuran, N,N-dimethylformamide, N,N-dimethylacetamide, and HMPA, or a combination thereof; preferably, the inert solvent is N,N-dimethylformamide.

In another preferred embodiment, the condensing agent is selected from the group consisting of HATU, HBTU, TBTU, EDCI, HOAt, HOBt, CDI, TCFH, TFFH, DCC, DIC, BOP, AOP, PyAOP, BrOP, PyClOP, PyBrOP, and DMTMM. or a combination thereof, preferably, the condensing agent is EDCI.

In another preferred embodiment, the base is selected from the group consisting of TEA, DIPEA, NMM, DBU, DMAP, pyridine, 2,6-dimethylpyridine, 2,4,6-Collidine, imidazole, and N-methylimidazole, or a combination thereof, preferably, the base is 2,4,6-Collidine.

Another aspect of the present invention provides a method for preparing a molecule of formula **7i,**

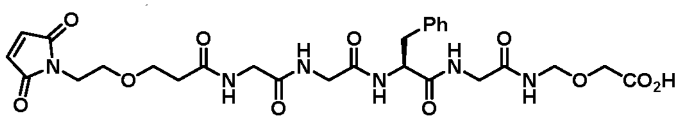

**7i**

characterized in that the method comprises the step of:

Reacting the compound of formula **14j** with the compound of formula **8e** in an inert solvent to obtain the compound of formula 7i;

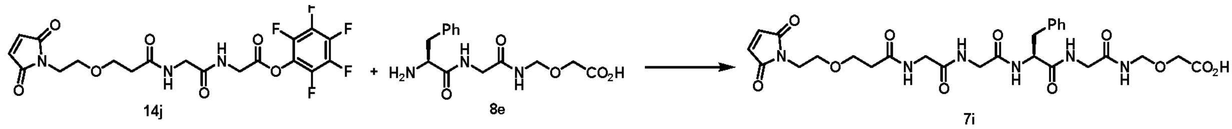

14j 8e 7i

In another preferred embodiment, the inert solvent is selected from the group consisting of acetonitrile, water, tetrahydrofuran, 2-methyl tetrahydrofuran, and dichloromethane, or a combination thereof; and further preferably, the inert solvent is tetrahydrofuran.

In another preferred embodiment, the reaction is carried out in the absence or in the presence of a base; preferably, the base is selected from triethylamine, N,N-diisopropylethylamine, 2,6-dimethylpyridine, 2,4,6-Collidine, DBU, and NMM; more preferably, the reaction is carried out in the absence of a base;

Another aspect of the present invention also provides a preparation method for a molecule of formula **14j** as follows:

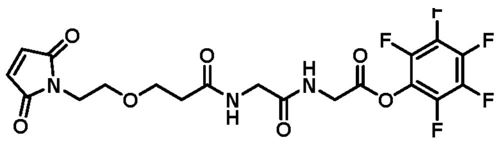

**14j**

characterized by comprises the steps of:

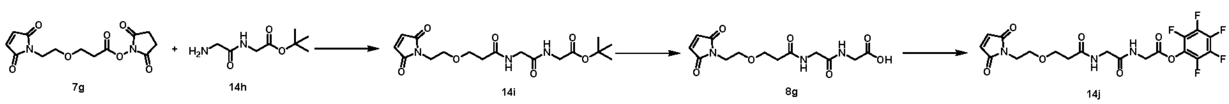

7g 14h 14i 8g 14j

(1) In an inert solvent, subjecting a compound of formula **7g** to a condensation reaction with a compound of formula **14h** to obtain a compound of formula **14i;**

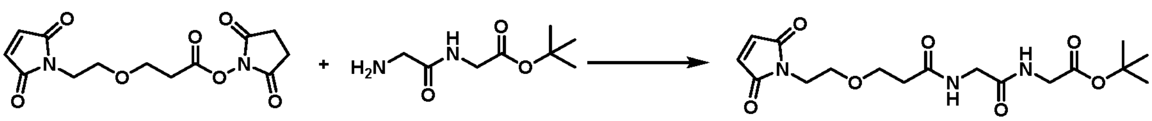

**7g** **14h** **14i**

In a preferred embodiment, the inert solvent is selected from the group consisting of acetonitrile, water, tetrahydrofuran, 2-methyltetrahydrofuran, dichloromethane, and ethyl acetate, or combination thereof; more preferably, the inert solvent is ethyl acetate.

In another preferred embodiment, the reaction is carried out in the absence or presence of a base; preferably, the base is selected from triethylamine, N,N-diisopropylethylamine, 2,6-lutidine, 2,4,6-collidine, DBU, and NMM; more preferably, the reaction is carried out in the absence of a base;

(2) Deprotection of tert-butyl group of the compound of formula **14i** in an inert solvent under acidic conditions to give the compound of formula **8g;**

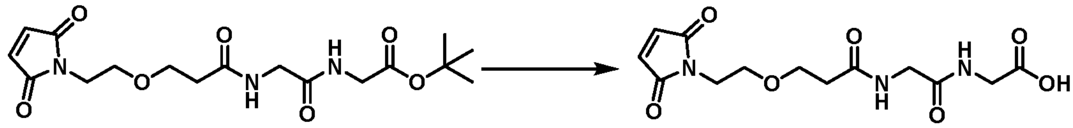

**14i** **8g**

In another preferred embodiment, the inert solvent is selected from the group consisting of dichloromethane, tetrahydrofuran, dioxane, ethyl acetate, and toluene, or combination thereof; more preferably toluene.
In another preferred embodiment, the acid is selected from the group consisting of hydrochloric acid, sulfuric acid, HCl in ethyl acetate solution, HCl in tetrahydrofuran solution, HCl in dioxane solution, trifluoroacetic acid, trifluoromethanesulfonic acid, methanesulfonic acid, and dichloroacetic acid; preferably, the acid is trifluoroacetic acid;

(3) In an inert solvent, the compound of formula **8g** below was used in a condensation reaction with pentafluorophenol in the presence of a condensing agent to give the compound of formula **14j;**

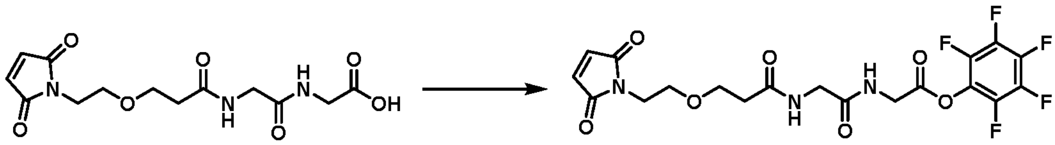

**8g** **14j**

In another preferred embodiment, the condensing agent is selected from the group consisting of HATU, HBTU, TBTU, EDCI, HOAt, HOBt, CDI, TCFH, TFFH, DCC, DIC, BOP, AOP, PyAOP, BrOP, PyClOP, PyBrOP, and DMTMM, or a combination thereof; preferably, the condensing agent is EDCI.
The inert solvent is selected from the group consisting of dichloromethane, tetrahydrofuran, 2-methyltetrahydrofuran, and toluene, or a combination thereof; more preferably, the inert solvent is dichloromethane.
Another aspect of the present invention provides a compound as shown in formula **14b:**

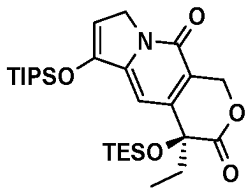

**14b**

Another aspect of the present invention provides a compound represented by the following formula **14c:**

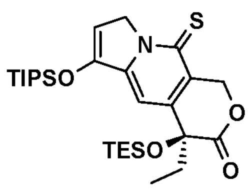

**14c**

Another aspect of the present invention provides a compound represented by the following formula **14g:**

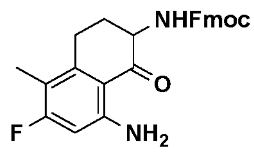

14g

Another aspect of the present invention provides a compound represented by the following formula **14i:**

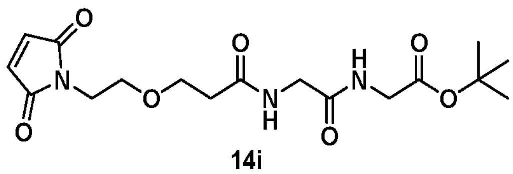

14i

Another aspect of the present invention provides a compound represented by the following formula **14j:**

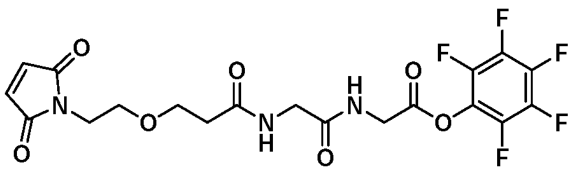

14j

Another aspect of the present invention provides a compound represented by the following formula **14k:**

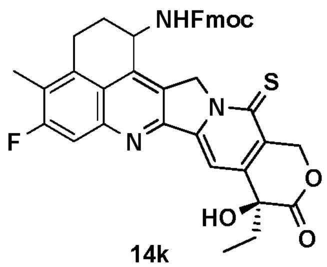

14k

Another aspect of the present invention provides a compound represented by the following formula **15c:**

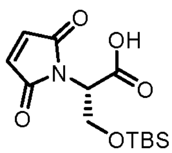

15c

Another aspect of the present invention provides a compound represented by the following formula **15d:**

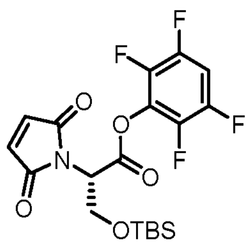

15d

Another aspect of the present invention provides a compound represented by the following formula **15f:**

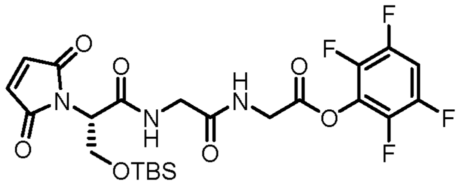

15f

Another aspect of the present invention provides a compound represented by the following formula **15g:**

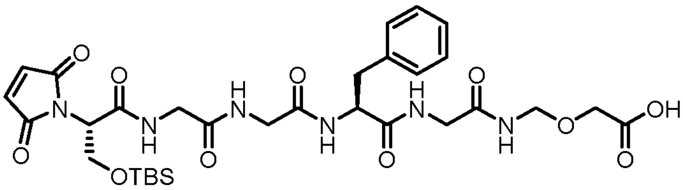

15g

Another aspect of the present invention provides a compound represented by the following formula **7c:**

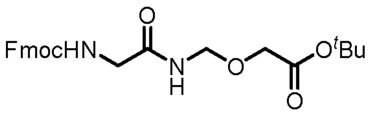

7c

Another aspect of the present invention provides a compound represented by the following formula **7e:**

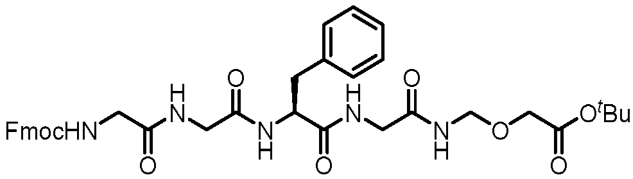

7e

Another aspect of the present invention provides a compound represented by the following formula **7h:**

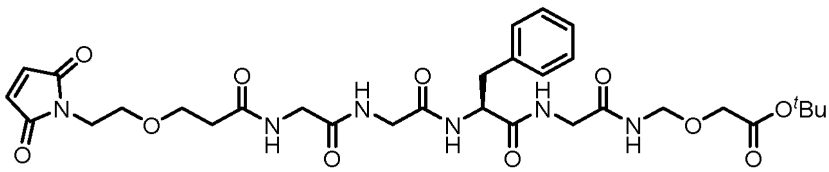

7h

Another aspect of the present invention provides a compound represented by the following formula **8b:**

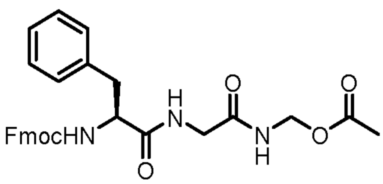

8b

Another aspect of the present invention provides a compound represented by the following formula **8e:**

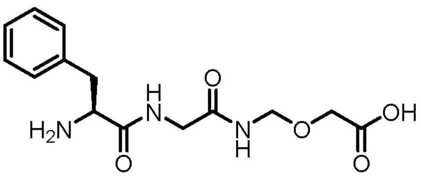

**8e**

Another aspect of the present invention provides a compound represented by the following formula **8g:**

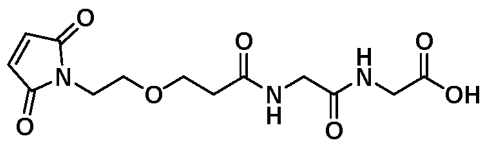

**8g**

Another aspect of the present invention provides a compound represented by the following formula **9b:**

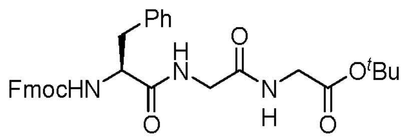

**9b**

Another aspect of the present invention provides a compound represented by the following formula **9c:**

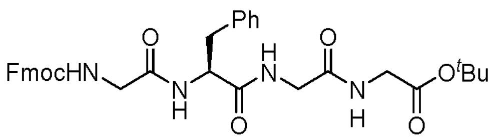

**9c**

Another aspect of the present invention provides a compound represented by the following formula **9e:**

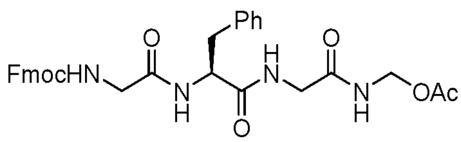

**9e**

Another aspect of the present invention provides a compound represented by the following formula **9f:**

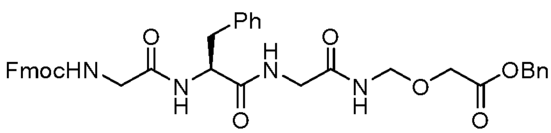

**9f**

Another aspect of the present invention provides a compound represented by the following formula **9g:**

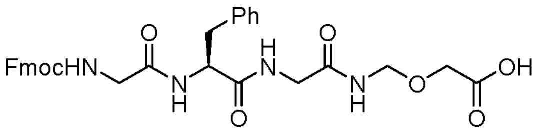

**9g**

Another aspect of the present invention provides a compound represented by the following formula **9h:**

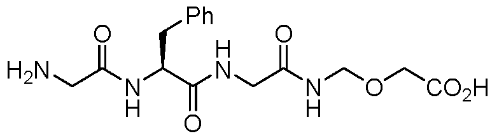

**9h**

Another aspect of the present invention provides a compound represented by the following formula **9i:**

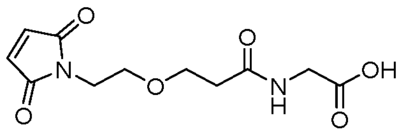

**9i**

It should be understood that within the scope of the present invention, each of the above technical features of the present invention and each of the technical features specifically described below (e.g., in the embodiments) may be combined with each other, thereby constituting a new or preferred technical solution. For the sake of space, we will not repeat them herein.

## Specific embodiments

The present inventor has prepared and obtained a class of structurally novel ligand-drug conjugate, drug molecules (toxins) suitable for ligand-drug conjugate, ligand precursors suitable for ligand-drug conjugate and ligand-drug conjugate precursors suitable for ligand-drug conjugate based on a long and in-depth research, and the drugs have a better stability, a better therapeutic efficacy and a higher safety. Based on the above findings, the inventors have completed the present invention.

## Definitions of Terms

As used herein, the term "alkyl" includes a linear or branched alkyl. For example, $C_1$-$C_8$ alkyl represents a linear or branched chain alkyl with 1-8 carbon atoms, such as methyl, ethyl, propyl, isopropyl, butyl, isobutyl, tert-butyl, etc.

As used herein, the term "alkenyl" includes a linear or branched chain alkenyl. For example, $C_2$-$C_6$ alkenyl refers to a linear or branched chain alkenyl with 2-6 carbon atoms, such as vinyl, allyl, 1-propenyl, isopropenyl, 1-butenyl, 2-butenyl, and the like.

As used herein, the term "alkynyl" includes a linear or branched chain alkynyl. For example, $C_2$-$C_6$ alkynyl refers to a linear or branched chain alkynyl with 2-6 carbon atoms, such as ethynyl, propynyl, butynyl, and the like.

As used herein, the term "$C_3$-$C_{10}$ cycloalkyl" refers to a cycloalkyl with 3-10 carbon atoms. It can be a single ring, such as cyclopropyl, cyclobutyl, cyclopentyl, cyclohexyl, or similar groups. It can also be in the form of a double ring, such as a bridge ring or a spiro ring.

As used herein, the term "$C_1$-$C_8$ alkylamino" refers to anamino substituted by $C_1$-$C_8$ alkyl, which may be mono- or disubstituted; for example, methylamino, ethylamino, propylamino, isopropylamino, butylamino, isobutylamino, *tert*-butylamino, dimethylamino, diethylamino, dipropylamino, diisopropylamino, dibutylamino, diisobutylamino, di-*tert*-butylamino, etc.

As used herein, the term "$C_1$-$C_8$ alkoxy" refers to a linear or branched chain alkoxy with 1-8 carbon atoms; for example, methoxy, ethoxy, propoxy, isopropoxy, butoxy, isobutyloxy, *tert*-butoxy, etc.

As used herein, the term "3-10 membered heterocyclic group with 1-3 heteroatoms selected from the group consisting of N, S, and O" refers to saturated or partially saturated cyclic group with 3-10 ring atoms, of which 1-3 atoms are heteroatoms selected from N, S and O. It can be in the form of a single ring or a double ring, such as a bridge ring or a spiro

ring. Specific examples can be oxetane, azetidine, tetrahydro-2H-pyranyl, piperidinyl, tetrahydrofuranyl, morpholinyl, pyrrolidinyl, etc.

As used herein, the term "$C_6$-$C_{10}$ aryl" refers to an aryl with 6-10 carbon atoms, e.g., phenyl or naphthyl and the like.

As used herein, the term "5-10 membered heteroaryl with 1-3 heteroatoms selected from the group consisting of N, S, and O" refers to a cyclic aromatic group with 5-10 atoms, among which 1-3 atoms are heteroatoms selected from the group consisting of N, S, and O. It can be in the form of a single ring or a fused ring. Specific examples can be pyridinyl, pyridazinyl, pyrimidinyl, pyrazinyl, triazinyl, pyrrolyl, pyrazolyl, imidazolyl, (1,2,3)-triazolyl and (1,2,4)-triazolyl, tetrazolyl, furanyl, thienyl, isoxazolyl, thiazolyl, oxazolyl, etc.

Unless otherwise specified, the chain groups (e.g., alkyl, haloalkyl, or deuterated alkyl) in the present application have 1-10 carbon atoms, preferably with 1-6 carbon atoms, or 1-4 carbon atoms; the non-aromatic cyclic groups (e.g., cycloalkyl, heterocyclic group, alicyclic, aliphatic heterocyclic, etc.) in the present application are 3-12 membered, preferably with 3-8 membered, and 3-6 membered; and the aromatic cyclic groups (e.g., aryl, heteroaryl, etc.) in the present application are 5-15 membered, such as 6-10 membered aryl, 5-7 membered heteroaryl, 5-10 membered heteroaryl, etc.

Unless otherwise specified, the group in the present application is "substituted or unsubstituted", the groups of the present application can be substituted by substituents selected from the group consisting of halogen, cyano, nitro, hydroxyl, amino, $C_1$-$C_6$ alkylamino, $C_1$-$C_6$ alkyl, $C_2$-$C_6$ alkenyl, $C_2$-$C_6$ alkynyl, $C_1$-$C_6$ alkoxy, $C_1$-$C_6$ haloalkyl, $C_2$-$C_6$ haloalkenyl, $C_2$-$C_6$ haloalkynyl, $C_1$-$C_6$ haloalkoxy, allyl, benzyl, $C_6$-$C_{12}$ aryl, $C_1$-$C_6$ alkoxy-$C_1$-$C_6$ alkyl, $C_1$-$C_6$ alkoxycarbonyl, phenoxycarbonyl, $C_2$-$C_6$ alkynylcarbonyl, $C_2$-$C_6$ alkenylcarbonyl, $C_3$-$C_6$ cycloalkylcarbonyl, $C_1$-$C_6$ alkylsulfonyl, etc.

As used herein, "halogen" or "halogen atom" refers to F, Cl, Br, and I. More preferably, the halogen or halogen atoms selected from F, Cl, and Br. "Halogenated" refers to being substituted by atoms selected from F, Cl, Br, and I.

Unless otherwise specified, the structural formula described in the application is intended to include all isomeric forms (such as enantiomers, diastereomers and geometric isomers (or conformational isomers)), for example, R and S configurations of asymmetric centers, (Z) and (E) isomers of double bonds, etc. Therefore, a single stereochemicalisomer of the compound of the application or a mixture of its enantiomers, diastereomers or geometric isomers (or conformational isomers) all fall within the scope of the application.

As used herein, the term "tautomer" means that structural isomers with different energies can cross the low energy barrier and transform into each other. For example, proton tautomer (i.e. proton shift) involves interconversion through proton migration, such as 1H-indazole and 2H-indazole. The valence tautomerism involves interconversion through some bonding electrons recombination.

As used herein, the term "hydrate" refers to a complex formed by the coordination of a compound of the present application with water.

The compound of this application can be prepared through various synthetic methods well-known to those skilled in the art, including the specific embodiments listed below, the embodiments formed by combining the specific embodiments with other chemical synthesis methods, and equivalent substitution methods well-known for those skilled in the art. Preferred embodiments include but are not limited to the embodiments of present application.

The solvent used in this application are commercially available, compounds are named manually or by ChemDraw® software, commercially available compounds are named by supplier catalog.

In the present application, the term "ligand" generally refers to a macromolecular compound that recognizes and binds to an antigen or receptor associated with a target cell. A ligand may function to present a drug to a target cell population bound to a ligand, which includes, but is not limited to, a protein hormone, a lectin, a growth factor, an antibody, or other molecule that binds to a cell, a receptor, and/or an antigen. In the present application, the ligand may be denoted as Ab, and the ligand antigen, which forms a linkage bond to a linkage unit via a heteroatom on the ligand, may be an antibody or an antigen-binding fragment thereof, and the antibody may be selected from a chimeric antibody, a humanized antibody, a fully human antibody, or a murine-derived antibody; the antibody may be a monoclonal antibody. The antibody may be, for example, an antibody or an antigen-binding fragment thereof targeting a target selected from the group consisting of: HER2, TROP2, PSMA, FR-$\alpha$ or B7H3 and IGF-1R.

In the present application, the terms "Trop2", "TROP2" generally refer to Type I single-pass transmembrane cell membrane proteins. In this application, the term "Trop2" may also encompass homologs, variants and isoforms of Trop 2, including splice isoforms. The term "Trop" also encompasses proteins having a sequence of one or more of the Trop 2 homologs, variants, and isoforms, as well as fragments of the sequence, as long as it is the variant protein (including the isoform). Trop2 may be human Trop2. For example, Uniprot Accession No. P09758 provides a description of Trop2 and the sequence.

In the present application, the term "HER2" generally refers to human epidermal growth factor receptor 2 (HER2). For example, the term "HER2" refers to any natural HER2 from any human source, and the term also encompasses "full-length" and unprocessed HER2, as well as any form of HER2 derived from processing in cells (e.g., maturation proteins).

The term also covers naturally occurring variants and equivalents of HER2, such as splice variants or allelic variants. For example, Uniprot Accession No. P04626 provides a description of HER2 and sequence.

In the present application, the term "PSMA" refers to Glutamate Carboxypeptidase II. In this application, the term "PSMA" may also encompass homologs, variants and isoforms of PSMA. The term "PSMA" also encompasses proteins having one or more sequences of PSMA homologs, variants, and isoforms from various sources (e.g., human), as well as fragments of such sequences. For example, Uniprot Accession No. Q04609 provides a description of PSMA and the sequence.

In the present application, the term "FR-α" refers to folate receptor α, encoded by FOLR1. In the present application, the term "FR-α" may also cover homologs, variants and isoforms of FR-α. The term "FR-α" also includes proteins having one or more sequences of FR-α homologs, variants, and isoforms from various sources (e.g., human), as well as fragments of such sequences. For example, Uniprot Accession No. P15328 provides a description of FR-α and the sequence.

In the present application, the term "B7H3" refers to CD276, and for the purposes of this application, the term "B7H3" may encompass any of its homologs, variants, and isoforms, as well as any of its possible forms of expression (e.g., 2Ig and 4Ig) in humans. The term "B7H3" also encompasses proteins having sequences of one or more of the B7H3 homologs, variants and isoforms from various sources (e.g., human), as well as fragments of such sequences. For example, Uniprot Accession No. Q5ZPR3 provides a description of B7H3 (CD276) and the sequence.

In the present application, the term "IGF-1R" refers to insulin-like growth factor I receptor. In this application, the term "IGF-1R" may also encompass its homologs, variants and isoforms. The term "IGF-1R" also encompasses proteins having one or more sequences of IGF-1R homologs, variants, and isoforms from various sources (e.g., human), as well as fragments of such sequences. For example, Uniprot Accession No. P08069 provides a description of IGF-1R and the sequence.

In the present application, the term "peptide residue" generally refers to a residue comprises one or more amino acid residues linked together. For example, one or more amino acids in the peptide residue may be optionally substituted. For example, the peptide residues of the present application may comprise Glycine-Glycine-Phenylalanine-Glycine (Gly-Gly-Phe-Gly).

In the present application, the term "drug moiety" generally refers to a chemical moiety that is directly or indirectly conjugated to an antibody or antigen-binding fragment to form an immunoconjugate. For example, the "drug moiety" includes, but is not limited to, compounds with anti-tumor activity as described herein. Illustrative drug moieties include topoisomerase inhibitors.

In the present application, the term "anti-tumor compound" generally refers to a compound having the ability to cause a reduction in the rate of proliferation, viability, or metastatic activity of tumor cells. For example, antitumor activity may be indicated by a reduction in the rate of growth of abnormal cells or a stabilization or reduction in tumor size that occurs during treatment, or by a longer survival period due to treatment as compared to a control in the absence of treatment. Anti-tumor activity may be assessed using recognized in vitro or in vivo tumor models, such as xenograft models.

In the present application, the term "includes" generally refers to the inclusion of expressly designated features, but not to the exclusion of other elements. The terms "above" and "below" generally refer to the inclusion of this number.

In the present application, the term "about" generally refers to a variation of 0.5% to 10% above or below the specified value, e.g., within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the specified value.

In the present application, the compounds of this application include the tautomers, racemates, optima, enantiomers, and/or diastereomers of the compounds.

In the present application, some atoms of the compounds of this application may be in the form of more than one isotope. For example, hydrogen may exist in the form of protium ($^1H$), deuterium ($^2H$), and tritium ($^3H$), and carbon may exist naturally in the form of three different isotopes ($^{12}C$, $^{13}C$, and $^{14}C$). Examples of isotopes that may be incorporated into the compounds of the present application include, but are not limited to, $^{15}N$, $^{18}O$, $^{17}O$, $^{18}F$, $^{32}P$, $^{33}P$, $^{129}I$, $^{131}I$, $^{123}I$, $^{124}I$, $^{125}I$, or similar isotopes. Accordingly, the compounds of the present application may be in the form of enrichment in one or more of these isotopes relative to the natural abundance of these isotopes. As is known to those skilled in the art, such isotopically enriched compounds may be used for a variety of purposes. For example, replacement with a heavy isotope such as deuterium ($^2H$) may offer certain therapeutic advantages, which may be due to greater metabolic stability.

In the present application, the term "pharmaceutical composition" generally refers to a mixture containing one or more compounds described in the present application or their physiological/pharmacological salts or prodrugs and other chemical components, as well as other components such as physiological/pharmacological carriers and excipients. The pharmaceutical composition may facilitate administration to an organism, facilitate absorption of the active ingredient and thus exert biological activity. Conventional preparation of pharmaceutical compositions can be found in commonly used techniques in the art.

In the present application, the term "pharmaceutically acceptable salt" or "medicinally acceptable salt" generally

refers to a salt of the compound or ligand-drug conjugate of the present application, or a salt of the compound described herein, which can be safe and/or effective when used in mammals and can have the desired biological activity. The antibody-drug conjugate compound of the present application can form a salt with an acid. Nonlimiting examples of pharmaceutically acceptable salts include: hydrochloride, hydrobromide, hydroiodide, sulfate, hydrogen sulfate, citrate, acetate, succinate, ascorbate, oxalate, nitrate, perrate, hydrogen phosphate, dihydrogen phosphate, salicylate, hydrogen citrate, tartrate, maleate, fumarate, formate, benzoate, mesylate, ethylsulfonate, benzenesulfonate, or *p*-toluenesulfonate.

In the present application, the term "conjugate" generally refers to a compound prepared through one or more chemical reactions involving the compounds of the present application, or compounds interconnected via one or more bridging structures such as bridges, spacers, or linking moieties.

In the present application, the term "pharmaceutically acceptable carrier" generally refers to a carrier that is administered with a therapeutic agent, such as an antibody or a polypeptide, a gene and other therapeutic agents. The term refers to any pharmaceutical carrier that does not itself induce the production of antibodies harmful to the individual receiving the composition and can be administered without excessive toxicity. For example, a pharmaceutically acceptable carrier can be distinguished from a nucleic acid vector used in genetic engineering to contain a target gene. Suitable carriers can be large, slowly metabolized macromolecules such as proteins, polysaccharides, polylactic acid, polyglycolic acid, polyamino acids, amino acid copolymers, lipid aggregates and inactivated viral particles. A person skilled in the art is familiar with these carriers. Pharmaceutically acceptable carriers in therapeutic compositions may include liquids such as water, saline, glycerin and ethanol. Auxiliary substances such as wetting or emulsifying agents, pH buffers, etc. may also be present in these carriers.

In the present application, the term "antibody" generally covers monoclonal antibodies, polyclonal antibodies, dimers, oligomers, multi-specific antibodies (e.g., bispecific antibodies) and antibody fragments, provided they exhibit the desired biological activity. Antibodies may be murine, human, humanized, chimeric antibodies or derived from other species. Antibodies are proteins produced by the immune system that recognize and bind specific antigens. Target antigens typically have a large number of binding sites, also called epitopes, that are recognized by the CDRs of a wide range of antibodies. Each antibody that specifically binds a different epitope has a different structure. As a result, an antigen can have more than one corresponding antibody. Antibodies include full-length immunoglobulin molecules or immunologically active portions of full-length immunoglobulin molecules, i.e., molecules containing antigen-binding specificities that bind to the target of interest or portions thereof, such targets including, but not limited to, cancer cells or cells producing autoimmune antibodies associated with autoimmune diseases. The immunoglobulins described in this application can have any type (e.g., IgG, IgE, IgM, IgD, and IgA), class (e.g., IgG1, IgG2, IgG3, IgG4, IgA1, and IgA2, or any mutations thereof), or subclass of immunoglobulin molecule. The immunoglobulins can be derived from any species. However, in one aspect, the immunoglobulins are derived from a human, murine, or rabbit. "Antibody fragments" may comprise a portion of a full-length antibody, generally its antigen-binding region or variable region. Examples of antibody fragments include: Fab, Fab', F(ab')2 and Fv fragments; bisantibodies; linear antibodies; minibodies; fragments prepared from Fab expression libraries; anti-idiotypic (anti-Id) antibodies; CDR (complementarity-determining regions); and any of the foregoing that bind cancer cell antigens, a viral antigen or a microbial antigen, and any of the above epitope-binding fragments that bind cancer cell antigens, viral antigens or microbial antigens in an immunospecific manner; single-chain antibody molecules; and multi-specific antibodies formed from antibody fragments. The antibodies comprise the antibody-drug conjugates of the present application can maintain their antigen-binding ability in their native wild-type state. Therefore, the antibodies of the present application can, for example, bind to antigens specifically. The antigens involved include, for example, tumor-associated antigens (TAAs), cell surface receptor proteins and other cell surface molecules, cell survival regulators, cell proliferation regulators, molecules associated with tissue growth and differentiation (e.g., known or predicted to be functional), lymphokines, cytokines, molecules involved in cell cycle regulation, molecules involved in angiogenesis, and molecules associated with angiogenesis (e.g., known antibody-binding antigens can be one or a subset of the above classifications, while other subsets include other molecules/antigens with specific properties (compared to the target antigen). Antibodies used in antibody drug conjugates include, but are not limited to, antibodies against cell surface receptors and tumor-associated antigens. Such tumor-associated antigens are well-known in the industry and can be prepared using antibody preparation methods and information well-known in the industry. These targets can be specifically expressed on the surface of one or more cancer cells, while being expressed at low levels or not expressed on the surface of one or more non-cancer cells. Typically, such tumor-associated polypeptides can be more highly overexpressed on the surface of cancer cells than on the surface of non-cancer cells.

In the present application, the term "chimeric antibody" generally refers to an antibody formed by fusing the variable region of a murine-derived antibody with the constant region of a human antibody, which can reduce the immune response induced by the murine-derived antibody. To create a chimeric antibody, a hybridoma secreting a murine-specific monoclonal antibody can be established, and then the variable region gene can be cloned from the murine hybridoma cells. The constant region gene of the human antibody can be cloned as needed, and the murine variable region gene and the human constant region gene can be linked to form a chimeric gene and inserted into an expression vector. The chimeric

antibody molecule can then be expressed in an eukaryotic system or prokaryotic system.

In the present application, the term "humanized antibody", also known as a CDR-grafted antibody, generally refers to an antibody produced by grafting a murine CDR sequence into the framework of a human antibody variable region, i.e., a framework sequence of a human antibody of a different type. This can overcome the heterologous reaction induced by chimeric antibodies carrying a large amount of murine protein components. Such framework sequences can be obtained from public DNA databases or published references that include the gene sequences of germline antibodies. For example, the germline DNA sequences of human heavy and light chain variable regions can be found in the "VBase" human germline sequence database.

In the present application, the terms "fully human antibody", "fully human antibodies" or "fully humanized antibody", also known as "fully human monoclonal antibody", refer to an antibody in which both the variable and constant regions can be human-derived, eliminating immunogenicity and toxic side effects. Monoclonal antibodies have gone through four stages of development: murine-derived monoclonal antibodies, chimeric monoclonal antibodies, humanized monoclonal antibodies, and fully human monoclonal antibodies. The antibody or ligand described in this application may be a fully human monoclonal antibody. The relevant technologies for the preparation of fully human antibodies may include human hybridoma technology, EBV-transformed B lymphocyte technology, phage display, transgenic murine antibody preparation technology, and single B cell antibody preparation technology.

In the present application, the term "CDR" generally refers to one of the six hypervariable regions within the variable domain of an antibody that are primarily responsible for antigen binding. One of the most commonly used definitions for the six CDRs is provided by Kabat E.A. et al., Chothia et al., or MacCallum et al. As used herein, the Kabat definition of CDR applies to the CDR1, CDR2, and CDR3 of the light chain variable domain (LCDR1, LCDR2, LCDR3 or L1, L2, L3) and the CDR1, CDR2, and CDR3 of the heavy chain variable domain (HCDR1, HCDR2, HCDR3 or H1, H2, H3).

In this application, the term "a group capable of conjugate with a thiol group" generally refers to the fact that the compound A has a thiol group, the compound B has a group capable of coupling with a thiol group, and the compound B reacts with the thiol group of the compound A through the group capable of coupling with a thiol group, so as to achieve the connection between the compound A and the compound B.

In the present application, the term 'linker' generally refers to a chemical structural fragment or bond that is connected to one group at one end and to another group at the other end, and can also be connected to other linkers and then to drugs and/or ligands. The ligands directly or indirectly connected can refer to the groups directly connecting the ligands by covalent bonds, or connecting the ligands through linkers. For example, the linker can be the structure shown in the linker described in the present application. For example, linkers may be chemical structural fragments or bonds comprises acid labile linker structures (e.g., hydrazones), protease sensitive (e.g., peptidases sensitive) linker structures, photo labile linker structures, dimethyl linker structures, or disulfide containing linker structures.

In the present application, the term "linking group" generally refers to a chemical moiety capable of forming covalent attachment to another group. For instance, a compound comprises a linking group may undergo a coupling reaction through the linking group, thereby enabling covalent conjugation of the compound to a second group. For example, a maleimide group can be used as a linking group.

In the present application, the term 'expression-associated' to a disease of a target usually refers to the fact that the occurrence and/or progression of the disease is associated with the expression level of the target. For example, the expression level of a target in cells from a diseased area such as a patient's specific tissue or organ is increased, i.e. highly expressed, relative to the expression level in normal cells from the tissue or organ. Or for example, the expression level of a target in cells from a diseased area such as a patient's specific tissue or organ is decreased, i.e. lowly expressed, relative to the expression level in normal cells from the tissue or organ. Or, for example, cells from a diseased area such as a patient's specific tissue or organ express a target, i.e. positive. Or, for example, the absence of expression of a target in cells from a diseased area such as a patient's specific tissue or organ, i.e. negative. For example, the expression of a target can be characterised by a standard assay known in the art.

In the present application, the term 'effective amount' generally refers to the amount of a therapeutic agent that treats, alleviates, or prevents a target disease or condition, or that exhibits a detectable therapeutic or preventive effect. The precise effective amount for a particular subject depends on the subject's body size and health, the nature and severity of the condition, and the treatment agent and/or combination of treatment agents chosen to be administered. Therefore, it is not useful to specify in advance the precise effective amount. However, for a given condition, the effective amount can be determined using routine experiments and is capable of being judged by a clinician.

Unless otherwise specified, all compounds appearing in this application are intended to include all possible optical isomers, such as a single chiral compound or a mixture of different chiral compounds (i.e., racemates). Among all the compounds in this application, each chiral carbon atom can be optionally of the R configuration or the S configuration, or a mixture of the R configuration and the S configuration.

In the present application, the term 'compound of the application' generally refers to the compound of the application. The term also includes various crystalline forms, pharmaceutically acceptable salts, hydrates or solvates of the compound of the application.

When a trade name is used herein, they are intended to include the trade name product formulation, its corresponding generic equivalent, and the active pharmaceutical ingredient of the trade name product.

**Pharmaceutical composition and method of administration**

Due to the excellent inhibitory activity of the compounds of the present application on tumor cell proliferation, the compounds of the present application and their various crystal forms, pharmaceutically acceptable inorganic or organic salts, hydrates or solvates, as well as pharmaceutical composition containing the compounds of the present application as the main active ingredient, can be used for the prevention and/or treatment (stabilization, alleviation, or cure) of diseases associated with tumor cell proliferation.

The pharmaceutical composition of the present application includes compounds of the present application within a safe and effective range, as well as pharmaceutically acceptable excipients or carriers. The "safe and effective amount" refers to the amount of compound that is sufficient to significantly improve the condition without causing serious side effects. Typically, the pharmaceutical composition contains 1-2000 mg compound of the present application/formulation, and more preferably, 1-200 mg compound of the present application/formulation. Preferably, the "one dose" is a capsule or tablet, or a unit dose of an injectable preparation.

"Pharmaceutically acceptable carrier" refers to one or more compatible solid or liquid fillers or gel substances, which are suitable for human use, and must be sufficient pure and low toxicity. "Compatible" herein refers to the ability of components of a composition to blend with each other and with the compounds of the application without significantly reducing the efficacy of the compounds. Examples of pharmaceutically acceptable carriers include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween®), wetting agent (such as lauryl sodium sulfate), colouring agents, flavoringagents, stabilizers, antioxidants, preservatives, pyrogen-free water, etc.

The methods of administration of the compounds or pharmaceutical compositions of the present applicationare are not particularly limited, and representative methods of administration include (but are not limited to) oral, parenteral (intravenous, intramuscular or subcutaneous).

Solid dosage forms for oral administration include capsules, tablets, pills, powders and granules. In these solid dosage forms, the active compounds are mixed with at least one conventional inert excipient (or carrier), such as sodium citrate or di-calcium phosphate, or mixed with any of the following components: (a) fillers or compatibilizer, such as starch, lactose, sucrose, glucose, mannitol and silicic acid; (b) binders, such as hydroxyl methyl cellulose, alginate, gelatin, polyvinylpyrrolidone, sucrose and arabic gum; (c) humectants, such as, glycerol; (d) disintegrating agents, such as agar, calcium carbonate, potato starch or tapioca starch, alginic acid, certain composite silicates, and sodium carbonate; (e) solution-retarding agents, such as paraffin; (f) absorption accelerators, such as quaternary ammonium compounds; (g) wetting agents, such as cetyl alcohol and glyceryl monostearate; (h) adsorbents, such as kaolin; and (i) lubricants, such as talc, stearin calcium, magnesium stearate, solid polyethylene glycol, lauryl sodium sulfate, or mixtures thereof. In capsules, tablets and pills, the dosage forms may also contain buffering agents.

The solid dosage forms such as tablets, dragees, capsules, pills and granules can be prepared by using coating and shell materials, such as enteric coatings and any other materials known in the art. They may include opacifying agents, and the active compound or compound in such a composition may be released in a certain part of the digestive tract in a delayed manner. Examples of the embedding components include polymers and waxes. If necessary, the active compound may also form a microcapsule form with one or more of the excipients described above.

Liquid dosage forms for oral administration include pharmaceutically acceptable emulsions, solutions, suspensions, syrups or tinctures. In addition to the active compounds, the liquid dosage form may contain inert diluents conventionally used in the art, such as water or other solvents, solubilizers and emulsifiers, for example, ethanol, isopropanol, ethyl carbonate, ethyl acetate, propylene glycol, 1,3-butanediol, dimethylformamide, as well as oil, in particular, cottonseed oil, peanut oil, corn germ oil, olive oil, castor oil and sesame oil, or mixtures thereof.

In addition to these inert diluents, the composition can also include adjuvants, such as wetting agents, emulsifying and suspending agents, sweetening agents, flavoring agents and perfuming agents.

In addition to the active compound, the suspension may comprise suspending agents, such as ethoxylated isooctadecanol, polyoxyethylene sorbitol and dehydrated sorbitol esters, microcrystalline cellulose, methanolic aluminum, agar, and mixtures thereof.

The composition for parenteral injection may comprise physiologically acceptable sterile aqueous or anhydrous solutions, dispersions, suspensions or emulsions, and sterile powders for redissolution into sterile injectable solutions or dispersions. Suitable aqueous and non-aqueous carriers, diluents, solvents, or excipients include water, ethanol, polyols, and suitable mixtures thereof.

The compounds of the present application can be administered alone or in combination with other pharmaceutically acceptable therapeutic agents.

When administered in combination, the pharmaceutical composition further includes one or more (2, 3, 4, or more) other pharmaceutically acceptable therapeutic agents. One or more (2, 3, 4, or more) of the other pharmaceutically acceptable therapeutic agents can be used simultaneously, separately or sequentially with the compounds of the present application for the prevention and/or treatment of diseases associated with tumour cell proliferation.

When the pharmaceutical composition is used, a safe and effective amount of the compound of the present application is administrated to a mammal (such as a human) in need of treatment, wherein the dose is considered as a pharmaceutically effective dose. For a person weighing 60 kg, the daily dose is usually 1 to 2000 mg, preferably 1 to 500 mg. Of course, the particular dose should also depend on various factors, such as the route of administration, patient healthy status, which are well within the skills of an experienced physician.

**A preparation process for a toxin intermediate**

In the present invention, there is also provided a novel process for the preparation of the synthetic intermediate **14d** (compound **1k** in WO 2022262789 A1) of the toxin molecule **1d**, as described above. The synthetic route disclosed in the prior art (WO 2022262789 A1) is as follows:

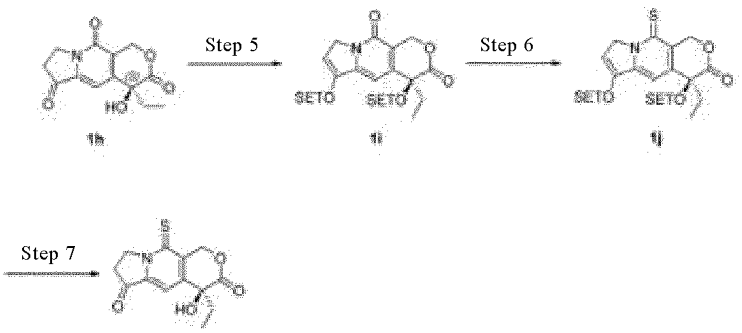

1h 1i 1j 1k

Compared with the above prior art, the synthetic route of the present invention replaces one of the two TES silyl protecting group in the prior art with a TIPS silyl protecting group, and the main advantages are reflected in the following:

1. The intermediates **14b** and **14c** in the synthetic route of the present application exhibit better stability compared to the intermediates **1i** and **1j** of the above prior art, and are easy for scale-up the production and long-term preservation;
2. Both of the intermediates **14b** and **14c** in the synthetic route of the present application can be isolated and purified, and are easy for quality control. While the intermediates **1i** and **1j** of the above prior art are used as crude products for the next reaction.
3. the yield of the synthetic route of the present application has been increased from 49% to 73% compared with the yield in the above prior art (WO 2022262789 A1).

**A preparation process for a toxin molecule**

The present invention also provides a new synthetic route for the toxin molecule **1d** (compound **1n-P1** in WO 2022262789 A1), and the synthetic route in the prior art (WO 2022262789 A1) is as follows:

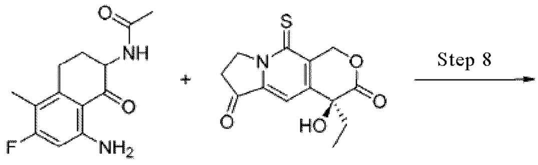


**1l** **1k**

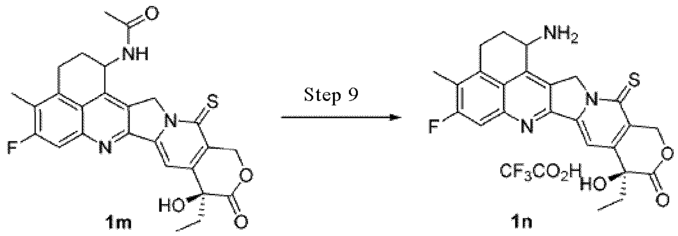


**1m** **1n**

Compared with the prior art, the synthetic route of the present invention replaces the acetyl (Ac) protecting group in the prior art with the Fmoc protecting group. The main advantages are as follows:

1, In the synthetic route of the present application, replacement of the acetyl protecting group in **11** in the synthetic route of the prior art with an Fmoc protecting group, thereby obtaining compound **14g,** increases the synthetic yield of compound **1d** (compound **1n-P1** in WO 2022262789 A1) from 7.9% to 15.6%.
2, The deacetylation step of compound **1m** in the synthetic route of the prior art (WO 2022262789 A1) requires harsh reaction conditions, exhibits poor reproducibility and incomplete removal of the protecting group during scale-up. The method of the present application for removal the Fmoc protecting group of **14k** requires milder reaction conditions, which is more suitable for scale-up production.

The present application will be further illustrated by the following specific examples. It should be understood that these examples only aim to illustrate the application but not to limit the scope of the application. The experimental methods in the following examples without specific conditions are usually following conventional conditions or conditions recommended by the manufacturer. Unless otherwise specified, percentages and portions are calculated by weight.

**Definition of abbreviations**

| | **Abbreviati on** |
|---|---|
| Dichloromethane | DCM |
| Ethyl acetate | EA or EtOAc |
| Methanol | MeOH |
| Tetrahydrofuran | THF |
| N,N-Dimethylformamide | DMF |
| N,N-Dimethylacetamide | DMA or DMAc |
| Dimethyl sulfoxide | DMSO |
| Hexamethylphosphoramide | HMPA |
| 2-(7-Azabenzotriazol-1-yl)-N,N,N',N'-tetramethyluronium hexafluorophosphate | HATU |
| [benzotriazol-1-yloxy(dimethylamino)methylidene]-dimethylazanium; hexafluorophosphate | HBTU |
| benzotriazol-1-yl-tetramethyluronium tetrafluoroborate | TBTU |
| 1-(3-Dimethylaminopropyl)-3-ethylcarbodiimide hydrochloride | EDCI |
| N-Hydroxy-7-azobenzotriazole | HOAt |
| 1-Hydroxybenzotriazole | HOBt |

(continued)

| | Abbreviati on |
|---|---|
| 1,1'-Carbonyldiimidazole | CDI |
| N,N,N',N'-Tetramethylchloroformamidinium hexafluorophosphate | TCFH |
| Fluoro-N,N,N',N'-tetramethylformamidinium hexafluorophosphate | TFFH |
| Dicyclohexylcarbodiimide | DCC |
| N,N'-Diisopropylcarbodiimide | DIC |
| 1H-Benzotriazol-1-yloxytris(dimethylamino)phosphonium Hexafluorophosphate | BOP |
| 7-Azabenzotriazol-1-yloxytris(dimethylamino)phosphonium hexafluorophosphate | AOP |
| (3-Hydroxy-3H-1,2,3-triazolo[4,5-b]pyridinato-O)tri-1-pyrrolidinylphosphonium hexafluorophosphate | PyAOP |
| Bromotris(dimethylamino)phosphonium hexafluorophosphate | BrOP |
| Chlorotripyrrolidinophosphonium hexafluorophosphate | PyClOP |
| Bromo-tris-pyrrolidino-phosphonium hexafluorophosphate | PyBrOP |
| 4-(4,6-Dimethoxy-1,3,5-triazin-2-yl)-4-methyl morpholinium chloride | DMTMM |
| Triethylamine | TEA |
| N,N-Diisopropylethylamine | DIPEA |
| 4-Methylmorpholine | NMM |
| 1,8-Diazabicyclo[5.4.0]undecane-7-ene | DBU |
| 4-Dimethylaminopyridine | DMAP |
| Pyridine | Py |
| 2,6-Lutidine | N/A |
| 2,4,6-Collidine | N/A |
| Imidazole | N/A |
| 1-Methylimidazole | NMI |
| 9-Fluorenylmethyl chloroformate | FmocCl |
| Thin-layer chromatography | TLC |
| (S)-(-)-2,2'-Bis(diphenylphosphino)-1,1'-binaphthyl | BINAP |
| methyl | Me |
| ethyl | Et |
| isopropyl | $^{i}$Pr |
| t-butyl | $^{t}$Bu |
| - | Allyl |
| benzyl | Bn |
| *t*-butoxycarbonyl | Boc |
| 9-fluorenylmethoxycarbonyl | Fmoc |
| benzyloxycarbonyl | Cbz |
| acetyl | Ac |
| triethylsilyl | TES |
| triisopropylsilyl | TIPS |
| tert-butyldimethylsilyl | TBS |
| Chlorotriethylsilane | TESCl |

(continued)

| | **Abbreviati on** |
|---|---|
| Triisopropylsilyl chloride | TIPSCl |
| *tert*-Butyldimethylsilyl chloride | TBSCl |
| Trifluoromethanesulfonic acid tert-butyldimethylsilyl ester | TBSOTf |

**Example**

**Example1.1**

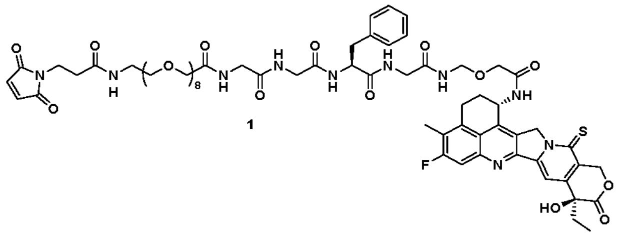

1

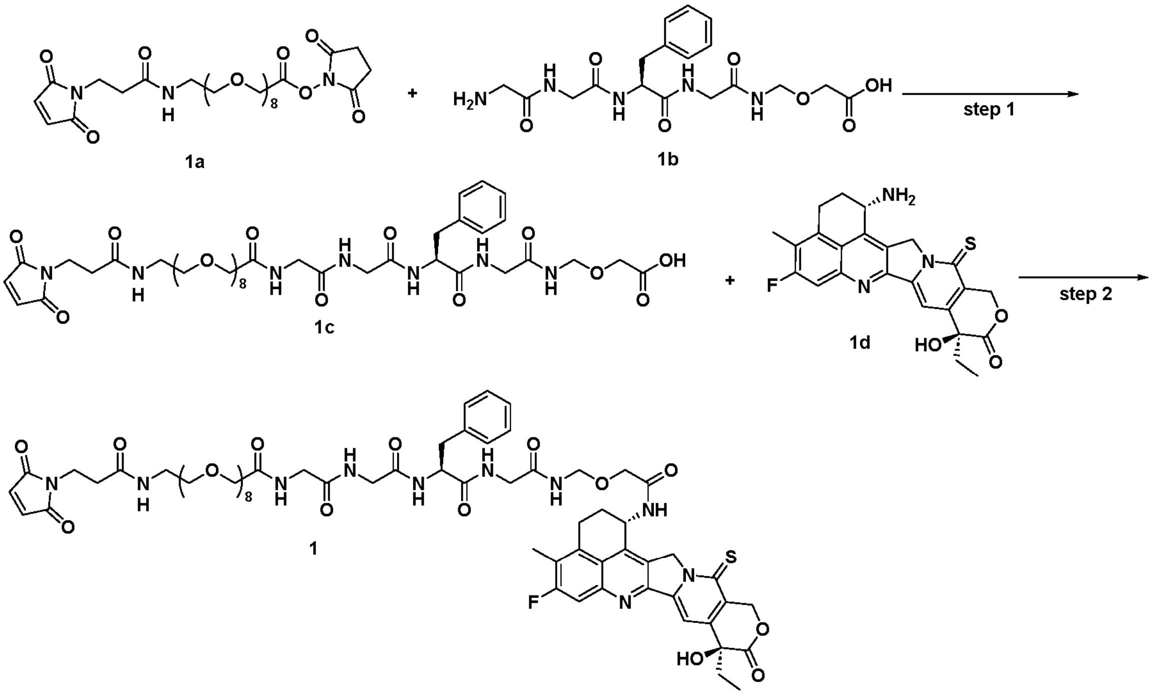


1a

1b

1c

1d

1

Step 1

The compound **1b** (342 mg, 0.81 mmol) and **1a** (500 mg, 0.85 mmol) were dissolved in a mixed solvent of acetonitrile (3.42 mL) and water (6.84 mL). The mixture was cooled to 0°C, and *N,N-diisopropylethylamine* (83 mg, 0.64 mmol) was added dropwise under stirring. After the addition was complete, the reaction was stirred for 3 hours. The reaction mixture was directly purified by prep-HPLC to afford **1c** (283 mg) in 39% yield.
MS-ESI: calcd. for $[M+Na]^+$ 1020, found 1020.

Step 2

Compound **1c** (85 mg, 0.19 mmol) was dissolved in *N*,*N*-dimethylformamide (1.70 mL), followed by the addition

of trifluoroacetic acid (22 mg, 0.19 mmol). The mixture was cooled to 0°C, and **1d** (226 mg, 0.23 mmol), 1-(3-dimethylaminopropyl)-3-ethylcarbodiimide hydrochloride (72 mg, 0.38 mmol), and 2,4,6-Collidine (23 mg, 0.19 mmol) were added sequentially. After the addition was complete, the reaction was stirred at 0-10°C for 1 h. The mixture was then adjusted to pH 6-7 with $HCl_{aq}$ (0.05 M) and purified by prep-HPLC to afford compound **1** (134 mg) in 49% yield.

$^1H$ NMR (400 MHz, DMSO-$d_6$) δ 8.66 (t, *J* = 6.4 Hz, 1H), 8.60 (d, *J* = 8.8 Hz, 1H), 8.28 (t, *J* = 6.0 Hz, 1H), 8.15 (t, *J* = 5.6 Hz, 1H), 8.09 (d, *J* = 8.0 Hz, 1H), 8.04-7.95 (m, 2H), 7.84-7.77 (m, 2H), 7.28-7.14 (m, 5H), 6.99 (s, 2H), 6.69 (s, 1H), 5.91 (d, *J* = 16.8 Hz, 1H), 5.70-5.62 (m, 1H), 5.56-5.45 (m, 2H), 5.35 (d, *J* = 18.8 Hz, 1H), 4.68 (d, *J* = 6.8 Hz, 2H), 4.51-4.42 (m, 1H), 4.17-4.04 (m, 2H), 3.80-3.55 (m, 12H), 3.55-3.42 (m, 32H), 3.17-3.10 (m, 2H), 3.06-2.98 (m, 1H), 2.80-2.71 (m, 1H), 2.41-2.28 (m, 7H), 2.27-2.15 (m, 2H), 1.94-1.82 (m, 2H), 0.86 (t, *J* = 7.2 Hz, 3H).

**Example 1.2**

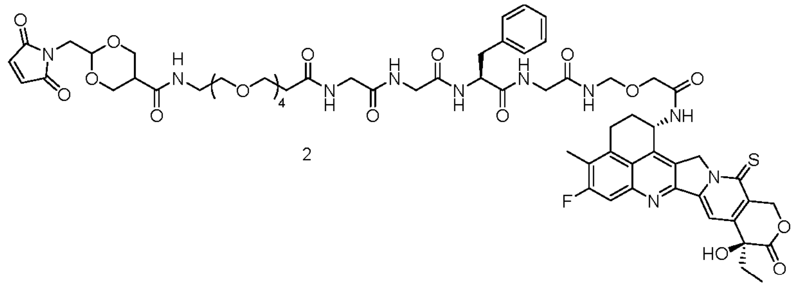

2

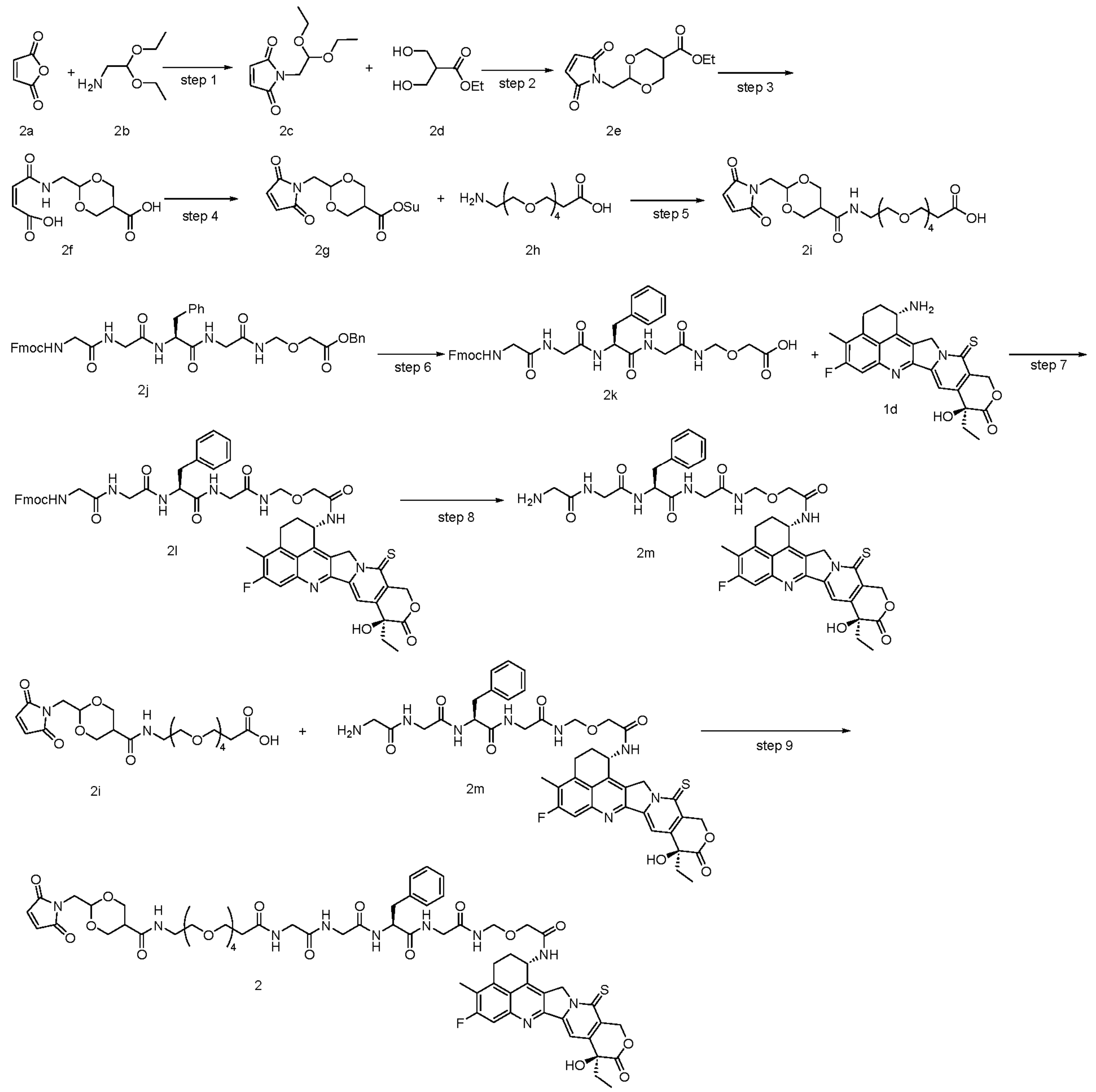


2a 2b 2c 2d 2e 2f 2g 2h 2i 2j 2k 1d 2l 2m 2i 2m 2

Step 1

Compound **2a** (14.72 g, 150.16 mmol) was dissolved in acetone (118 mL). Under an ice bath, **2b** (20.00 g, 150.16 mmol) was added, and the mixture was stirred for 5 min until TLC indicated complete consumption of the starting material. The reaction mixture was concentrated under reduced pressure to afford the crude product as a solid. The crude material was dissolved in acetic anhydride (28 mL), followed by the addition of sodium acetate (24.63 g, 300.32 mmol). The reaction mixture was heated to 90°C and refluxed for 2 h. After cooling, the mixture was filtered to remove insoluble solids, and the filter cake was washed with toluene. The filtrate was concentrated in vacuo to give the crude product, which was purified by silica gel column chromatography (EA/Hexanes = 0-100%) to yield **2c** (17.43 g) in 43% yield.

MS-ESI: calcd. for $[M+Na]^+$ 236, found 236.

Step 2

**2c** (12.95 g, 60.74 mmol) and **2d** (9.00 g, 60.74 mmol) were dissolved in toluene (180 mL), followed by addition of *p*-toluenesulfonic acid (2.10 g, 12.15 mmol). The reaction mixture was heated to 90°C and refluxed for 2 h. After cooling to room temperature, the mixture was concentrated under reduced pressure. The residue was dissolved in ethyl acetate, and the organic phase was sequentially washed with saturated aqueous $NaHCO_3$ and brine (200 mL × 1), dried over

anhydrous $Na_2SO_4$, filtered, and concentrated in vacuo to afford the crude product. The crude product was purification by silica gel column chromatography (EA/Hexanes = 0-100%) to afford **2e** (5.53 g) in 33% yield.

MS-ESI: calcd for $[M+H]^+$ 270, found 270.

Step 3

**2e** (3.87 g, 14.37 mmol) was dissolved in THF (77.4 mL). A solution of lithium hydroxide (1.37 g, 57.49 mmol) in $H_2O$ (38.7 mL) was added to the above solution, the resulting mixture was then stirred at room temperature for 30 min. Ethyl acetate (15 mL) was added, and the pH was adjusted to approximately 2 with $HCl_{aq}$ (1 N). The aqueous layer was extracted with ethyl acetate (38 mL × 3), and the combined organic phases were washed with brine (15 mL), dried over anhydrous sodium sulfate, filtered, and concentrated under reduced pressure to afford the crude product **2f** (3.06 g).

MS-ESI: calcd for $[M+H]^+$ 260, found 260.

Step 4

N-Hydroxysuccinimide (6.49 g, 56.37 mmol) was dissolved in *N,N*-dimethylformamide (36.5 mL). Under ice-cooling, trifluoroacetic anhydride (11.84 g, 56.37 mmol) was added dropwise, and the mixture was stirred for 30 min. Subsequently, 2,4,6-Collidine (6.83 g, 56.37 mmol) was added dropwise, and stirring was continued for an additional 40 min to afford solution A, which was stored for subsequent use. Crude product **2f** (3.06 g) was dissolved in *N*,*N*-dimethylformamide (36.5 mL), and 2,4,6-Collidine (3.41 g, 28.15 mmol) was added dropwise under ice-cooling. After stirring for 30 min while maintaining the ice bath, solution A was added dropwise to the reaction mixture. The resulting solution was allowed to warm to room temperature naturally and stirred for 24 h. The reaction was quenched with dichloromethane (180 mL) and $HCl_{aq}$ (0.7 N, 140 mL), followed by stirring for 30 min. The layers were separated, and the aqueous phase was extracted with dichloromethane (70 mL). The combined organic phases were washed with water until the pH reached 5-7, followed by one wash with brine, dried over anhydrous sodium sulfate, filtered, and concentrated under reduced pressure. The crude product was purified by silica gel column chromatography (EA/Hexanes = 0-100%) to afford **2g** (3.54 g) in 78% yield.

MS-ESI: calcd for $[M+H]^+$ 339, found 339.

$^1H$ NMR (400 MHz, $CDCl_3$) $\delta$ 6.77 (s, 2H), 4.79 (t, *J* = 4.8 Hz, 1H), 4.49-4.40 (m, 2H), 3.91 (t, *J* = 11.6 Hz, 2H), 3.79-3.70 (m, 2H), 3.45-3.34 (m, 1H), 2.86 (s, 4H).

Step 5

A mixture of **2h** (850 mg, 3.38 mmol) and **2g** (1.20 g, 3.55 mmol) were dissolved in anhydrous *N*,*N*-dimethylformamide (8.5 mL). Under ice-cooling, *N,N*-diisopropylethylamine (437 mg, 3.38 mmol) was added dropwise to the reaction mixture. The resulting solution was stirred at 0-10°C for 2 h. The crude product was purified by preparative high-performance liquid chromatography (prep-HPLC) to afford **2i** (740 mg) in 46% yield.

MS-ESI: calcd for $[M+H]^+$ 475, found 475.

Step 6

**2j** (10.00 g, 13.59 mmol) was dissolved in THF (450 mL). To this solution was added wet Pd/C (2 g, 20% w/w). The reaction mixture was stirred under a hydrogen atmosphere for 66 h at room temperature. The reaction mixture was filtered, and the filter cake was washed thoroughly with a mixed solvent of DCM/MeOH . The combined filtrates were dried over anhydrous sodium sulfate, filtered, and concentrated under reduced pressure to afford the crude product. The crude product was purified by flash column chromatography on silica gel (MeOH/DCM = 0-100%) to afford **2k** (6.10 g) in 69% yield.

MS-ESI: calcd for $[M+H]^+$ 668, found 668.

Step 7

Under a nitrogen atmosphere, a solution of **1d** (2.70 g, 5.97 mmol) and trifluoroacetic acid (680 mg, 5.97 mmol) in N,N-dimethylformamide (54 mL) was stirred at 0°C for 10 min. To this cooled solution was sequentially added: **2k** (5.40 g, 8.36 mmol), 1-(3-dimethylaminopropyl)-3-ethylcarbodiimide hydrochloride (2.30 g, 11.94 mmol), and 2,4,6-Collidine (722 mg, 5.97 mmol). The resulting reaction mixture was stirred at 0-10°C for 2 h. The reaction was quenched by addition of $HCl_{aq}$ (0.05 N, 54 mL) at 0°C, resulting in precipitation. The suspension was diluted with 2-methyltetrahydrofuran (100 mL) and stirred until complete dissolution. The layers were separated, and the aqueous phase was extracted with 2-methyltetrahydrofuran (100 mL × 2). The combined organic phases were washed with brine (50 mL), dried over anhydrous

sodium sulfate, filtered, and concentrated under reduced pressure. The crude product was purified by silica gel column chromatography (MeOH/DCM = 0-100%) to afford **2l** (6.00 g) in 84% yield.

MS-ESI: calcd for $[M+H]^+$ 1079, found 1079.

Step 8

Compound **2l** (6.00 g, 5.56 mmol) was dissolved in a mixed solvent of DCM/MeOH (120 mL/12 mL). Under ice-cooling, diethylamine (24 mL) was added dropwise to the solution. After complete addition, the reaction mixture was allowed to warm to room temperature naturally and stirred for 4 h. The reaction mixture was concentrated directly under reduced pressure to afford a brown solid crude product. The crude material was triturated with methyl tert-butyl ether, filtered, and the filter cake was washed with methyl tert-butyl ether 3 times. The filtrate was concentrated to yield a yellow solid crude product. A portion of the crude product (1 g) was subjected to prep-HPLC to obtain **2m** (150 mg). The remaining crude material was purified by silica gel column chromatography (MeOH/DCM = 0-100%) to afford additional **2m** (800 mg) in 59% overall yield.

MS-ESI: calcd for $[M+H]^+$ 857, found 857.

Step 9

**2i** (74 mg, 0.16 mmol) was dissolved in N,N-dimethylformamide (1.30 mL). After three consecutive vacuum-nitrogen purge cycles, 2-(7-aza-1H-benzotriazol-1-yl)-1,1,3,3-tetramethyluronium hexafluorophosphate (88 mg, 0.23 mmol) was added under ice-cooling, followed by stirring for 10 min. **2m** (132 mg, 0.16 mmol) was then added, and stirring continued until complete dissolution was achieved. 2,4,6-Collidine (54 mg, 0.45 mmol) was subsequently charged, and the resulting mixture was stirred at 0-10°C for 2 h. The reaction mixture was then adjusted to pH 6-7 with $HCl_{aq}$ (0.05 M) and purified by prep-HPLC to afford **2** (71 mg) in 34% yield.

MS-ESI: calcd for $[M+H]^+$ 1313, found 1313.

**Example 1.3**

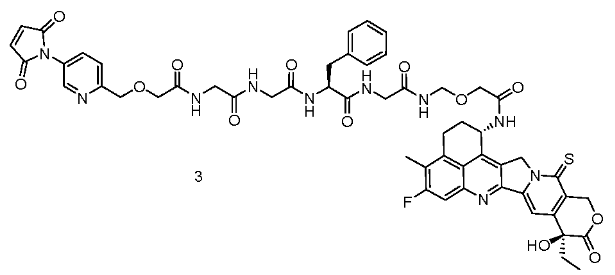

3

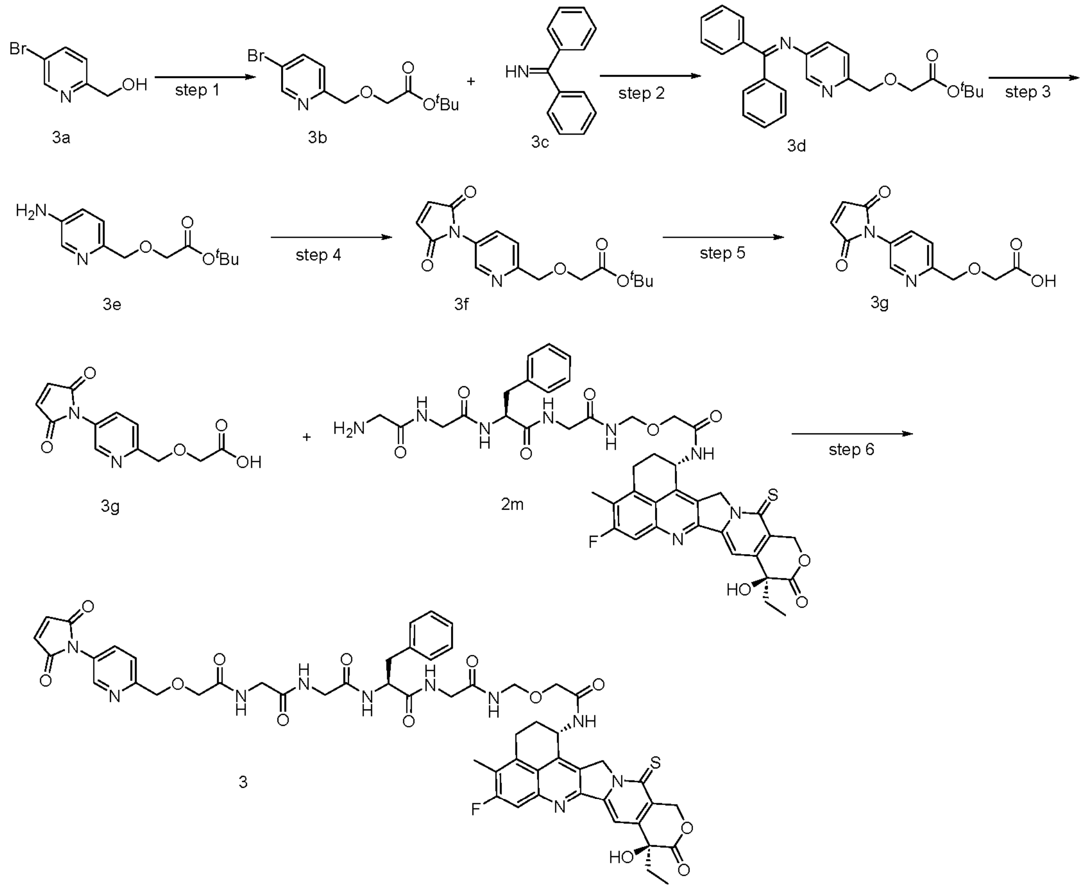


3a 3b 3c 3d 3e 3f 3g 3g 2m 3

Step 1

Under a nitrogen atmosphere, **3a** (5.00 g, 26.74 mmol) was dissolved in *N,N*-dimethylformamide (50 mL), and the solution was cooled to 0-5°C. NaH (1.28 g, 32.09 mmol) was added, followed by stirring for 10 min. Subsequently, *tert*-butyl bromoacetate (6.23 g, 32.09 mmol) was added, and the reaction mixture was stirred at 0-5°C for 2 h. Upon completion, water (200 mL) was added to the reaction mixture, and the aqueous phase was extracted with ethyl acetate (200 mL). The organic phase was washed with saturated brine (200 mL × 2), dried over anhydrous sodium sulfate, and filtered. The filtrate was concentrated under reduced pressure to afford **3b** (4.60 g) in 43% yield.

MS-ESI: calcd for $[M+H]^+$ 302, 304, found 302, 304.

$^1H$ NMR (400 MHz, $CDCl_3$) δ 8.60 (d, *J* = 2.0 Hz, 1H), 7.84 (dd, *J* = 8.4, 2.4 Hz, 1H), 7.45 (d, *J* = 8.4 Hz, 1H), 4.69 (s, 2H), 4.10 (s, 2H), 1.49 (s, 9H).

Step 2

Under a nitrogen atmosphere, a mixture of **3b** (5.00 g, 16.60 mmol), **3c** (3.61 g, 19.93 mmol), $Pd_2(dba)_3$ (0.76 g, 0.83 mmol), BINAP (1.03 g, 1.66 mmol), and cesium carbonate (13.53 g, 41.52 mmol) was dissolved in anhydrous toluene (50 mL). The reaction mixture was heated to an internal temperature of 80°C and stirred for 16 hours. The reaction mixture was then filtered, and the filtrate was concentrated under reduced pressure to afford the crude product. The crude product was purified by silica gel column chromatography (EA/hexanes = 0-100%) to afford **3d** (4.50 g) in 67% yield.

MS-ESI: Calcd. for $[M+H]^+$ 403, Found 403.

Step 3

**3d** (2.81 g, 6.98 mmol) was dissolved in a mixed solvent of THF (28 mL) and $HCl_{aq}$ (1 N, 28 mL). The resulting mixture was stirred at room temperature for 3 h. Water (100 mL) was then added to the reaction mixture, and the aqueous phase was washed with ethyl acetate (100 mL × 2), with the organic phase being discarded. The aqueous phase was adjusted to pH 8-9 with aqueous ammonia and then extracted with ethyl acetate (100 mL × 2). The combined organic phases were dried over anhydrous sodium sulfate, filtered, and concentrated under reduced pressure to afford **3e** (1.43 g)

in 86% yield.
MS-ESI: Calcd for $[M+H]^+$ 239, Found 239.

Step 4

**3e** (1.83 g, 7.68 mmol) and maleic anhydride (0.75 g, 7.68 mmol) were dissolved in acetonitrile (18.3 mL), the reaction mixture was stirred at room temperature for 2 h. The reaction mixture was then concentrated under reduced pressure to afford 2.45 g of a white solid intermediate. This intermediate (2.45 g) was charged into a reaction flask, followed by sequential addition of acetic anhydride (5 mL) and sodium acetate (2.09 g, 15.36 mmol). The resulting mixture was stirred at room temperature for 2 h. Water (50 mL) was then added, and the aqueous phase was extracted with ethyl acetate (50 mL). The organic phase was dried over anhydrous sodium sulfate, filtered, and concentrated under reduced pressure to yield a crude residue. The crude residue was purified by silica gel column chromatography (EA/hexanes = 0-100%) to afford **3f** (1.97 g) in 80% yield.
MS-ESI: Calcd for $[M+H]^+$ 319, Found 319.
$^1H$ NMR (400 MHz, DMSO-$d_6$) δ 8.51 (d, *J* = 2.0 Hz, 1H), 7.83 (dd, *J* = 8.4, 2.4 Hz, 1H), 7.6 (d, *J* = 8.4 Hz, 1H), 7.24 (s, 2H), 4.67 (s, 2H), 4.15 (s, 2H), 1.44 (s, 9H).

Step 5

**3f** (0.90 g, 2.83 mmol) was dissolved in dichloromethane (9 mL), trifluoroacetic acid (1.8 mL) was then added and the resulting mixture was stirred at room temperature for 5 hours. The reaction solution was concentrated under reduced pressure followed by coevaporation with dichloromethane (45 mL × 5), and the residue was concentrated using an oil pump until no obvious oily substance remaining to obtain the crude product **3g** (0.93 g).
MS-ESI: Calcd for $[M+H]^+$ 263, Found 263.

step 6

To the reaction flask were successively added **3g** (0.06 g), **2m** (0.20 g, 0.23 mmol), and N,N-dimethylformamide (4 mL). After stirring to dissolve, the mixture was cooled in an ice bath. Then, 2-(7-aza-1H-benzotriazol-1-yl)-N,N,N',N'-tetramethyluronium hexafluorophosphate (0.13 g, 0.35 mmol) and 2,4,6-Collidine (0.26 g, 2.15 mmol) were added. The reaction mixture was stirred at 0-5 °C for 1 h. After completion, the reaction mixture was adjusted to pH 4-5 using $HCl_{aq}$ (0.5 N) and then subjected to prep-HPLC under neutral aqueous conditions for purification, affording **3** (70 mg) in 27% yield.
MS-ESI: Calcd for $[M+H]^+$ 1101, Found 1101.
$^1H$ NMR (400 MHz, DMSO-$d_6$) δ 8.65 (t, *J* = 6.8 Hz, 1H), 8.60 (d, *J* = 8.8 Hz, 1H), 8.34-8.24 (m, 2H), 8.09 (d, *J* = 8.0 Hz, 1H), 7.99 (t, *J* = 5.6 Hz, 1H), 7.81 (d, *J* = 11.2 Hz, 1H), 7.79 (s, 1H), 7.30-7.14 (m, 5H), 7.04 (s, 2H), 6.68 (s, 1H), 5.91 (d, *J* = 16.8 Hz, 1H), 5.70-5.61 (m, 1H), 5.56-5.45 (m, 2H), 5.36 (d, *J* = 19.6 Hz, 1H), 5.05 (t, *J* = 6.0 Hz, 1H), 4.68 (d, *J* = 6.4 Hz, 2H), 4.59 (dd, *J* = 9.2, 6.0 Hz, 1H), 4.52-4.42 (m, 1H), 4.17-4.05 (m, 2H), 4.00-3.92 (m, 1H), 3.89-3.79 (m, 1H), 3.78-3.68 (m, 5H), 3.64-3.54 (m, 2H), 3.01 (dd, *J* = 8.8, 4.4 Hz, 1H), 2.74 (dd, *J* = 13.6, 9.6 Hz, 1H), 2.39 (s, 3H), 2.26-2.18 (m, 2H), 1.94-1.82 (m, 2H), 0.86 (t, *J* = 7.6 Hz, 3H).

**Example 1.4**

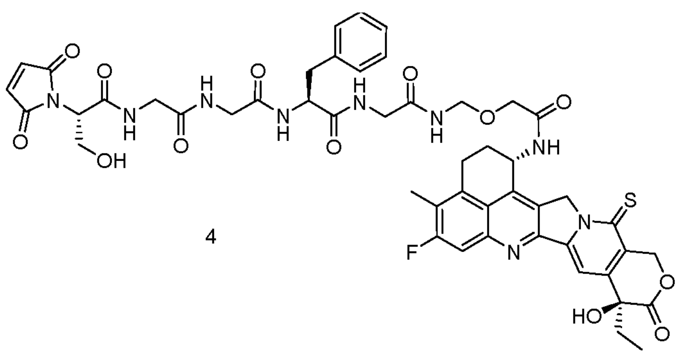

4

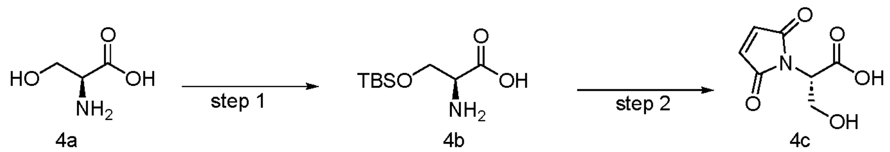


4a 4b 4c

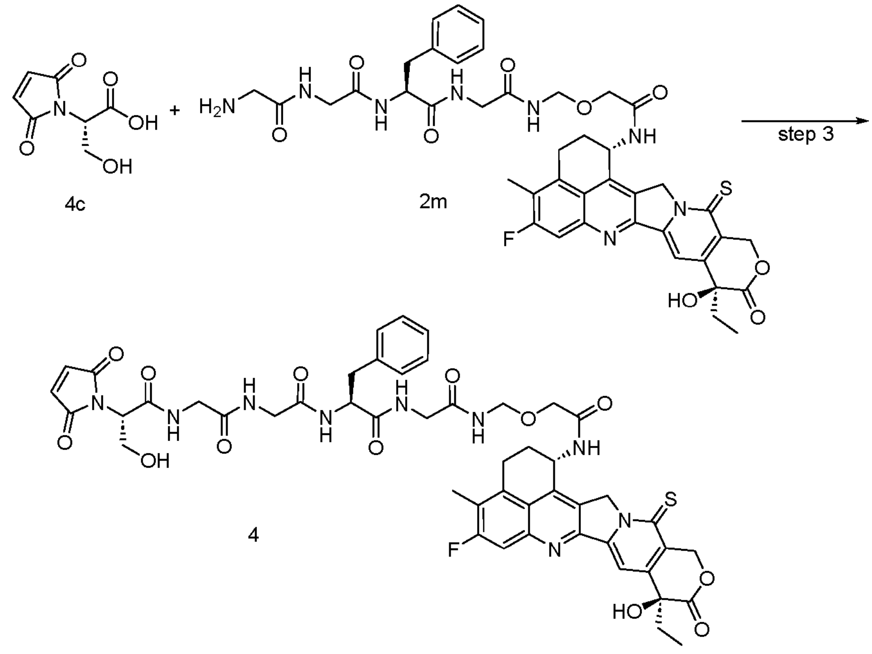


4c 2m

4

Step 1

**4a** (10.00 g, 95.20 mmol) was dissolved in acetonitrile (100 mL), followed by the addition of tert-butyldimethylsilyl chloride (15.06 g, 99.92 mmol). The reaction mixture was cooled to 0°C, and 1,8-diazabicyclo[5.4.0]undec-7-ene (13.73 g, 90.21 mmol) was added dropwise. After complete addition, the reaction mixture was allowed to warm to room temperature and stirred for 16 h. The product gradually precipitated out. The reaction mixture was directly filtered, and the filter cake was collected to afford **4b** (11.50 g) in 55% yield.

MS-ESI: Calcd for $[M+H]^+$ 220, Found 220.

Step 2

To the reaction flask were successively added **4b** (5.55 g, 25.33 mmol), acetone (55.5 mL), and maleic anhydride (2.48 g, 25.33 mmol). After complete addition, the mixture was stirred at room temperature for 2 h. The reaction mixture was then concentrated under reduced pressure to afford 8.25 g of a yellow oil. The obtained yellow oil was dissolved in toluene (82 mL), followed by the addition of triethylamine (5.12 g, 50.66 mmol). The reaction mixture was heated to 120 °C and refluxed for 2 h. After completion, the mixture was concentrated under reduced pressure to yield a crude residue, which was purified by silica gel column chromatography (MeOH/DCM = 0-100%) to afford **4c** (1.53 g) in 33% yield.

MS-ESI: Calcd for $[M-H]^-$ 184, Found 184.

$^1$H NMR (400 MHz, DMSO-$d_6$) δ 7.00 (s, 2H), 6.05 (s, 1H), 4.30 (dd, *J* = 9.6, 5.6 Hz, 1H), 3.98 (dd, *J*= 10.8, 5.6 Hz, 1H), 3.84 (dd, *J* = 10.8, 10.8 Hz, 1H).

Step 3

To the reaction flask were sequentially added **4c** (65 mg, 0.234 mmol), **2m** (0.20 g, 0.23 mmol), and *N*,*N*-dimethylformamide (4 mL). After stirring to complete dissolution, the mixture was cooled in an ice bath. Subsequently, 2-(7-aza-1H-benzotriazol-1-yl)-N,N,N',N'-tetramethyluronium hexafluorophosphate (0.13 g, 0.35 mmol) and 2,4,6-collidine (85 mg, 0.70 mmol) were added. The reaction mixture was stirred at 0-5 °C for 1 h. Upon completion, the reaction mixture was quenched with $HCl_{aq}$ (0.5 N) to adjust the pH to 4-5, followed by purification via prep-HPLC using a neutral water-based mobile phase to afford **4** (70 mg) in 14% yield.

MS-ESI: Calcd for $[M+H]^+$ 1024, Found 1024.

$^1$H NMR (400 MHz, DMSO-$d_6$) δ 8.65 (t, *J* = 6.8 Hz, 1H), 8.60 (d, *J* = 8.8 Hz, 1H), 8.29 (q, *J* = 5.6 Hz, 2H), 8.09 (d, *J* = 8.0 Hz, 1H),7.99 (t, *J* = 5.6 Hz, 1H), 7.81 (d, *J* = 11.2 Hz, 1H), 7.80 (s, 1H), 7.29-7.14 (m, 5H), 7.04 (s, 2H), 6.68 (s, 1H), 5.91 (d, *J* = 16.8 Hz, 1H), 5.71-5.60 (m, 1H), 5.56-5.45 (m, 2H), 5.36 (d, *J* = 19.6 Hz, 1H), 5.05 (t, *J* = 5.6 Hz, 1H), 4.68 (d, *J* = 6.4 Hz, 2H), 4.59 (dd, *J* = 9.2, 6.0 Hz, 1H), 4.52-4.43 (m, 1H), 4.17-4.04 (m, 2H), 4.00-3.92 (m, 1H), 3.98-3.80 (m, 1H), 3.78-3.68 (m, 5H), 3.64-3.54 (m, 2H), 3.20-3.09 (m, 1H), 3.00 (dd, *J* = 14.0, 4.4 Hz, 1H), 2.73 (dd, *J* = 13.6, 9.6 Hz, 1H), 2.39 (s, 3H), 2.26-2.17 (m, 2H), 1.94-1.83 (m, 2H), 0.86 (t, *J* = 7.2 Hz, 3H).

**Example 1.5**

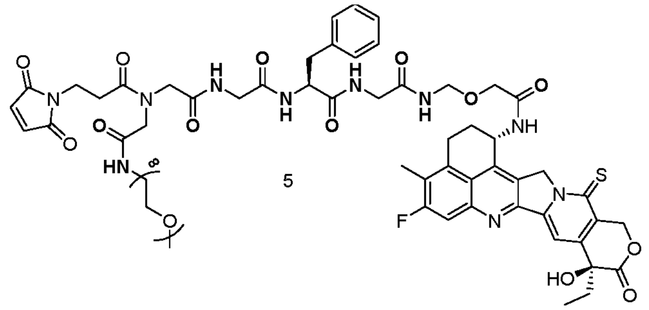

5

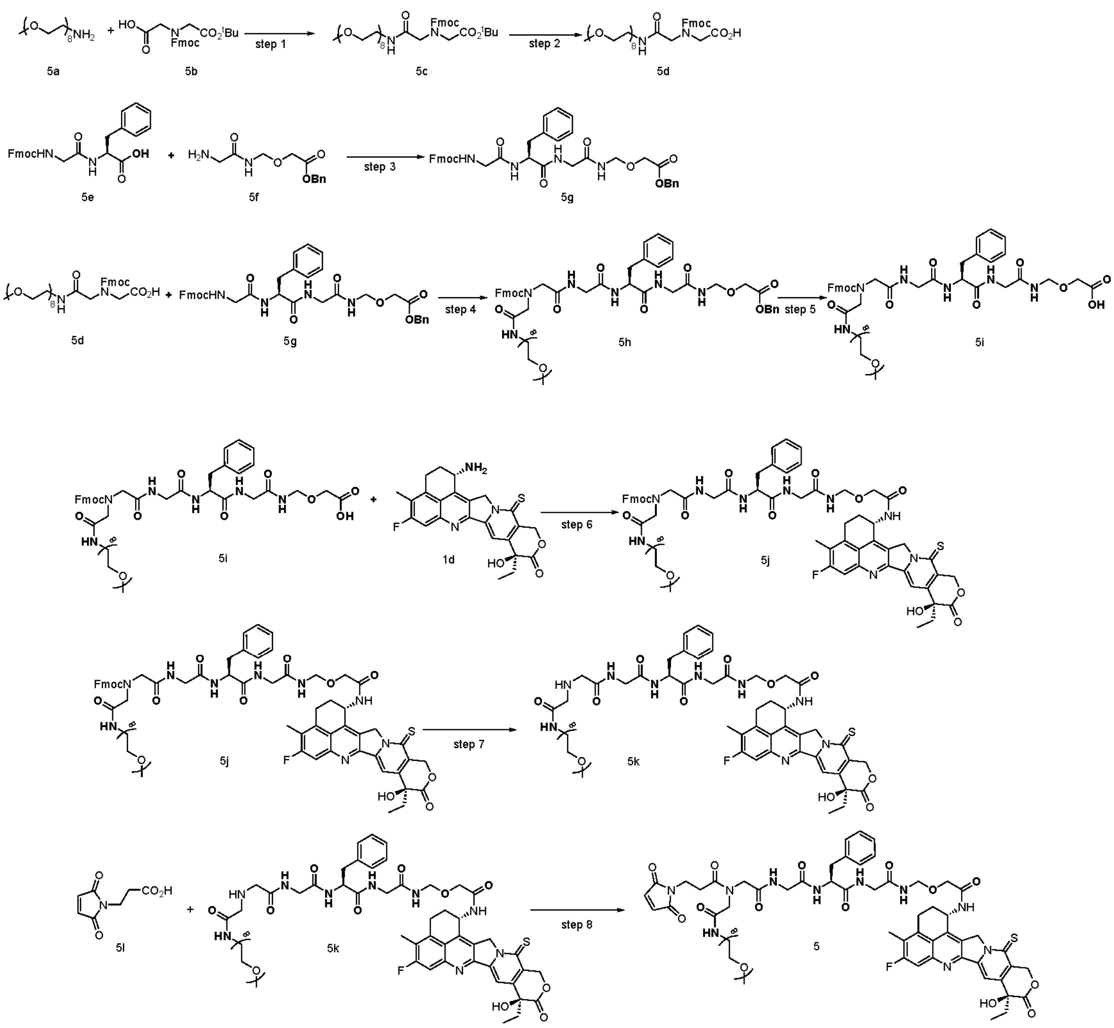


5a 5b 5c 5d

5e 5f 5g

5d 5g 5h 5i

5i 1d 5j

5j 5k

5l 5k 5

Step 1

**5a** (650 mg, 1.70 mmol) and **5b** (699 mg, 1.70 mmol) were dissolved in anhydrous dichloromethane (6 mL). Under an ice bath, 2-(7-aza-1H-benzotriazol-1-yl)-N,N,N',N'-tetramethyluronium hexafluorophosphate (771 mg, 2.03 mmol) and N,N-diisopropylethylamine (440 mg, 2.40 mmol) were added sequentially. After complete addition, the reaction mixture was allowed to warm to room temperature and stirred for 1 h. The reaction mixture was concentrated directly, and the residue was purified by silica gel column chromatography (MeOH/DCM = 0-100%) to afford **5c** (1.10 g) in 83% yield.
MS-ESI: calcd for $[M+H]^+$ 777, found 777.

Step 2

**5c** (1.10 g, 1.42 mmol) was dissolved in anhydrous dichloromethane (2.6 mL). Under an ice bath, trifluoroacetic acid (2 mL) was added dropwise. After complete addition, the reaction mixture was allowed to warm to room temperature and stirred for 2 h. The reaction mixture was concentrated directly, and the residue was purified by silica gel column chromatography (MeOH/DCM = 0-100%) to afford the crude product **5d** (1.16 g).
MS-ESI: calcd for $[M+H]^+$ 721, found 721.

Step 3

**5f** (800 mg, 1.69 mmol) was dissolved in anhydrous acetonitrile (12 mL). Under an ice bath, 1,8-diazabicyclo [5.4.0]undec-7-ene (129 mg, 0.85 mmol) was added dropwise. After complete addition, the reaction mixture was allowed to warm to room temperature and stirred for 1 h. Subsequently, **5e** (750 mg, 1.69 mmol) was added to the above solution, followed by sequential addition of 2-(7-aza-1H-benzotriazol-1-yl)-N,N,N',N'-tetramethyluronium hexafluorophosphate (771 mg, 2.03 mmol) and N,N-diisopropylethylamine (654 mg, 5.07 mmol) under ice-cooling. Upon completion of addition, the reaction mixture was warmed to room temperature and stirred for 2 h. The mixture was diluted with ethyl acetate (200 mL), and the organic phase was washed with saturated brine (50 mL × 3), dried over anhydrous sodium sulfate, and filtered. The filtrate was concentrated under reduced pressure to afford the crude residue, which was purified by silica gel column chromatography (MeOH/DCM = 0-100%) to afford the crude product **5g** (440 mg) in 38% yield.
MS-ESI: calcd for $[M+Na]^+$ 701, found 701.

Step 4

**5g** (420 mg, 0.62 mmol) was dissolved in anhydrous acetonitrile (10 mL). Under an ice bath, 1,8-diazabicyclo [5.4.0]undec-7-ene (94 mg, 0.62 mmol) was added dropwise.After complete addition, the reaction mixture was allowed to warm to room temperature and stirred for 2 h. Subsequently, **5d** (446 mg, 0.62 mmol) was added to the above reaction solution, followed by sequential addition of 2-(7-aza-1H-benzotriazol-1-yl)-N,N,N',N'-tetramethyluronium hexafluorophosphate (353 mg, 0.93 mmol) and N,N-diisopropylethylamine (240 mg, 1.86 mmol) under ice-cooling. Upon completion of addition, the reaction mixture was warmed to room temperature and stirred for 1 h. The reaction mixture was diluted with ethyl acetate (100 mL), and the organic phase was washed with saturated brine (25 mL × 3), dried over anhydrous sodium sulfate, and filtered. The filtrate was concentrated under reduced pressure to afford the crude residue, which was purified by silica gel column chromatography (MeOH/DCM = 0-100%) to afford **5h** (600 mg) in 83% yield.
MS-ESI: calcd for $[M+Na]^+$ 1181, found 1181.

Step 5

**5h** (600 mg, 0.52 mmol) was dissolved in the mixed solvent of THF/water (8 mL/2 mL). To this solution was added wet Pd/C (60 mg, 10% w/w). The reaction mixture was stirred under a hydrogen atmosphere for 5 h. The reaction mixture was filtered to remove insoluble solid. The obtained filtrate was concentrated under reduced pressure to afford the crude product. The crude product was purified by flash column chromatography on silica gel (MeOH/DCM = 0-100%) to afford **5i** (360 mg) in 65% yield.
MS-ESI: calcd for $[M+Na]^+$ 1091.5, found 1091.8.

Step 6

To the reaction flask were successively added **1d** (50 mg, 0.11 mmol), **5i** (140 mg, 0.13 mmol), and *N*,*N*-dimethylformamide (2 mL). After stirring to dissolve, the mixture was cooled in an ice bath, followed by the addition of 2-(7-aza-1H-benzotriazol-1-yl)-N,N,N',N'-tetramethyluronium hexafluorophosphate (65 mg, 0.17 mmol) and N,N-diisopropylethylamine (44 mg, 0.34 mmol). The resulting mixture was stirred at 0-5°C for 1 h. The reaction solution was then

concentrated under reduced pressure to afford the crude residue, which was purified by silica gel column chromatography (MeOH/DCM = 0-100%) to yield the crude product **5j** (140 mg) in 85% yield.

MS-ESI: calcd for $[M+H]^+$ 1502.6, found 1503.

Step 7

**5j** (55 mg, 0.037 mmol) was dissolved in anhydrous acetonitrile (2 mL), followed by dropwise addition of diethylamine (27 mg, 0.37 mmol) under an ice bath. After complete addition, the reaction mixture was allowed to warm to room temperature and stirred overnight. The reaction solution was then concentrated under reduced pressure, and the resulting residue was purified by silica gel column chromatography (MeOH/DCM = 0-100%) to afford **5k** (30 mg) in 63% yield.

MS-ESI: calcd for $[M+H]^+$ 1280.6, found 1281.

Step 8

**5k** (30 mg, 0.023 mmol) and **51** (20 mg, 0.12 mmol) were dissolved in anhydrous dichloromethane (1 mL). Under ice-cooling, 2-(7-aza-1H-benzotriazol-1-yl)-N,N,N',N'-tetramethyluronium hexafluorophosphate (35 mg, 0.092 mmol) and N,N-diisopropylethylamine (16 mg, 0.12 mmol) were added sequentially. The resulting mixture was stirred at room temperature for 1 h. The reaction mixture was then acidified to pH 4-5 with $HCl_{aq}$ (0.5 N) and purified by prep-HPLC using a neutral water-based mobile phase to afford **5** (15 mg) in 45% yield.

MS-ESI: calcd for $[M+H]^+$ 1431.6, found 1432.

**Example 1.6**

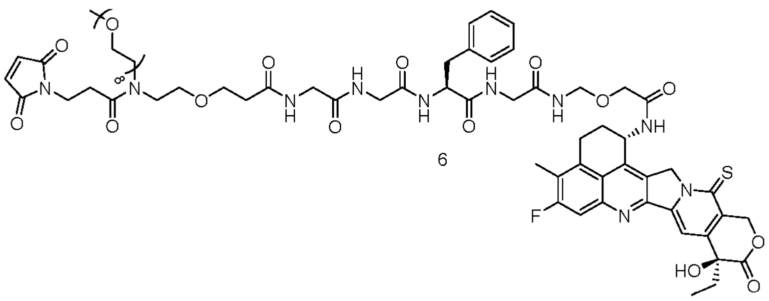

6

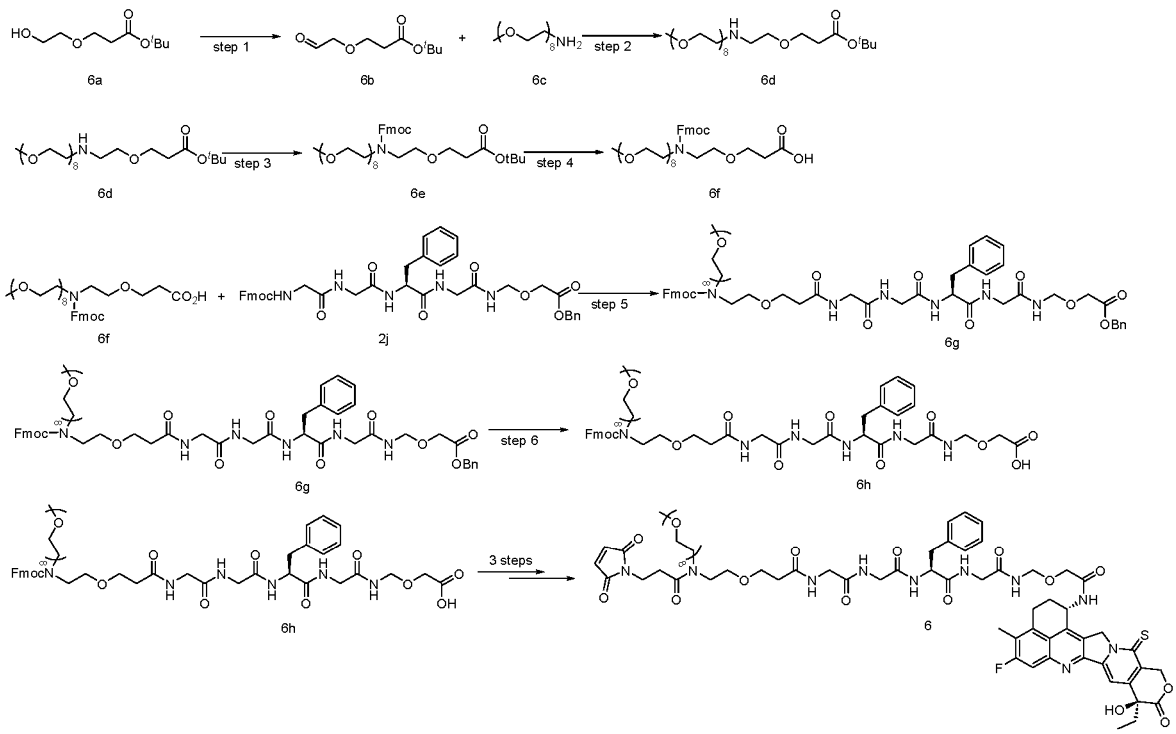


Step 1

**6a** (4.00 g, 21.03 mmol) was dissolved in anhydrous dichloromethane (64 mL), followed by addition of Dess-Martin periodinane (11.15 g, 26.30 mmol). After complete addition, the reaction mixture was stirred at room temperature for 2 h. The reaction was then quenched by dilution with dichloromethane (100 mL). The organic phase was sequentially washed with saturated sodium thiosulfate solution (50 mL × 1) and saturated brine (50 mL × 2), dried over anhydrous sodium sulfate, and filtered. The filtrate was concentrated under reduced pressure to afford the crude product **6b** (5.30 g).

Step 2

The crude product **6b** (500 mg) and compound **6c** (1.20 g, 3.19 mmol) were dissolved in anhydrous methanol (25 mL). Acetic acid (7.56 g, 126.00 mmol) and sodium cyanoborohydride (418 mg, 6.65 mmol) were then added. After complete addition, the reaction mixture was stirred at room temperature for 3 h. The reaction was quenched by dilution with dichloromethane (150 mL) followed by addition of saturated sodium bicarbonate solution (50 mL) to neutralize excess acetic acid. The layers were separated, and the organic phase was washed with saturated brine (50 mL × 2), dried over anhydrous sodium sulfate, and filtered. The filtrate was concentrated under reduced pressure to afford the crude product **6d** (1.38 g).

MS-ESI: calcd for $[M+H]^+$ 556, found 556.

Step 3

The crude product **6d** (1.38 g) was dissolved in anhydrous tetrahydrofuran (30 mL), followed by addition of N,N-diisopropylethylamine (482 mg, 3.73 mmol) and 9-fluorenylmethyl chloroformate (1.29 g, 4.97 mmol). After complete addition, the reaction mixture was stirred at room temperature for 3 h. The reaction was then diluted with dichloromethane (150 mL), and the organic phase was washed with saturated brine (50 mL × 2), dried over anhydrous sodium sulfate, and filtered. The filtrate was concentrated under reduced pressure, and the resulting residue was purified by silica gel column chromatography (MeOH/DCM = 0-100%) to afford **6e** (1.00 g) in 65% yield.

MS-ESI: calcd for $[M+H]^+$ 778, found 778.

Step 4

**6e** (1.00 g, 1.28 mmol) was dissolved in anhydrous dichloromethane (4 mL), and trifluoroacetic acid (1.46 g, 12.85 mmol) was added. The reaction mixture was stirred at room temperature for 1 h, followed by addition of another

portion of trifluoroacetic acid (1.46 g, 12.85 mmol). After completion, the reaction mixture was concentrated under reduced pressure. The crude residue was purified by flash column chromatography (MeOH/DCM = 0-100%) to afford **6f** (500 mg) in 54% yield.

MS-ESI: calcd for $[M+H]^+$ 722.4, found 722.9.

Step 5

**2j** (500 mg, 0.68 mmol) was dissolved in anhydrous acetonitrile (5 mL). under ice-cooling. 1,8-Diazabicyclo [5.4.0]undec-7-ene (134 mg, 0.88 mmol) was added dropwise. After complete addition, the reaction mixture was allowed to warm to room temperature and stirred for 2 h. Subsequently, compound **6f** (490 mg, 0.68 mmol) was added to the above reaction solution, followed by sequential addition of 2-(7-aza-1H-benzotriazol-1-yl)-1,1,3,3-tetramethyluronium hexafluorophosphate (388 mg, 1.02 mmol) and N,N-diisopropylethylamine (220 mg, 1.70 mmol) under an ice bath. Upon completion of addition, the reaction system was returned to room temperature and stirred for 1 h. The reaction mixture was diluted with dichloromethane (100 mL), and the organic phase was washed with saturated brine (25 mL × 3), dried over anhydrous sodium sulfate, and filtered. The filtrate was concentrated under reduced pressure to afford the crude product, which was purified by silica gel column chromatography (MeOH/DCM = 0-100%) to afford **6g** (470 mg) in 57%.

MS-ESI: calcd for $[M+H]^+$ 1239.6, found 1239.9.

$^1$H NMR (400 MHz, DMSO-$d_6$) δ 7.84 (d, *J* = 7.6 Hz, 2H), 7.82-7.74 (m, 1H), 7.63 (d, *J* = 7.6 Hz, 2H), 7.61-7.52 (m, 2H),7.50-7.44 (m, 2H), 7.44-7.24 (m, 15H), 4.90-4.81 (m, 2H), 4.73-4.62 (m, 1H), 4.59 (m, *J* = 5.6 Hz, 2H), 4.30 (s, 2H), 4.29-4.25 (m, 1H), 4.15-4.02 (m, 1H), 4.00-3.89 (m, 2H), 3.86 (d, *J* = 5.2 Hz, 2H), 3.80-3.45 (m, 34H), 3.43 (s, 3H), 3.41-3.38 (m, 1H), 3.11 (dd, *J* = 14.0, 10.0 Hz, 1H).

Step 6

**6g** (470 mg, 0.39 mmol) was dissolved in the mixed solvent of THF/water (4 mL/2 mL). To this solution was added wet Pd/C (62 mg, 13% w/w). The reaction mixture was stirred under a hydrogen atmosphere for 2 h. The reaction mixture was filtered to remove insoluble solid. The obtained filtrate was concentrated under reduced pressure to afford crude product **6h** (360 mg).

MS-ESI: calcd for $[M+Na]^+$ 1149.5, found 1149.9.

Following a three-step reaction sequence analogous to that used for the synthesis of compound **5** from compound **5i** and compound **1d**, compound **6h** was reacted with compound **1d** to afford compound **6.**

MS-ESI: Calcd for $[M+H]^+$ 1489.6, found 1490.

**Example 1.7**

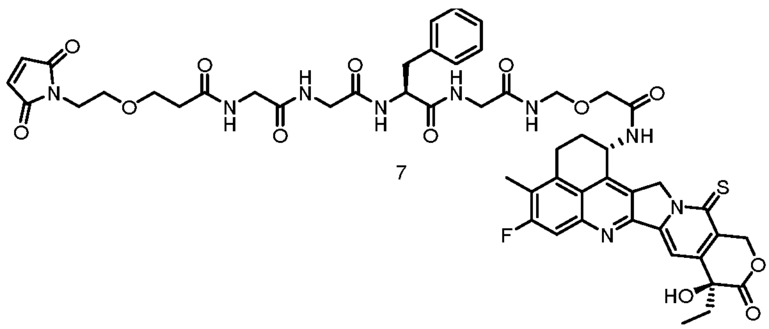

7

Synthetic Route 1

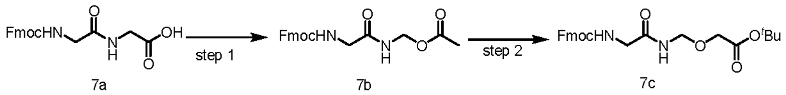


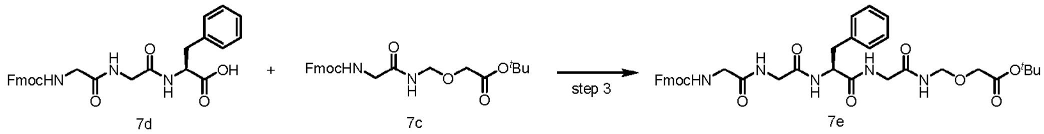


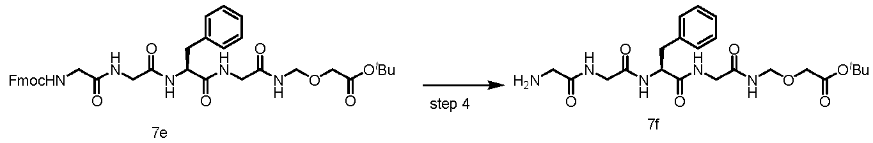


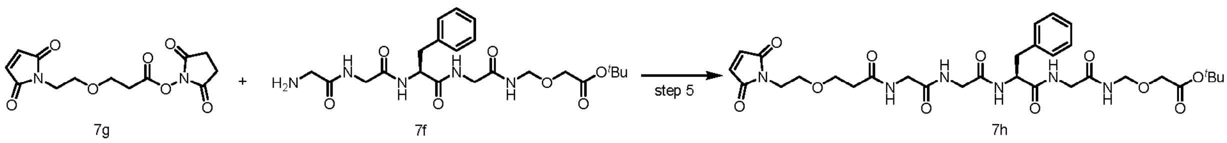


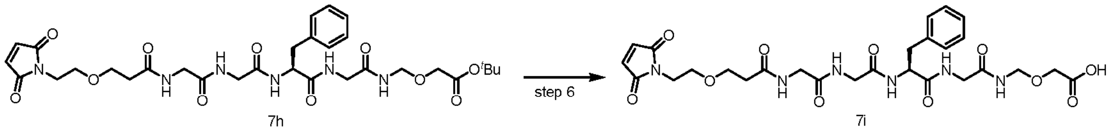


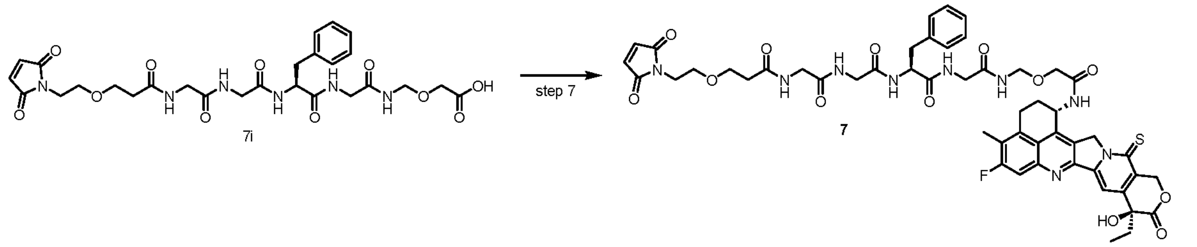


Step 1

Under a nitrogen atmosphere, compound **7a** (10.60 g, 30.00 mmol) was dissolved in a mixed solvent of tetrahydrofuran/toluene (200 mL/50 mL). Lead(IV) acetate (17.30 g, 39.00 mmol) and pyridine (3.08 g, 39.00 mmol) were added, and the reaction mixture was heated to 75°C with stirring for 4 h. After completion, the mixture was filtered to remove insoluble solids, and the filtrate was concentrated under reduced pressure to remove most of the solvent. The residue was dissolved in ethyl acetate (400 mL), and the organic layer was successively washed with water (100 mL × 2), saturated aqueous sodium bicarbonate (100 mL), hydrochloric acid (0.5 N, 100 mL × 2) and saturated brine (100 mL × 2). The organic phase was dried over anhydrous sodium sulfate, filtered, and concentrated under reduced pressure. The crude product was purified by silica gel column chromatography (EA/hexanes = 0-100%) to afford **7b** (9.70 g) in 88% yield.
MS-ESI: Calcd for $[M+Na]^+$ 391, Found 391.

Step 2

Under a nitrogen atmosphere, compound **7b** (8.00 g, 21.74 mmol) was dissolved in anhydrous tetrahydrofuran (40 mL). p-Toluenesulfonic acid (374 mg, 2.18 mmol) and *tert*-butyl glycolate (8.60 g, 65.14 mmol) were added sequentially under an ice bath. After complete addition, the reaction mixture was allowed to warm to room temperature and stirred for 1 h. The mixture was then diluted with ethyl acetate (200 mL), and the organic layer was washed sequentially with water (50 mL × 2), saturated aqueous sodium bicarbonate (50 mL), saturated brine (100 mL × 2). The organic phase was dried over anhydrous sodium sulfate, filtered, and concentrated under reduced pressure. The crude product was purified by silica gel column chromatography (EA/hexanes = 0-100%) to afford **7c** (5.90 g) in 62% yield.
MS-ESI: Calcd for $[M+Na]^+$ 463, Found 463.

Step 3

Under a nitrogen atmosphere, **7c** (4.90 g, 11.14 mmol) was dissolved in anhydrous acetonitrile (40 mL). The solution was then cooled in an ice bath, and 1,8-diazabicyclo[5.4.0]undec-7-ene (847 mg, 5.57 mmol) was added. After complete addition, the reaction mixture was allowed to warm to room temperature and stirred for 3 h. Subsequently, **7d** (6.13 g, 11.14 mmol) was added to the above reaction mixture. The reaction mixture was cooled again in an ice bath, followed by the sequential addition of 2-(7-aza-1H-benzotriazol-1-yl)-N,N,N',N'-tetramethyluronium hexafluorophosphate (6.34 mg, 16.68 mmol) and N,N-diisopropylethylamine (2.88 g, 22.32 mmol). After complete addition, the reaction was warmed to room temperature and stirred for an additional 1 h. The reaction mixture was then diluted with ethyl acetate (200 mL), and the organic phase was washed with saturated brine (25 mL × 3), dried over anhydrous sodium sulfate, and filtered. The filtrate was concentrated under reduced pressure to afford the crude residue, which was purified by silica gel column chromatography (MeOH/DCM = 0-100%) to yield **7e** (5.8 g) in 74% yield).
MS-ESI: Calcd for $[M+Na]^+$ 724, Found 724.

Step 4

Under a nitrogen atmosphere, **7e** (2.80 g, 3.99 mmol) was dissolved in anhydrous acetonitrile (30 mL). The solution was cooled in an ice bath, and 1,8-diazabicyclo[5.4.0]undec-7-ene (606 mg, 3.99 mmol) was added. After complete addition, the reaction mixture was allowed to warm to room temperature and stirred for 5 h. The mixture was then cooled to 0°C, and 1-hydroxybenzotriazole (1.08 g, 7.98 mmol) was added portionwise. The reaction mixture precipitated a gel-like solid, and stirring was continued in the ice bath for an additional 1 h. The mixture was filtered, and the collected solid was triturated with a mixture of isopropyl ether/hexane (10 mL/40 mL). After filtration, the solid was isolated to afford **7f** (2.0 g).
MS-ESI: Calcd for $[M+H]^+$ 480, Found 480.

Step 5

**7g** (0.99 g, 3.20 mmol) and **7f** (2.40 g, 3.20 mmol) was dissolved in a mixed solvent of acetonitrile/water (15 mL/15 mL). The solution was cooled in an ice bath, and 2,4,6-collidine (387 mg, 3.20 mmol) was added. After complete addition, the reaction mixture was allowed to warm to room temperature and stirred for 2 h. The reaction mixture was then diluted with ethyl acetate (200 mL). The organic phase was sequentially washed with water (30 mL × 2), hydrochloric acid (0.5 N, 50 mL × 2) and saturated brine (50 mL × 3). The organic layer was dried over anhydrous sodium sulfate, filtered, and concentrated under reduced pressure. The resulting residue was purified by silica gel column chromatography (MeOH/DCM = 0-100%) to afford **7h** (1.25 g) in 58% yield.
MS-ESI: Calcd for $[M+Na]^+$ 697, Found 697.

Step 6

Under a nitrogen atmosphere, zinc bromide (8.00 g, 35.60 mmol) was charged into a reaction flask and dissolved in nitromethane (15 mL). The mixture was stirred at room temperature for 10 minutes, forming a milky suspension. The flask was then immersed in an ice bath, and a solution of **7h** (1.20 g, 1.78 mmol) in nitromethane (20 mL) was added dropwise. After complete addition, the reaction mixture was maintained in the ice bath with continued stirring for 4 h. The reaction was quenched by addition of water (50 mL), and the aqueous phase was adjusted to pH 5-6 using aqueous ammonia solution (2% w/w). The layers were separated, and the aqueous phase was washed with dichloromethane (50 mL × 3). The aqueous layer was then lyophilized, and the resulting residue was purified by silica gel column chromatography (MeOH/DCM = 0-100%) to afford **7i** (450 mg) in 41% yield.
MS-ESI: Calcd for $[M+Na]^+$ 641, Found 641.
$^1$H NMR (400 MHz, DMSO-$d_6$) δ 7.28-7.14 (m, 5H), 6.97 (s, 2H), 4.66-4.54 (m, 2H), 4.43 (dd, *J* = 9.2, 4.4 Hz, 1H), 3.81 (s, 2H), 3.77-3.52 (m, 12H), 3.07 (dd, *J* = 14.0, 4.8 Hz, 1H), 2.81 (dd, *J* = 13.6, 9.6 Hz, 1H), 2.33 (t, *J* = 6.0 Hz, 2H).

Step 7

**1d** (50 mg, 0.11 mmol), **7i** (74 mg, 0.12 mmol), 1-(3-Dimethylaminopropyl)-3-ethylcarbodiimide hydrochloride (31 mg, 0.16 mmol) and *N*,*N*-Dimethylformamide (2 mL) were added sequentially to a 10 mL round-bottom flask. The mixture was cooled in an ice bath, and 2,4,6-collidine (60 mg, 0.49 mmol) was added dropwise. After complete addition, the reaction was allowed to warm to room temperature and stirred for 3 h. The reaction mixture was then slowly added dropwise to methyl *tert*-butyl ether (60 mL), resulting in precipitation of a solid. The solid was collected by filtration and dissolved in a dichloromethane/methanol (24 mL/4 mL) mixture. The solution was concentrated to afford the crude

product, which was further purified by preparative HPLC to afford 7 (16 mg) in 17% yield.

MS-ESI: Calcd for $[M+H]^+$ 1052, Found 1052.

$^1H$ NMR (400 MHz, DMSO-$d_6$) δ 8.65 (t, *J* = 6.8 Hz, 1H), 8.61 (d, *J* = 9.2 Hz, 1H), 8.28 (t, *J* = 4.4 Hz, 1H), 8.14-8.06 (m, 2H), 8.06 (t, *J* = 5.6 Hz, 1H), 7.82 (d, *J* = 11.2 Hz, 1H), 7.79 (s, 1H), 7.28-7.13 (m, 5H), 6.99 (s, 2H), 5.92 (d, *J*= 16.8 Hz, 1H), 5.70-5,60 (m, 1H), 5.53 (d, *J* = 8.0 Hz, 1H), 5.48 (d, *J* = 4.8 Hz, 1H), 5.37 (d, *J* = 19.6 Hz, 1H), 4.68 (d, *J* = 6.4 Hz, 1H), 4.51-4.41 (m, 1H), 4.17-4.04 (m, 2H), 3.72-3.68 (m, 2H), 3.68-3.63 (m, 2H), 3.62-3.58 (m, 1H), 3.58-3.54 (m, 3H), 3.54-3.39 (m, 3H), 3.48-3.43 (m, 2H), 3.19-3.08 (m, 1H), 3.06-2.97 (m, 1H), 2.80-2.70 (m, 1H), 2.70-2.65 (m, 1H), 2.39 (s, 3H), 2.35-2.28 (m, 3H), 2.27-2.18 (m, 2H), 1.94-1.82 (m, 2H), 0.85 (t, *J* = 7.2 Hz, 3H).

**Synthetic route 2**

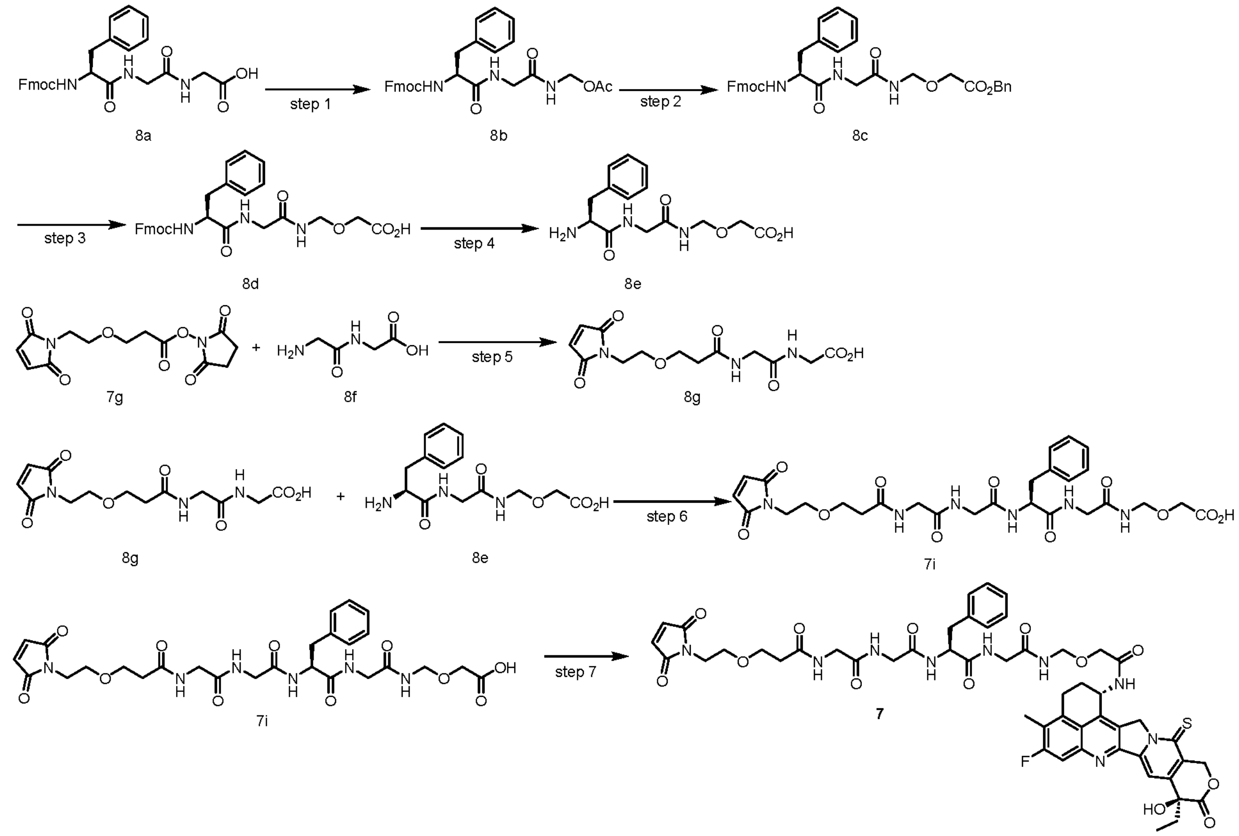


8a, 8b, 8c, 8d, 8e, 7g, 8f, 8g, 8g, 8e, 7i, 7i, 7

Step 1

Under a nitrogen atmosphere, **8a** (4.50 g, 8.98 mmol) was dissolved in anhydrous *N,N*-dimethylformamide (25 mL). Acetic acid (1.19 g, 19.76 mmol), lead(IV) acetate (7.96 g, 17.96 mmol), and copper(II) acetate (1.10 g, 8.98 mmol) were then added sequentially. The reaction mixture was heated to 50°C and stirred for 0.5 h. The reaction was quenched by adding water (50 mL), and the aqueous phase was extracted with ethyl acetate (500 mL). The organic layer was separated and washed sequentially with saturated sodium bicarbonate solution (100 mL × 1), water (100 mL × 1), and saturated brine (50 mL × 4), dried over anhydrous sodium sulfate and filtrated. The filtrate was concentrated under reduced pressure to afford the crude product **8b** (4.70 g).

MS-ESI: Calcd for $[M+Na]^+$ 538, Found 538.

Step 2

Under nitrogen atmosphere, **8b** (4.70 g, 9.13 mmol) was dissolved in anhydrous tetrahydrofuran (40 mL). The solution was cooled in an ice bath, and p-toluenesulfonic acid (235 mg, 1.37 mmol) followed by benzyl glycolate (5.30 g, 31.96 mmol) were added sequentially. After complete addition, the reaction mixture was allowed to warm to room temperature and stirred for 4 hours. The reaction mixture was then diluted with ethyl acetate (200 mL), and the organic

phase was washed sequentially with water (50 mL × 1), saturated sodium bicarbonate solution (50 mL × 1), and saturated brine (50 mL × 2), dried over anhydrous sodium sulfate and filtrated. The filtrate was concentrated under reduced pressure. The residue was purified by silica gel column chromatography (EA/hexanes = 0-100%) to afford **8c** (4.50 g) in 79% yield.

MS-ESI: Calcd for $[M+Na]^+$ 641, Found 641.

Step 3

**8c** (4.50 g, 7.25 mmol) was dissolved in the mixed solvent of THF/water (50 mL/15 mL). To this solution was added wet Pd/C (380 mg, 8% W/W). The reaction mixture was stirred under a hydrogen atmosphere overnight. The reaction mixture was filtered to remove insoluble solid. The obtained filtrate was concentrated under reduced pressure to afford crude product **8d** (2.65 g).

MS-ESI: calcd for $[M+Na]^+$ 554, found 554.

Step 4

Under a nitrogen atmosphere, **8d** (2.65 g, 4.99 mmol) was dissolved in anhydrous acetonitrile (30 mL). The solution was cooled in an ice bath, and 1,8-diazabicyclo[5.4.0]undec-7-ene (1.52 g, 9.98 mmol) was added. After complete addition, the reaction mixture was allowed to warm to room temperature and stirred for 5 hours. The reaction mixture was then cooled to 0°C, and 1-hydroxybenzotriazole (1.35 g, 9.98 mmol) was added portionwise. The reaction mixture precipitated solids during this process. The mixture was maintained at 0°C with continued stirring for an additional 2 hours before filtration. The filter cake was collected and dried under vacuum to afford the crude product **8e** (1.85 g).

MS-ESI: calcd for $[M+H]^+$ 310, found 310.

Step 5

**7g** (3.10 g, 10.00 mmol) and **8f** (1.45 g, 11.00 mmol) were dissolved in a mixed solvent of acetonitrile/water (25 mL/8 mL). Triethylamine (1.51 g, 15.00 mmol) was added dropwise under an ice bath. After complete addition, the reaction mixture was allowed to warm to room temperature and stirred for 2 h. The majority of the acetonitrile was removed under reduced pressure, and the residue was diluted with dichloromethane/water (100 mL/50 mL). The layers were separated, and the aqueous phase was washed with dichloromethane (50 mL × 2) and then lyophilized. The resulting residue was purified by silica gel column chromatography (MeOH/DCM = 0-100%) to afford the crude product **8g** (4.25 g).

MS-ESI: calcd for $[M+H]^+$ 328, found 328.

Step 6

**8g** (1.03 g) and N-hydroxysuccinimide (552 mg, 4.80 mmol) were dissolved in anhydrous dichloromethane (10 mL). N,N'-Dicyclohexylcarbodiimide (0.99 g, 4.80 mmol) was added dropwise under an ice bath. After complete addition, the reaction mixture was allowed to warm to room temperature and stirred for 1.5 h. The mixture was filtered to remove insoluble solids, and the filtrate was retained for the next step. To the filtrate, 8e (0.52 g) and 2,4,6-Collidine (203 mg, 1.68 mmol) were added under an ice bath. The resulting mixture was warmed to room temperature and stirred for 4 h. The reaction was quenched with water (50 mL), and the aqueous phase was washed with dichloromethane (50 mL × 2) and then lyophilized. The crude residue was purified by silica gel column chromatography (MeOH/DCM = 0-100%) to afford **7i** (630 mg).

MS-ESI: calcd for $[M+Na]^+$ 641, found 641.

Step 7

**1d** (110 mg, 0.24 mmol) and **7i** (195 mg, 0.32 mmol) were successively added to a 10 mL round-bottom flask, followed by anhydrous N,N-dimethylformamide (3 mL). The mixture was cooled to 0°C, then treated sequentially with dropwise additions of trifluoroacetic acid (29 mg, 0.25 mmol), 2,4,6-Collidine (133 mg, 1.10 mmol), and 1-ethyl-3-(3-dimethylaminopropyl)carbodiimide hydrochloride (68 mg, 0.35 mmol). After complete addition, the reaction mixture was allowed to warm to room temperature and stirred for 3 h. The crude mixture was directly purified by silica gel column chromatography (MeOH/DCM = 0-100%) to afford compound **7** (86 mg) in 34% yield.

MS-ESI: calcd for $[M+H]^+$ 1052, found 1052.

**Synthetic route 3**

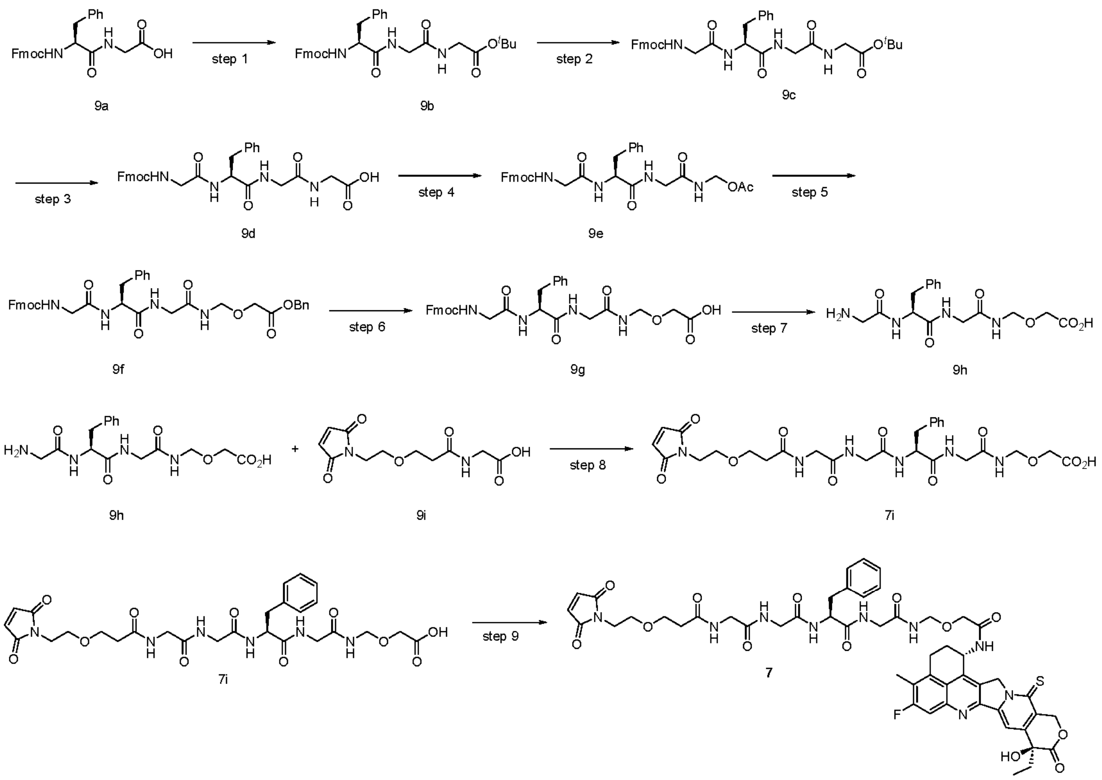


9a 9b 9c 9d 9e 9f 9g 9h 9h 9i 7i 7i 7

Step 1

Under a nitrogen atmosphere, **9a** (5.10 g, 11.48 mmol) was dissolved in anhydrous dichloromethane (100 mL), followed by sequential addition of 2-(7-azabenzotriazol-1-yl)-N,N,N',N'-tetramethyluronium hexafluorophosphate (6.54 g, 17.22 mmol), N,N-diisopropylethylamine (4.44 g, 34.44 mmol), and glycine *tert*-butyl ester (1.80 g, 13.78 mmol). After complete addition, the reaction mixture was stirred at room temperature for 2 h. The mixture was then diluted with dichloromethane (100 mL), and the organic phase was washed sequentially with dilute hydrochloric acid (2 N, 50 mL × 1), water (50 mL × 1), and saturated brine (50 mL × 2). The organic layer was dried, filtered, and concentrated under reduced pressure to afford the crude product **9b** (6.80 g) as a white solid.

MS-ESI: calcd for $[M+H]^+$ 558, found 558.

Step 2

Under a nitrogen atmosphere, the crude product **9b** (6.30 g) was dissolved in anhydrous acetonitrile (100 mL) and cooled in an ice bath. 1,8-Diazabicyclo[5.4.0]undec-7-ene (DBU, 3.44 g, 22.62 mmol) was added, and the reaction mixture was allowed to warm to room temperature and stirred for 5 h. The flask was then recooled in an ice bath, followed by sequential addition of Fmoc-glycine (4.03 g, 13.57 mmol), 2-(7-azabenzotriazol-1-yl)-N,N,N',N'-tetramethyluronium hexafluorophosphate (5.16 g, 13.57 mmol), and N,N-diisopropylethylamine (2.19 g, 16.97 mmol). After complete addition, the mixture was stirred at room temperature for an additional 1 h. The reaction was quenched by dilution with ethyl acetate (200 mL), and the organic phase was washed sequentially with dilute hydrochloric acid (2 N, 50 mL × 1), water (50 mL × 2), and saturated brine (50 mL × 2). The organic layer was dried over anhydrous $Na_2SO_4$, filtered, and concentrated under reduced pressure. The resulting residue was purified by silica gel column chromatography (MeOH/DCM = 0-100%) to afford the crude product **9c** (8.5 g).

MS-ESI: calcd for $[M+H]^+$ 615, found 615.

Step 3

Under nitrogen atmosphere, **9c** (8.50 g, 13.83 mmol) was dissolved in anhydrous dichloromethane (20 mL) and

cooled to 0°C in an ice-water bath. Trifluoroacetic acid (20 mL, 0.261 mol) was added dropwise, and the reaction mixture was allowed to warm to room temperature naturally and stirred for 4 hours. After concentration under reduced pressure, the residue was treated with ethyl acetate (100 mL) and water (100 mL). The pH of the solution was adjusted to 4-5 with saturated sodium bicarbonate solution (resulting in solid precipitation and formation of an emulsion in the aqueous phase). The layers were separated, and the aqueous emulsion was collected. The aqueous phase was washed with ethyl acetate (50 mL × 2), then acidified to pH 1-2 with 1N hydrochloric acid solution. Dichloromethane (200 mL) was added, and the layers were separated. The aqueous phase was further extracted with dichloromethane (150 mL × 2). The combined organic phases were dried over anhydrous sodium sulfate, filtered, and concentrated under reduced pressure to afford the crude product **9d** (4.10 g) as a white solid.

MS-ESI: calcd for $[M+H]^+$ 559, found 559.

Step 4

Under nitrogen atmosphere, a reaction flask was charged sequentially with **9d** (4.10 g, 7.34 mmol), acetic acid (1.78 g, 29.35 mmol), lead tetraacetate (13.00 g, 17.96 mmol), and copper acetate (1.80 g, 14.68 mmol). The reaction mixture was heated to 50°C and stirred for 0.5 hour. The reaction was quenched by addition of water (50 mL), and the aqueous phase was extracted with dichloromethane (500 mL). The layers were separated, and the organic phase was washed sequentially with saturated sodium bicarbonate solution (100 mL × 1), water (100 mL), and saturated brine (50 mL × 4). After drying over anhydrous sodium sulfate, the solution was filtered and concentrated under reduced pressure to afford the crude product **9e** (3.84 g).

MS-ESI: calcd for $[M+H]^+$ 595, found 595.

Step 5

Under a nitrogen atmosphere, **9e** (3.80 g, 6.60 mmol) was dissolved in anhydrous tetrahydrofuran (40 mL) and cooled in an ice bath. p-Toluenesulfonic acid (171 mg, 1.00 mmol) and benzyl glycolate (3.86 g, 25.20 mmol) were added sequentially. After complete addition, the reaction mixture was allowed to warm to room temperature and stirred for 4 h. The reaction was quenched by dilution with ethyl acetate (200 mL), and the organic phase was washed successively with water (50 mL × 1), saturated sodium bicarbonate solution (50 mL × 1), and saturated brine (50 mL × 2). The organic layer was dried over anhydrous sodium sulfate, filtered, and concentrated under reduced pressure. The residue was purified by silica gel column chromatography (MeOH/DCM = 0-100%) to afford **9f** (2.14 g) in 48% yield.

MS-ESI: calcd for $[M+Na]^+$ 701, found 701.

Step 6

**9f** (2.10 g, 3.09 mmol) was dissolved in the mixed solvent of THF/water (50 mL/15 mL). To this solution was added wet Pd/C (168 mg, 8% W/W). The reaction mixture was stirred under a hydrogen atmosphere overnight. The reaction mixture was filtered to remove insoluble solids. The obtained filtrate was concentrated under reduced pressure to afford crude product **9g** (2.40 g).

MS-ESI: calcd for $[M+Na]^+$ 611, found 611.

Step 7

Under nitrogen atmosphere, **9g** (2.18 g, 3.70 mmol) was dissolved in anhydrous acetonitrile (30 mL) and cooled in an ice bath. 1,8-Diazabicyclo[5.4.0]undec-7-ene (1.13 g, 7.41 mmol) was added, and the reaction mixture was allowed to warm to room temperature and stirred for 5 h. The reaction was then cooled to 0°C, and 1-hydroxybenzotriazole (1.35 g, 9.98 mmol) was added portionwise, resulting in precipitation of solids. Additional acetonitrile (30 mL) was added, and the slurry was stirred at room temperature for 2 h. The mixture was filtered, and the filter cake was washed with acetonitrile (20 mL × 2) and dried under vacuum to afford the crude product **9h** (1.83 g) as a white powder.

MS-ESI: calcd for $[M+H]^+$ 367, found 367.

Step 8

**9i** (500 mg, 1.43 mmol, molar ratio: **9i**:2,4,6-Collidine = 1:0.655), 2,4,6-Collidine (494 mg, 4.08 mmol), and N-hydroxysuccinimide (206 mg, 1.79 mmol) were dissolved in anhydrous dichloromethane (25 mL). Under an ice bath, *N,N'*-dicyclohexylcarbodiimide (443 mg, 2.15 mmol) was added. After complete addition, the reaction mixture was stirred at 0°C for 3 h. Subsequently, **9h** (600 mg, 1.60 mmol) was added under ice cooling, and the resulting mixture was warmed to room temperature and stirred overnight. The reaction was quenched with water (100 mL), and the aqueous phase was washed

with dichloromethane (100 mL × 3). The aqueous layer was lyophilized, and the residue was purified by silica gel column chromatography (MeOH/DCM = 0-50%) to afford **7i** (550 mg) in 62% yield.

MS-ESI: calcd for $[M+Na]^+$ 641, found 641.

Step 9

**1d** (110 mg, 0.24 mmol) and **7i** (195 mg, 0.32 mmol) were successively added to a 10 mL round-bottom flask, followed by anhydrous *N*,*N*-dimethylformamide (3 mL). The mixture was cooled to 0°C, then treated sequentially with dropwise additions of trifluoroacetic acid (29 mg, 0.25 mmol), 2,4,6-Collidine (133 mg, 1.10 mmol), and 1-ethyl-3-(3-dimethylaminopropyl)carbodiimide hydrochloride (68 mg, 0.35 mmol). After complete addition, the reaction mixture was allowed to warm to room temperature and stirred for 3 h. The crude mixture was directly purified by silica gel column chromatography (MeOH/DCM = 0-100%) to afford 7 (90 mg) in 35% yield.

MS-ESI: calcd for $[M+H]^+$ 1052, found 1052.

**Example 1.8**

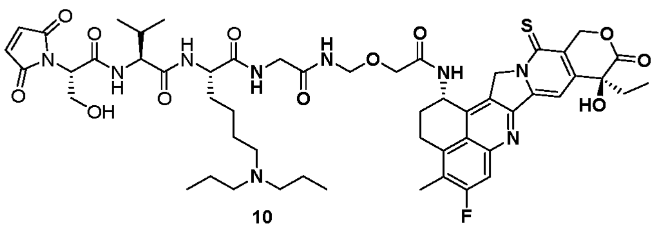

**10**

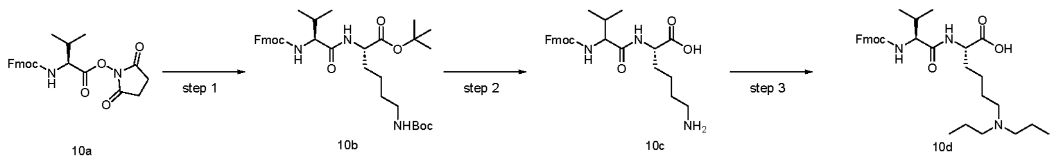


10a 10b 10c 10d

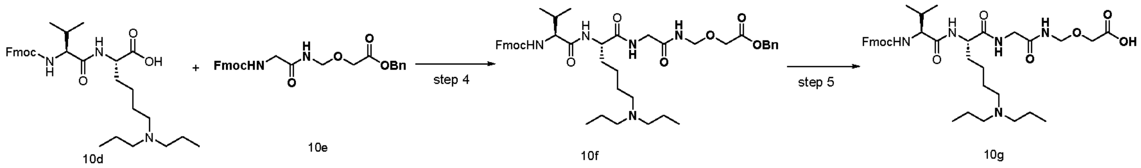


10d 10e 10f 10g

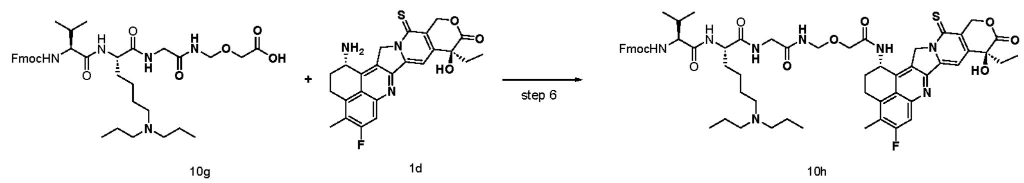


10g 1d 10h

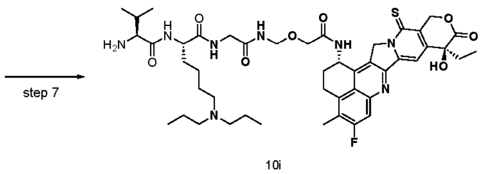


10i

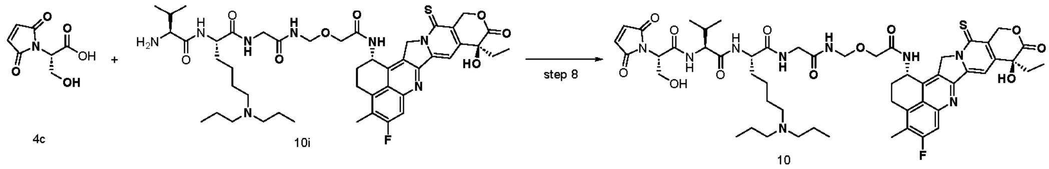


4c 10i 10

Step 1

Under nitrogen protection, **10a** (10 g, 22.91 mmol) and N6-Boc-L-lysine *tert*-butyl ester hydrochloride (9.32 g, 27.50 mmol) were dissolved in a mixed solvent of tetrahydrofuran/water ($V_{THF}$ : $V_{H2O}$ = 4:1, 100 mL). The mixture was cooled to 0°C and stirred for 10 minutes, followed by addition of sodium bicarbonate (3.85 g, 45.83 mmol). The reaction was maintained at 0°C with stirring for 2 hours. The mixture was then diluted with water (100 mL) and ethyl acetate (100 mL), and the layers were separated. The aqueous phase was extracted with ethyl acetate (100 mL × 2). The combined organic phases were dried over anhydrous sodium sulfate, filtered, and concentrated under reduced pressure. The residue was purified by silica gel column chromatography (ethyl acetate/petroleum ether = 0-100%) to afford compound **10b** (12.50 g) in 87% yield.

MS-ESI: calcd for $[M+Na]^+$ 646, found 646.

Step 2

**10b** (12.50 g, 20.04 mmol) was dissolved in hydrogen chloride/dioxane solution (4 M, 63 mL), and the reaction mixture was stirred at room temperature for 3 h. After concentration under reduced pressure, the residue was triturated with methyl *tert*-butyl ether (150 mL) to afford the crude product **10c** (11.40 g).

MS-ESI: calcd for $[M+H]^+$ 468, found 468.

Step 3

**10c** (11.40 g) was dissolved in anhydrous dichloromethane (190 mL), followed by addition of propionaldehyde

(7.01 g, 120.70 mmol). After stirring at room temperature for 15 min, sodium triacetoxyborohydride (21.33 g, 100.64 mmol) was added portionwise. The reaction mixture was stirred at room temperature for an additional 1 h, then quenched with saturated aqueous ammonium chloride (10 mL) and stirred for 1 h. The mixture was diluted with water (200 mL) and ethyl acetate (100 mL), and the layers were separated. The aqueous phase was extracted with ethyl acetate (100 mL × 2), and the combined organic phases were dried over anhydrous sodium sulfate, filtered, and concentrated under reduced pressure. The residue was purified by silica gel column chromatography (MeOH/DCM = 0-100%) to afford **10d** (9.40 g) in 85% yield over two steps.

MS-ESI: calcd for $[M+H]^+$ 552, found 552.

Step 4

Under nitrogen protection, **10e** (2.5 g, 5.27 mmol) was dissolved in anhydrous dichloromethane (35 mL), followed by addition of 1,8-diazabicyclo[5.4.0]undec-7-ene (1.20 g, 7.88 mmol). The reaction mixture was cooled to 0°C and stirred for 10 min, then warmed to room temperature and stirred for an additional 1 h. The mixture was recooled to 0°C, and **10d** (3.49 g, 6.33 mmol), N,N-diisopropylethylamine (1.02 g, 7.89 mmol), and 2-(7-azabenzotriazol-1-yl)-N,N,N',N'-tetramethyluronium hexafluorophosphate (3.01 g, 7.92 mmol) were added sequentially. After complete addition, the reaction was stirred at 0°C for 1 h. The mixture was then diluted with water (50 mL) and ethyl acetate (50 mL), and the layers were separated. The aqueous phase was extracted with ethyl acetate (100 mL × 2), and the combined organic phases were dried over anhydrous sodium sulfate, filtered, and concentrated under reduced pressure. The residue was purified by silica gel column chromatography (MeOH/DCM = 0-100%) to afford **10f** (2.00 g) in 48% yield.

MS-ESI: calcd for $[M+H]^+$ 786, found 786.

Step 5

**10f** (2.00 g, 2.54 mmol) was dissolved in a mixed solvent of tetrahydrofuran/water ($V_{tetrahydrofuran}$ : $V_{water}$ = 3:1, 40 mL), followed by addition of 10% wet palladium on carbon (200 mg). The reaction mixture was stirred under a hydrogen atmosphere at room temperature for 3 h. The mixture was then filtered to remove insoluble solids, and the filter cake was washed with ethyl acetate. The filtrate was concentrated under reduced pressure to afford an aqueous residue, which was extracted with ethyl acetate (20 mL × 3). The combined organic phases were dried over anhydrous sodium sulfate, filtered, and concentrated under reduced pressure. The residue was purified by silica gel column chromatography (MeOH/DCM = 0-100%) to afford **10g** (930 mg) in 53% yield.

MS-ESI: calcd for $[M+H]^+$ 696, found 696.

Step 6

Under nitrogen atmosphere, **10g** (652 mg, 0.94 mmol) and **1d** (330 mg, 0.73 mmol) were dissolved in anhydrous dichloromethane (5 mL). The mixture was cooled to 0°C, followed by addition of N,N-diisopropylethylamine (189 mg, 1.46 mmol) and 2-(7-azabenzotriazol-1-yl)-N,N,N',N'-tetramethyluronium hexafluorophosphate (330 mg, 0.87 mmol). The reaction mixture was stirred at 0°C for 1 h. After concentration under reduced pressure, the residue was purified by silica gel column chromatography (MeOH/DCM = 0-100%) to afford **10h** (730 mg) in 88% yield.

MS-ESI: calcd for $[M+H]^+$ 1129, found 1129.

Step 7

Under nitrogen protection, **10h** (720 mg, 0.64 mmol) was dissolved in anhydrous tetrahydrofuran (7 mL) and cooled to 0°C. Piperidine (272 mg, 3.19 mmol) was then added, and the reaction mixture was stirred at 0°C for 1 h. After concentration under reduced pressure, the residue was purified by silica gel column chromatography (MeOH/DCM = 0-100%) to afford **10i** (350 mg) in 61% yield.

MS-ESI: calcd for $[M+H]^+$ 907, found 907.

$^1H$ NMR (400 MHz, DMSO-$d_6$) δ 7.78 (s, 1H), 7.72 (d, *J* = 10.8 Hz, 1H), 5.91 (d, *J* = 16.4 Hz, 1H), 5.70-5.60 (m, 1H), 5.49 (d, *J* = 16.4 Hz, 1H), 4.74-4.62 (m, 2H), 4.24-4.15 (m, 1H), 4.13 (d, *J* = 6.0 Hz, 2H), 3.81-3.61 (m, 2H), 3.34-3.20 (m, 1H), 3.18 (s, 2H), 3.16-3.07 (m, 1H), 3.00 (d, *J* = 4.8 Hz, 1H), 2.93-2.81 (m, 2H), 2.37 (s, 3H), 2.31-2.21 (m, 5H), 1.99-1.83 (m, 3H), 1.67-1.46 (m, 4H), 1.39-1.22 (m, 8H), 0.91-0.82 (m, 6H), 0.82 (t, *J* = 7.2 Hz, 6H), 0.76 (d, *J* = 6.8 Hz, 3H).

Step 8

**10i** (150 mg, 0.17 mmol) was dissolved in anhydrous *N*,*N*-dimethylformamide (2 mL), followed by addition of 2,4,6-collidine (60 mg, 0.50 mmol) and 2-(7-azabenzotriazol-1-yl)-N,N,N',N'-tetramethyluronium hexafluorophosphate

(94 mg, 0.25 mmol). The reaction mixture was maintained at 0°C with stirring for 5 min, then **4c** (57 mg, 0.20 mmol) was added. After complete addition, the reaction was stirred at 0°C for 1 h. The mixture was diluted with water (5 mL) and ethyl acetate (10 mL), and the layers were separated. The aqueous phase was extracted with ethyl acetate (10 mL × 2), and the combined organic phases were dried over anhydrous sodium sulfate, filtered, and concentrated under reduced pressure. The residue was purified by preparative HPLC to afford compound **10** (125 mg) in 67% yield.

MS-ESI: calcd for $[M+H]^+$ 1074, found 1074.

**Example 1.9**

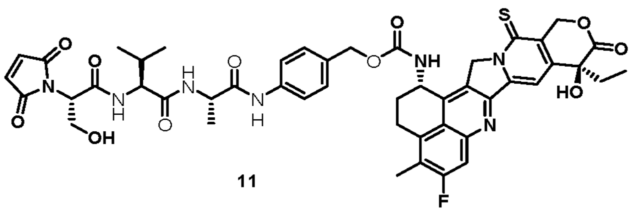

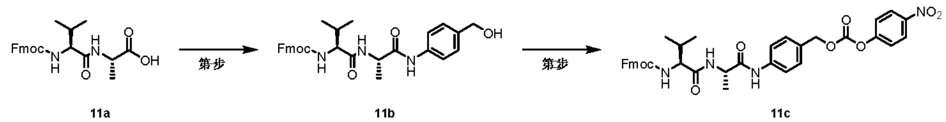


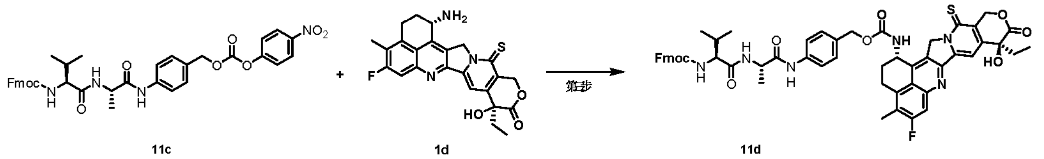

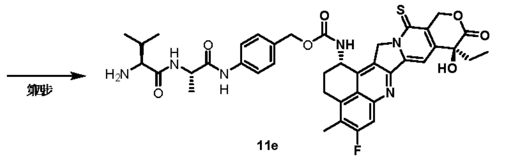

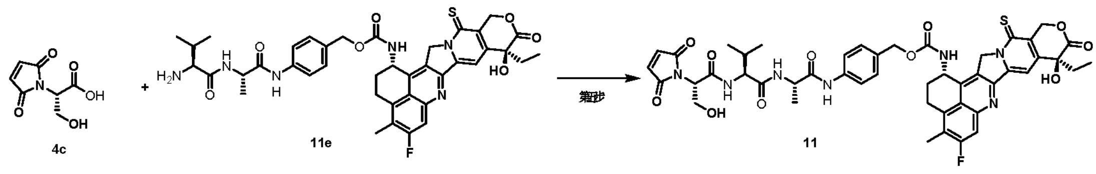

Step 1

Under nitrogen protection, **11a** (5.00 g, 12.18 mmol) and 4-aminobenzyl alcohol (2.25 g, 18.27 mmol) were dissolved in anhydrous dichloromethane (100 mL). The mixture was cooled to 0°C, followed by addition of N,N-diisopropylethylamine (3.94 g, 30.49 mmol) and 2-(7-azabenzotriazol-1-yl)-N,N,N',N'-tetramethyluronium hexafluorophosphate (5.56 g, 14.62 mmol). After complete addition, the reaction was stirred at 0°C for 1 h. The mixture was then diluted with water (200 mL) and a mixed solvent of dichloromethane/methanol (10:1, 300 mL), and the layers were separated. The aqueous phase was extracted with dichloromethane/methanol (10:1, 300 mL × 2), and the combined organic phases were dried over anhydrous sodium sulfate, filtered, and concentrated under reduced pressure. The residue was purified by silica gel column chromatography (MeOH/DCM = 0-100%) to afford **11b** (6.68 g) in quantitative yield.

MS-ESI: calcd for $[M-OH]^+$ 498, found 498.

Step 2

Under nitrogen protection, **11b** (3.23 g, 6.26 mmol) and bis(4-nitrophenyl) carbonate (2.86 g, 9.40 mmol) were dissolved in a mixed solvent of anhydrous dichloromethane/N,N-dimethylformamide (2:1, 48 mL). N,N-Diisopropylethylamine (2.43 g, 18.80 mmol) was added, and the reaction mixture was stirred at room temperature for 12 h. The reaction was quenched with water (100 mL), followed by addition of ethyl acetate (100 mL). The layers were separated, and the aqueous phase was extracted with ethyl acetate (100 mL × 2). The combined organic phases were dried over anhydrous sodium sulfate, filtered, and concentrated under reduced pressure. The residue was purified by trituration with methyl tert-butyl ether (10 mL) to afford **11c** (2.00 g) in 47% yield.

MS-ESI: calcd for $[M+Na]^+$ 703, found 703.

Step 3

Under nitrogen protection, **11c** (1.27 g, 1.87 mmol) and **1d** (430 mg, 0.95 mmol) were dissolved in anhydrous N,N-dimethylformamide (12 mL). The mixture was cooled to 0°C, followed by dropwise addition of triethylamine (3 drops). After stirring at 0°C for 15 min, 1-hydroxybenzotriazole (299 mg, 2.21 mmol) and pyridine (2.15 g, 27.18 mmol) were added. The reaction mixture was maintained at 0°C for an additional 15 min, then warmed to room temperature and stirred for 2 h. The reaction was quenched with water (20 mL) and ethyl acetate (30 mL), and the layers were separated. The aqueous phase was extracted with ethyl acetate (30 mL × 2), and the combined organic phases were dried over anhydrous sodium sulfate, filtered, and concentrated under reduced pressure. The residue was purified by silica gel column chromatography (MeOH/DCM = 0-100%) to afford **11d** (550 mg) in 58% yield.

MS-ESI: calcd for $[M+H]^+$ 993, found 993.

Step 4

**11d** (500 mg, 0.50 mmol) was dissolved in 1,4-dioxane (5 mL), followed by addition of piperidine (5 mL). The reaction mixture was stirred at room temperature for 2 h. Petroleum ether (5 mL) was then added dropwise, resulting in significant precipitation. The solid was collected by filtration and dried to afford **11e** (386 mg) in 94% yield.

MS-ESI: calcd for $[M+H]^+$ 771, found 771.

$^1$H NMR (400 MHz, DMSO-$d_6$) δ 7.76 (s, 1H), 7.66 (d, *J* = 10.4 Hz, 1H), 7.60 (d, *J* = 8.0 Hz, 2H), 7.43 (d, *J* = 8.4 Hz, 2H), 5.91 (d, *J* = 16.4 Hz, 1H), 5.49 (d, *J* = 16.4 Hz, 1H), 5.29 (q, *J* = 4.8 Hz, 1H), 5.20-5.02 (m, 2H), 4.50-4.39 (m, 1H), 3.30-3.14 (m, 1H), 3.14-3.02 (m, 1H), 2.99 (d, *J* = 5.2 Hz, 1H), 2.31 (s, 3H), 2.30-2.21 (m, 1H), 2.13-1.98 (m, 1H), 1.98-1.80 (m, 3H), 1.30 (d, *J* = 7.2 Hz, 3H), 0.88 (d, *J* = 6.8 Hz, 3H), 0.84 (t, *J* = 7.2 Hz, 3H), 0.78 (d, *J* = 6.8 Hz, 3H).

Step 5

**11e** (150 mg, 0.20 mmol) was dissolved in anhydrous *N,N*-dimethylformamide (2 mL), followed by addition of 2,4,6-Collidine (71 mg, 0.59 mmol) and 2-(7-azabenzotriazol-1-yl)-N,N,N',N'-tetramethyluronium hexafluorophosphate (111 mg, 0.29 mmol). The mixture was stirred at 0°C for 5 min, then **4c** (67 mg, 0.23 mmol) was added. After complete addition, the reaction was stirred at 0°C for 1 h. The mixture was diluted with water (5 mL) and ethyl acetate (10 mL), and the layers were separated. The aqueous phase was extracted with ethyl acetate (10 mL × 2), and the combined organic phases were dried over anhydrous sodium sulfate, filtered, and concentrated under reduced pressure. The residue was purified by prep-HPLC to afford compound **11** (38 mg) in 21% yield.

MS-ESI: calcd for $[M+H]^+$ 938, found 938.

$^1$H NMR (400 MHz, DMSO-$d_6$) δ 8.56-8.00 (m, 3H), 7.78 (s, 1H), 7.72 (d, *J* = 10.8 Hz, 1H), 7.59 (d, *J* = 8.0 Hz, 2H), 7.42 (d, *J* = 8.0 Hz, 2H), 7.02 (s, 2H), 5.92 (d, *J* = 16.4 Hz, 1H), 5.65-5.42 (m, 2H), 5.42-5.26 (m, 2H), 5.23-4.97 (m, 2H), 4.68-4.51 (m, 1H), 4.41-4.27 (m, 1H), 4.23-4.11 (m, 1H), 4.04-3.94 (m, 1H), 3.92-3.77 (m, 1H), 3.31-3.18 (m, 1H), 3.16-2.92 (m, 1H), 2.35 (s, 3H), 2.30-2.20 (m, 1H), 2.15-2.02 (m, 1H), 2.03-1.91 (m, 1H), 1.92-1.82 (m, 2H), 1.31-1.26 m, 3H), 0.86 (t, *J* = 6.8 Hz, 3H), 0.83-0.78 (m, 3H), 0.77 (d, *J* = 6.8 Hz, 3H).

**Example 1.10**

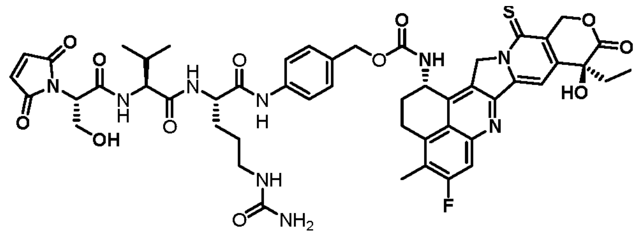

12

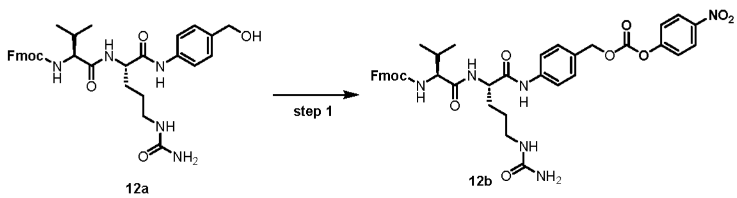


12a 12b

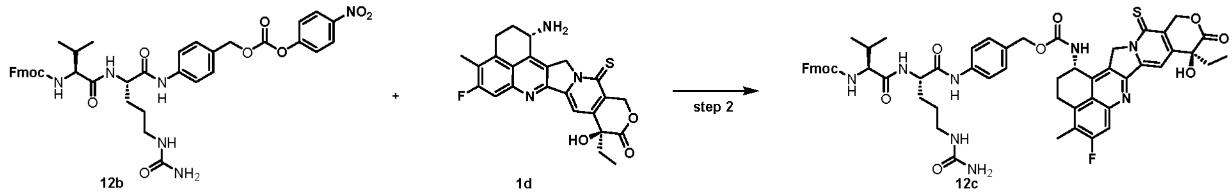


12b 1d 12c

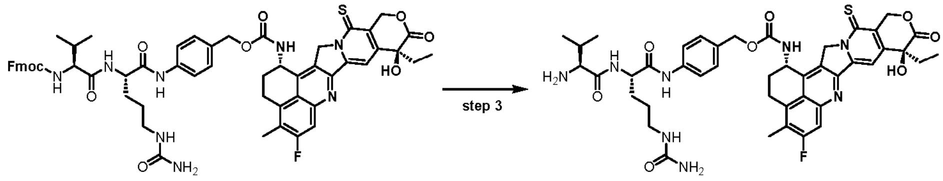


12c 12d

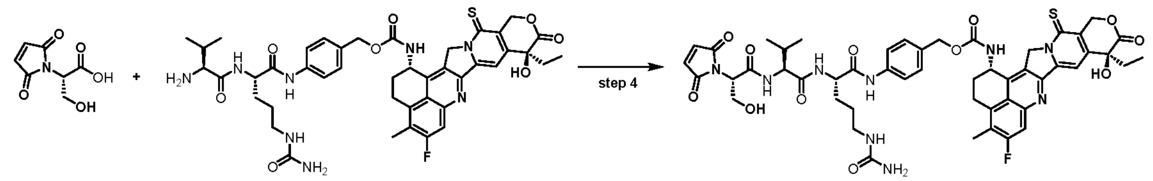


4c 12d 12

Step 1

**12a** (4.00 g, 6.65 mmol) and bis(4-nitrophenyl) carbonate (10.08 g, 33.14 mmol) were dissolved in anhydrous *N*,*N*-dimethylformamide (80 mL), followed by addition of N,N-diisopropylethylamine (2.40 g, 18.57 mmol). After complete addition, the reaction mixture was stirred at room temperature for 2 h. Ethyl acetate (120 mL) and petroleum ether (240 mL) were then added dropwise sequentially to the reaction mixture. Upon complete addition, the resulting mixture was stirred at room temperature for an additional 10 min and then filtered. The filter cake was dried to afford compound **12b** (3.30 g) in 65% yield.

MS-ESI: calcd for $[M+H]^+$ 767, found 767.

Step 2

Under nitrogen protection, **12b** (675 mg, 0.88 mmol) and **1d** (270 mg, 0.60 mmol) were dissolved in anhydrous *N*,*N*-dimethylformamide (5 mL). The mixture was cooled to 0°C, followed by dropwise addition of triethylamine (3 drops). After stirring at 0°C for 15 min, 1-hydroxybenzotriazole (189 mg, 1.40 mmol) and pyridine (1.36 g, 17.19 mmol) were added. The reaction mixture was maintained at 0°C for an additional 15 min, then warmed to room temperature and stirred for 2 h. The reaction was quenched with water (20 mL) and ethyl acetate (30 mL), and the layers were separated. The

aqueous phase was extracted with ethyl acetate (30 mL × 2), and the combined organic phases were dried over anhydrous sodium sulfate, filtered, and concentrated under reduced pressure. The residue was purified by silica gel column chromatography (MeOH/DCM = 0-100%) to afford **12c** (350 mg) in 54% yield.

MS-ESI: calcd for $[M+Na]^+$ 1101, found 1101.

Step 3

**12c** (570 mg, 0.53 mmol) was dissolved in 1,4-dioxane (10 mL), followed by addition of piperidine (450 mg, 5.28 mmol). The reaction mixture was stirred at room temperature for 3 h. To the reaction mixture was added methyl *tert*-butyl ether (15 mL), resulting in precipitation of solids. After stirring for 30 min, the mixture was filtered, and the filter cake was dried to afford compound **12d** (400 mg) in 88% yield.

MS-ESI: calcd for $[M+H]^+$ 857, found 857.

Step 4

**12d** (210 mg, 0.25 mmol) was dissolved in anhydrous *N,N*-dimethylformamide (3 mL), followed by addition of 2,4,6-collidine (89 mg, 0.73 mmol) and 2-(7-azabenzotriazol-1-yl)-N,N,N',N'-tetramethyluronium hexafluorophosphate (140 mg, 0.37 mmol). The mixture was stirred at 0°C for 5 min, then **4c** (71 mg, 0.25 mmol) was added. After complete addition, the reaction was stirred at 0°C for 1 h. The mixture was diluted with water (5 mL) and ethyl acetate (10 mL), and the layers were separated. The aqueous phase was extracted with ethyl acetate (10 mL × 2), and the combined organic phases were dried over anhydrous sodium sulfate, filtered, and concentrated under reduced pressure. The residue was purified by prep-HPLC to afford compound **12** (30 mg) in 12% yield.

MS-ESI: calcd for $[M+H]^+$ 1024, found 1024.

$^1H$ NMR (500 MHz, DMSO-$d_6$) δ 7.79 (s, 1H), 7.75 (d, *J* = 10.5 Hz, 1H), 7.59 (d, *J* = 7.5 Hz, 1H), 7.42 (d, *J* = 8.5 Hz, 1H), 7.02 (s, 2H), 5.92 (d, *J* = 17.0 Hz, 1H), 5.57 (d, *J* = 20.0 Hz, 1H), 5.51 (d, *J* = 16.5 Hz, 1H), 5.37 (d, *J* = 19.5 Hz, 1H), 5.33-5.28 (m, 1H), 5.16 (d, *J*= 12.5 Hz, 1H), 5.08 (d, *J* = 12.5 Hz, 1H), 4.60-4.54 (m, 1H), 4.37-4.30 (m, 1H), 4.20-4.14 (m, 1H), 4.00-3.93 (m, 1H), 3.88-3.81 (m, 1H), 3.30-3.21 (m, 1H), 3.15-3.06 (m, 1H), 3.05-2.89 (m, 2H), 2.36 (s, 3H), 2.31-2.23 (m, 1H), 2.14-2.03 (m, 1H), 2.02-1.93 (m, 1H), 1.92-1.82 (m, 2H), 1.74-1.64 (m, 1H), 164-1.53 (m, 1H), 1.48-1.31 (m, 2H), 0.89-0.80 (m, 6H), 0.76 (d, *J* = 6.5 Hz, 3H).

**Example 1.11**

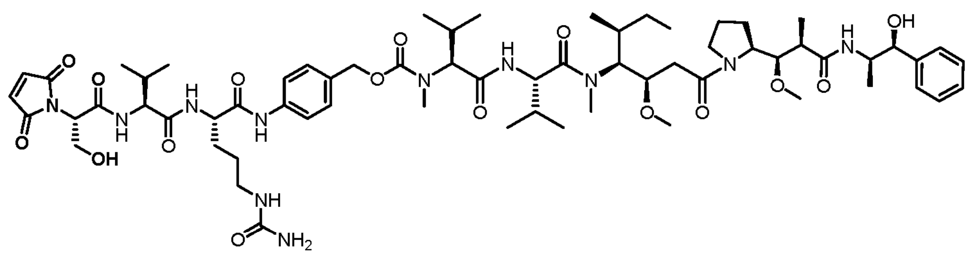

**13**

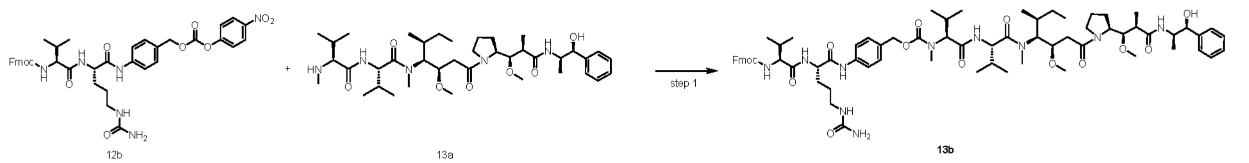


12b 13a 13b

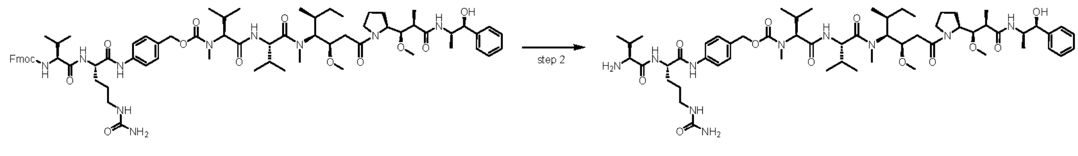


13b 13c

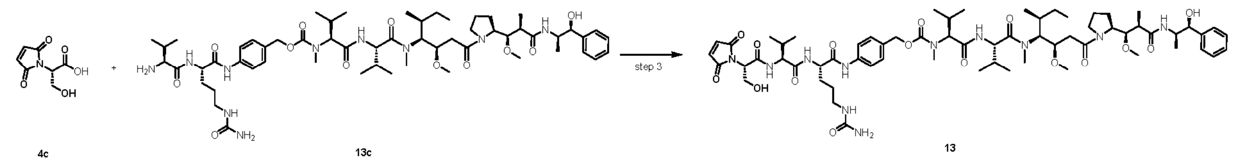


4c 13c 13

Step 1

**12b** (427 mg, 0.56 mmol) and **13a** (270 mg, 0.38 mmol) were dissolved in anhydrous *N*,*N*-dimethylformamide (15 mL), followed by addition of 1-hydroxybenzotriazole (51 mg, 0.38 mmol) and pyridine (744 mg, 9.41 mmol). The reaction mixture was stirred at room temperature for 12 h. The reaction was quenched with water (20 mL) and ethyl acetate (30 mL), and the layers were separated. The aqueous phase was extracted with ethyl acetate (30 mL × 2), and the combined organic phases were dried over anhydrous sodium sulfate, filtered, and concentrated under reduced pressure. The residue was purified by silica gel column chromatography (MeOH/DCM = 0-100%) to afford **13b** (220 mg) in 43% yield.

MS-ESI: calcd for $[(1/2M)+H]^+$ 673, found 673.

Step 2

**13b** (200 mg, 0.15 mmol) was dissolved in a mixed solvent of anhydrous tetrahydrofuran/*N*,*N*-dimethylformamide (2:1, 5.4 mL), followed by addition of piperidine (66 mg, 0.78 mmol). The reaction mixture was stirred at room temperature for 1 h. The crude product was directly purified by silica gel column chromatography (MeOH/DCM = 0-100%) to afford **13c** (110 mg) in 66% yield.

MS-ESI: calcd for $[M+H]^+$ 1123.7, found 1123.4.

Step 3

**13c** (110 mg, 0.10 mmol) was dissolved in anhydrous N,N-dimethylformamide (3 mL), followed by addition of 2,4,6-Collidine (25 mg, 0.21 mmol) and 2-(7-azabenzotriazol-1-yl)-N,N,N',N'-tetramethyluronium hexafluorophosphate (59 mg, 0.16 mmol). The mixture was stirred at 0°C for 5 min, then **4c** (55 mg, 0.19 mmol) was added. After complete addition, the reaction was stirred at 0°C for 1 h. The mixture was diluted with water (5 mL) and ethyl acetate (10 mL), and the layers were separated. The aqueous phase was extracted with ethyl acetate (10 mL × 2), and the combined organic phases were dried over anhydrous sodium sulfate, filtered, and concentrated under reduced pressure. The residue was purified by prep-HPLC to afford compound **13** (54 mg) in 43% yield.

MS-ESI: calcd for $[M+H]^+$ 1290.7, found 1290.4.

$^1$H NMR (500 MHz, DMSO-$d_6$) δ 8.05 (d, *J* = 8.5 Hz, 1H), 7.58-7.52 (m, 2H), 7.36-7.21 (m, 5H), 7.21-7.10 (m, 1H), 7.02 (s, 2H), 5.14-4.91 (m, 2H), 4.60-4.52 (m, 1H), 4.51-4.44 (m, 1H), 4.44-4.36 (m, 1H), 4.36-4.30 (m, 1H), 4.28-4.20 (m, 1H), 4.20-4.14 (m, 1H), 4.05-3.90 (m, 3H), 3.85 (dd, *J* = 11.0, 9.0 Hz, 1H), 3.59-3.51 (m, 1H), 3.25 - 3.20 (m, 3H), 3.21 - 3.15 (m, 3H), 3.10 (s, 1H), 3.07-2.90 (m, 3H), 2.88-2.80 (m, 2H), 2.43-2.34 (m, 1H), 2.30-2.20 (m, 1H), 2.16-1.88 (m, 4H), 1.85-1.63 (m, 4H), 1.62-1.22 (m, 6H), 1.06-0.96 (m, 6H), 0.89-0.86 (m, 2H), 0.85-0.80 (m, 10H), 0.80-0.70 (m, 12H).

**Example 1.12**

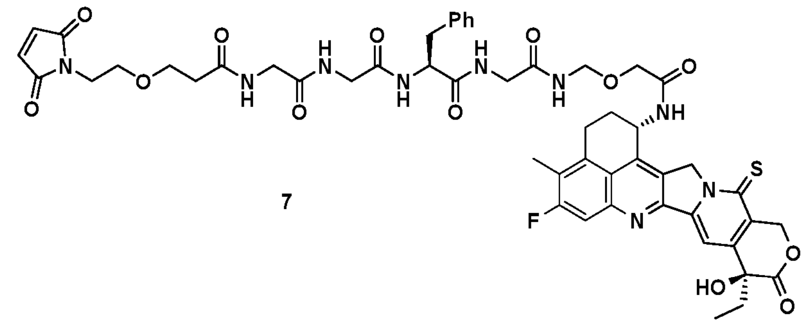

7

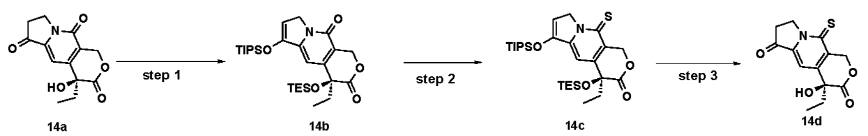


14a 14b 14c 14d

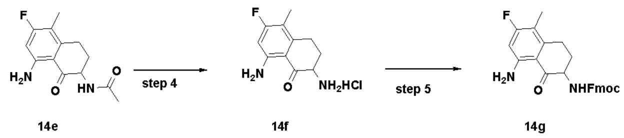


14e 14f 14g

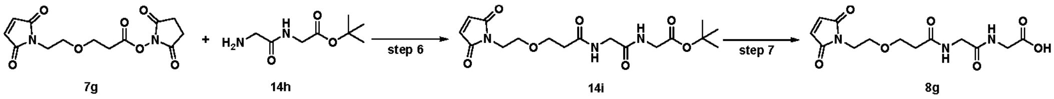


7g 14h 14i 8g

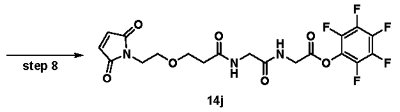


14j

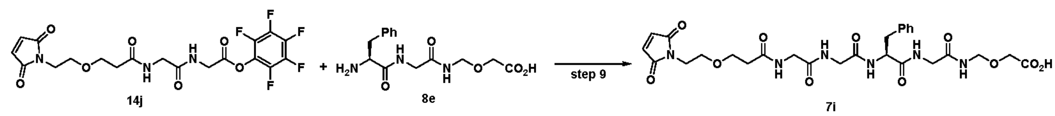


14j 8e 7i

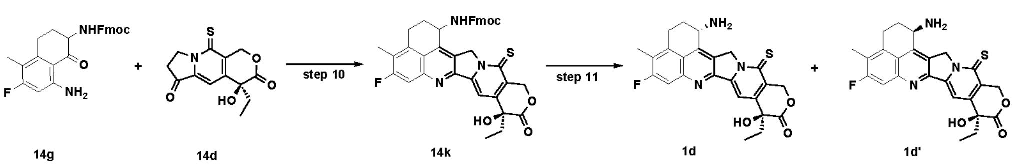


14g 14d 14k 1d 1d'

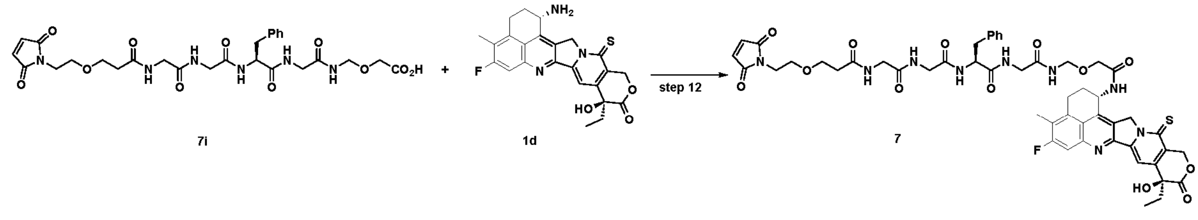


7i 1d 7

Step 1

Under nitrogen protection, imidazole (161.60 g, 2373.68 mmol) was dissolved in anhydrous *N*,*N*-dimethylformamide (1.25 L), and the mixture was cooled to 0±5°C. Triisopropylsilyl chloride (458.20 g, 2376.56 mmol) was added dropwise, and the reaction mixture was stirred at 0±5°C for 30 min. A solution of **14a** (250.00 g, 949.67 mmol) in *N,N*-dimethylformamide (1250 mL) was then added dropwise, and the reaction was maintained at 0±5°C for 18 h. Additional imidazole (129.30 g, 1899.24 mmol) was added, and after stirring for 10 min, triethylsilyl chloride (286.50 g, 1900.86 mmol) was added dropwise. The reaction was continued at 0±5°C for another 18 h, followed by dropwise addition of methanol (250 mL) and stirring at 0°C for 1 h. After warming to room temperature, the reaction mixture was diluted with water (5000 mL) and extracted with methyl *tert*-butyl ether (5000 mL × 2). The combined organic phases were washed with aqueous sodium chloride (5%, 2500 mL × 2), dried over anhydrous sodium sulfate, filtered, and concentrated under reduced pressure. The residue was purified by silica gel column chromatography (ethyl acetate/ n-heptane = 0-100%) to afford **14b** (479.10 g, 88% assay yield), Purity: 99.95%, Assay: 93.5%.

MS-ESI: calcd for $[M+H]^+$ 534, found 534.

$^1H$ NMR (400 MHz, $CDCl_3$) δ 6.70 (s, 1H), 6.14 (d, *J* = 16.4 Hz, 1H), 5.69-5.66 (m, 1H), 5.59 (d, *J* = 16.4 Hz, 1H), 5.16 (d, *J* = 16.0 Hz, 1H), 4.53 (d, *J* = 2.4 Hz, 2H), 1.87-1.75 (m, 2H), 1.37-1.23 (m, 3H), 1.13 (d, *J* = 7.6 Hz, 18H), 0.95-0.87 (m, 12H), 0.67 (q, *J* = 7.6 Hz, 6H).

Step 2

Under nitrogen protection, **14b** (450.0 g, Assay: 93.5% content, 788.13 mmol) was dissolved in toluene (4.5 L), followed by addition of sodium carbonate (25.10 g, 236.81 mmol) and Lawesson's reagent (382.50 g, 945.68 mmol). The reaction mixture was heated to 110°C and stirred for 3 h. After cooling to 50-60°C, n-heptane (4500 mL) was added dropwise, and the mixture was stirred for 1 h before filtration. The filter cake was washed with n-heptane (900 mL), and the combined organic phases were concentrated under reduced pressure. The residue was purified by silica gel column chromatography (ethyl acetate/n-heptane = 0-100%) to afford compound **14c** (517.0 g), which was used directly in the next step.

MS-ESI: calcd for $[M+H]^+$ 550, found 550.

$^1H$ NMR (400 MHz, $CDCl_3$) δ 7.20 (s, 1H), 6.14 (d, *J* = 16.4 Hz, 1H), 5.87 (t, *J* = 2.0 Hz, 1H), 5.26 (d, *J* = 16.8 Hz, 1H), 4.82 (d, *J* = 2.0 Hz, 1H), 1.90-1.75 (m, 2H), 1.41-1.27 (m, 3H), 1.18-1.11 (m, 18H), 0.97-0.85 (m, 12H), 0.77-0.66 (m, 6H).

Step 3

Under nitrogen protection, the crude product **14c** (517.0 g) from the previous step was dissolved in anhydrous tetrahydrofuran (4.5 L). Hydrofluoric acid (295.50 g, 48 wt%, 7092.00 mmol) was added at room temperature, and the reaction mixture was stirred at the same temperature for 36 h. The reaction was quenched with water (4500 mL), and the aqueous phase was extracted with ethyl acetate (4500 mL × 2). The combined organic phases were washed with saturated brine (2250 mL × 2), dried over anhydrous sodium sulfate, filtered, and concentrated under reduced pressure. The residue was purified by silica gel column chromatography (EtOAc/DCM = 0-100%). The obtained crude product was triturated with methyl *tert*-butyl ether (2250 mL) for 1 h to afford **14d** (194.60 g, assay: 94%) in 83% yield over two steps.

MS-ESI: calcd for $[M+H]^+$ 280, found 280.

$^1H$ NMR (400 MHz, $CDCl_3$) δ 6.63 (s, 1H), 5.82 (d, *J* = 17.2 Hz, 1H), 5.41 (d, *J* = 17.2 Hz, 1H), 4.45-4.30 (m, 2H), 2.99-2.92 (m, 2H), 1.80 (q, *J* = 7.2 Hz, 2H), 0.78 (t, *J* = 7.2 Hz, 3H).

Step 4

Under nitrogen protection, water (8.0 L) and $HCl_{(aq)}$ (8.0 L, 6 N) were charged into a 50 L reactor, followed by addition of **14e** (800.00 g, 3196.55 mmol). The mixture was heated to 100±5°C and stirred for 4 h. After cooling to 30°C, aqueous ammonia (3500 mL, 25-28%) was slowly added to adjust the pH to 4-5 (while maintaining the reaction temperature below 40°C during addition). The resulting mixture was stirred for 30 min and filtered, and the filter cake was washed with water (2500 mL × 1). The solid was triturated with ethanol (8 L) at room temperature for 1 h, filtered, and washed with ethanol (1000 mL × 1). The product was subjected to air-forced convection drying at 50±5°C to afford **14f** (709.00 g, 91% yield).

MS-ESI: calcd for $[M+H]^+$ 209, found 209.

Step 5

A mixture of tetrahydrofuran (10,200 mL), water (10,200 mL), and **14f** (680.00 g, 2779.03 mmol) was charged into a 50 L reactor. The mixture was cooled to 0-10°C, followed by addition of potassium carbonate (653.00 g, 4724.69 mmol) and subsequent dropwise addition of a solution of 9-fluorenylmethyl chloroformate (733.31 g, 2834.60 mmol) in

tetrahydrofuran (3400 mL) while maintaining the internal temperature at 0-10°C. The reaction mixture was stirred at 0-10°C for 1 h, then extracted with 2-methyltetrahydrofuran (10,200 mL × 2). The combined organic phases were successively washed with water (10,200 mL × 1) and saturated brine (10,000 mL × 1), dried over anhydrous sodium sulfate, filtered, and concentrated under reduced pressure. The residue was treated with methyl *tert*-butyl ether (6800 mL) and stirred at room temperature for 4 h. The resulting solid was collected by filtration, washed with methyl *tert*-butyl ether (680 mL × 1), and subjected to air-forced convection drying at 50±5°C to afford **14g** (1090.00 g) in 91% yield.

MS-ESI: calcd for $[M+H]^+$ 431, found 431.

$^1H$ NMR (400 MHz, DMSO-$d_6$) δ 7.90 (d, *J* = 7.2 Hz, 2H), 7.80-7.73 (m, 2H), 7.53-7.28 (m, 6H), 6.39 (d, *J* = 12.8 Hz, 1H), 4.40-4.15 (m, 4H), 3.03-2.75 (m, 2H), 2.20-2.07 (m, 1H), 2.04-1.87 (m, 1H), 1.98 (s, 3H).

Step 6

Under nitrogen protection, **7g** (150.00 g, 483.47 mmol) was charged into a 3 L three-necked flask, followed by addition of ethyl acetate (1200 mL). A solution of **14h** (111.20 g, assay: 90%, 531.69 mmol) in ethyl acetate (300 mL) was added dropwise. After complete addition, the reaction mixture was stirred at room temperature for 2-6 h. The reaction was quenched with saturated brine (450 mL) and stirred for 10 min at room temperature. The layers were separated, and the organic phase was washed with saturated brine (450 mL × 1), dried over anhydrous sodium sulfate, filtered, and concentrated under reduced pressure. The residue was purified by silica gel column chromatography (ethyl acetate/petroleum ether = 0-100%) to afford **14i** (134.00 g) in 73% yield.

MS-ESI: calcd for $[M+H]^+$ 384, found 384.

$^1H$ NMR (400 MHz, DMSO-$d_6$) δ 8.13-8.05 (m, 2H), 7.01 (s, 2H), 3.71 (d, *J* = 6.0 Hz, 2H), 3.70 (d, *J* = 6.0 Hz, 2H), 3.61-3.52 (m, 4H), 3.50-3.44 (m, 2H), 2.33 (t, *J* = 6.4 Hz, 2H), 1.40 (s, 9H).

Step 7

Under nitrogen protection, **14i** (184.30 g, 480.70 mmol) was charged into a 3 L three-necked flask, followed by addition of toluene (1.84 L). Trifluoroacetic acid (823.27 g, 7220.21 mmol) was added dropwise, and the reaction mixture was stirred at room temperature for 4.5 h. The solution was concentrated under reduced pressure, and the residue was subjected to three successive azeotropic distillations with toluene (600 mL × 3) until solid precipitation occurred. The solid was triturated with ethyl acetate (1.7 L) for 2 h and filtered to afford **8g** (150.20 g) in 96% yield.

MS-ESI: calcd for $[M-H]^-$ 326, found 326.

$^1H$ NMR (400 MHz, DMSO-$d_6$) δ 8.12-8.05 (m, 2H), 7.01 (s, 2H), 3.75 (d, *J* = 6.0 Hz, 2H), 3.70 (d, *J* = 5.6 Hz, 2H), 3.60-3.52 (m, 4H), 3.50-3.44 (m, 2H), 2.33 (t, *J* = 6.4 Hz, 2H).

Step 8

Under nitrogen protection, 8g (20.00 g, 61.11 mmol) was dissolved in dichloromethane (200 mL) and cooled to 0 ±5°C. Pentafluorophenol (16.90 g, 91.82 mmol) was added, and the mixture was stirred for 10 min, followed by addition of 1-(3-dimethylaminopropyl)-3-ethylcarbodiimide hydrochloride (17.60 g, 91.82 mmol). The reaction mixture was maintained at 0±5°C for 4-16 h, then diluted with water (200 mL) and stirred for 10 min. The layers were separated, and the organic phase was washed with saturated brine (100 mL × 1), dried over anhydrous sodium sulfate, filtered, and concentrated under reduced pressure. The residue was purified by silica gel column chromatography (acetone/DCM = 0-100%), and the resulting crude product was triturated with methyl *tert*-butyl ether (150 mL) at room temperature for 1 h. The solid was collected by filtration and dried at 35°C to afford **14j** (23.00 g) in 76% yield.

MS-ESI: calcd for $[M+H]^+$ 494, found 494.

$^1H$ NMR (400 MHz, $CDCl_3$) δ 7.12 (d, *J* = 5.6 Hz, 1H), 6.96 (t, *J* = 5.6 Hz, 1H), 6.70 (s, 1H), 4.42 (d, *J* = 6.0 Hz, 2H), 4.03 (d, *J* = 6.0 Hz, 1H), 3.72 (t, *J* = 5.2 Hz, 2H), 3.69 (t, *J* = 5.6 Hz, 2H), 3.59 (t, *J* = 5.2 Hz, 2H), 2.49 (t, *J* = 5.6 Hz, 2H).

Step 9

Under nitrogen protection, **8e** (7.50 g, 24.25 mmol) was dissolved in tetrahydrofuran (400 mL) and stirred at room temperature for 1 h. **14j** (10.00 g, 20.27 mmol) was then added, and the reaction mixture was stirred at room temperature for 16 h. Ethyl acetate (400 mL) was added, and the mixture was stirred for an additional 1 h before filtration. The filter cake was dried and dissolved in a mixed solvent of dichloromethane/methanol (v/v = 20:1, 100 mL). The solution was purified by silica gel column chromatography (MeOH/DCM = 0-100%) to afford the crude product (14.51 g), which was subsequently dissolved in methanol (30 mL). Ethyl acetate (450 mL) was added dropwise to the methanol solution, and the mixture was stirred at room temperature for 24 h. The resulting solid was collected by filtration and dried to afford **7i** (10.20 g) in 81% yield.

MS-ESI: calcd for $[M+Na]^+$ 641, found 641.

Step 10

Under nitrogen protection, toluene (3200 mL), acetic acid (3200 mL), **14d** (320.00 g, 1145.68 mmol), 14g (592.00 g, 1375.21 mmol), and pyridinium p-toluenesulfonate (144.00 g, 573.02 mmol) was sequentially charged into a 10 L reactor. The reaction mixture was heated to 110°C and stirred for 24 h. After cooling to 30±10°C, the solution was transferred to an 80 L extraction followed by addition of 2-methyltetrahydrofuran (6400 mL) and water (6400 mL). The biphasic mixture was stirred for 10-20 min, and the organic phase was separated and successively washed with water (4800 mL × 3) and saturated sodium chloride solution (4800 mL × 1). The organic layer was dried over anhydrous sodium sulfate, filtered, and concentrated under reduced pressure at 50±5°C until no significant distillate was observed. The residue was evaporated twice with methyl *tert*-butyl ether (640 mL × 2). The resulting crude product 1 was triturated with methyl *tert*-butyl ether (3200 mL) for 2-2.5 h, filtered, and washed with methyl *tert*-butyl ether (640 mL × 2) to afford crude product 2. This material was dissolved in *N*,*N*-dimethylformamide (6400 mL), and methyl *tert*-butyl ether (25600 mL) was added. The mixture was stirred at 20-30°C for 16-18 h to allow crystallization. The solid was collected by filtration, re-triturated with methyl *tert*-butyl ether (3200 mL) for 2-2.5 h, filtered, washed with methyl *tert*-butyl ether (640 mL × 2), and subjected to air-forced convection drying at 50±5°C to afford **14k** (490 g) in 63% yield.

MS-ESI: calcd for $[M+H]^+$ 674, found 674.

$^1$H NMR (400 MHz, DMSO-$d_6$) δ 8.16-8.06 (m, 1H),7.85 (d, J = 7.2 Hz, 2H), 7.82-7.74 (m ,2H), 7.74-7.64 (m, 2H), 7.42-7.33 (m, 2H), 7.33-7.23 (m, 2H), 6.76-6.64 (m, 2H), 5.97-5.81 (m, 1H), 5.58-5.40 (m, 2H), 5.38-5.20 (m, 2H), 4.68-4.52 (m, 1 H), 4.34 (s, 2H), 3.28-3.14 (m, 1H), 3.12-2.96 (m, 1H), 2.32 (s, 3H), 2.28-2.18 (m, 1H), 2.18-2.04 (m, 1H), 2.00-1.82 (m, 2H), 0.94-0.82 (m, 3H).

Step 11

Under nitrogen atmosphere, tetrahydrofuran (19,200 mL) and **14k** (480 g, 712.42 mmol) were sequentially charged into the reactor, and the mixture was cooled to 0-5°C. Piperidine (546 g, 6,412.21 mmol) was added dropwise while maintaining the temperature at 0-5°C. After complete addition, the reaction mixture was stirred at 0-5°C for 22-24 h, then treated with hydrochloric acid (1,440 mL, 6 N) dropwise at 0-10°C. The mixture was stirred for 10-15 min and concentrated under reduced pressure. The residue was triturated with water (9,600 mL) for 2-2.5 h, filtered. The filter cake was washed with acetonitrile (480 mL × 2), and then dried. The crude product was purified by prep-HPLC (Preparation instrument: Hanbon Industrial Preparative Liquid Chromatograph, Model: DAC150; Column: Welch Ultimate XB-C18 column, 150×250 mm, 10 μm). The collected fractions were adjusted to pH 7-8 with 5% aqueous ammonia, stirred for 30-35 min, filtered, and washed with water (1,000 mL × 1). The filter cake was subjected to air-forced convection drying at 60°C for 24±2 h to afford **1d** (97 g) in 30% yield.

MS-ESI: calcd for $[M+H]^+$ 452, found 452.

Step 12

Under nitrogen protection, *N*,*N*-dimethylformamide (1200 mL) was added to the reaction flask followed by sequential addition of **1d** (60.00 g, 132.89 mmol) and trifluoroacetic acid (15.20 g, 133.31 mmol). The mixture was cooled to 0-10°C and stirred for 10 min, then **7i** (115.10 g, 186.07 mmol), 1-(3-dimethylaminopropyl)-3-ethylcarbodiimide hydrochloride (51.00 g, 266.04 mmol), and 2,4,6-collidine (16.10 g, 132.86 mmol) were added. The reaction mixture was stirred at 0-10°C for 1 h, then quenched by dropwise addition of hydrochloric acid (1200 mL, 0.05 N) followed by water (1800 mL). After stirring for 10-20 min, the mixture was filtered and the filter cake was washed with water (300 mL × 2). The solid was dissolved in dichloromethane/methanol (v/v = 30:1, 1800 mL), dried over anhydrous sodium sulfate, filtered, and purified by silica gel column chromatography (MeOH/DCM = 0-100%). The crude product was triturated with methyl *tert*-butyl ether (600 mL) for 1-1.5 h, filtered, and washed with methyl *tert*-butyl ether (300 mL × 2). The filter cake was dried under reduced pressure with a rotary evaporator for 5±1 h to afford compound 7 (90.0 g) in 64% yield.

MS-ESI: calcd for $[M+H]^+$ 1052, found 1052.

**Example 1.13**

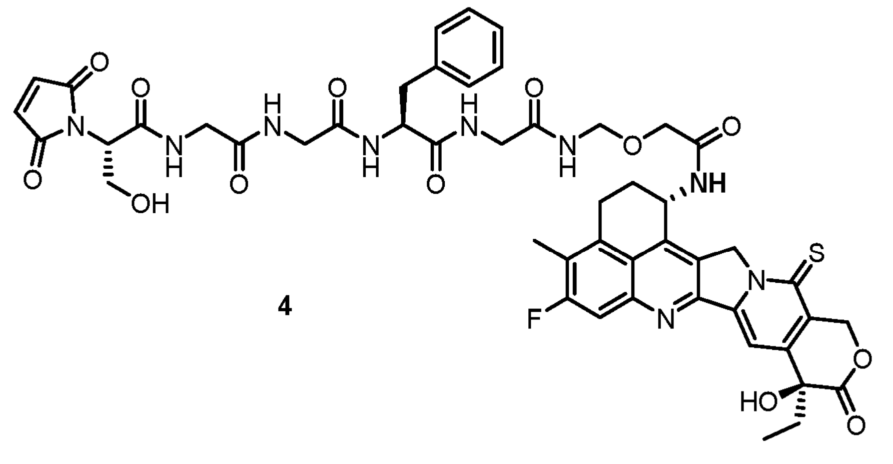

4

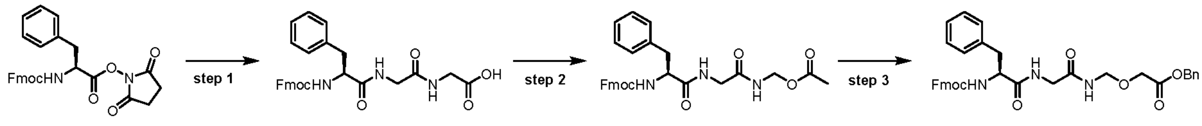


15a 8a 8b 8c

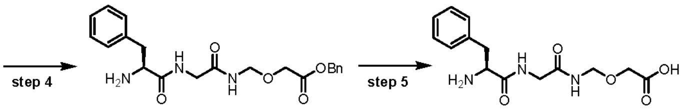


15b 8e

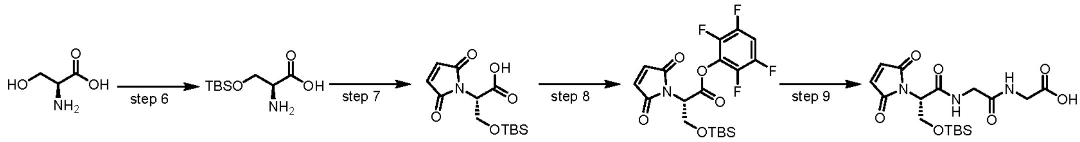


4a 4b 15c 15d 15e

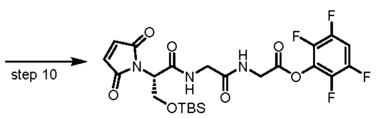


15f

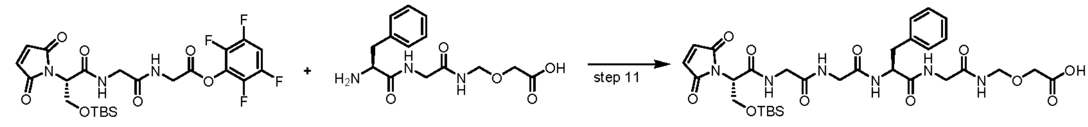


15f 8e 15g

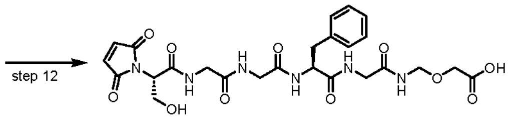


15h

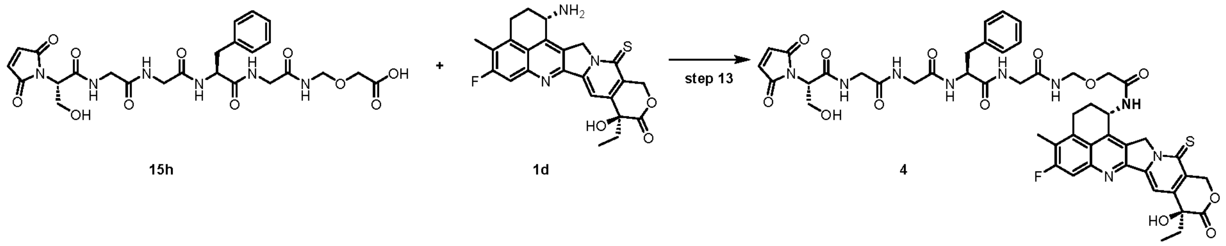


15h 1d 4

Step 1

Under nitrogen protection, **15a** (438.00 g, 904.0 mmol) was dissolved in ethylene glycol dimethyl ether (4380 mL), followed by addition of water (2630 mL). The mixture was cooled to 0-5°C, and glycylglycine (238.87 g, 1808.0 mmol) and sodium bicarbonate (151.87 g, 1808.0 mmol) were added sequentially. After complete addition, the reaction mixture was warmed to 20-25°C and stirred for 1 h. The solution was re-cooled to -5-0°C, and dilute hydrochloric acid (0.5 M, 4380 mL) was added. After stirring for 10 min, the aqueous phase was extracted with 2-methyltetrahydrofuran (8760 mL × 1) and 2-methyltetrahydrofuran (4380 mL × 1). The combined organic phases were washed with saturated brine (4380 mL × 1), dried over anhydrous sodium sulfate, filtered, and concentrated under reduced pressure. The crude product was triturated with acetone (8760 mL) for 16 h, filtered, and dried to afford **8a** (375.00 g) in 83% yield.
MS-ESI: calcd for $[M+H]^+$ 502, found 502.

Step 2

**8a** (375.00 g, 747.69 mmol) was dissolved in N,N-dimethylformamide (4000 mL), followed by sequential addition of lead tetraacetate (730.11 g, 1646.70 mmol), copper(II) acetate (135.95 g, 748.50 mmol), and acetic acid (98.80 g, 1646.70 mmol). The reaction mixture was heated to 45-50°C and stirred for 0.5 h, then poured into a mixture of 2-methyltetrahydrofuran (7500 mL) and water (7500 mL). The layers were separated, and the aqueous phase was extracted with 2-methyltetrahydrofuran (3750 mL × 1). The combined organic phases were washed sequentially with saturated sodium bicarbonate solution (3750 mL × 1), water (3750 mL × 1), and saturated brine (3750 mL × 1), dried over anhydrous sodium sulfate, filtered, and concentrated under reduced pressure. The crude product was triturated with methyl tert-butyl ether (3750 mL) for 16 h, filtered, and subjected to air-forced convection drying for 16 h to afford **8b** (345.00 g) in 89% yield.
MS-ESI: calcd for $[M-CH_3COO]^+$ 456, found 456.
$^1H$ NMR (400 MHz, DMSO-$d_6$) δ 8.67 (t, *J* = 6.8 Hz, 1H), 8.34 (t, *J* = 5.6 Hz, 1H), 7.87 (d, *J* = 7.6 Hz, 1H), 7.71-7.58 (m, 3H), 7.50-7.10 (m, 9H), 5.18-5.04 (m, 2H), 4.40-4.00 (m, 4H), 3.78 (d, *J* = 5.6 Hz, 2H), 3.83-3.69 (m, 2H), 3.05 (dd, *J* = 13.6, 4.0 Hz, 1H), 2.80 (dd, *J* = 13.2, 10.8 Hz, 1H), 1.98 (s, 3H).

Step 3

Under nitrogen protection, **8b** (345.00 g, 669.17 mmol) was dissolved in tetrahydrofuran (3450 mL) and cooled to 0-5°C. Benzyl glycolate (244.47 g, 1472.17 mmol) and p-toluenesulfonic acid (17.20 g, 100.37 mmol) were added sequentially. The reaction mixture was warmed to 20-25°C and stirred for 1 h, then quenched with ethyl acetate (3450 mL) and water (3450 mL). The layers were separated, and the aqueous phase was extracted with ethyl acetate (3450 mL × 1). The combined organic phases were washed with water (3450 mL × 1) and brine (3450 mL × 1), dried over anhydrous sodium sulfate, filtered, and concentrated under reduced pressure. The crude product was purified by silica gel column chromatography (ethyl acetate/petroleum ether = 0-100%) to afford **8c** (178.00 g) in 42% yield.
MS-ESI: calcd for $[M+Na]^+$ 644, found 644.
$^1H$ NMR (400 MHz, DMSO-$d_6$) δ 8.63 (t, *J* = 6.8 Hz, 1H), 8.35 (t, *J* = 5.6 Hz, 1H), 7.87 (d, *J* = 7.6 Hz, 1H), 7.71-7.60 (m, 3H), 7.45-7.13 (m, 14H), 5.13 (s, 2H), 4.67-4.61 (m, 2H), 4.32-4.24 (m, 1H), 4.22-4.09 (m, 5H), 3.83-3.69 (m, 2H), 3.06 (dd, *J* = 13.6, 4.0 Hz, 1H), 2.80 (dd, *J* = 13.6, 10.4 Hz, 1H).

Step 4

Under nitrogen protection, **8c** (102.00 g, 164.07 mmol) was dissolved in dichloromethane (1020 mL) and cooled to -5-0°C. 1,8-Diazabicyclo[5.4.0]undec-7-ene (24.97 g, 164.07 mmol) was added, and the reaction mixture was stirred at 0-5°C for 1 h. The crude product was directly purified by silica gel column chromatography (dichloromethane/isopropyl alcohol = 0-100%) to afford **15b** (43.50 g) in 66% yield.
MS-ESI: calcd for $[M+H]^+$ 400, found 400.
$^1$H NMR (400 MHz, DMSO-$d_6$) δ 8.64 (t, *J* = 6.4 Hz, 1H), 8.26-8.14 (m, 1H), 7.45-7.15 (m, 10H), 5.15 (s, 2H), 4.64 (d, *J* = 6.8 Hz, 2H), 4.15 (s, 2H), 3.81-3.65 (m, 2H), 3.44 (dd, *J* = 8.4, 4.4 Hz, 1H), 2.98 (dd, *J* = 13.6, 4.8 Hz, 1H), 2.59 (dd, *J* = 13.6, 8.8 Hz, 1H).

Step 5

Under nitrogen protection, **15b** (43.00 g, 107.65 mmol) was dissolved in a mixed solvent of tetrahydrofuran (430 mL) and water (430 mL). The mixture was cooled to 0-10°C, and wet Pd/C (6.50 g) was added. After three cycles of hydrogen purging, the reaction was stirred under a hydrogen atmosphere at 0-10°C for 24 h. The mixture was filtered, and the filter cake was washed sequentially with tetrahydrofuran (43 mL) and water (43 mL). The filtrate was concentrated under reduced pressure to remove tetrahydrofuran, and the residual aqueous phase was washed with 2-methyltetrahydrofuran (430 mL × 2) and lyophilized to afford **8e** (31.50 g) in 95% yield.
MS-ESI: calcd for $[M-H]^-$ 308, found 308.
$^1$H NMR (400 MHz, DMSO-$d_6$) δ 9.07 (s, 1H), 8.67 (s, 1H), 7.33-7.26 (m, 2H), 7.25-7.18 (m, 3H), 4.68-4.56 (m, 2H), 3.92-3.71 (m, 4H), 3.52 (dd, *J* = 16.4, 4.0 Hz, 1H), 3.01 (dd, *J* = 13.6, 6.4 Hz, 1H), 2.79 (dd, *J* = 13.2, 7.6 Hz, 1H).

Step 6

**4a** (1000.00 g, 9515.65 mmol) and acetonitrile (10.00 L) were charged into a 20 L reactor, followed by addition of *tert*-butyldimethylsilyl chloride (1505.92 g, 9991.42 mmol). The mixture was cooled to 0-10°C, and 1,8-diazabicyclo[5.4.0]undec-7-ene (1376.18 g, 9039.84 mmol) was added slowly. After complete addition, the reaction was warmed to room temperature and stirred for 40 h. The mixture was filtered directly, and the filter cake was dissolved in methanol (18.00 L) and filtered. Acetonitrile (54.00 L) was added to the filtrate, and the resulting mixture was stirred for 2 h to precipitate the product. The solid was collected by filtration and dried to afford **4b** (1481.60 g) in 71% yield.
MS-ESI: calcd for $[M-H]^-$ 218, found 218.

Step 7

**4b** (500.00 g, 2279.36 mmol) and tetrahydrofuran (3.75 L) were charged into the reaction flask. The mixture was cooled to 0-5°C, and N-methoxycarbonylmaleimide (395.00 g, assay: 85%, 2164.59 mmol) was added. After complete addition, the reaction was warmed to room temperature and stirred for 16 h. The mixture was re-cooled to 0-5°C, and 10% aqueous sodium bicarbonate solution (3.75 L) was added while maintaining the temperature below 20°C. The reaction was then heated to 30°C and stirred for 88 h. After cooling to 0-5°C, the pH was adjusted to 5-6 by addition of 5% aqueous citric acid solution, followed by addition of ethyl acetate (3.75 L). The layers were separated, and the aqueous phase was extracted with ethyl acetate (1.50 L). The combined organic phases were washed with water (3.75 L × 1) and saturated brine (3.75 L × 1), dried over anhydrous sodium sulfate, filtered, and concentrated under reduced pressure. The residue was purified by silica gel column chromatography (ethyl acetate/petroleum ether = 0-100%) to afford **15c** (350.00 g) in 45% yield, assay: 88%.
MS-ESI: calcd for $[M+H]^+$ 300, found 300.
$^1$H NMR (400 MHz, DMSO-$d_6$) δ 13.35 (br s, 1H), 7.14 (s, 2H), 4.69-4.81 (m, 1H), 4.13-3.97 (m, 2H), 0.77 (m, 9H), 0.00 (s, 3H), -0.06 (s, 3H).

Step 8

Dichloromethane (1260 mL) and **15c** (210.00 g, 701.40 mmol) were charged into the reaction flask. The mixture was cooled to 0-5°C. A solution of N,N'-dicyclohexylcarbodiimide (159.30 g, 772.56 mmol) in dichloromethane (420 mL) and a solution of tetrafluorophenol (122.46 g, 737.40 mmol) in dichloromethane (420 mL) were added sequentially. The reaction mixture was maintained at 0-5°C with stirring for 1 h, then filtered, and the filter cake was washed with dichloromethane (1050 mL). The filtrate was concentrated under reduced pressure, and the residue was treated with methyl *tert*-butyl ether (2100 mL). After stirring for 10 min, the mixture was filtered, and the filter cake was washed with methyl *tert*-butyl ether (630 mL). The filtrate was concentrated, and the resulting residue was triturated with n-hexane

(2100 mL) for 30 min, filtered. The filter cake was washed with n-hexane (630 mL) and dried to afford **15d** (246.78 g) in 79% yield.

MS-ESI: calcd for $[M+H]^+$ 448, found 448.

$^1H$ NMR (400 MHz, $CD_3OD$) δ 7.48-7.36 (m, 1H), 6.98 (s, 2H), 5.30 (dd, *J* = 9.2, 6.0 Hz, 1H), 4.34-4.23 (m, 2H), 0.84 (s, 9H), 0.07 (s, 3H), 0.02 (s, 3H).

Step 9

Water (1470 mL) and glycylglycine (79.58 g, 602.91 mmol) were charged into the reaction flask under stirring, followed by addition of ethylene glycol dimethyl ether (2450 mL). The mixture was cooled to 5-10°C, and **15d** (245.00 g, 547.53 mmol) and sodium bicarbonate (92.09 g, 1096.18 mmol) were added. The reaction mixture was stirred for 40 h. The mixture was washed with methyl *tert*-butyl ether (2450 mL × 2), and the aqueous phase was treated with 2-methyltetrahydrofuran (7350 mL). Pre-cooled 2.5% aqueous citric acid solution (3920 mL) was added to adjust the pH to 5-6 while maintaining the temperature around 0°C during addition. The layers were separated, and the aqueous phase was extracted with 2-methyltetrahydrofuran (2450 mL × 1). The combined organic phases were sequentially washed with water (7350 mL × 4) and saturated sodium chloride solution (3680 mL × 1), dried over anhydrous sodium sulfate, filtered, and concentrated under reduced pressure. The residue was triturated with ethyl acetate (2450 mL) for 10 min and filtered. The filter cake was washed with ethyl acetate (735 mL), and the filtrate was concentrated again. The resulting residue was dissolved in ethyl acetate (1230 mL), and n-hexane (6125 mL) was added to precipitate the solid. After stirring for 16 h, the mixture was filtered, and the filter cake was washed with n-hexane (735 mL) and dried to afford **15e** (115.40 g) in 51% yield.

MS-ESI: calcd for $[M+H]^+$ 414, found 414.

$^1H$ NMR (400 MHz, DMSO-$d_6$) δ 8.41 (t, *J* = 5.6 Hz, 1H), 8.05 (t, *J* = 5.6 Hz, 1H), 7.11 (s, 2H), 4.73 (dd, *J* = 10.4, 5.2 Hz, 1H), 4.11 (dd, *J* = 10.4, 5.6 Hz, 1H), 4.00 (t, *J* = 10.4 Hz, 1H), 3.83-3.73 (m, 3H), 3.57 (dd, *J* = 16.8, 5.6 Hz, 1H), 0.76 (s, 9H), -0.01 (s, 3H), -0.07 (s, 3H).

Step 10

Dichloromethane (1150 mL) and **15e** (115.00 g, 278.11 mmol) were charged into the reaction flask. The mixture was cooled to -40°C--30°C. A solution of N,N'-dicyclohexylcarbodiimide (86.10 g, 417.29 mmol) in dichloromethane (155 mL) and a solution of tetrafluorophenol (46.24 g, 278.44 mmol) in dichloromethane (155 mL) were added sequentially. The reaction mixture was maintained at -40--30°C with stirring for 5 h, then quenched with water (2300 mL) and filtered to remove insoluble solids. The filtrate was stirred and separated, and the aqueous phase was extracted with dichloromethane (1150 mL). The combined organic phases were washed with saturated brine (1730 mL × 1), dried over anhydrous sodium sulfate, filtered, and concentrated under reduced pressure. The residue was triturated with ethyl acetate (1150 mL) for 30 min and filtered. The filter cake was washed with ethyl acetate (345 mL), and the filtrate was concentrated. The resulting residue was triturated with a mixed solvent of methyl *tert*-butyl ether/n-hexane (2:13, 1730 mL) for 3 h, filtered. The filter cake was rinsed with n-hexane (345 mL) and dried to afford **15f** (146.80 g) in 94% yield.

MS-ESI: calcd for $[M+H]^+$ 562, found 562.

$^1H$ NMR (400 MHz, $CDCl_3$) δ 7.70-7.61 (m, 1H), 7.14-7.06 (m, 1H), 7.06-6.96 (m, 1H), 6.75 (s, 2H), 4.72 (dd, *J* = 8.4, 6.4 Hz, 1H), 4.50-4.35 (m, 2 H), 4.35-4.24 (m, 1H), 4.16-3.94 (m, 2H), 3.87 (dd, *J* = 10.4, 6.4 Hz, 1H), 0.87 (s, 9H), 0.085 (s, 3H), 0.078 (s, 3H).

Step 11

Acetonitrile (2628 mL), water (876 mL), and **15f** (146.00 g, 260.00 mmol) were charged into the reaction flask. The mixture was cooled to -5-0°C. **8e** (96.60 g, 312.30 mmol) and N,N-diisopropylethylamine (33.70 g, 260.65 mmol) were added, and the reaction was stirred at -5-0°C for 18 h. The mixture was diluted with methyl *tert*-butyl ether (2920 mL) and water (1460 mL), and the layers were separated. The organic phase was extracted with water (1460 mL × 1), and the combined aqueous phases were washed with methyl *tert*-butyl ether (2190 mL × 3). The aqueous layer was treated with phosphate buffer solution (2190 mL; formulation: 6.51 g sodium dihydrogen phosphate ($NaH_2PO_4$) and 0.30 g disodium hydrogen phosphate ($Na_2HPO_4$) dissolved in 100.0 mL water), stirred for 10-20 min, and extracted with 2-methyltetrahydrofuran (2190 mL × 3). The combined organic phases were washed with saturated brine (2190 mL × 2), dried over anhydrous sodium sulfate, filtered, and concentrated. The residue was purified by prep-HPLC (Preparation instrument: Hanbon Industrial Preparative Liquid Chromatograph, Model: DAC150; Column: Welch Xtimate C18). The collected fractions were mixed with a 1:1 (v/v) ethyl acetate/2-methyltetrahydrofuran solution (half the volume of the collected fractions), and the layers were separated. The aqueous phase was extracted with ethyl acetate (half-volume of the collected fractions), and the combined organic phases were washed with deionized water (half-volume of the collected fractions × 3) and concentrated. The residue was lyophilized to afford **15g** (83.54 g) in 46% yield.

MS-ESI: calcd for $[M+Na]^+$ 727, found 727.

Step 12

Acetonitrile (664 mL), water (996 mL), and **15g** (83.00 g, 117.8 mmol) were charged into the reaction flask. The mixture was cooled to 5-10°C. Formic acid (65.11 g, 1414.5 mmol) was added, and the reaction was stirred at 5-10°C for 40 h. The mixture was extracted with methyl *tert*-butyl ether (830 mL), and the organic phase was extracted with water (420 mL × 2). The combined aqueous phases were lyophilized to afford the crude product, which was further purified by repeated azeotropic distillation with dichloromethane (1660 mL × 5) to remove residual formic acid. The residue was dissolved in water (830 mL) and lyophilized to afford **15h** (65.30 g) in 94% yield.

MS-ESI: calcd for $[M+H]^+$ 591, found 591.

$^1$H NMR (400 MHz, DMSO-$d_6$) δ 8.54 (t, *J* = 6.4 Hz, 1H), 8.37-8.28 (m, 1H), 8.12 (d, *J* = 7.6 Hz, 1H), 8.02 (t, *J* = 6.4 Hz, 1H), 7.29-7.15 (m, 5H), 7.05 (s, 2H), 4.65-4.57 (m, 3H), 4.55-4.45 (m, 1H), 4.02-3.94 (m, 3H), 3.92-3.82 (m, 1H), 3.80-3.68 (m, 4H), 3.65-3.57 (m, 2H), 3.04 (dd, *J* = 13.6, 4.4 Hz, 1H), 2.78 (dd, *J* = 14.0, 10.0 Hz, 1H).

Step 13

Under nitrogen protection, a solution of **1d** (120.00 g, 265.77 mmol) in *N,N*-dimethylformamide (2400 mL) was charged into the reactor and cooled to -10--5°C. **15h** (157.20 g, 266.19 mmol) and 2-(7-azabenzotriazol-1-yl)-N,N,N',N'-tetramethyluronium hexafluorophosphate (151.20 g, 397.64 mmol) were added, followed by addition of 2,4,6-Collidine (64.80 g, 534.74 mmol) while maintaining the temperature at -10--5°C. After complete addition, the reaction mixture was stirred at -10~-5°C for 4 h. The reaction was quenched by slow addition of 2% aqueous citric acid solution (7200 mL), stirred for 10-15 min, and filtered. The filter cake was washed with water (1200 mL × 3) and dried. The crude product was purified by preparative reverse-phase chromatography (Preparation instrument: Hanbon Industrial Preparative Liquid Chromatograph, Model: DAC150; Column: Kromasil 100-10-C18(W)). The obtained preparative solution was processed in batches (25.0 ± 5.0 kg per batch). Each batch of the preparative solutions was transferred to a 50 L reactor, and acetonitrile was added slowly to make the solution clear. Ethyl acetate (10,000 mL) was then added, and the mixture was stirred for 5-10 min and allowed to settle for 5-10 min. The organic phase was washed with water (10,000 mL × 3), and the combined aqueous phases were back-extracted with ethyl acetate (10,000 mL). The organic phases were combined and washed with water (10,000 mL × 2), then concentrated under reduced pressure until no liquid distilled. The residue was transferred to a 10 L Büchner funnel and filtered under vacuum. The filter cake was washed with distilled water (1200 mL × 2) until no liquid remained. The wet products from multiple batches were combined, suspended in water (2400 mL), and stirred at 0-10°C for 2-3 h. The solid was collected by filtration, washed with water (480 mL), and dried in a vacuum oven at 30-35°C for 40 h to afford **4** (152.00 g) in 56% yield.

MS-ESI: calcd for $[M+H]^+$ 1024, found 1024.

$^1$H NMR (400 MHz, DMSO-$d_6$) δ 8.70-8.62 (m, 1H), 8.56 (d, *J* = 8.8 Hz, 1H), 8.35-8.24 (m, 2H), 8.09 (d, *J* = 8.0 Hz, 1H), 8.04-7.95 (m, 1H), 7.76 (s, 1H), 7.69 (d, *J* = 8.8 Hz, 1H), 7.28-7.13 (m, 5H), 7.05 (s, 2H), 6.69 (s, 1H), 5.90 (d, *J* = 16.4 Hz, 1H), 5.72-5.62 (m, 1H), 5.48 (d, *J* = 16.8 Hz, 1H), 5.42 (d, *J* = 20.0 Hz, 1H), 5.17 (d, *J* = 19.6 Hz, 1H), 5.06 (t, *J* = 5.6 Hz, 1H), 4.68 (d, *J* = 7.2 Hz, 2H), 4.60 (dd, *J* = 9.6, 6.0 Hz, 1H), 4.52-4.43 (m, 1H), 4.18-4.04 (m, 2H), 4.01-3.92 (m, 1H), 3.90-3.81 (m, 1H), 3.79-3.68 (m, 4H), 3.65-3.55 (m, 2H), 3.28-3.07 (m, 2H), 3.01 (dd, *J* = 13.6, 4.0 Hz, 1H), 2.73 (dd, *J* = 13.6, 9.6 Hz, 1H), 2.35 (s, 3H), 2.28-2.12 (m, 2H), 1.88 (q, *J* = 7.2 Hz, 2H), 0.85 (t, *J* = 7.2 Hz, 3H).

**Example 1.14**

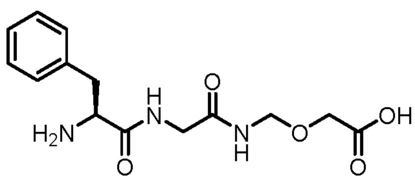

**8e**

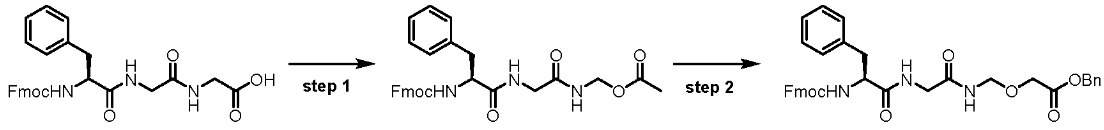


8a 8b 8c

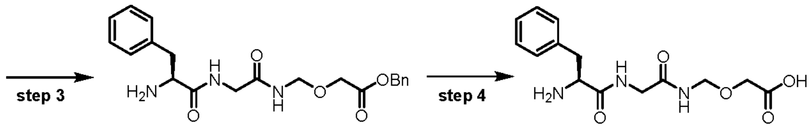


15b 8e

Step 1

A solution of **8a** (200.00 g, 399.12 mmol) in N,N-dimethylformamide (600 mL) was prepared and set aside. To the reaction flask were sequentially added *N,N*-dimethylformamide (400 mL), acetic acid (119.80 g, 1996.67 mmol), and lead tetraacetate (260.00 g, 598.66 mmol). The above **8a** solution was then added dropwise to the reaction mixture. After complete addition, the mixture was heated up to 40°C and stirred for 4 h. The reaction was quenched with dichloromethane (4000 mL) and water (4000 mL), stirred for 30 min, and filtered to remove insoluble solids. The filtrate was separated, and the aqueous phase was extracted with dichloromethane (1000 mL × 1). The combined organic phases were washed with water (2000 mL × 3), concentrated under reduced pressure, and triturated with methyl *tert*-butyl ether (1000 mL) for 1 h. The solid was collected by filtration and dried to afford **8b** (179.63 g) in 87% yield.

MS-ESI: calcd for $[M-CH_3COO]^+$ 456, found 456.

Step 2

Under nitrogen protection, **8b** (300.00 g, 582.29 mmol) and tetrahydrofuran (1200 mL) were charged into the reaction flask. The mixture was cooled to 0-5°C, followed by addition of benzyl glycolate (106.39 g, 640.50 mmol; residual material rinsed with 300 mL THF and transferred to the flask). Aqueous lithium hydroxide solution (16.70 g, 698.74 mmol in 300 mL $H_2O$) was then added dropwise. After complete addition, the reaction was stirred at 0-5°C for 2 h. the mixture was diluted with water (3000 mL) and dichloromethane (3000 mL), and the layers were separated. The organic phase was washed with brine (4000 mL x 1), concentrated to ~450 g, and purified by silica gel column chromatography (ethyl acetate/petroleum ether = 0-100%) to afford **8c** (269.20 g) in 74% yield.

MS-ESI: calcd for $[M+Na]^+$ 644, found 644.

Step 3

Under nitrogen protection, **8c** (264.00 g, 424.98 mmol) was dissolved in dichloromethane (26440 mL) and cooled to -5-0°C. 1,8-Diazabicyclo[5.4.0]undec-7-ene (64.60 g, 424.72 mmol) was added, and the reaction mixture was stirred at 0-5°C for 2 h. The reaction mixture was directly purified by silica gel column chromatography (isopropyl alcohol/dichloromethane = 0-100%) to afford **15b** (111.00 g) in 66% yield.

MS-ESI: calcd for $[M+H]^+$ 400, found 400.

Step 4

**15b** (108.00 g, 270.60 mmol) and water (2160 mL) were charged into the reaction flask sequentially, followed by addition of wet Pd/C (10.80 g). After three cycles of hydrogen purging, the mixture was stirred under a hydrogen atmosphere for 3 h. The reaction was filtered to remove insoluble solids, and the aqueous phase was directly lyophilized to afford **8e** (82.16 g) in 98% yield.

MS-ESI: calcd for $[M-H]^-$ 308, found 308.

**Example 2**

**Preparation Method for Antibody-Drug Conjugates (ADCs)**

The anti-IGF-1R antibody was prepared using an antibody sequence with the following CDR regions (as defined

by Kabat):

CDR1: SFVMA (SEQ ID No. 1)
CDR2: AISGSGSRARYADSVKG (SEQ ID No. 2)
CDR3: NPRRATPDLTQYAY (SEQ ID No. 3)
**Heavy Chain Variable Region (VH):**

EVQLVESGGGLVQPGGSLRLSCAASGRTFSSFVMAWFRQAPGKGLEFVSAISGSGSRARYADSVKGRFTISRDNSKNTLYLQMNSLRAEDTAVYYCAANPRRATPDLTQYAYWGQGTLVTVSS (SEQ ID No. 4)

**The full length sequence is as follows:**

EVQLVESGGGLVQPGGSLRLSCAASGRTFSSFVMAWFRQAPGKGLEFVSAISGSGSRARYADSVKGRFTISRDNSKNTLYLQMNSLRAEDTAVYYCAANPRRATPDLTQYAYWGQGTLVTVSSEPKSSDKTHTCPPCPAPEAAGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK (SEQ ID No. 5)

The antibody was dialyzed into 50 mM phosphate buffer (PB, pH 7.4) to obtain the antibody intermediate. An appropriate volume of the intermediate was sequentially supplemented with 10 mM tris(2-carboxyethyl)phosphine hydrochloride (TCEP) stock solution and 10 mM diethylenetriaminepentaacetic acid (DTPA) stock solution, followed by addition of 50 mM PB buffer to make the final antibody concentration in the reaction system at 20 mg/mL, with molar ratio to antibody at 4:1, and final concentration of DTPA at 1 mM. After thorough mixing, the reduction reaction was performed at 25±2°C with 400 rpm agitation for 2 hours in a thermomixer. Upon completion of reduction, the reaction system was transferred to an ice-water bath, and an appropriate volume of 5 mM linker-payload stock solution was added at a 4.5:1 molar ratio of linker-payload to antibody, with dimethyl sulfoxide (DMSO) supplementation to reach a final concentration of 20% (v/v) DMSO in the conjugation reaction. The mixture was thoroughly agitated and incubated at 25±2°C with 400 rpm agitation for 1 hour in the thermomixer to facilitate conjugation. The resulting ADC product was dialyzed into the dialysis buffer (10 mM His/His-HCl, pH 6.0 ± 0.2) using ultrafiltration centrifugal tubes to obtain the ADC bulk solution, which was aliquoted and stored at -80°C.

The prepared ADCs were analyzed by size exclusion-high-performance liquid chromatography (SEC-HPLC) and hydrophobic interaction chromatography -high-performance liquid chromatography (HIC-HPLC) to determine molecular size variant purity and average drug-to-antibody ratio (DAR). Key analytical parameters are summarized in Tables 1 and 2. The results of ADC purity and DAR distribution are presented in Table 3. The data demonstrate that the manufactured ADCs exhibit high purity and homogeneous DAR distribution.

Table 1. Key parameters of the size exclusion-high-performance liquid chromatography (SEC-HPLC)

| | |
|---|---|
| Column | TSKgel G3000SWxl column (5 μm) 7.8 mm*30 cm |
| Mobile phase A | 25 mM sodium phosphate, 0.3 M NaCl, 5% isopropanol (v/v), pH 7.0 |

Table 2. Key parameters of the hydrophobic interaction chromatography -high-performance liquid chromatography (HIC-HPLC)

| | |
|---|---|
| Column | TSKgel Butyl-NPR column (2.5 μm) 4.6 mm*35 mm |
| Mobile phase A | 20 mM sodium phosphate, 1.5 M ammonium sulfate, pH 7.0 |
| Mobile phase B | 20 mM sodium phosphate, 25% isopropanol (v/v), pH 7.0 |

Table 3. Purity of antibody-drug conjugates, average drug-antibody ratio, and DAR4 percentage

| ID | Purity (SEC) | Average drug-antibody ratio | DAR4 (%) |
|---|---|---|---|
| ADC-1 (Example 1.1 linker-payload conjugate) | 99.4% | 3.9 | 97.7% |
| ADC-2 (Example 1.2 linker-payload conjugate) | 99.1% | 3.8 | 94.3% |
| ADC-3 (Example 1.3 linker-payload conjugate) | 97.6% | 4.0 | 99.5% |
| ADC-4 (Example 1.4 linker-payload conjugate) | 98.7% | 3.9 | 95.0% |
| ADC-7 (Example 1.7 linker-payload conjugate) | 99.4% | 3.9 | 97.7% |

**Example 3**

**ADC Plasma Stability Study**

The linker-payload was prepared according to the method described in Example 1 of this application, and the antibody-drug conjugate (ADC) was obtained following the procedure outlined in Example 2.

An appropriate volume of the ADC stock solution was added to 8 mL of anticoagulated human plasma to achieve a final ADC concentration of 200 μg/mL. The mixture was incubated in a biochemical incubator at 37°C for 0 h, 24 h, 72 h, or 168 h, after which samples were collected and purified by Protein A affinity chromatography. The purified ADC samples were concentrated via ultrafiltration centrifugation, followed by the addition of 5 μL of 5 M tris(2-carboxyethyl)phosphine hydrochloride (TCEP) stock solution and sufficient ultrapure water to adjust the total sample volume to approximately 200 μL. The mixture was incubated at 56°C in a dry-block thermostat for 40 min, after which it was subjected to another round of ultrafiltration centrifugation (12,000 rpm, 15 min). Following centrifugation, 100 μL of ultrapure water was added to the sample and mixed thoroughly, which was then analyzed by reversed-phase mass spectrometry (RP-MS). The mass spectrometry parameters were set to positive ion mode, with mobile phases consisting of A: 0.1% formic acid in water and B: 0.1% formic acid in acetonitrile, a column temperature of 40°C, and a flow rate of 0.4 mL/min.

Based on molecular weight differences, the conjugation numbers of small-molecule drugs were determined by the mass spectrometry analysis, and the average drug-to-antibody ratio (DAR) was calculated according to the area percentage of each peak and the corresponding drug conjugation number. The plasma stability of the ADC was investigated by monitoring DAR changes at different incubation time points, with the test results shown in Table 4. The calculation formula for the average DAR value is as follows:

$$\mathrm{DAR} = (0 \times \mathrm{DAR0\%} + 1 \times \mathrm{DAR1\%} + 2 \times \mathrm{DAR2\%}) / (\mathrm{DAR0\%} + \mathrm{DAR1\%} + \mathrm{DAR2\%}) \times 2$$

The experimental data demonstrate that the ADCs prepared in this application exhibit excellent plasma stability.

Table 4. Changes in Drug-to-Antibody Ratio (DAR) of Antibody-Drug Conjugates Following Incubation in Human Plasma

| ID | DAR (0h) | DAR (24h) / Percentage reduction (%) | DAR (72h) / Percentage reduction (%) | DAR (168h) / Percentage reduction (%) |
|---|---|---|---|---|
| ADC-1 | 4.0 | 3.52 / 12.0% | 3.54 / 11.5% | 3.58 / 10.5% |
| ADC-2 | 3.84 | 3.41 / 11.2% | 3.49 / 9.1% | 3.57 / 7.0% |
| ADC-3 | 4.0 | 3.97 / 0.7 % | 3.86 / 3.5% | 3.92 / 2.0% |
| ADC-4 | 4.0 | 4.0 / 0% | 4.0 / 0% | 4.0 / 0% |

**Example 4**

**In Vitro Proliferation Inhibition Assay on human-derived tumor cells**

The linker-payload was prepared according to the method described in Example 1 of this application, and the antibody-drug conjugate (ADC) was obtained following the procedure outlined in Example 2.

Log-phase human tumor cells were harvested, trypsinized, and resuspended in fresh complete culture medium to an appropriate concentration before being seeded into 96-well cell culture plates. The plates were put into a 37°C incubator with 5% $CO_2$ and incubated overnight. The following day, different concentrations of test ADC samples

(maximum final concentration 1 μM, serially diluted at 1:4 or 1:8 ratios) or buffer controls were added to designated wells, followed by continued incubation for 168 h in the $CO_2$ incubator. After equilibrating the assay plates to room temperature, luminescence signals were measured using the CellTiter-Glo Assay Kit (Promega, G7558) and a multimode microplate reader. The cell inhibition rate was calculated according to the formula: Inhibition rate (%) = $[1 - (RLU_{ADC} - RLU_{blank}) / (RLU_{buffer} - RLU_{blank})] \times 100\%$. Dose-response curves of inhibitory rate were plotted and EC50 values were fitted using GraphPad Prism software. The $EC_{50}$ values of each ADC against different human tumor cell lines are summarized in Table 5.

The ADCs prepared in this application demonstrated excellent *in vitro* tumor cell proliferation inhibition activity.

Table 5. In vitro proliferation inhibitory activity of ADC on human-derived tumor cells

| cell lines / $EC_{50}$ (nM) | ADC-1 | ADC-2 | ADC-3 | ADC-4 | ADC-7 |
|---|---|---|---|---|---|
| The human breast cancer cell line MCF-7 | 5.2 | 3.2 | 3.1 | 5.5 | 15.6 |
| The human breast cancer cell line T47D | 3.5 | 1.8 | 2.3 | 2.7 | 8.3 |
| The human colorectal cancer cell line HCT-116 | 13.2 | 2.8 | 3.3 | 5.2 | 23.1 |
| The human pancreatic cancer cell line BxPC-3 | 2.7 | 1.5 | 1.3 | 2.0 | 5.2 |

### Example 5

### In vivo anti-tumor efficacy assay

The linker-payload was prepared according to the method described in Example 1 of this application, and the antibody-drug conjugate (ADC) was obtained following the procedure outlined in Example 2.

Log-phase human tumor cells were harvested, counted and adjusted to the appropriate concentration, then resuspended on ice in a 1:1 mixture of serum-free medium and Matrigel (Corning 356234) before being subcutaneously inoculated into BALB/c nude mice at 200 μL per animal. When tumors reached measurable sizes, The long diameter and short diameter of each tumor were measured with vernier calipers, and tumor volumes were calculated according to the formula: $V = (a \times b^2)/2$, wherein 'a' represents the long diameter of the tumor and 'b' represents the short diameter of the tumor.

When the average tumor volume reached 100-200 $mm^3$, tumor-bearing nude mice were randomized into groups using a block design stratified by tumor volume. The tumor-bearing mice were injected with vehicle control (normal saline) or different dosage of ADC (3 mg/kg or 10 mg/kg) via tail vein injection according to the predetermined dosing regimen. The long diameter and short diameter of the tumors were measured twice weekly with concurrent recording of body weights. Tumor volumes were statistically analyzed, and tumor growth inhibition (TGI) was calculated using the formula: $TGI = 100\% \times [1 - (TV_{tT} - TV_{0T})/(TV_{tC} - TV_{0C})]$, where $TV_{tT}$ represents the tumor volume of treated groups at measurement day, $TV_{0T}$ denotes baseline tumor volume of treated groups at randomization, $TV_{tC}$ indicates tumor volume of control group at measurement day, and $TV_{0C}$ corresponds to baseline tumor volume of control group at randomization.

The ADC developed in this study demonstrated superior in vivo antitumor efficacy.

### Example 6

### Pharmacokinetic Evaluation in Rats

A healthy adult Sprague Dawley rat (6-8 weeks old) was administrated with the ADCs via tail vein injection over approximately 1 min ± 10 s, with a dosing volume of 5 mL/kg and a concentration of 20 mg/kg. Blood samples were collected at 0.083 h, 1 h, 2 h, 8 h, 24 h, 48 h, 72 h, 96 h, 120 h, 144 h, and 168 h post-dose. Serum was separated by centrifugation within 30-120 minutes. The concentrations of total antibody (Tab) and ADC in the blood samples were determined using a conventional ELISA method.

The detection method for total antibody (Tab) is briefly described as follows: Trop2-His was coated overnight at 4°C (0.75 μg/mL), followed by blocking with 5% skim milk at 37°C for 2 h. Standard curves and quality control (QC) samples were then added and incubated for 2 h. The standard curve ranged from 64 ng/mL to 0.5 ng/mL, starting at 64 ng/mL with 2-fold serial dilutions. QC samples were prepared at concentrations of 60 ng/mL, 6 ng/mL, and 0.6 ng/mL, with acceptable recovery rates between 80% and 120%. Subsequently, a 1:8000 dilution of horseradish peroxidase (HRP)-conjugated goat anti-human kappa light chain antibody (manufacturer: Sigma, catalog #: A7164-1ML) was added and incubated for 1 h. After washing 8 times with PBST, TMB substrate was added for color development, and the reaction was stopped with 0.1 M sulfuric acid. Absorbance was measured at 450 nm using a microplate reader, and the data were analyzed using SoftMax Pro software to calculate the concentrations in blood samples at different time points.

The detection method for ADC is briefly outlined as follows: Trop2-His was coated overnight at 4°C (0.75 μg/mL), followed by blocking with 5% skim milk at 37°C for 2 h. Standard curves and quality control (QC) samples were then added and incubated for 2 h. The standard curve ranged from 64 ng/mL to 0.5 ng/mL, starting at 64 ng/mL with 2-fold serial dilutions. QC samples were prepared at concentrations of 60 ng/mL, 6 ng/mL, and 0.6 ng/mL, with acceptable recovery rates between 80% and 120%. Subsequently, a 1:8000 dilution of horseradish peroxidase (HRP)-conjugated goat anti-human kappa light chain antibody (manufacturer: Sigma, catalog #: A7164-1ML) was added and incubated for 1 h. After washing 8 times with PBST, TMB substrate was added for color development, and the reaction was terminated with 0.1 M sulfuric acid. Absorbance was measured at 450 nm using a microplate reader, and the data were analyzed using SoftMax Pro software to determine ADC concentrations in blood samples at different time points.

The ELISA-derived pharmacokinetic curve demonstrated that the ADC concentration closely overlapped with the total antibody concentration, indicating minimal drug payload loss and high stability in circulation.

**Example 7**

**Cell Proliferation Inhibition Assay**

KPL-4 tumor cells in the logarithmic growth phase were harvested and resuspended in fresh RPMI1640 medium. After counting, the cell suspension was adjusted to a density of $2\times10^4$ cells/mL. The suspension was then seeded into 96-well cell culture plates at 100 μL/well and incubated overnight in a $CO_2$ incubator (37°C, 5% $CO_2$). The following day, one of the cell-seeded 96-well plates was Taken out and equilibrated to room temperature. CellTiter-Glo reagent (Promega, USA), pre-equilibrated and mixed thoroughly, was added to each well (100 μL/well). After 30 minutes of incubation in the dark, luminescence was measured using a microplate reader (recorded as Go value). A parallel plate was treated with either test compounds at varying concentrations or DMSO (final concentration 0.5%) in corresponding wells. Following 72 hours of incubation in the $CO_2$ incubator, the test plate was equilibrated to room temperature, and cell viability was assessed using the Cell Titer-Glo reagent (recorded as $G_3$ value).

The cell proliferation rate was calculated according to the following formula: Cell Proliferation Rate (%) = (Mean $G_3$ value of test compound wells - Mean Go value) / (Mean $G_3$ value of DMSO control wells - Mean Go value) $\times$ 100.The inhibition curve was fitted and the $GI_{50}$ value was calculated using GraphPad Prism software.

The compounds prepared in this application demonstrated excellent in vivo antitumor activity.

**Example 8**

**Bystander Killing Activity Assay**

BxPC3 (human pancreatic cancer cells, ATCC CRL-1687) and MiaPaCa2-luc cells (human pancreatic cancer cells, Biocytogen B-HCL-014) were cultured in RPMI1640 + 10% FBS and DMEM/high glucose + 10% FBS, respectively. Cells were detached with trypsin, neutralized with fresh medium, and centrifuged at 1,000 rpm for 3 minutes. After supernatant removal, cells were resuspended in RPMI1640 + 10% FBS and counted, with densities adjusted to $6\times10^4$ cells/mL for BxPC3 and $1.5\times10^4$ cells/mL for MiaPaCa2-luc. For 12-well plate 1, 500 μL of BxPC3 cells and 500 μL of MiaPaCa2-luc cells were added to each well, while for 12-well plate 2, 500 μL of MiaPaCa2-luc cells and 500 μL of RPMI 1640 medium supplemented with 10% FBS were added to each well. All plates were incubated at 37°C with 5% $CO_2$ for 24 hours.

The ADC Samples were prepared as a 40× concentrated intermediate solution (0.2 μM), and 25 μL aliquots were added to designated wells of the 12-well plate alongside solvent controls, followed by 6-day incubation at 37°C with 5% $CO_2$. Cells were then trypsinized, neutralized with fresh medium, and centrifuged at 1,000 rpm for 3 minutes before discarding the supernatant and resuspending in 1 mL FACS buffer (PBS + 2.5% FBS). For cell counting, 20 μL of cell suspension was mixed with 20 μL trypan blue. For cells from plate 1, centrifugation was performed at 1000 rpm for 3 minutes, the supernatant was discarded, and the pellet was resuspended in 100 μL FACS buffer. 2 μL of monoclonal antibody was added, followed by incubation on ice for 30 minutes. After centrifugation at 2000 rpm for 1 minute at 4°C, the supernatant was removed, and cells were resuspended in 150 μL FACS buffer. Detection was performed using a BD FACSVerse flow cytometer, and data were analyzed with FlowJo 7.6.

The results demonstrate that the ADC disclosed herein exhibits significant bystander killing activity. While the ADC showed no cytotoxicity against target-negative MiaPaCa2 cells, it effectively killed target-negative cells when they were co-cultured with target-expressing BxPC3 cells.

All references cited in the present invention are incorporated herein by reference as if each individual reference were specifically and individually indicated to be incorporated by reference. Furthermore, it should be understood that after reading the above teachings of the present invention, those skilled in the art may make various modifications or alterations to the present invention, and such equivalent forms shall likewise fall within the scope defined by the appended claims of

this application.

## Claims

1. A ligand-drug conjugate, or a tautomer, a mesomer, a racemate, an enantiomer, a diastereomer, or a pharmaceutically acceptable salt or hydrate thereof, **characterized in that** the ligand-drug conjugate comprises the structure shown in formula (I):

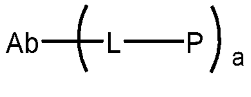
(I),

wherein, L is an optionally substituted linker that is connected to any O atom, S atom, or N atom in the P structure; Ab is a ligand, "a" is a number greater than 0, and "a" is a decimal or integer;
preferably, the "a" is 1-16 (such as 1, 2, 3, 4, 5, 6, 7, 8, 9, 10, 11, 12, 13, 14, 15, or 16); more preferably, the "a" is 2-8;
P is a group formed by dehydrogenating the structure described in formula (II):

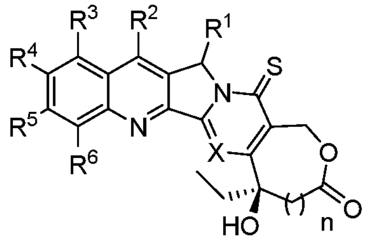
(II)

wherein, n is 0 or 1;
X is N or $CR^0$;
$R^0$ is selected from the group consisting of H, D, halogen, $C_1$-$C_8$ alkyl, $C_1$-$C_8$ alkoxy, OH, $NH_2$, $N_3$, and $NO_2$;
$R^1$ is selected from the group consisting of hydrogen atom, deuterium atom, halogen, $C_1$-$C_8$ alkyl, $C_1$-$C_8$ alkoxy, $C_1$-$C_8$ haloalkyl, $C_1$-$C_8$ haloalkoxy, $N_3$, $NO_2$, $NH_2$, NH-OH, - NR'R", -COOR', -CONR'R", and -NHR‴NR'R"; wherein, R', R", and R‴ are each independently selected from the group consisting of hydrogen, deuterium, alkyl, aryl, arylalkyl, acyl, alkoxycarbonyl, and aryloxycarbonyl;
$R^2$, $R^3$, $R^4$, $R^5$, and $R^6$ are each independently selected from the group consisting of hydrogen atom, deuterium atom, halogen, hydroxyl, cyano, $NH_2$, $NO_2$, substituted or unsubstituted $C_1$-$C_8$ alkyl, substituted or unsubstituted $C_1$-$C_8$ alkoxy, substituted or unsubstituted $C_1$-$C_8$ alkylthio, substituted or unsubstituted $C_1$-$C_8$ deuterated alkyl, $-(CH_2)_m$-tris-($C_1$-$C_4$ alkyl)silyl, $-(CH_2)_m$($C_3$-$C_8$ cycloalkyl), $-(CH_2)_m$(3-12 membered heterocyclic group), $-(CH_2)_mN(R^7)_2$, $-(CH_2)_mS(CH_2)_pR^7$, $-(CH_2)_mS(O)(CH_2)_pR^7$, $-(CH_2)_mS(O)_2(CH_2)_pR^7$, $-(CH_2)_mNH(CH_2)_pR^7$, $-(CH_2)_mNHC(O)(CH_2)_pR^7$, $-(CH_2)_mOC(O)(CH_2)_pR^7$, $-(CH_2)_mC(O)(CH_2)_pR^7$, and -CH=N(OtBu); wherein, m and p are each independently 0, 1, 2, 3, or 4;
or, $R^2$ and $R^3$ together with the carbon atoms to which they are attached form a structure selected from substituted or unsubstituted $C_5$-$C_8$ carbocycle or substituted or unsubstituted 5-12 membered heterocycle;
or, $R^3$ and $R^4$, or $R^4$ and $R^5$, together with the carbon atoms to which they are attached form a structure selected from the group consisting of a saturated or unsaturated 5-12 membered carbocycle which is unsubstituted or substituted with one or more $R^e$, and a saturated or unsaturated 5-12 membered heterocycle which is unsubstituted or substituted with one or more $R^e$; the $R^e$ is a substituted or unsubstituted substituent selected from hydrogen atom, deuterium atom, halogen, cyano, nitro, hydroxyl, amino, $C_1$-$C_6$ alkyl-NH-, $(C_1$-$C_6$ alkyl$)_2$N-, $C_1$-$C_6$ alkyl, $C_2$-$C_6$ alkenyl, $C_2$-$C_6$ alkynyl, $C_1$-$C_6$ alkoxy, allyl, benzyl, $C_6$-$C_{12}$ aryl, $C_1$-$C_6$ alkoxy-$C_1$-$C_6$ alkyl, $C_1$-$C_6$ alkoxycarbonyl, phenoxycarbonyl, $C_2$-$C_6$ alkynylcarbonyl, $C_2$-$C_6$ alkenylcarbonyl, $C_3$-$C_6$ cycloalkylcarbonyl, $C_1$-$C_6$ alkylsulfonyl, phenyl, 5-7 membered heteroaryl, $C_3$-$C_8$ cycloalkyl, 3-12 membered heterocyclic group, $-(CH_2)_mN(R^7)_2$, $-(CH_2)_mS(CH_2)_pR^7$, $-(CH_2)_mS(O)(CH_2)_pR^7$, $-(CH_2)_mS(O)_2(CH_2)_pR^7$, $-(CH_2)_mNH(CH_2)_pR^7$, $-(CH_2)_mNHC(O)(CH_2)_pR^7$, $-(CH_2)_mOC(O)(CH_2)_pR^7$, and $-(CH_2)_mC(O)(CH_2)_pR^7$; wherein, m and p are each independently 0, 1, 2, 3 or 4, preferably 0, 1 or 2;
$R^7$ is selected from the group consisting of hydrogen atom, deuterium atom, halogen, substituted or unsubstituted $C_1$-$C_8$ alkyl, $C_1$-$C_8$ haloalkyl, $C_1$-$C_8$ deuterated alkyl, substituted or unsubstituted $C_1$-$C_8$ alkoxy, hydroxyl, amino, cyano, nitro, thiol, substituted or unsubstituted $C_1$-$C_8$ alkylene-OH, substituted or unsubstituted $C_1$-$C_8$ alkylene-

$NH_2$, $SO_2Me$, -OC(O)(substituted or unsubstituted $C_1$-$C_4$ alkyl), -C(O)(substituted or unsubstituted $C_1$-$C_4$ alkyl), substituted or unsubstituted phenyl, substituted or unsubstituted 5-7 membered heteroaryl, substituted or unsubstituted $C_3$-$C_8$ cycloalkyl, and substituted or unsubstituted 3-12 membered heterocyclic group;

unless otherwise specified, each "substituted" refers to one or more hydrogen atoms on the group are substituted by substituents selected from the group consisting of hydrogen atom, deuterium atom, halogen, cyano, nitro, hydroxyl, amino, $C_1$-$C_6$ alkyl-NH-, $(C_1$-$C_6$ alkyl$)_2$N-, $C_1$-$C_6$ alkyl, $C_2$-$C_6$ alkenyl, $C_2$-$C_6$ alkynyl, $C_1$-$C_6$ alkoxy, $C_1$-$C_6$ haloalkyl, $C_2$-$C_6$ haloalkenyl, $C_2$-$C_6$ haloalkynyl, $C_1$-$C_6$ haloalkoxy, allyl, benzyl, $C_6$-$C_{12}$ aryl, $C_1$-$C_6$ alkoxy-$C_1$-$C_6$ alkyl, $C_1$-$C_6$ alkoxycarbonyl, phenoxycarbonyl, $C_2$-$C_6$ alkynylcarbonyl, $C_2$-$C_6$ alkenylcarbonyl, $C_3$-$C_6$ cycloalkylcarbonyl, $C_1$-$C_6$ alkylsulfonyl, phenyl, 5-7 membered heteroaryl, $C_3$-$C_8$ cycloalkyl, and 3-12 membered heterocyclic group.

**2.** The ligand-drug conjugate, or a tautomer, a mesomer, a racemate, an enantiomer, a diastereomer, or a mixture thereof, or a pharmaceutically acceptable salt or hydrate thereof according to claim 1, **characterized in that** L has a structure as shown in the following formula:

$$L_1\text{-}L_2\text{-}L_3\text{-}L_4\text{-}L_5;$$

wherein, $L_1$ is optionally substituted

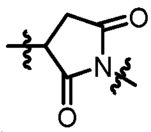,
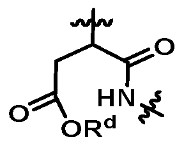
or
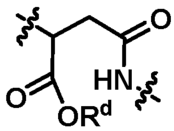,

$R^d$ is H, $C_1$-$C_6$ alkyl, $C_1$-$C_6$ deuterated alkyl, $C_3$-$C_8$ cycloalkyl, or $C_3$-$C_8$ deuterated cycloalkyl;

the $L_2$ group is selected from the group consisting of optionally substituted $-(CHR)_{m1}-X_1-(CH_2CH_2O)_{n3}-(CHR)_{m2}-C(O)-$, optionally substituted $-(CHR)_{m1}-X_1-X_2-(CH_2CH_2O)_{n3}-(CHR)_{m2}-C(O)-$, optionally substituted $-X_1-(CHROCHR)_{m2}-C(O)-$, optionally substituted - $(CHR)_{p1}-C(O)-$, optionally substituted $-(CHR)_{m1}-X_1-(CHR)_{m2}-C(O)-$, optionally substituted $-(CHR)_{m1}-X_1-(CHR)_{n3}-X_2-(CHR)_{m2}-C(O)-$, optionally substituted $-X_1-(CHR)_{m1}-X_2-(CHR)_{m2}-C(O)-$, and optionally substituted $-(CH_2CH_2O)_{n3}-C(O)-$;

$X_1$ and $X_2$ are each independently selected from the group consisting of -O-, -C(O)-, - C(O)-NR-, optionally substituted $C_6$-$C_{10}$ aryl, optionally substituted 5-9 membered heteroaryl, optionally substituted 3-8 membered heteroalicyclic group, and optionally substituted $C_3$-$C_6$ alicyclic group;

wherein, each R is independently selected from the group consisting of H, D, $(CH_2)_{n4}OH$, $(CH_2)_{n4}NH_2$, $(CH_2O)_{n4}(CH_2CH_2O)_{n5}H$, $(CH_2O)_{n4}(CH_2CH_2O)_{n5}CH_3$, $(CH_2)_{n4}OCH_3$, $(CH_2CH_2O)_{n5}CH_3$, $CH_2C(O)NH(CH_2O)_{n4}(CH_2CH_2O)_{n5}H$, and $CH_2C(O)NH(CH_2O)_{n4}(CH_2CH_2O)_{n5}CH_3$;

wherein, m1, m2, n3, n4, and n5 are each independently 0, 1, 2, 3, 4, 5, 6, 7, 8, 9, 10, 11 or 12; p1 is 0, 1, 2, 3, 4, 5, 6, 7 or 8;

the $L_3$ is a peptide residue which can be optionally substituted by one or more substituents selected from the group consisting of

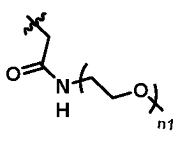,
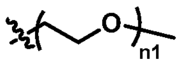,
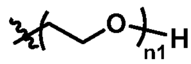,

and $CH_2C(O)R^c$; the $R^c$ is selected from the group consisting of

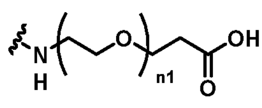
and
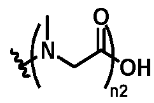;

wherein, n1 and n2 are each independently 0, 1, 2, 3, 4, 5, 6, 7, 8, 9, 10, 11 or 12;

the $L_4$ is an optionally substituted $-L_{4a}-(NR^b)_{n6}-R^{12}-L_{4b}-$, wherein $L_{4a}$ is absent or $L_{4a}$ is optionally substituted

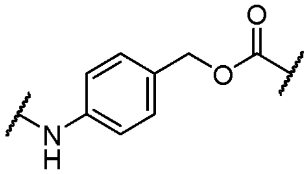

;

wherein n6 is 0 or 1; $R^{12}$ is chemical bond, $CH_2$, or $CD_2$;

$L_{4b}$ is absent, or $L_{4b}$ is optionally substituted

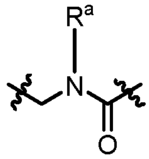

,

wherein $R^a$ and $R^b$ are each independently selected from the group consisting of hydrogen, optionally substituted $C_1$-$C_4$ alkyl, and optionally substituted $C_1$-$C_4$ deuterated alkyl;

the $L_5$ is absent, or $L_5$ is optionally substituted

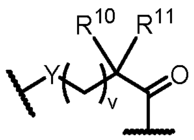

, wherein Y is selected from the group consisting of O, S, and NH; v is 0, 1, 2, 3, 4, 5, 6, 7, 8, 9 or 10;

$R^{10}$ and $R^{11}$ are each independently selected from the group consisting of hydrogen, deuterium, optionally substituted $C_1$-$C_4$ alkyl, optionally substituted $C_1$-$C_4$ haloalkyl, optionally substituted $C_3$-$C_6$ cycloalkyl, and optionally substituted $C_4$-$C_8$ cycloalkylalkyl, or $R^{10}$ and $R^{11}$ together with the carbon atoms to which they are attached form an optionally substituted 3-6 membered cycloalkyl, $R^{10}$ and $R^{11}$ are each independently selected from the group consisting of hydrogen atom, deuterium atom, halogen, optionally substituted $C_1$-$C_8$ alkyl, optionally substituted $C_1$-$C_8$ haloalkyl, and optionally substituted $C_1$-$C_8$ deuterated alkyl.

**3.** The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_1$ is

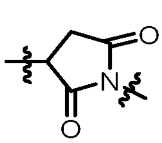 , 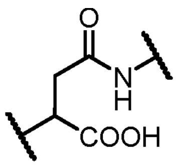 , 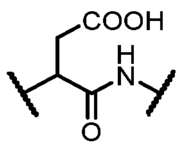 ,

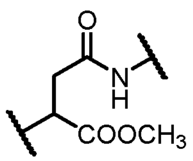 , 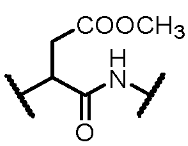 , 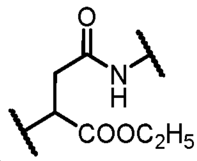 or 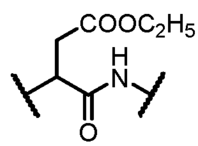 .

**4.** The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $X_1$ and $X_2$ are each independently selected from the group consisting of -O-, -C(O)-, -C(O)-NR-, optionally substituted phenyl, optionally substituted pyridyl, optionally substituted $C_3$-$C_6$ cycloalkyl, optionally substitute

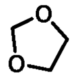

,

and optionally substituted

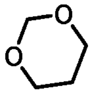

.

5. The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_2$ is optionally substituted $-(CH_2)_{m1}-X_1-(CH_2CH_2O)_{n3}-(CH_2)_{m2}-C(O)-$; wherein, $X_1$ is -C(O)-NH-; preferably, m1 and m2 are each independently 1, 2, or 3; n3 is 0, 1, 2, 3, 4, 5, 6, 7, 8, 9, 10, 11 or 12.

6. The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_2$ is optionally substituted $-(CHR)_{m1}-X_1-X_2-(CH_2CH_2O)_{n3}-(CHR)_{m2}-C(O)-$; wherein, $X_1$ is optionally substituted

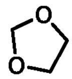

,

or optionally substituted

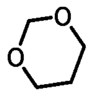

;

$X_2$ is -C(O)-NR-; preferably, m1 and m2 are each independently 0, 1 or 2; n3 is 1, 2, 3, 4, 5, 6, 7, 8, 9, 10, 11 or 12.

7. The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_2$ is optionally substituted $-X_1-(CHROCHR)_{m2}-C(O)-$; wherein, $X_1$ is an optionally substituted aryl or an optionally substituted heteroaryl; preferably, m2 is 0, 1, 2 or 3.

8. The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_2$ is optionally substituted $(CHR)_{p1}-C(O)-$; p1 is 0, 1 or 2; R is selected from: H, $(CH_2)_{n4}OH$, and $(CH_2O)_{n4}(CH_2CH_2O)_{n5}H$; preferably, n4 and n5 are each independently 0, 1, 2 or 3.

9. The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_2$ is optionally substituted $-(CH_2)_{m1}-X_1-(CH_2CH_2O)_{n3}-(CHR)_{m2}-C(O)-$; wherein, $X_1$ is -C(O)-; preferably, m1 and m2 are each independently selected from 0, 1, 2, or 3; n3 is selected from 0, 1 or 2.

10. The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_2$ is optionally substituted $-X_1-(CH_2)_{m1}-X_2-(CHR)_{m2}-C(O)-$; wherein, $X_1$ is an optionally substituted aryl or an optionally substituted heteroaryl; $X_2$ is -C(O)-; preferably, m1 and m2 are each independently selected from 0, 1 or 2.

11. The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_2$ is $-(CHR)_{m1}-X_1-(CHR)_{m2}-C(O)-$; wherein, $X_1$ is optionally substituted 3-8 membered heteroalicyclic group or an optionally substituted $C_3$-$C_6$ alicyclic group; preferably, m1 is 0, 1 or 2, and m2 is 0.

**12.** The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_2$ is optionally substituted $-(CHR)_{m1}-X_1-(CH_2CH_2O)_{n3}-(CHR)_{m2}-C(O)-$; wherein $X_1$ is O; preferably, m1, n3 and m2 are each independently 0, 1 or 2.

**13.** The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_2$ is optionally substituted $-(CHR)_{m1}-X_1-(CHR)_{n3}-X_2-(CHR)_{m2}-C(O)-$; wherein, $X_1$ is optionally substituted -C(O)-NR-, and $X_2$ is O; preferably, m1, n3 and m2 are each independently 1, 2 or 3; R is defined as claim 2.

**14.** The ligand-drug conjugate, or a tautomer, a mesomer, a racemate, an enantiomer, a diastereomer, or a mixture thereof, or a pharmaceutically acceptable salt or hydrate thereof according to claim 2, wherein $L_2$ is an optionally substituted structure selected from the group consisting of

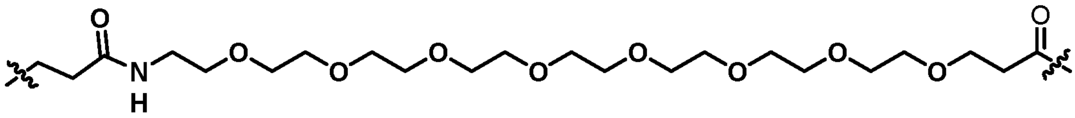,

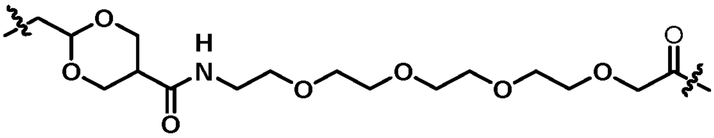, 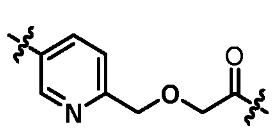, 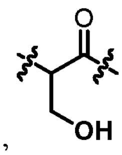,

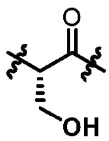, 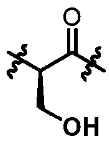, 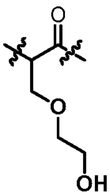, 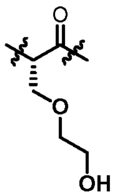, 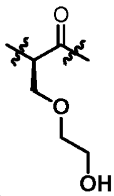, 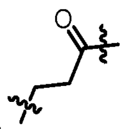, 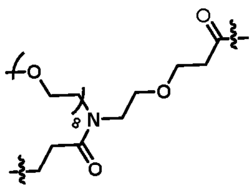, 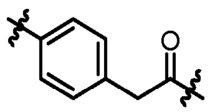,

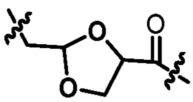, 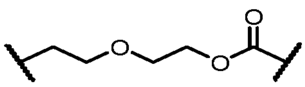, 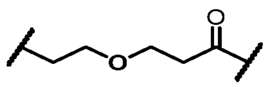, 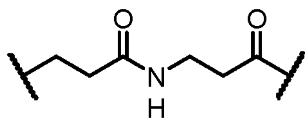,

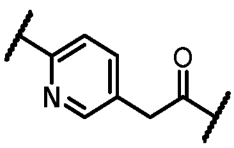, 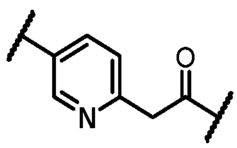, 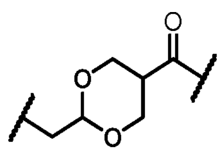, 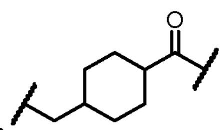

and

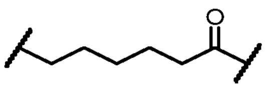.

**15.** The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_3$ is an unsubstituted or $CH_2C(O)R^c$- substituted peptide residue composed of amino acids selected from the group consisting of phenylalanine, isoleucine, leucine, tryptophan, valine, methionine, tyrosine, alanine, threonine, histidine, serine, glutamine, arginine, lysine, asparagine, glutamic acid, proline, citrulline, aspartic acid, and glycine.

**16.** The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_3$ is an unsubstituted or $CH_2C(O)R^c$- substituted peptide residue composed of amino acids selected from the group consisting of glycine, alanine, lysine, phenylalanine, valine, and citrulline.

**17.** The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_3$ is an unsubstituted or $CH_2C(O)R^c$- substituted peptide residue selected from the group consisting of -Glycine-Phenylalanine-Glycine-(-Gly-Phe-Gly-), -Glycine-Glycine-Phenylalanine-Glycine-(-Gly-Gly-Phe-Gly-), -Valine-Citrulline-(-Val-Cit-), -Citrulline-Valine-(-Cit-Val-), -Citrulline-Alanine-(-Cit-Ala-), - Valine-Alanine-(-Val-Ala-), -Valine-Arginine-(-Val-Arg-), -Valine-Lysine-(-Val-Lys-), - Valine-Lysine (Ac)-(-Val-Lys(Ac)-), -Lysine-Valine-(-Lys-Val-), -Leucine-Citrulline-(-Leu-Cit-), -Isoleucine-Citrulline-(-Ile-Cit-), -Tryptophan-Citrulline-(-Trp-Cit-), - Phenylalanine-Lysine-(-Phe-Lys-), -Phenylalanine-Lysine(Ac)-(-Phe-Lys(Ac)-), - Phenylalanine-Citrulline-(-Phe-Cit), -Phenylalanine-Alanine-(-Phe-Ala-), -Phenylalanine-Arginine-(-Phe-Arg-), -Alanine-Lysine-(-Ala-Lys-), -Alanine-Alanine-(-Ala-Ala-), - Alanine-Alanine-Alanine-(-Ala-Ala-Ala-), -Alanine-Alanine-Asparagine-(-Ala-Ala-Asn-), -Alanine-Alanine-Aspartic Acid-(-Ala-Ala-Asp-), -Lysine-Alanine-Alanine-Asparagine-(-Lys-Ala-Ala-Asn-), -Lysine-Alanine-Alanine-Aspartic acid-(-Lys-Ala-Ala-Asp-), -(D)-Valine-Leucine-Lysine-(-(D)-Val-Leu-Lys-), -Glycine-Glycine-Arginine-(-Gly-Gly-Arg-), -Glycine-Glycine-Asparagine- (-Gly-Gly-Asn-), -Glycine-Glycine-Phenylalanine-(-Gly-Gly-Phe-), -Valine-Lysine-Glycine-(-Val-Lys-Gly-), -Glutamic Acid-Alanine-Alanine-(Glu-Ala-Ala-), -Aspartate-Alanine-Alanine-(Asp-Ala-Ala)-, -Valine-Lysine-Glycine-Glycine-(-Val-Lys-Gly-Gly-), and -Lysine-Alanine-Asparagine-(-Lys-Ala-Asn-).

**18.** The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_3$ is an unsubstituted or $CH_2C(O)R^c$- substituted structure selected from the group consisting of

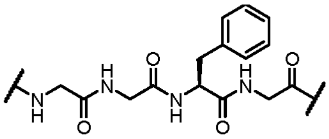
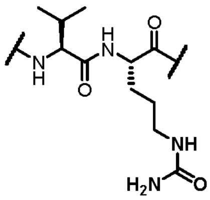
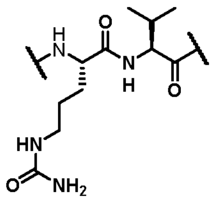
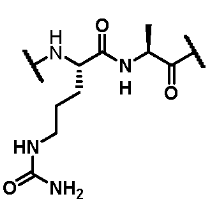

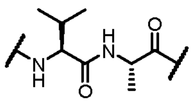
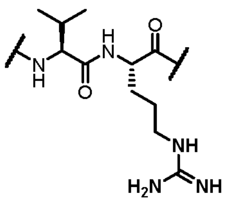
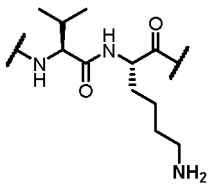
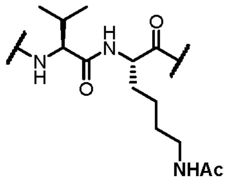

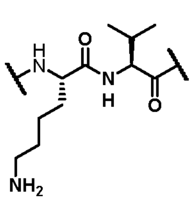
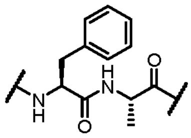
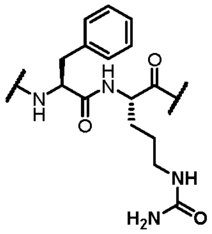
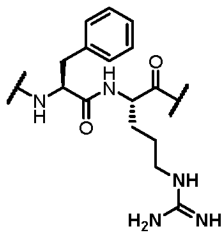

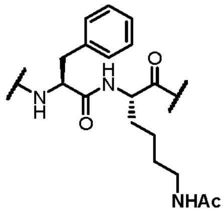
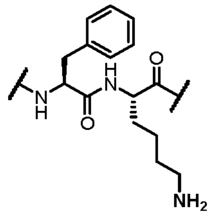
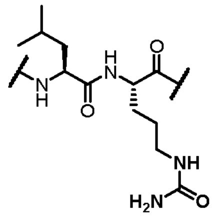
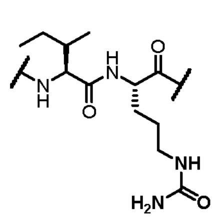

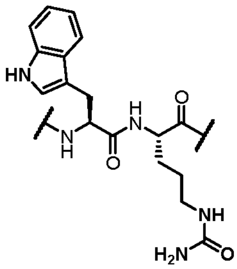
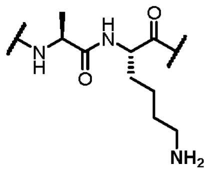
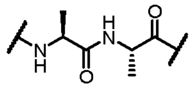
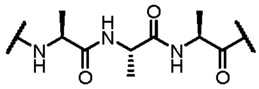

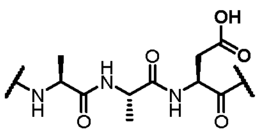
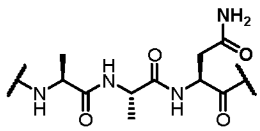
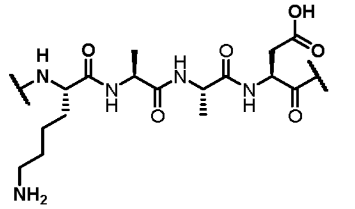

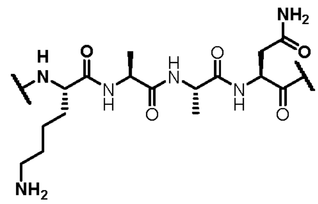
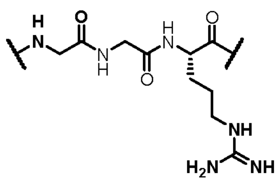
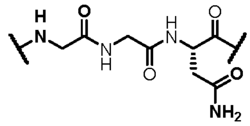

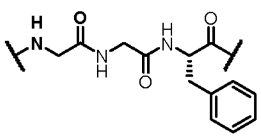
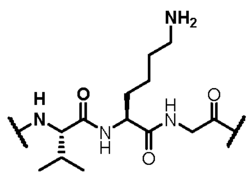
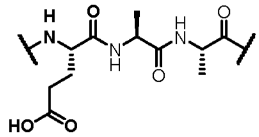

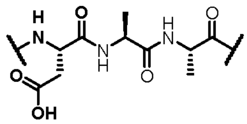
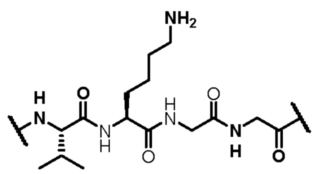
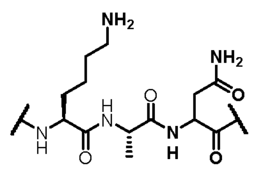

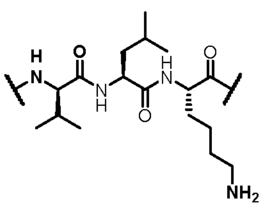
and
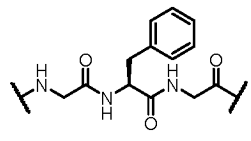

**19.** The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_4$ is a

chemical bond or an optionally substituted group selected from the group consisting of

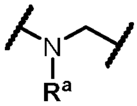 , 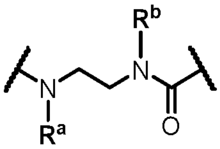 , 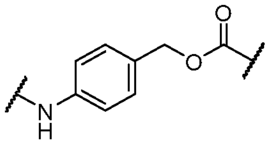 , 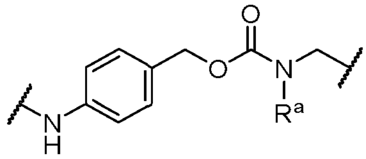

and

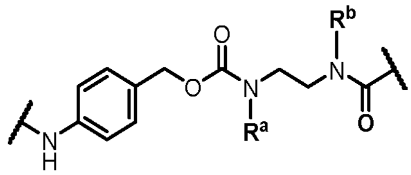 ;

wherein, $R^a$ and $R^b$ are each independently selected from the group consisting of hydrogen, optionally substituted $C_1$-$C_4$ alkyl, and optionally substituted $C_1$-$C_4$ deuterated alkyl.

**20.** The ligand-drug conjugate, or a tautomer, a mesomer, a racemate, an enantiomer, a diastereomer, or a mixture thereof, or a pharmaceutically acceptable salt or hydrate thereof according to claim 19, **characterized in that** $L_4$ is a chemical bond or an optionally substituted structure selected from the group consisting of

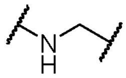 , 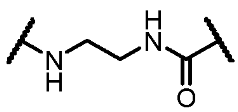 ,

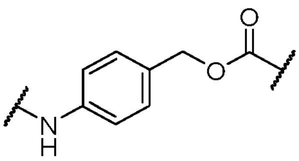 , 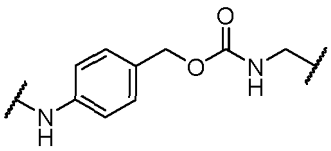 , 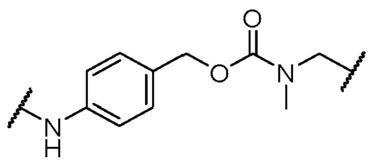

and

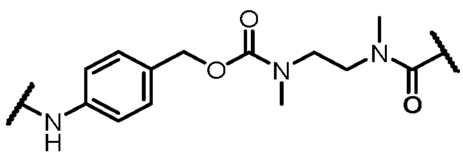 .

**21.** The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** $L_5$ is a chemical bond or a structure selected from the group consisting of optionally substituted

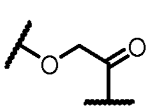 , 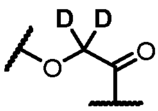 ,

optionally substituted

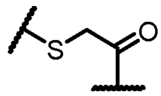,

optionally substituted

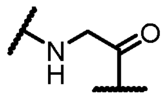,

optionally substituted

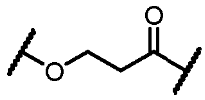,

optionally substituted

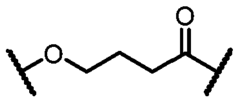,

, optionally substituted

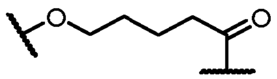,

optionally substituted

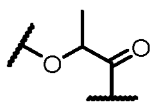,

optionally substituted

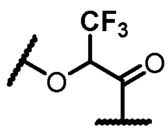,

optionally substituted

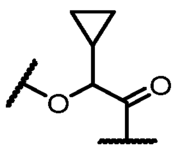,

optionally substituted

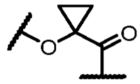
,

optionally substituted

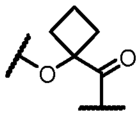
,

optionally substituted

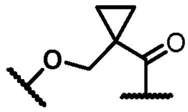
,

and optionally substituted

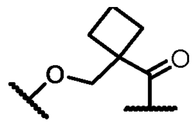
.

**22.** The ligand-drug conjugate, or a tautomer, a mesomer, a racemate, an enantiomer, a diastereomer, or a mixture thereof, or a pharmaceutically acceptable salt or hydrate thereof according to claim 2, **characterized in that** the compound of formula (II) is selected from the group consisting of

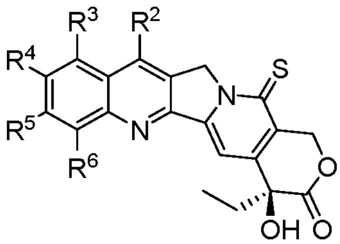
VI

and

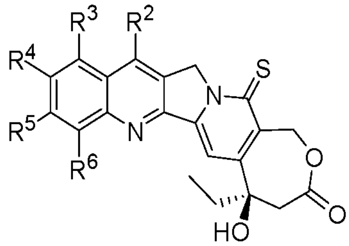
VII.

**23.** The ligand-drug conjugate, or a tautomer, a mesomer, a racemate, an enantiomer, a diastereomer, or a mixture thereof, or a pharmaceutically acceptable salt or hydrate thereof according to claim 22, **characterized in that** $R^4$ is selected from the group consisting of hydrogen atom, deuterium atom, halogen, hydroxyl, cyano, $NH_2$, $NO_2$, substituted or unsubstituted $C_1$-$C_8$ alkyl, substituted or unsubstituted $C_1$-$C_8$ alkoxy, substituted or unsubstituted $C_1$-$C_8$ alkylthio, substituted or unsubstituted $C_1$-$C_8$ deuterated alkyl, - $(CH_2)_m(C_3$-$C_8$ cycloalkyl), $-(CH_2)_m$(3-12 membered heterocyclic group), $-(CH_2)_mN(R^7)_2$, - $(CH_2)_mS(O)(CH_2)_pR^7$, $-(CH_2)_mS(O)_2(CH_2)_pR^7$, and $-(CH_2)_mNH(CH_2)_pR^7$; wherein, m and p are each independently 0, 1, or 2; $R^7$ is defined as above;

$R^5$ is selected from the group consisting of hydrogen atom, deuterium atom, halogen, $NH_2$, OH, substituted or unsubstituted $C_1$-$C_8$ alkyl, substituted or unsubstituted $C_1$-$C_8$ alkoxy;
or $R^4$ and $R^5$ together with the carbon atoms to which they are attached form a structure selected from the group consisting of saturated or unsaturated 5-6 membered carbocycle that is unsubstituted or substituted by one or more $R^e$, and saturated or unsaturated 5-6 membered heterocycle that is unsubstituted or substituted by one or more $R^e$; wherein, $R^e$ is defined as above.

**24.** The ligand-drug conjugate, or a tautomer, a mesomer, a racemate, an enantiomer, a diastereomer, or a mixture

thereof, or a pharmaceutically acceptable salt or hydrate thereof according to claim 22, **characterized in that** $R^2$ and $R^3$ are each independently selected from the group consisting of hydrogen atom, deuterium atom, halogen, $NH_2$, substituted or unsubstituted $C_1$-$C_8$ alkyl, substituted or unsubstituted $C_1$-$C_8$ deuterated alkyl, $-(CH_2)_m(C_3$-$C_6$ cycloalkyl), $-(CH_2)_m$(3-6 membered heterocyclic group), $-(CH_2)_mN(R^7)_2$, and - $(CH_2)_mOC(O)R^7$; wherein, each m is independently 0, 1, 2, 3, or 4; $R^7$ is defined as above;
or $R^2$ and $R^3$ together with the carbon atoms to which they are attached form a structure selected from the group consisting of saturated or unsaturated 5-6 membered ring that is unsubstituted or substituted with one or more $R^e$, and saturated or unsaturated 5-6 membered heterocycle that is unsubstituted or substituted with one or more $R^e$; wherein, $R^e$ is defined as above.

**25.** The ligand-drug conjugate, or a tautomer, a mesomer, a racemate, an enantiomer, a diastereomer, or a mixture thereof, or a pharmaceutically acceptable salt or hydrate thereof according to claim 22, **characterized in that** $R^4$ and $R^5$ are each independently selected from the group consisting of hydrogen atom, deuterium atom, halogen, hydroxyl, $NH_2$, and substituted or unsubstituted $C_1$-$C_4$ alkyl;
or $R^4$ and $R^5$ together with the carbon atoms to which they are attached form a structure selected from the group consisting of unsubstituted or one or more $R^e$ substituted 5-6 membered oxa-heterocycle; wherein, $R^e$ is described as above.

**26.** The ligand-drug conjugate, or a tautomer, a mesomer, a racemate, an enantiomer, a diastereomer, or a mixture thereof, or a pharmaceutically acceptable salt or hydrate thereof according to claim 22, **characterized in that**, $R^2$ is selected from the group consisting of deuterium atom, halogen, $NH_2$, substituted or unsubstituted $C_1$-$C_8$ alkyl, substituted or unsubstituted $C_1$-$C_8$ deuterated alkyl, $-(CH_2)_m(C_3$-$C_6$ cycloalkyl), $-(CH_2)_m$(3-6 membered heterocyclic group), $-(CH_2)_mN(R^7)_2$, and $-(CH_2)_mOC(O)R^7$; wherein, each m is independently 0, 1, 2, 3, or 4;

$R^3$ is selected from the group consisting of hydrogen atom, deuterium atom, halogen, substituted or unsubstituted $C_1$-$C_8$ alkyl, substituted or unsubstituted $C_1$-$C_8$ deuterated alkyl, $-(CH_2)_m(C_3$-$C_6$ cycloalkyl), $-(CH_2)_m$(3-6 membered heterocyclic group), $-(CH_2)_mN(R^7)_2$, and $-(CH_2)_mOC(O)R^7$; wherein, each m is independently 0, 1, 2, 3, or 4;
or $R^2$ and $R^3$ together with the carbon atoms to which they are attached form a structure selected from the group consisting of saturated or unsaturated 5-6 membered ring that is unsubstituted or substituted with one or more $R^e$, and saturated or unsaturated 5-6 membered heterocycle that is unsubstituted or substituted with one or more $R^e$; wherein, $R^e$ is defined as above;
$R^4$ is selected from the group consisting of hydrogen atom, deuterium atom, halogen, hydroxyl, CN, $NH_2$, $NO_2$, substituted or unsubstituted $C_1$-$C_8$ alkyl, and substituted or unsubstituted $C_1$-$C_8$ alkoxy;
$R^5$ is selected from the group consisting of hydrogen atom, deuterium atom, halogen, substituted or unsubstituted $C_1$-$C_8$ alkyl;
or $R^4$ and $R^5$ together with the carbon atoms to which they are attached form an unsubstituted or one or more $R^e$ substituted structure selected from the group consisting of $-OCH_2O-$ and $-O(CH_2)_2O-$;
$R^7$ is selected from the group consisting of hydrogen atom, deuterium atom, halogen, substituted or unsubstituted $C_1$-$C_8$ alkyl, hydroxyl, amino, cyano, nitro, and thiol;
wherein, $R^e$ is defined as above.

**27.** The ligand-drug conjugate, or a tautomer, a mesomer, a racemate, an enantiomer, a diastereomer, or a mixture thereof, or a pharmaceutically acceptable salt or hydrate thereof according to claim 22, **characterized in that** the compound of formula (II) is selected from the group consisting of

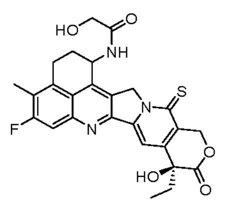
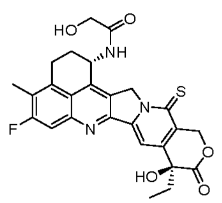
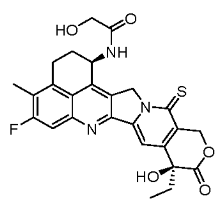
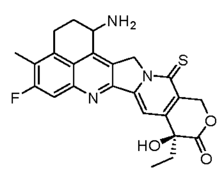

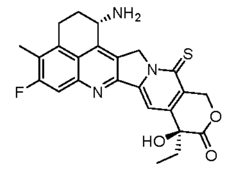
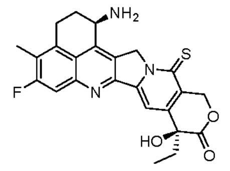
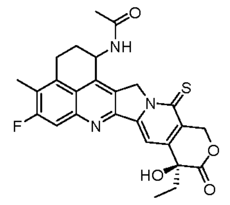
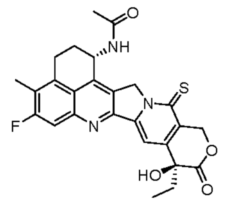

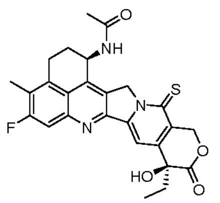
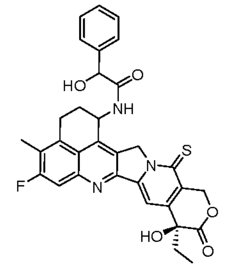
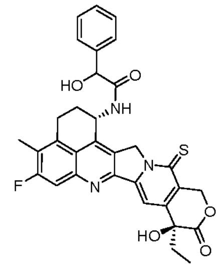
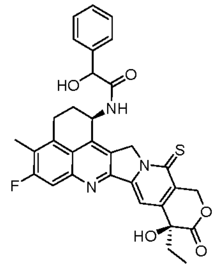

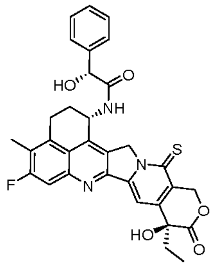
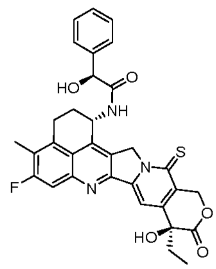
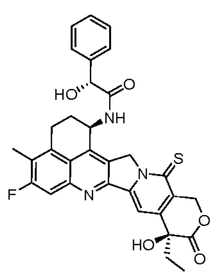
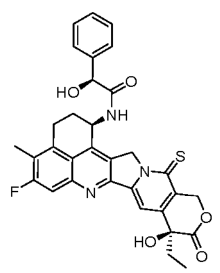

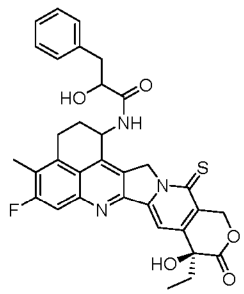
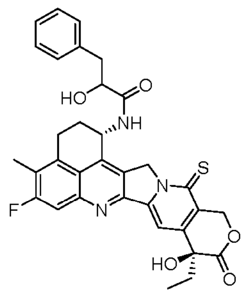
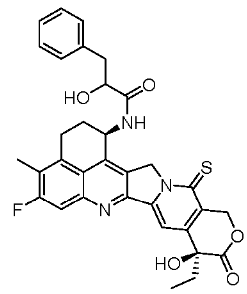
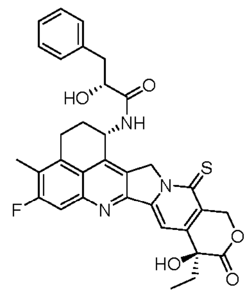

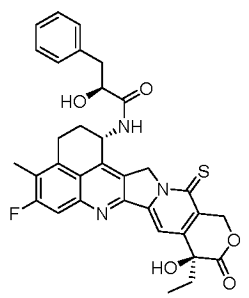
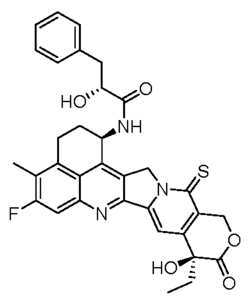
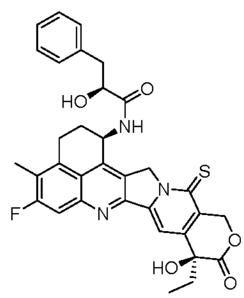
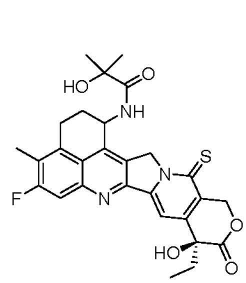

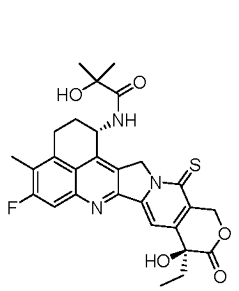
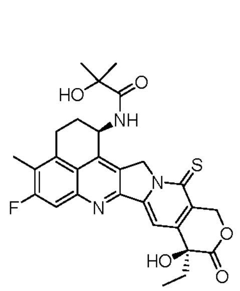
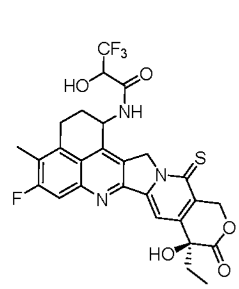
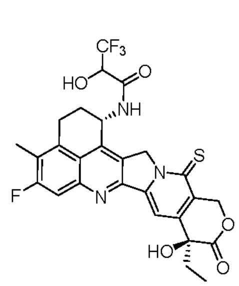

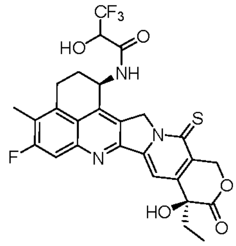 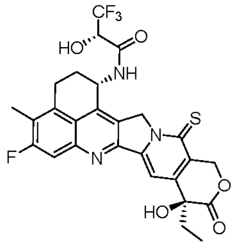 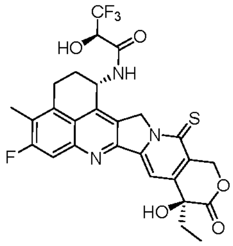 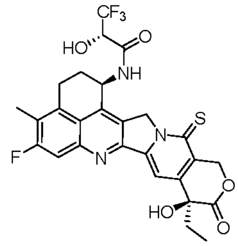

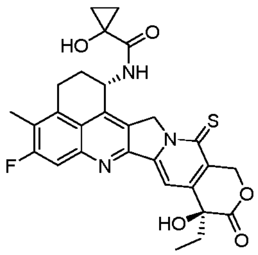 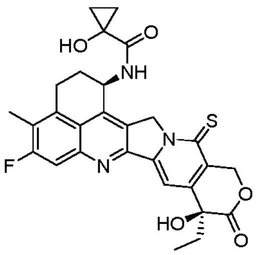 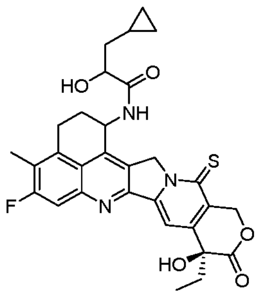 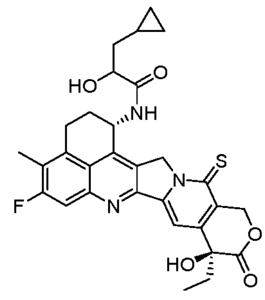

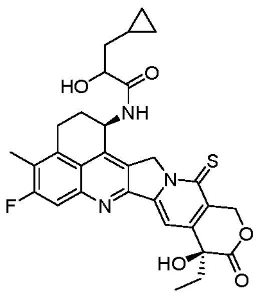 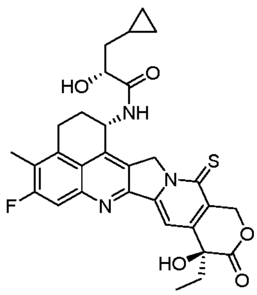 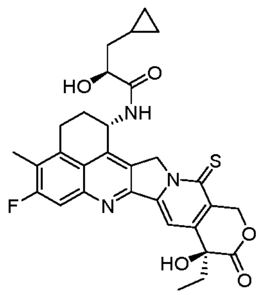 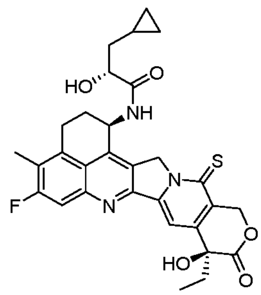

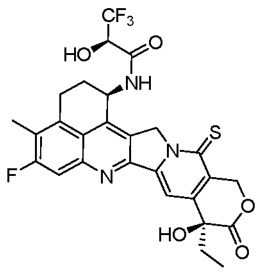 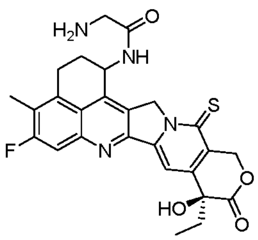 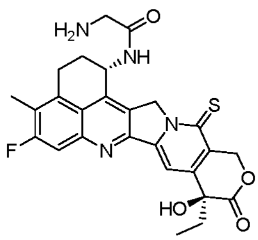 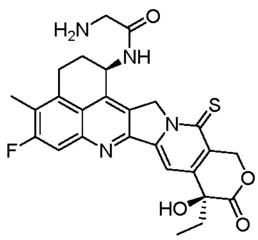

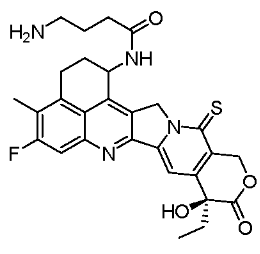 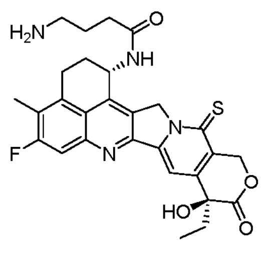 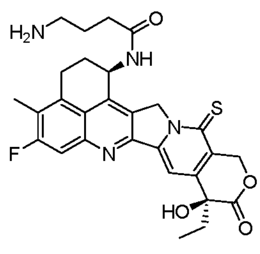 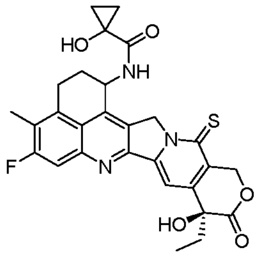

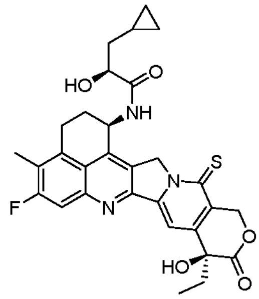
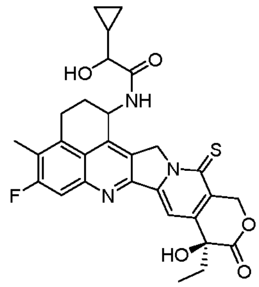
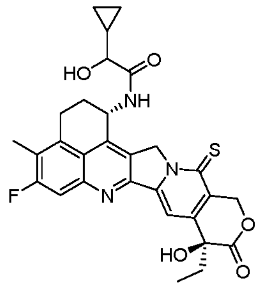
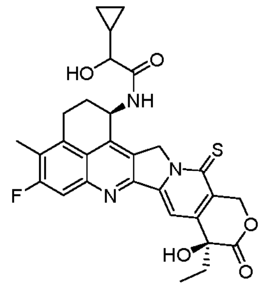

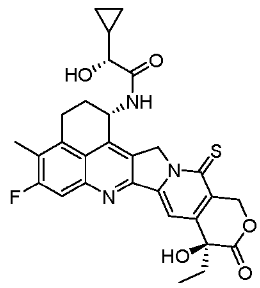
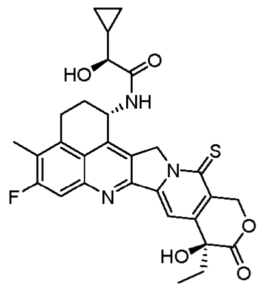
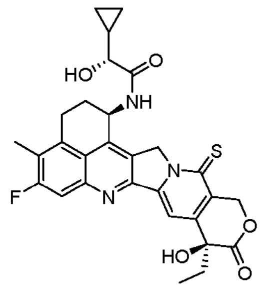
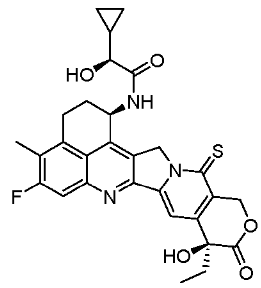

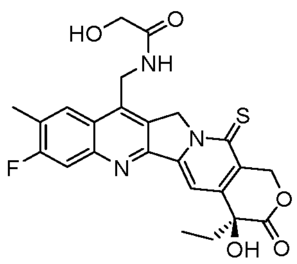
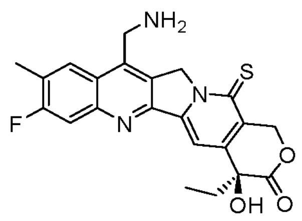
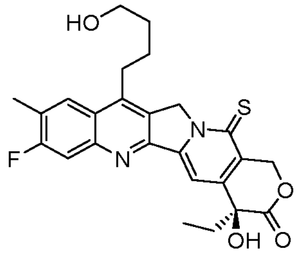

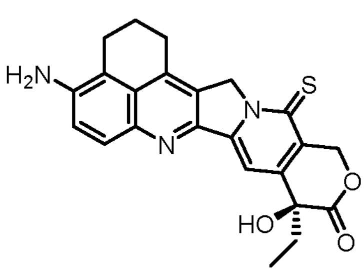
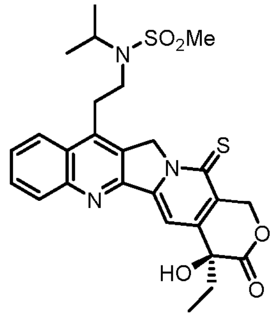
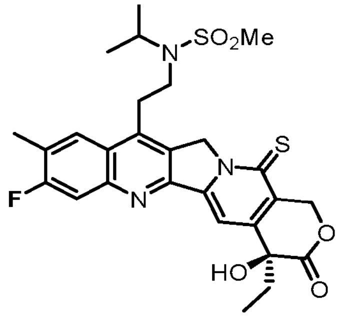

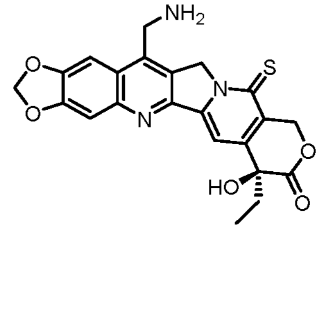
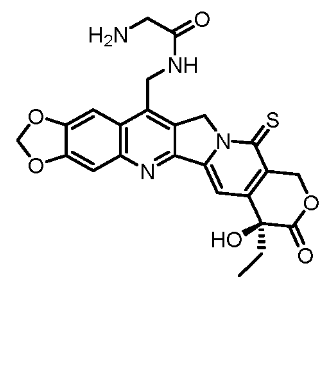
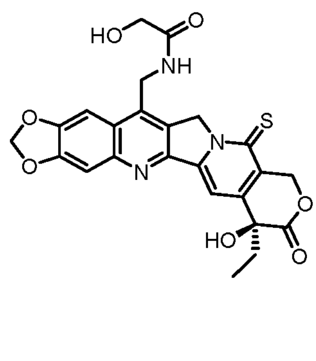

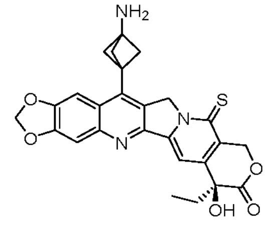
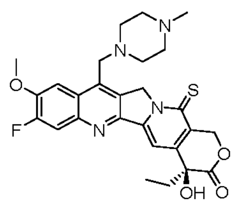
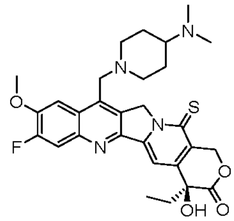

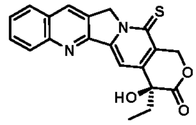
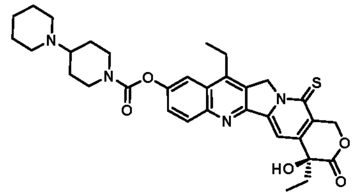
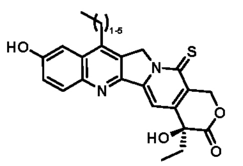
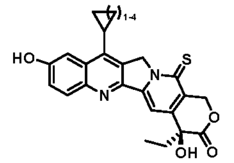

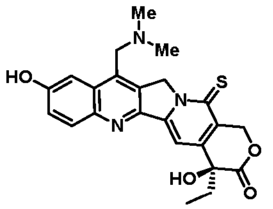
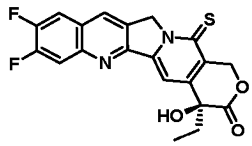
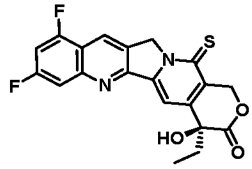
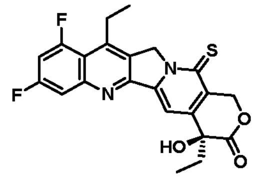

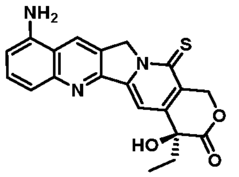
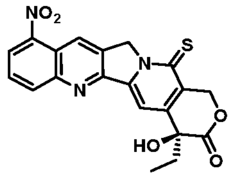
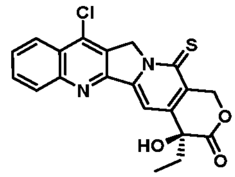
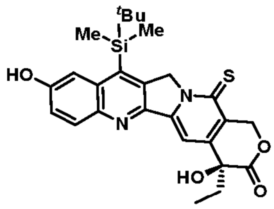

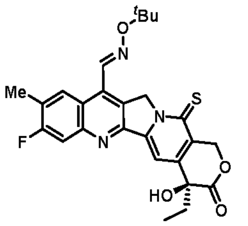
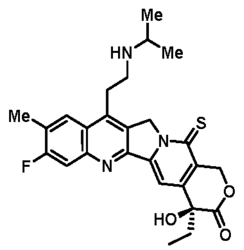
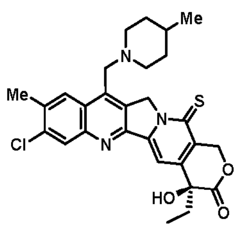
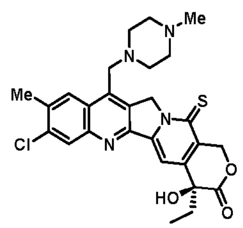

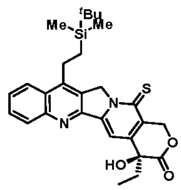
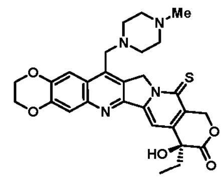
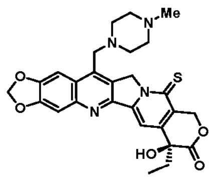
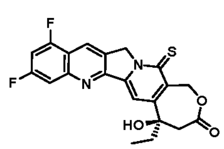
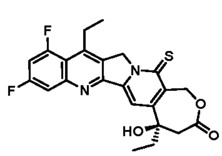

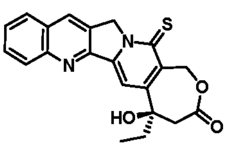
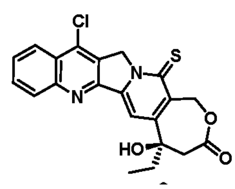
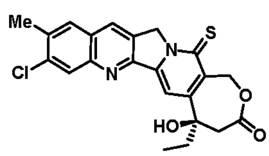
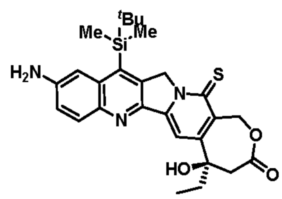

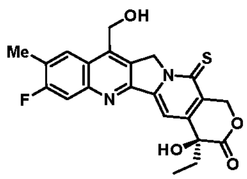
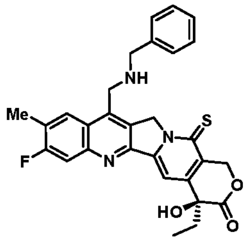
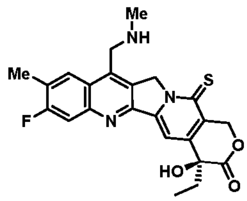
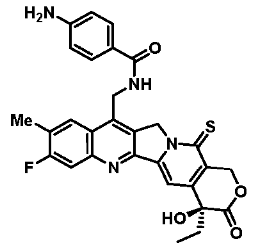
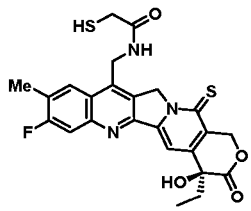
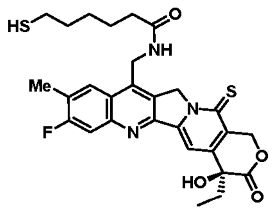
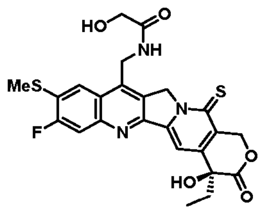
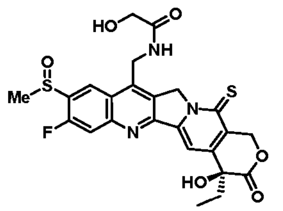
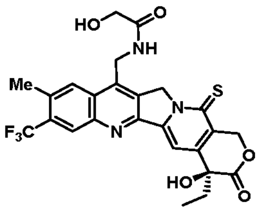
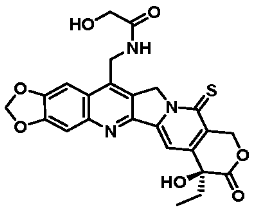
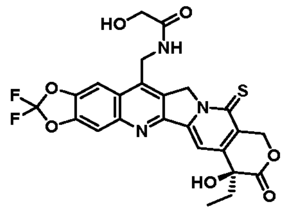
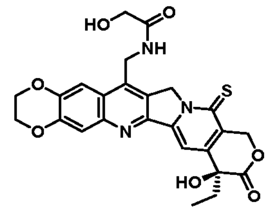
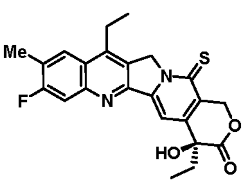
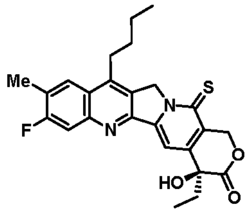
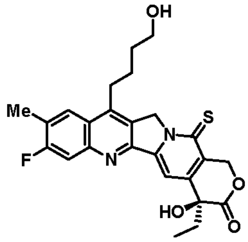
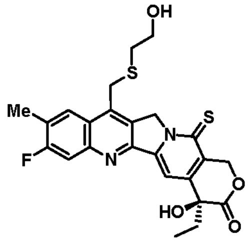
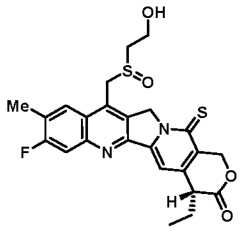
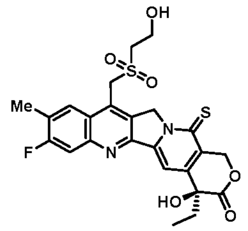
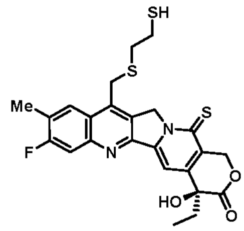
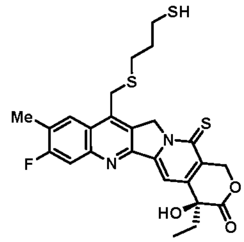
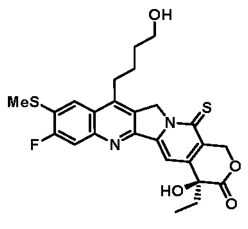
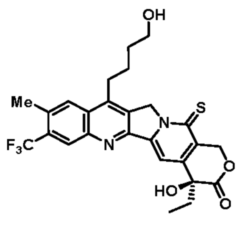
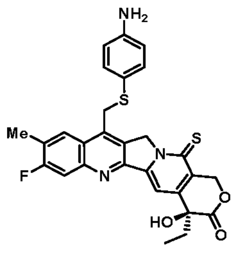

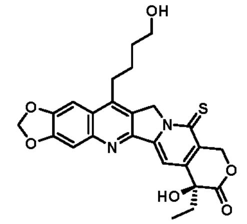

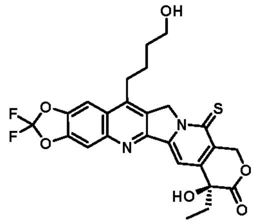

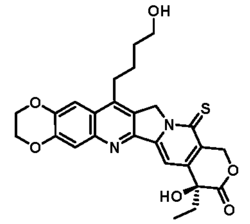

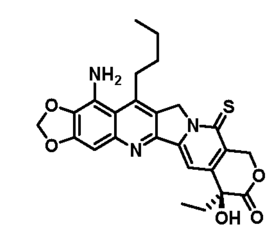

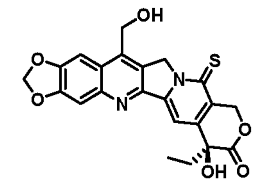

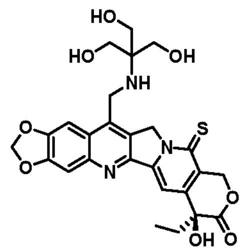

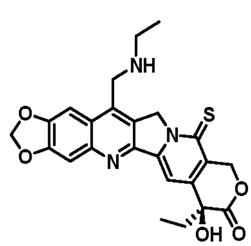

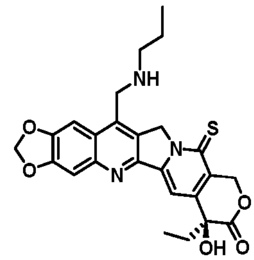

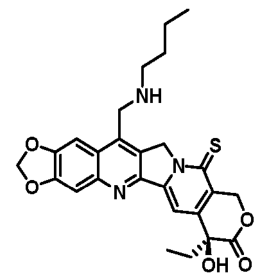

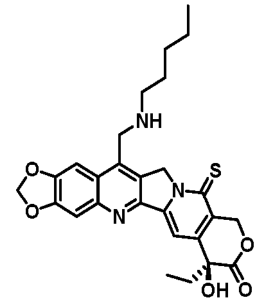

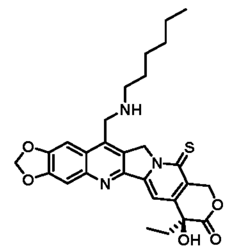

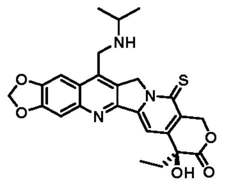

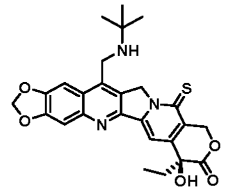

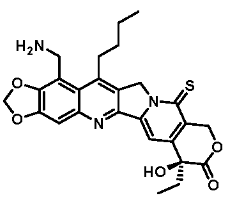

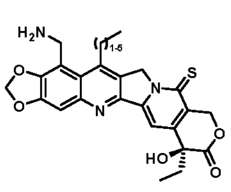

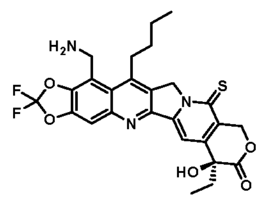

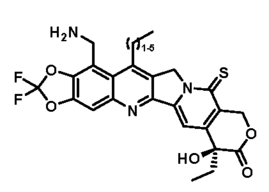

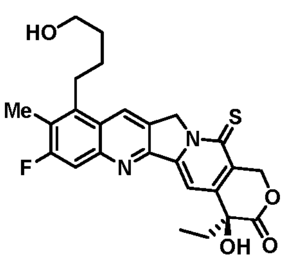

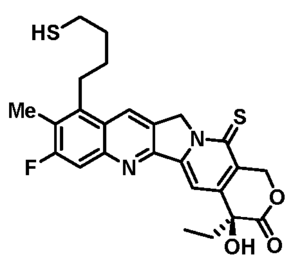

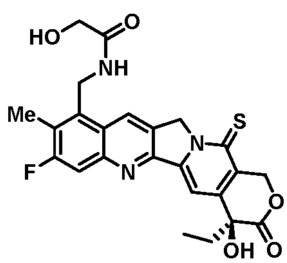

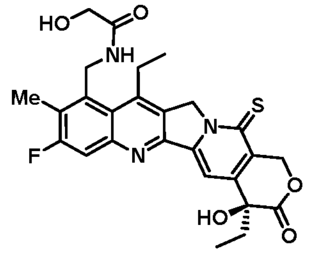
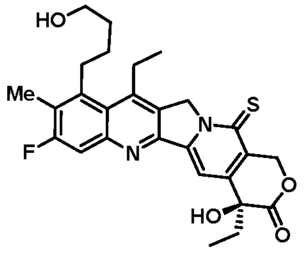
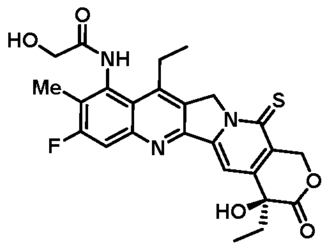
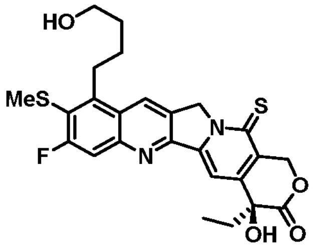
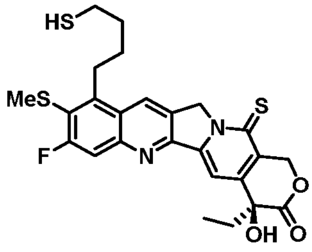
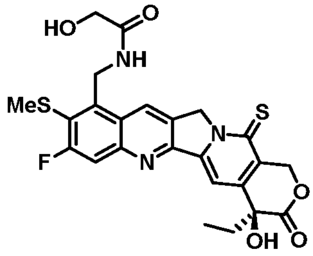
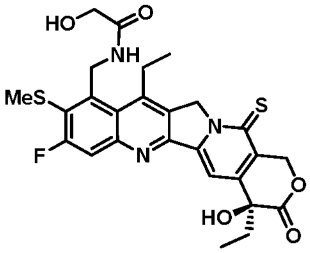
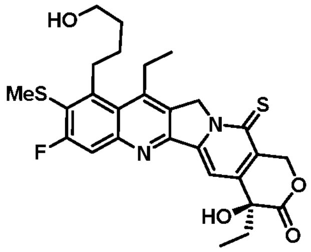
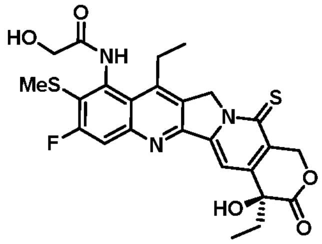
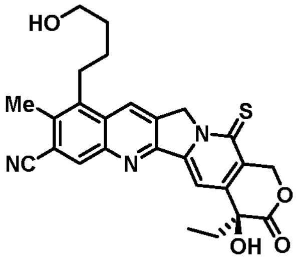
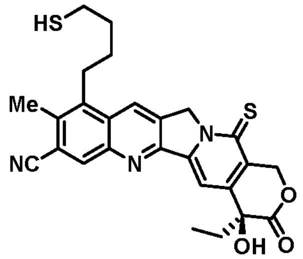
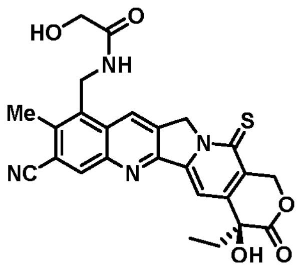
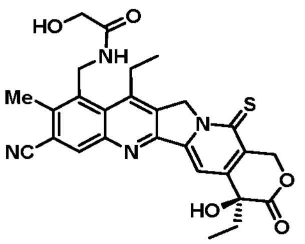
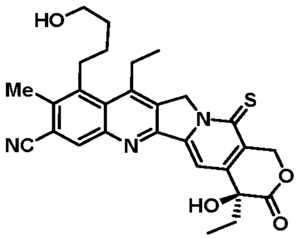
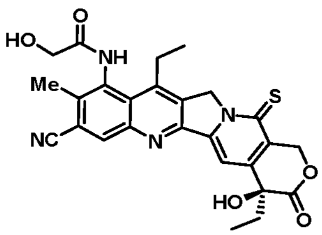
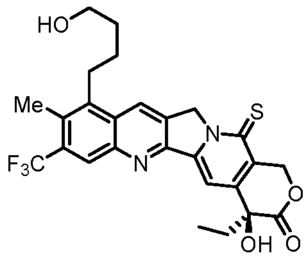
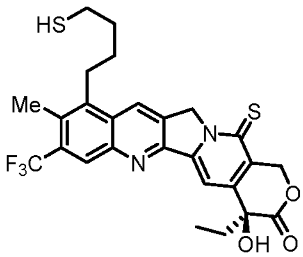
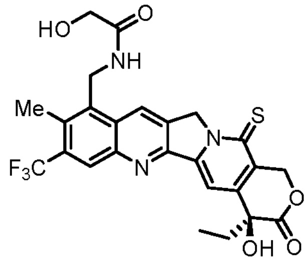

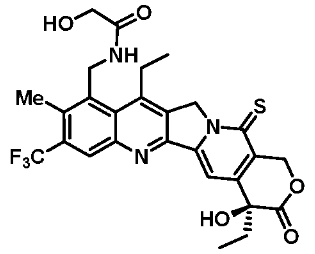
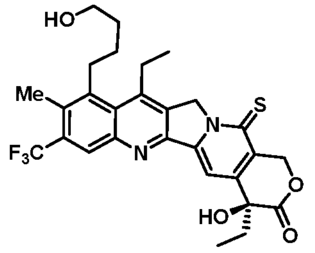
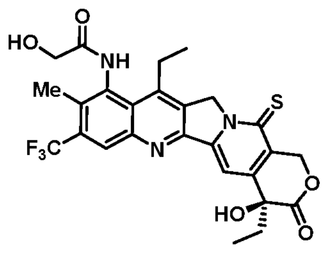

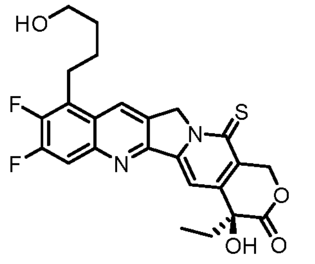
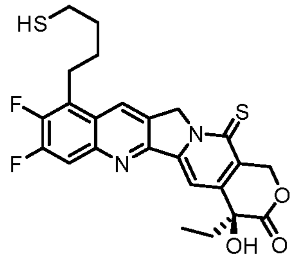
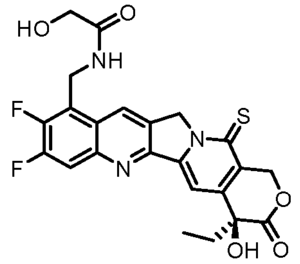

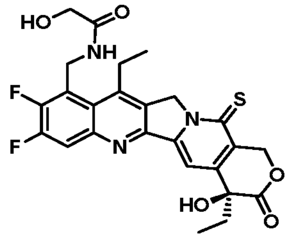
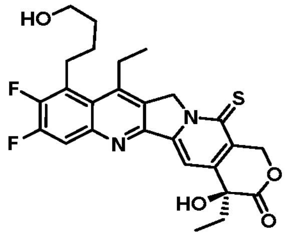
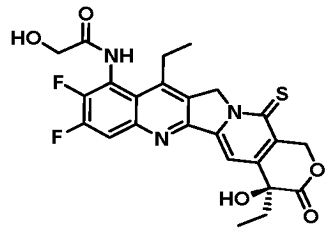

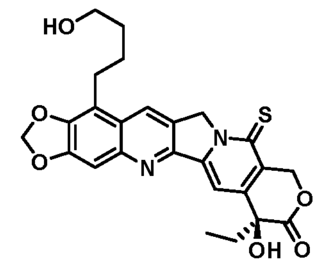
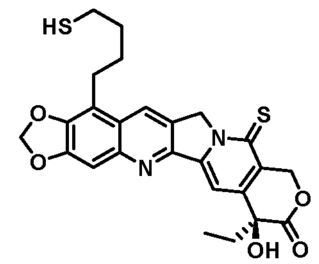
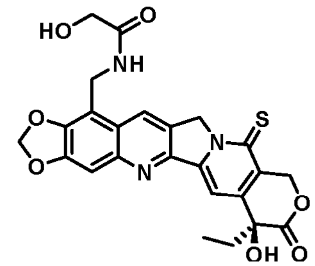

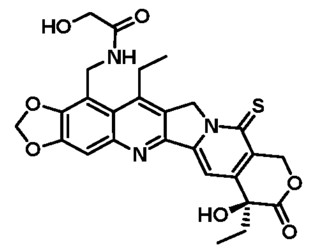
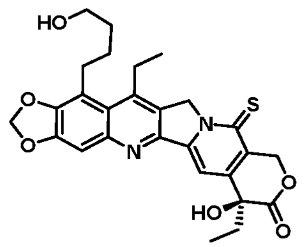
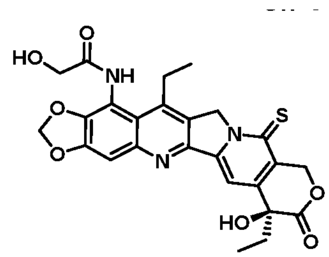

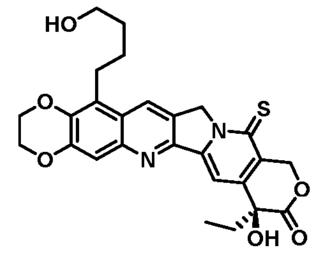
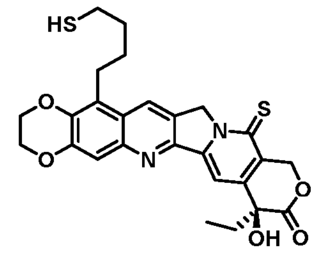
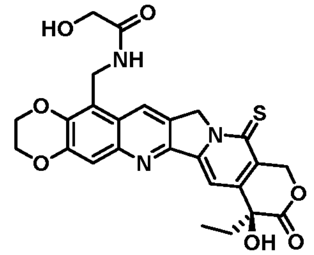

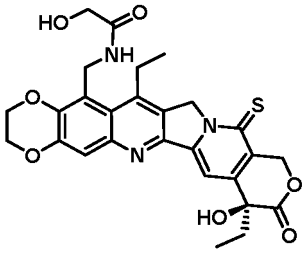

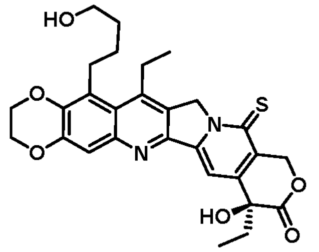

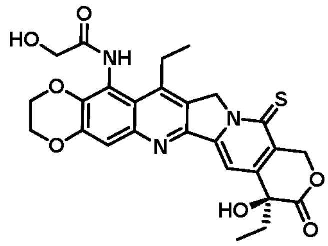

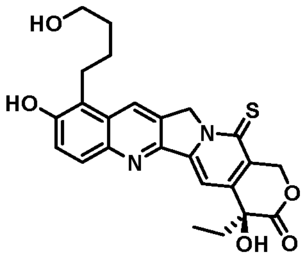

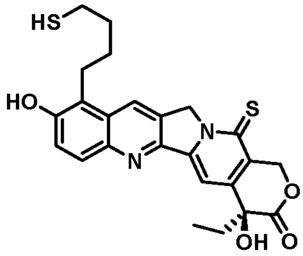

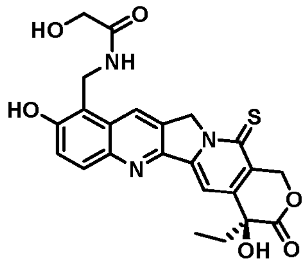

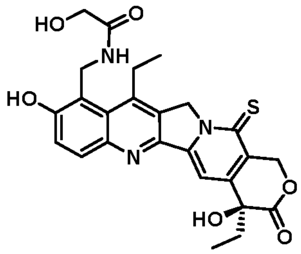

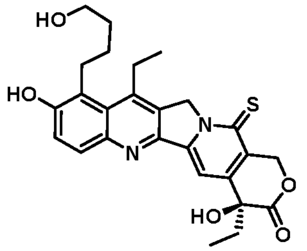

and

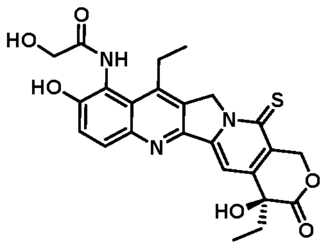

.

**28.** The ligand-drug conjugate, or a tautomer, a mesomer, a racemate, an enantiomer, a diastereomer, or a mixture thereof, or a pharmaceutically acceptable salt or hydrate thereof according to claim 1, wherein the Ab is an antibody or antigen binding fragment thereof.

**29.** The ligand-drug conjugate, or a tautomer, a mesomer, a racemate, an enantiomer, a diastereomer, or a mixture thereof, or a pharmaceutically acceptable salt or hydrate thereof according to claim 28, wherein the antibody is selected from the group consisting of murine antibody, chimeric antibody, humanized antibody, and fully human antibody.

**30.** The ligand-drug conjugate, or a tautomer, a mesomer, a racemate, an enantiomer, a diastereomer, or a mixture thereof, or a pharmaceutically acceptable salt or hydrate thereof according to claim 28, wherein the antibody is a monoclonal antibody, bispecific antibody, or polypeptide.

**31.** The ligand-drug conjugate, or a tautomer, a mesomer, a racemate, an enantiomer, a diastereomer, or a mixture thereof, or a pharmaceutically acceptable salt or hydrate thereof according to claim 28, wherein the antigen binding fragment is selected from the group consisting of Fab, Fab', Fv fragment, F $(ab')_2$, F $(ab)_2$, scFv, di-scFv, VHH, and dAb.

**32.** The ligand-drug conjugate, or a tautomer, a mesomer, a racemate, an enantiomer, a diastereomer, or a mixture thereof, or a pharmaceutically acceptable salt or hydrate thereof according to claim 1, **characterized in that** the ligand-drug conjugate is selected from the group consisting of

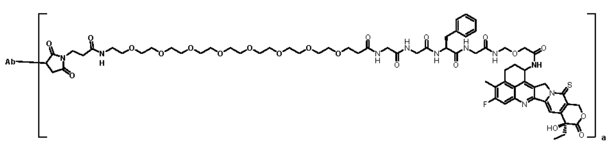

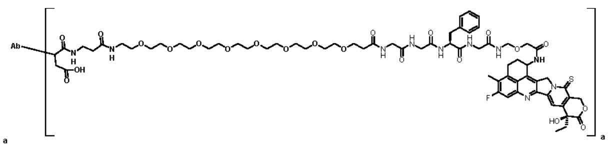

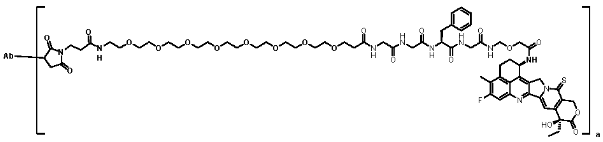
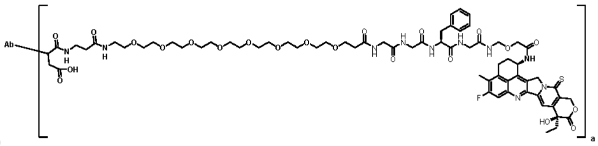

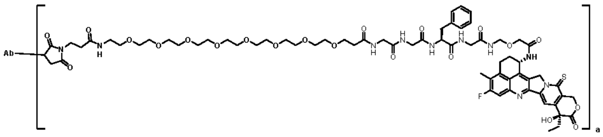
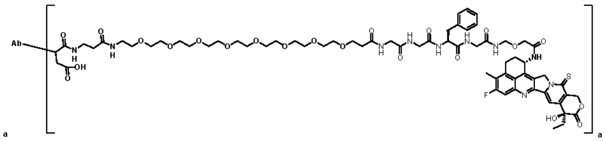

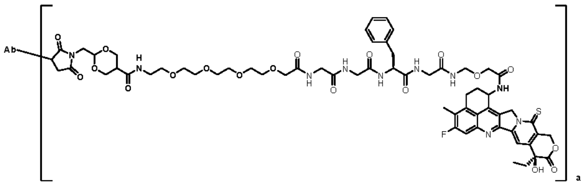

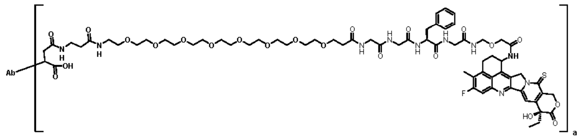

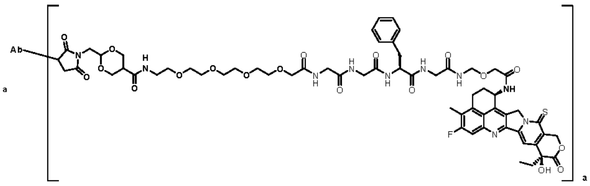

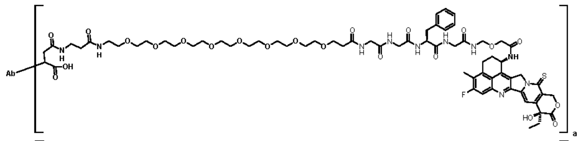

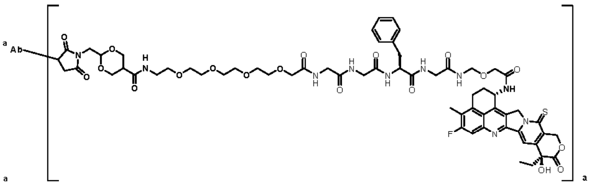

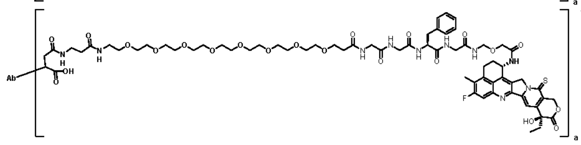

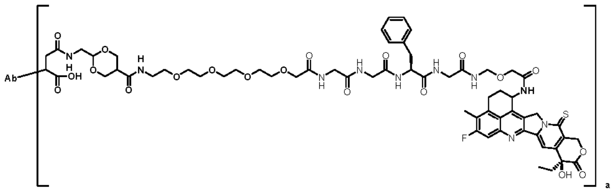

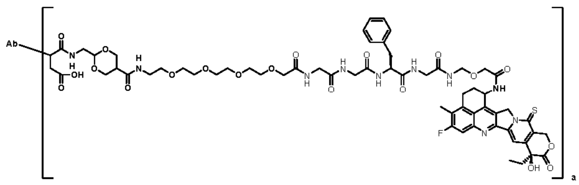

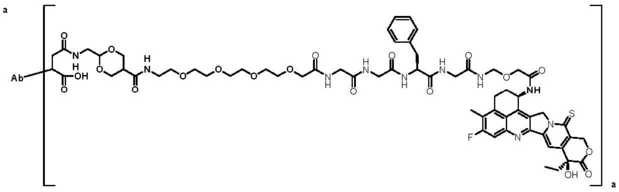

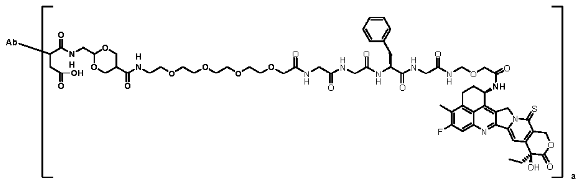

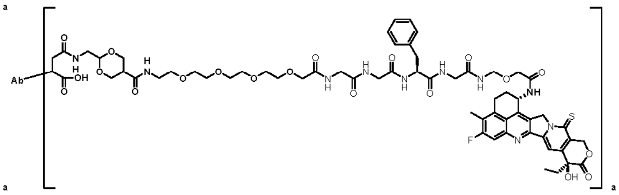

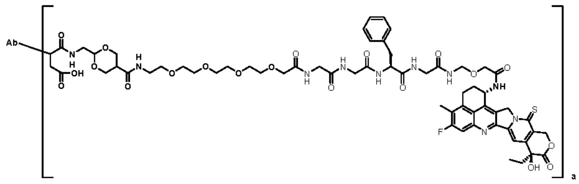

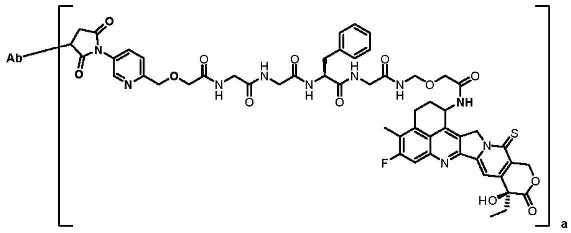

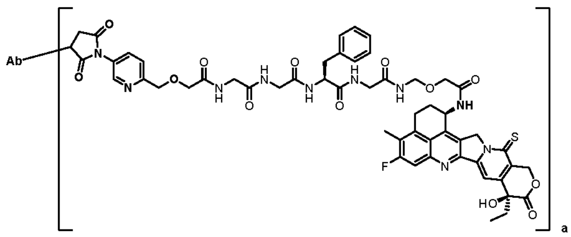

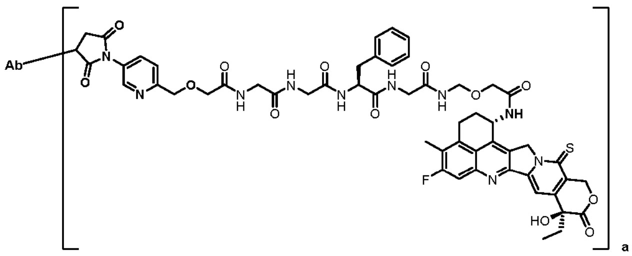

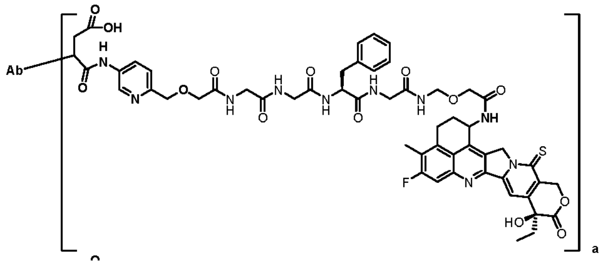
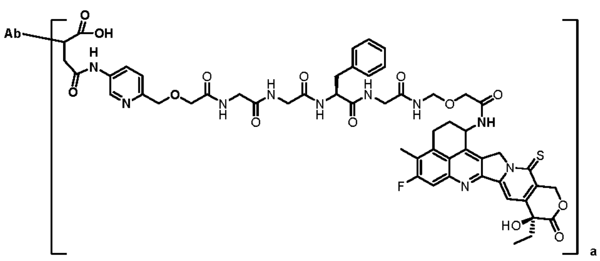
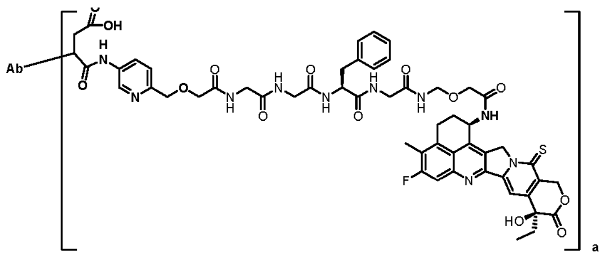
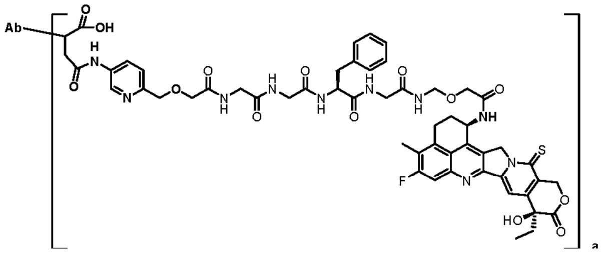
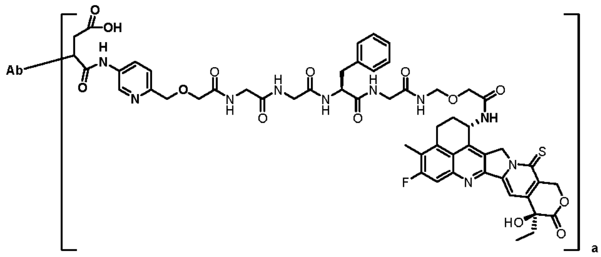
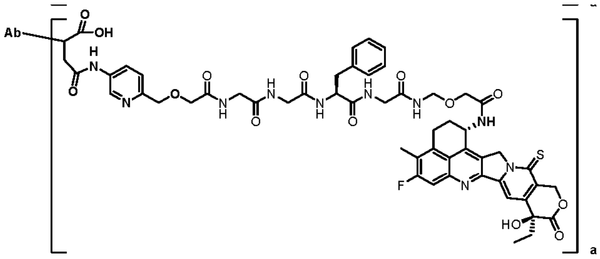
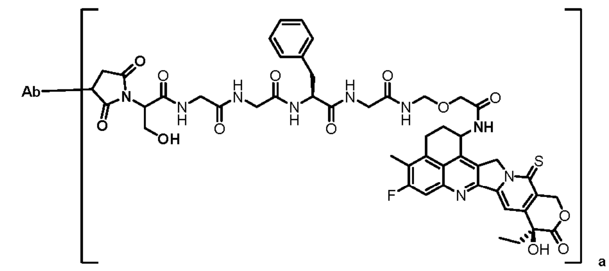
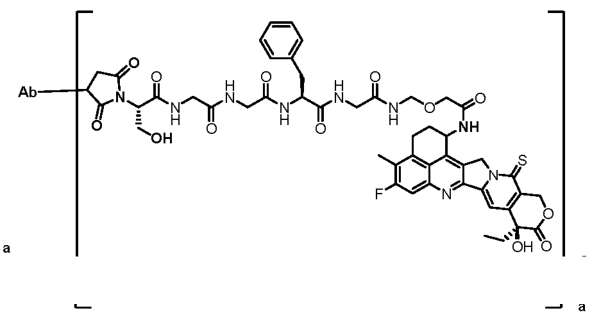
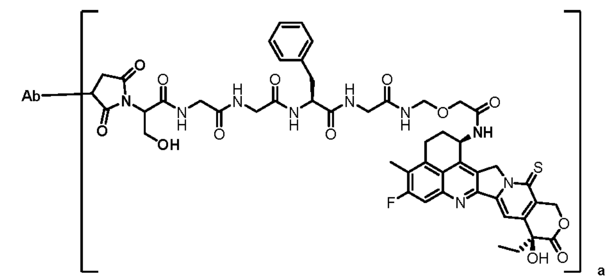
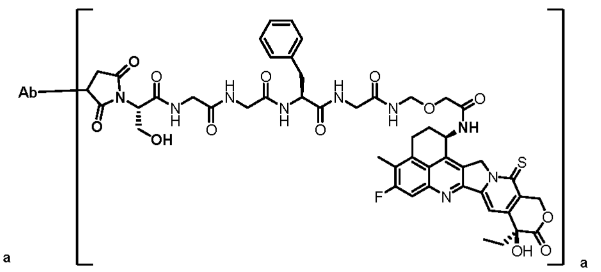
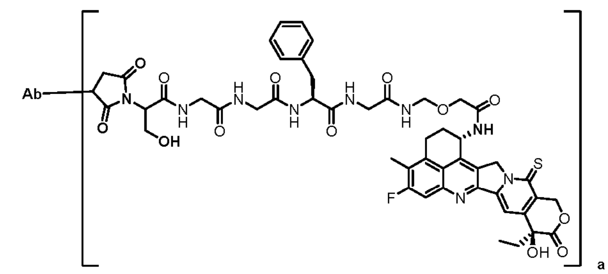
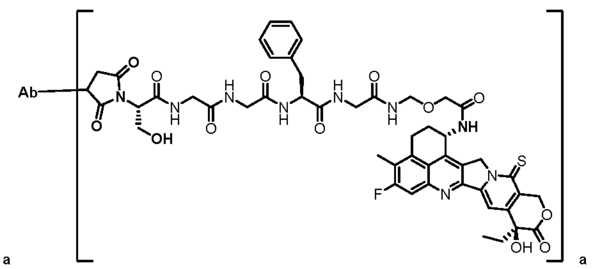

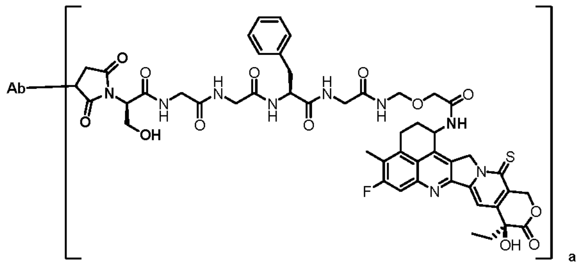

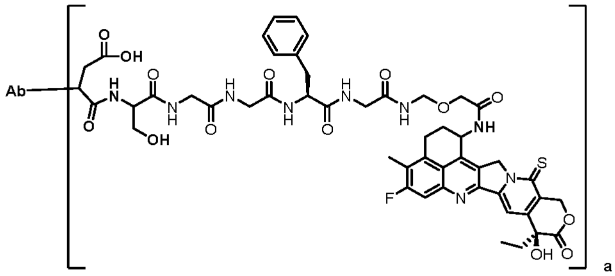

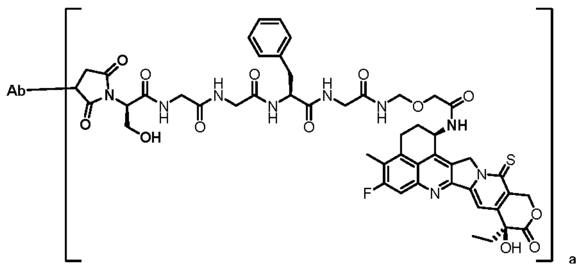

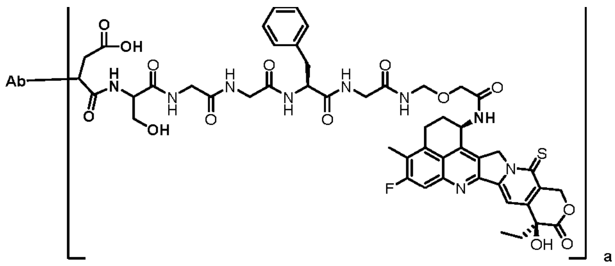

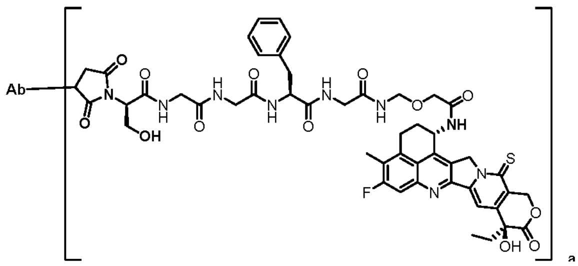

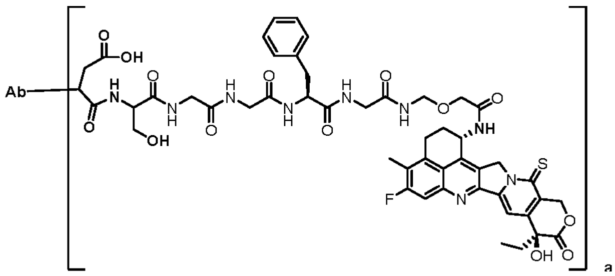

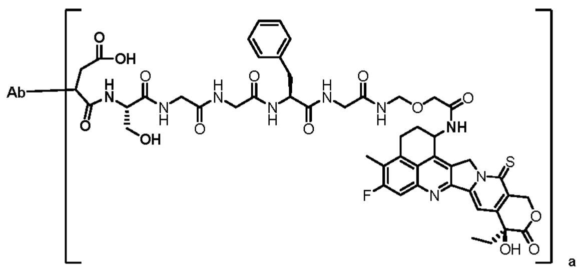

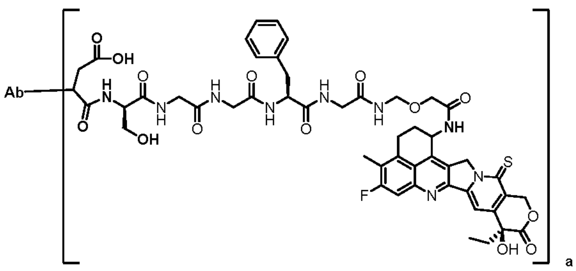

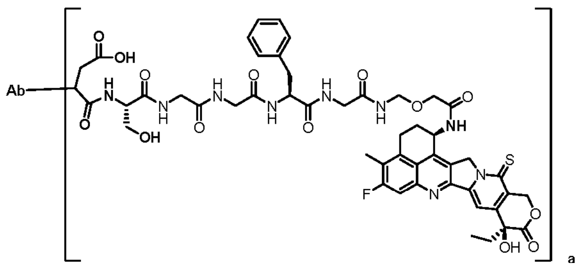

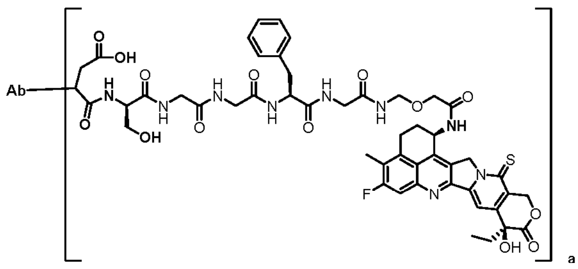

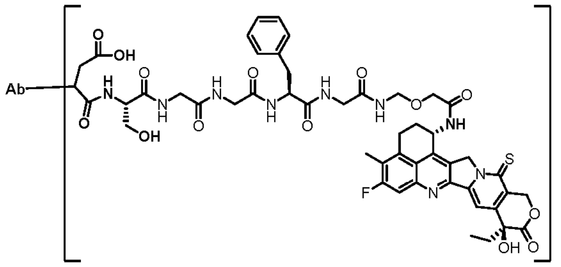

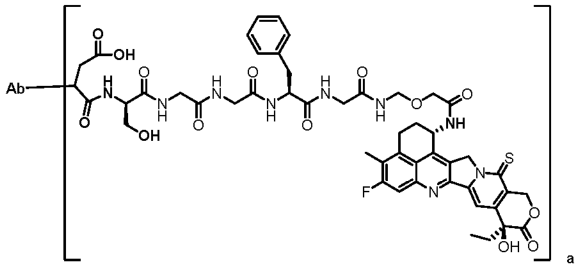

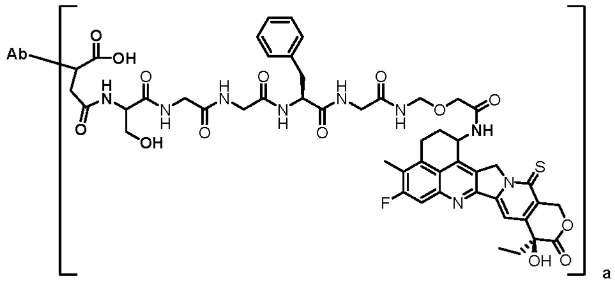

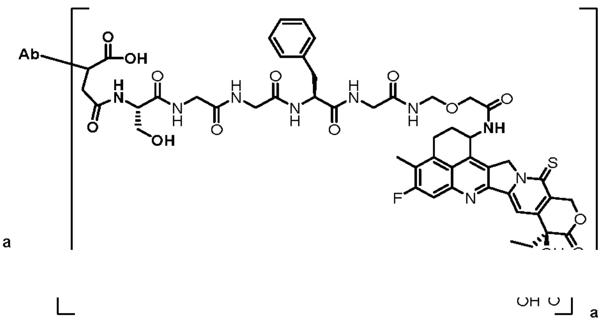

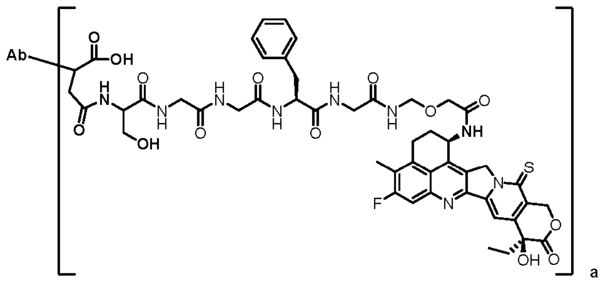

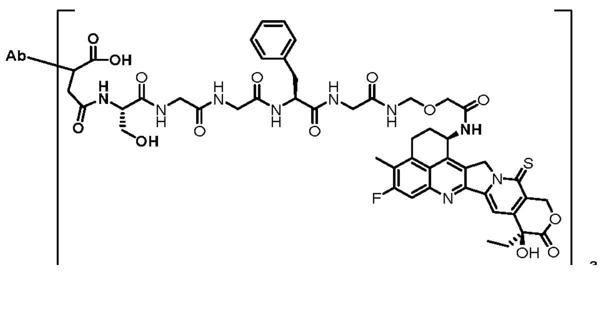

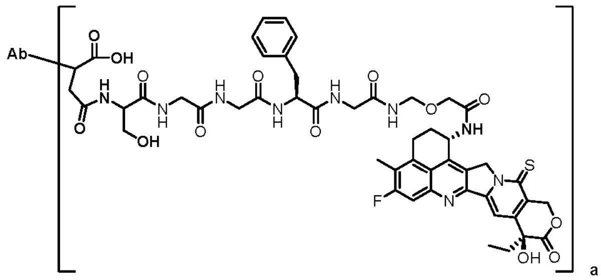

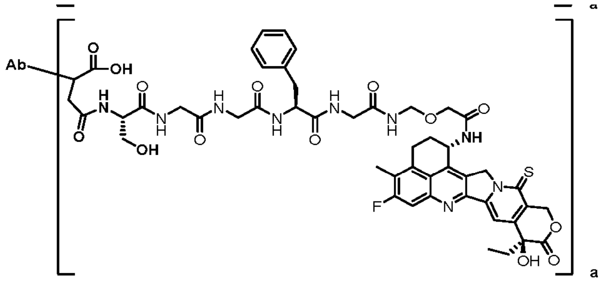

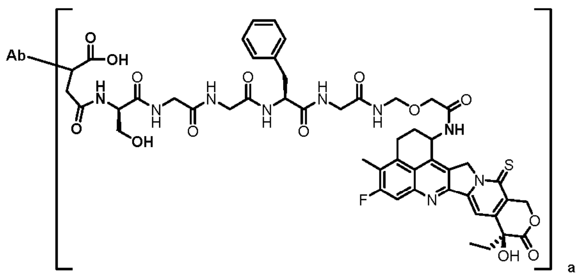

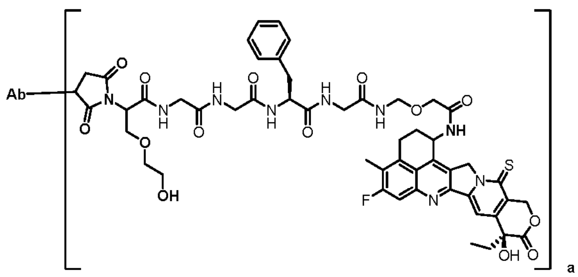

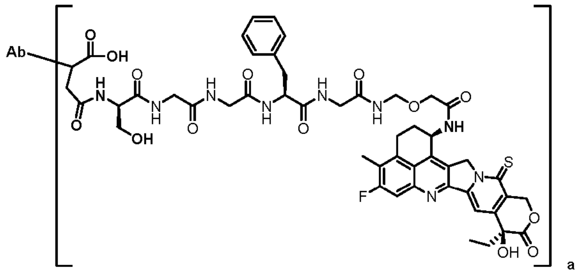
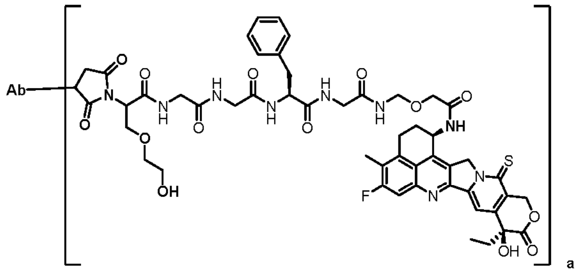
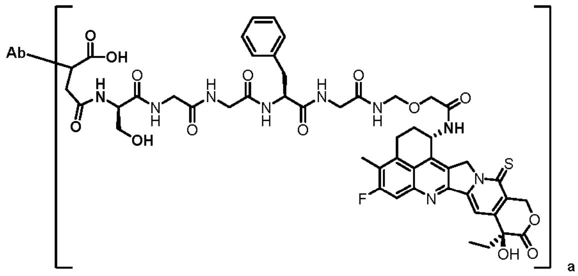
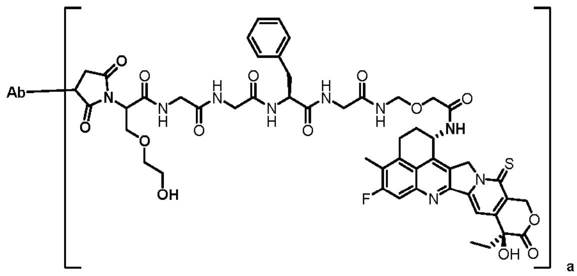
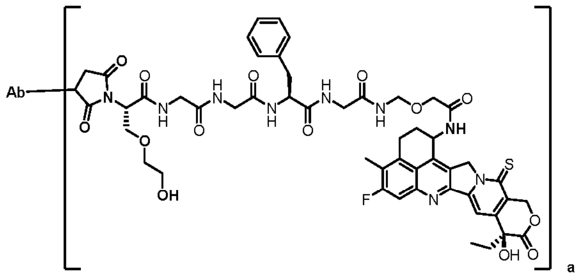
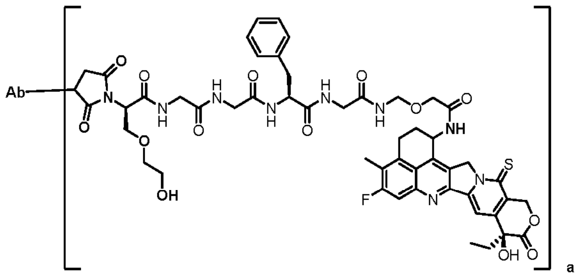
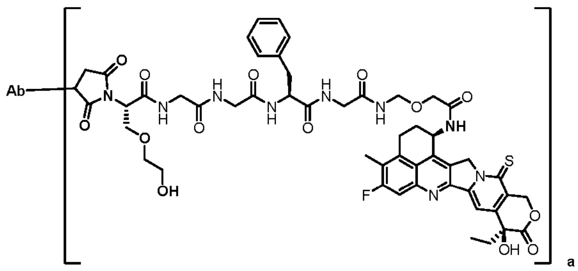
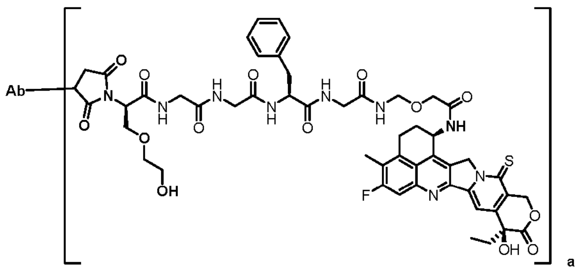
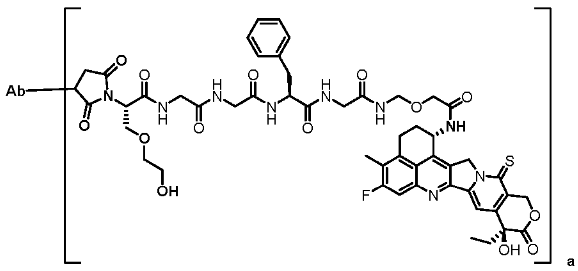
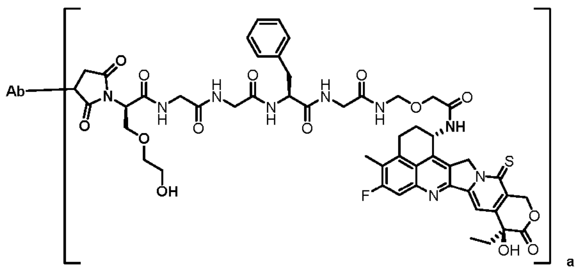

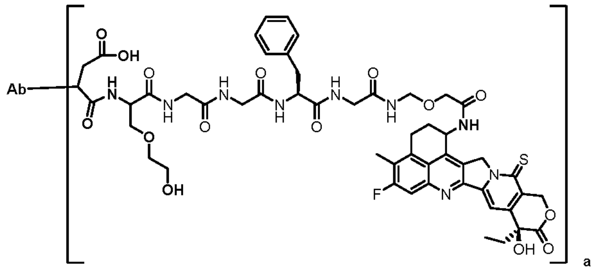

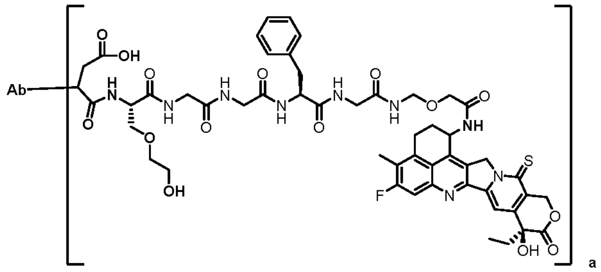

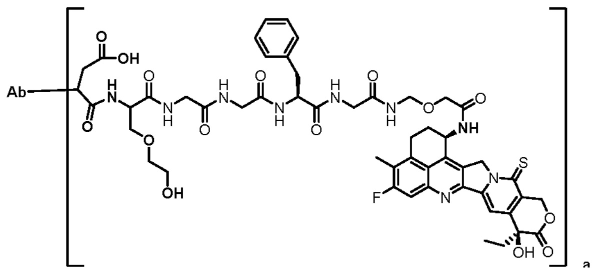

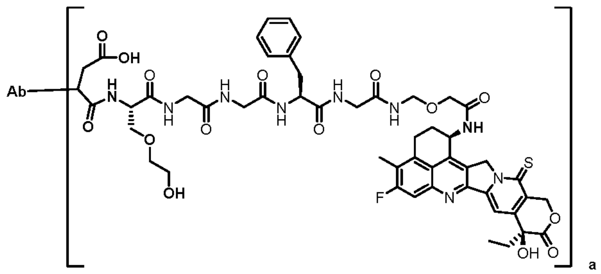

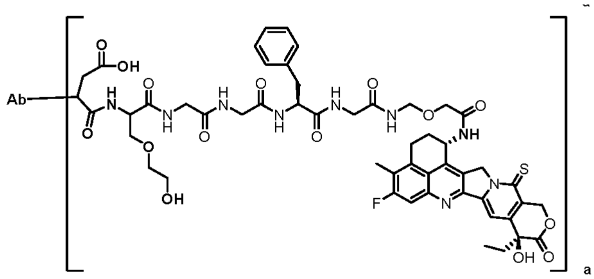

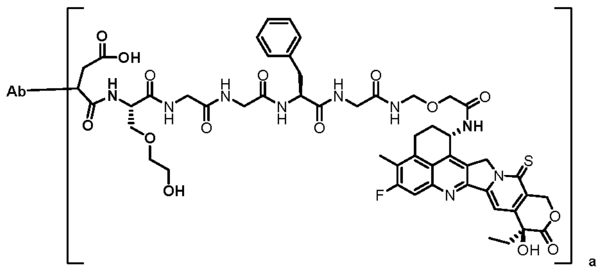

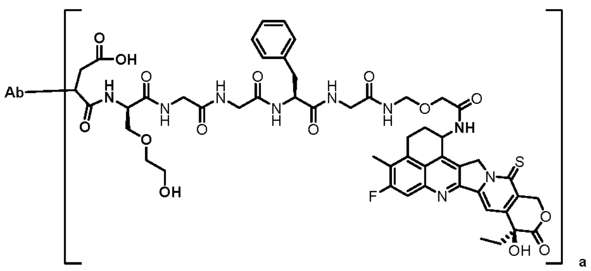

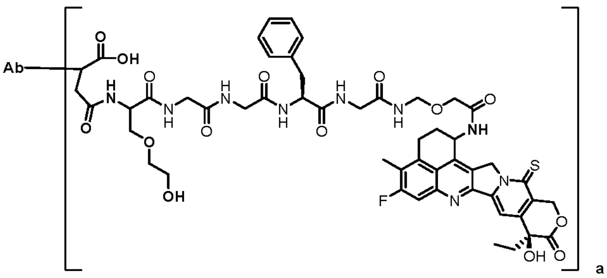

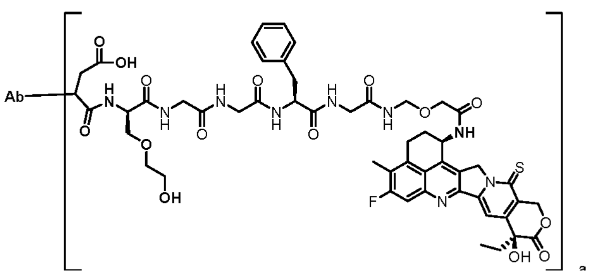

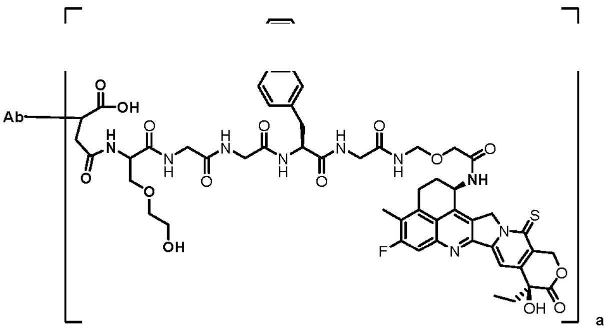

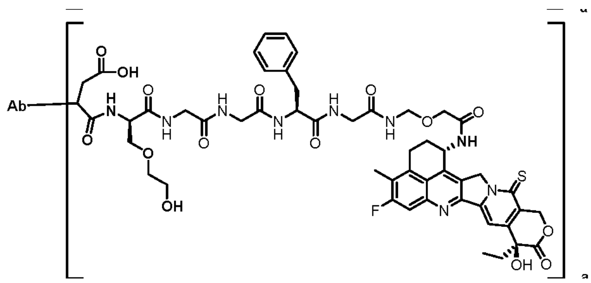

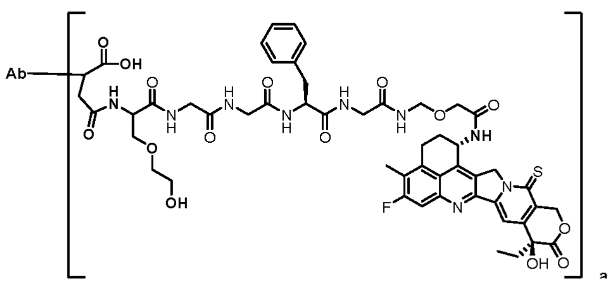

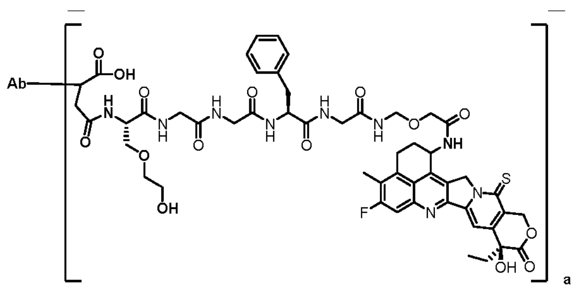

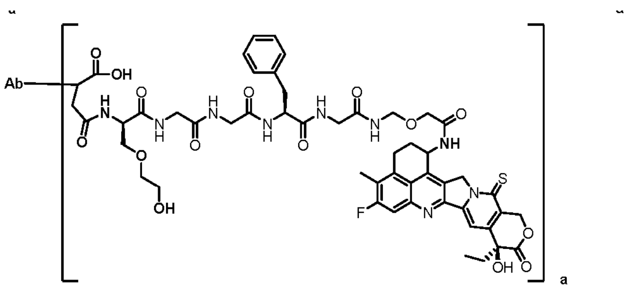

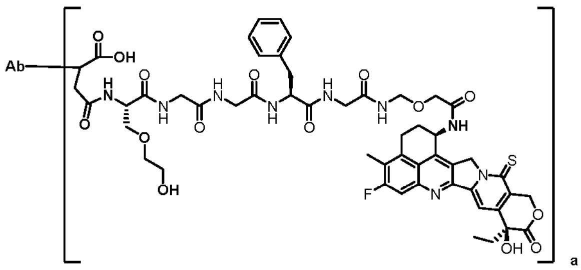

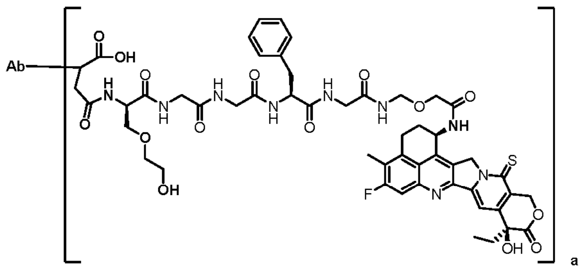

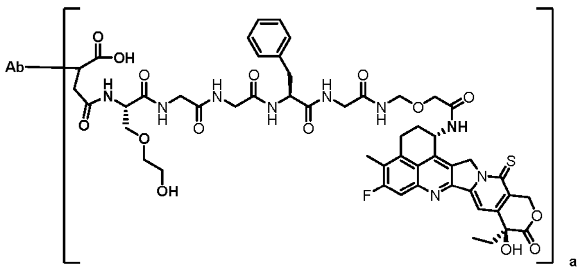

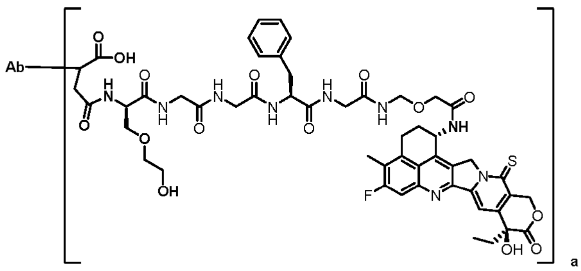

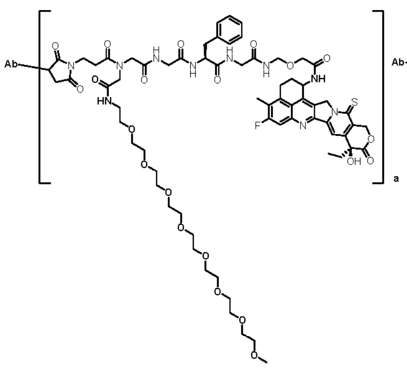

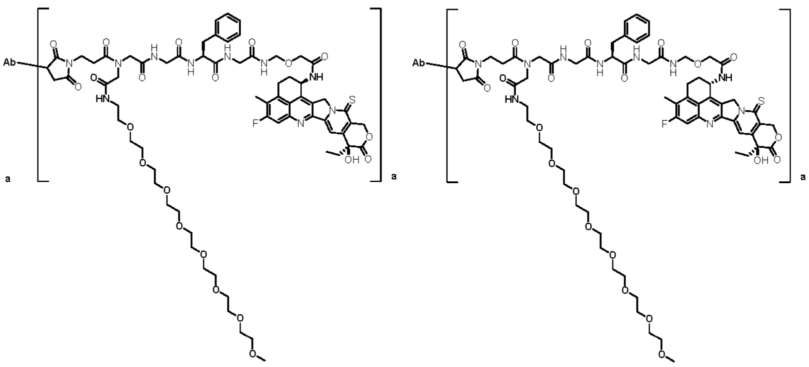

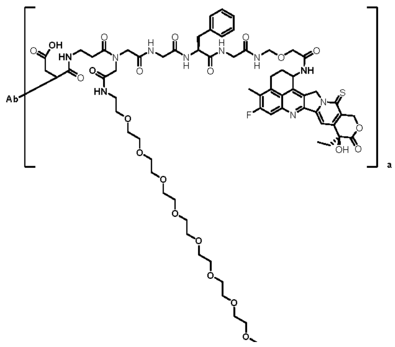 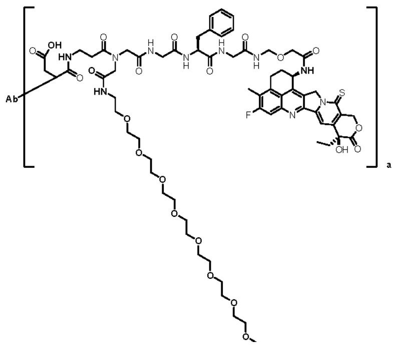 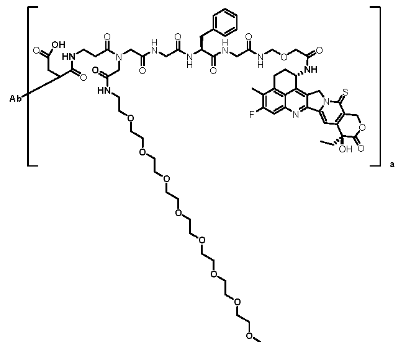

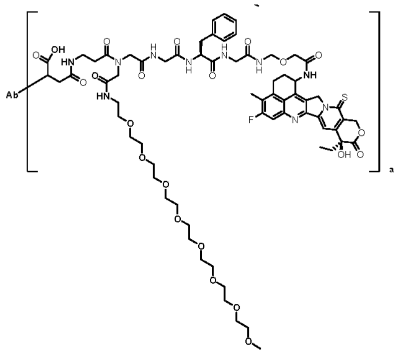 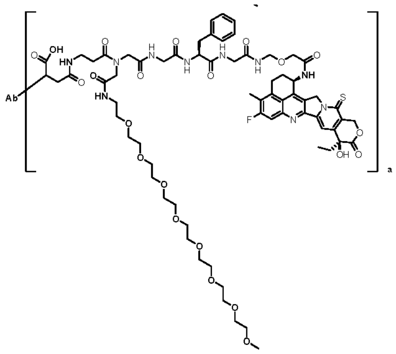 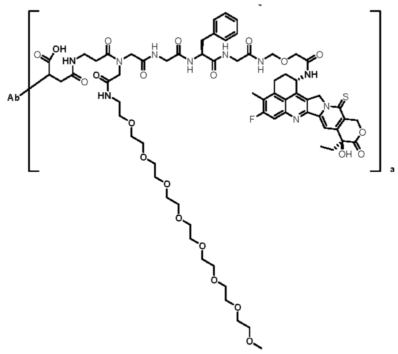

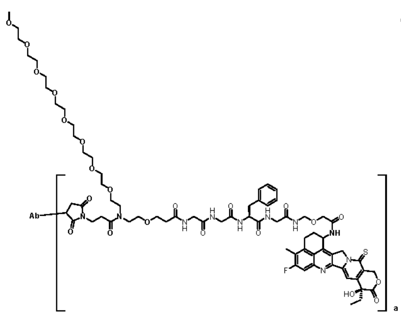 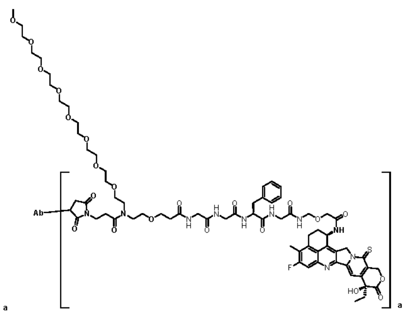 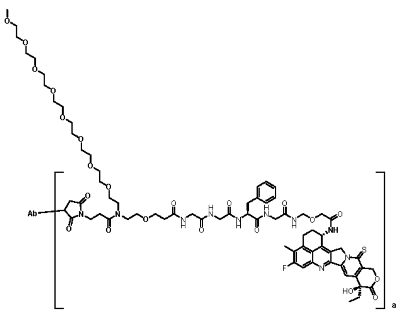

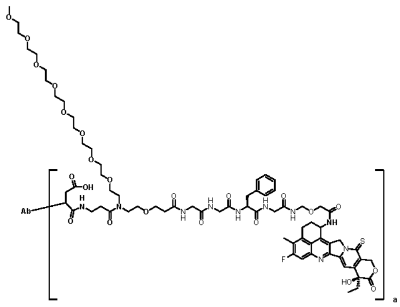 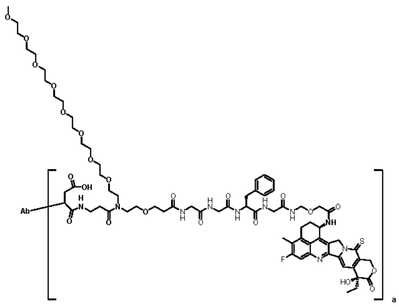 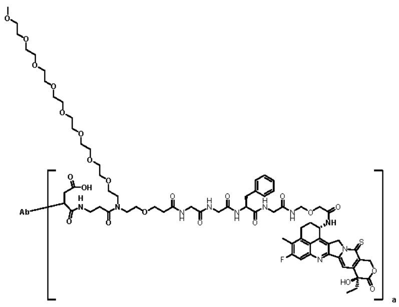

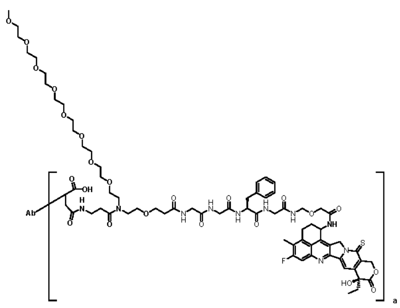 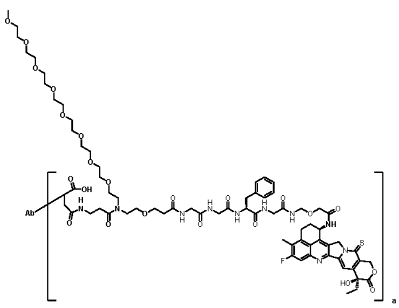 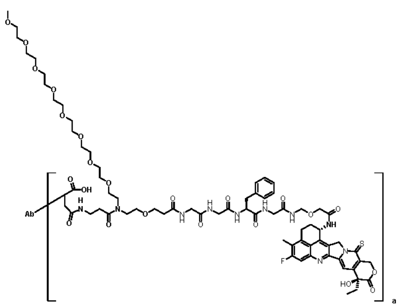

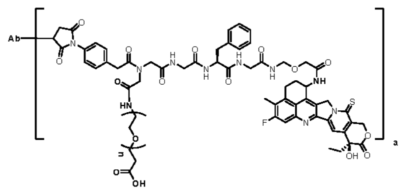
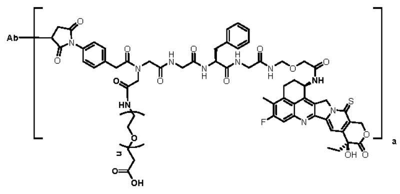
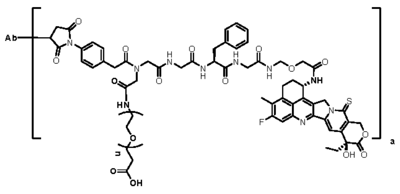

n = 1 ~ 24

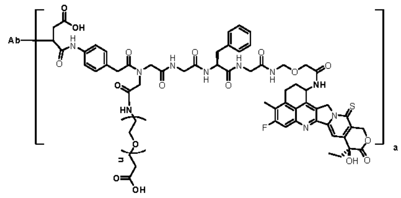
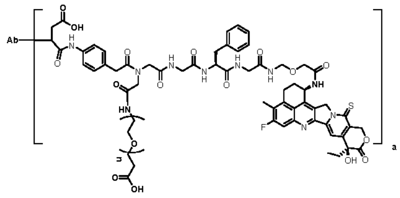
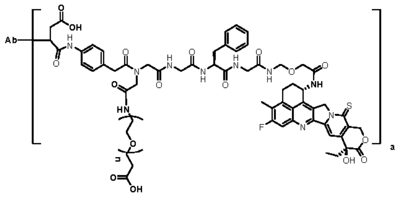

n = 1 ~ 24

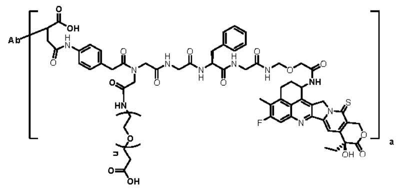
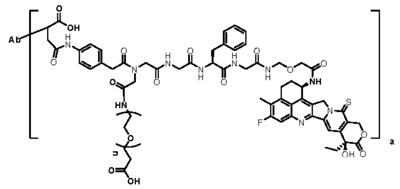
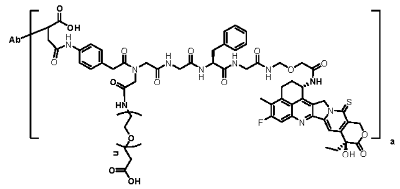

n = 1 ~ 24

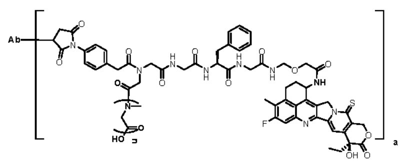
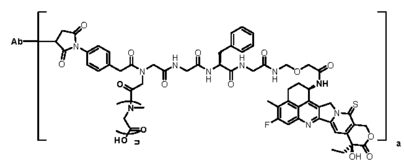
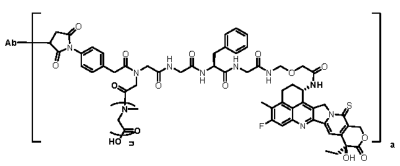

n = 1 ~ 24

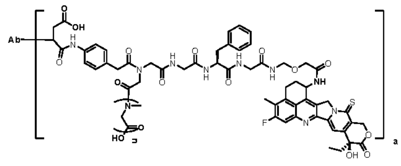
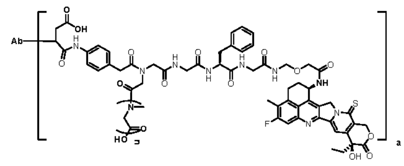
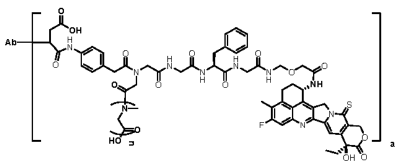

n = 1 ~ 24

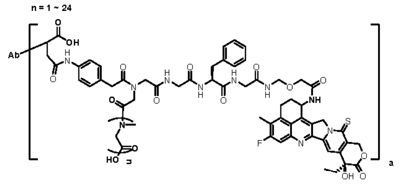
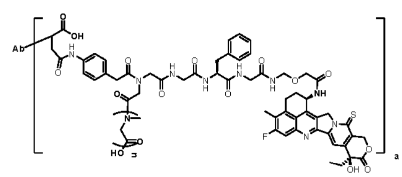
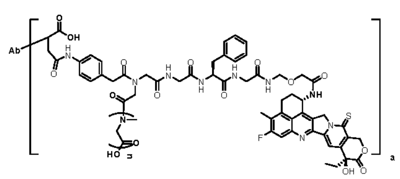

n = 1 ~ 24

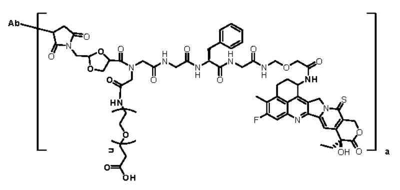
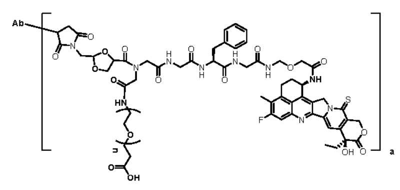
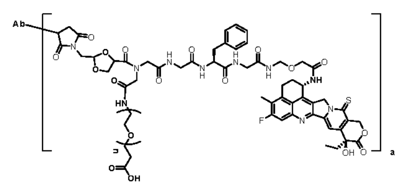

n = 1 ~ 24

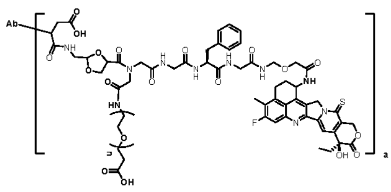
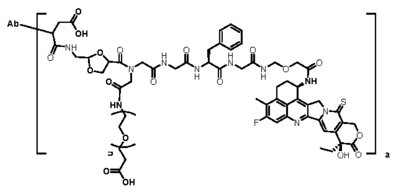
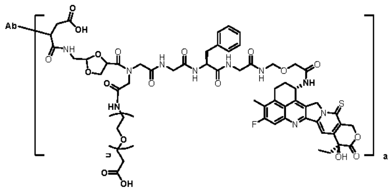

n = 1 ~ 24

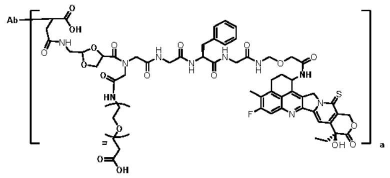
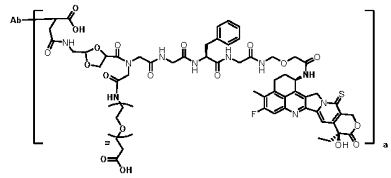
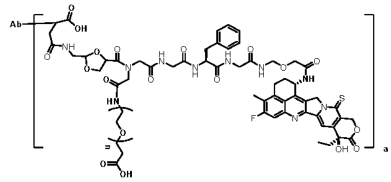

n = 1 ~ 24

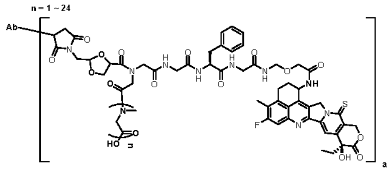
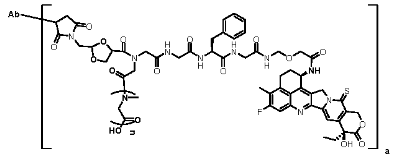
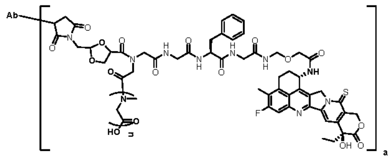

n = 1 ~ 24

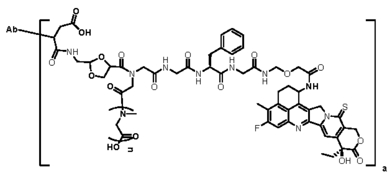
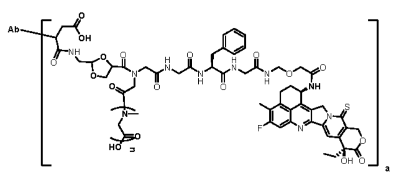
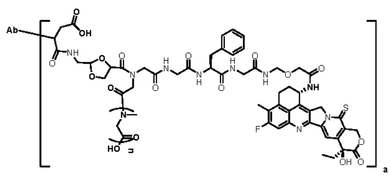

n = 1 ~ 24

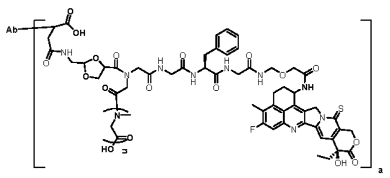
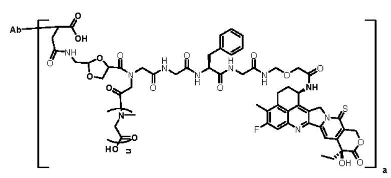
and
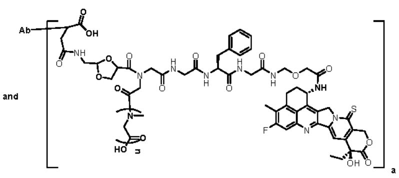

n = 1 ~ 24

**33.** A ligand-drug conjugate, or a tautomer, a mesomer, a racemate, an enantiomer, a diastereomer, or a mixture thereof, or a pharmaceutically acceptable salt or hydrate thereof according to claim 1, wherein the ligand-drug conjugate has the structure shown in formula (I-a):

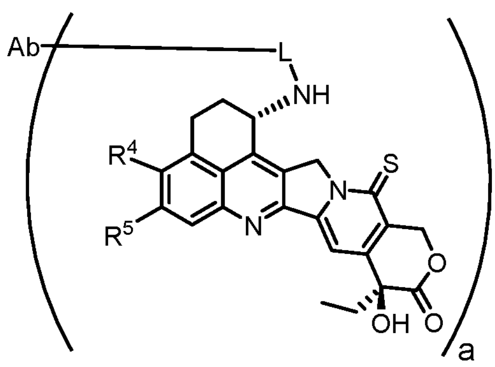
(I-a),

wherein, $R^4$, $R^5$ and L are defined as in claim 1, where "a" is a number greater than 0 and "a" is a decimal or integer.

**34.** The ligand-drug conjugate, or a tautomer, a mesomer, a racemate, an enantiomer, a diastereomer, or a mixture thereof, or a pharmaceutically acceptable salt or hydrate thereof according to claim 33, wherein the ligand-drug conjugate is a structure selected from the group consisting of

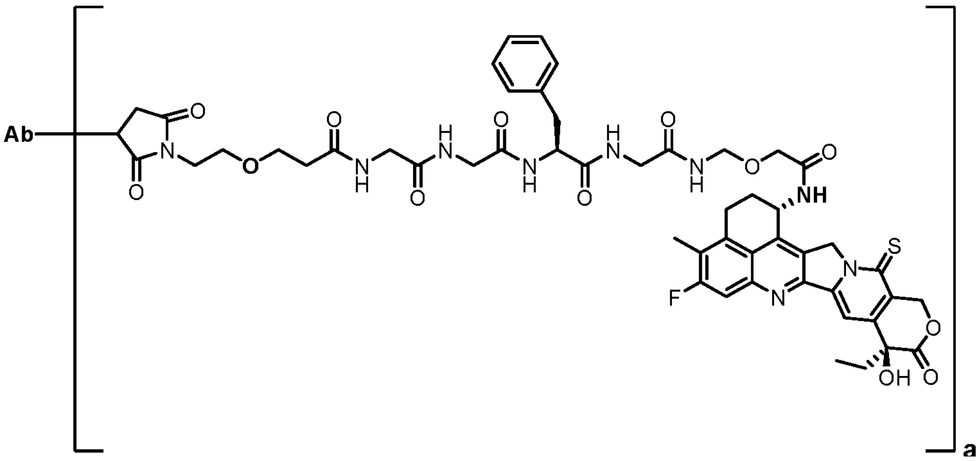

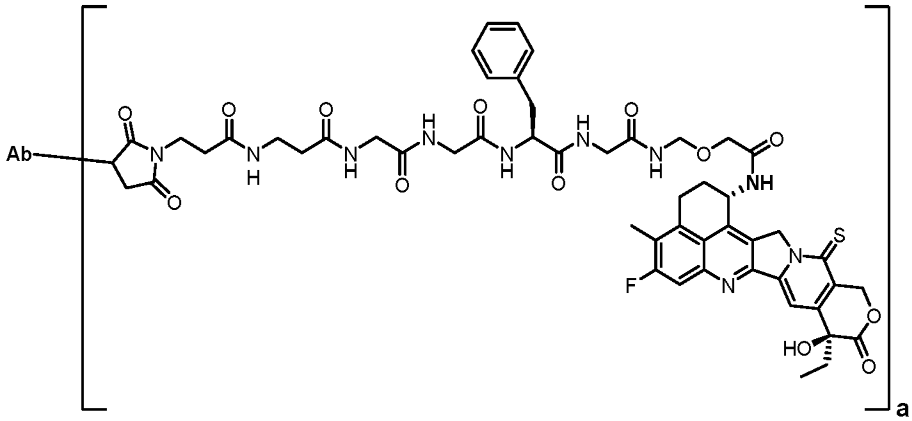

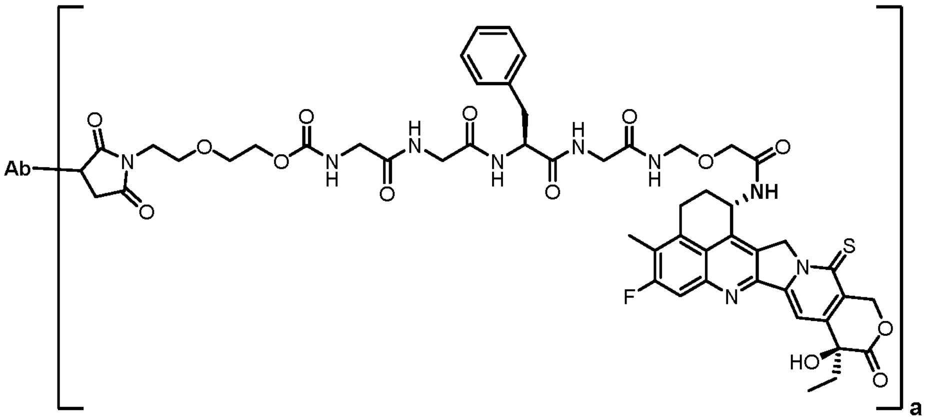

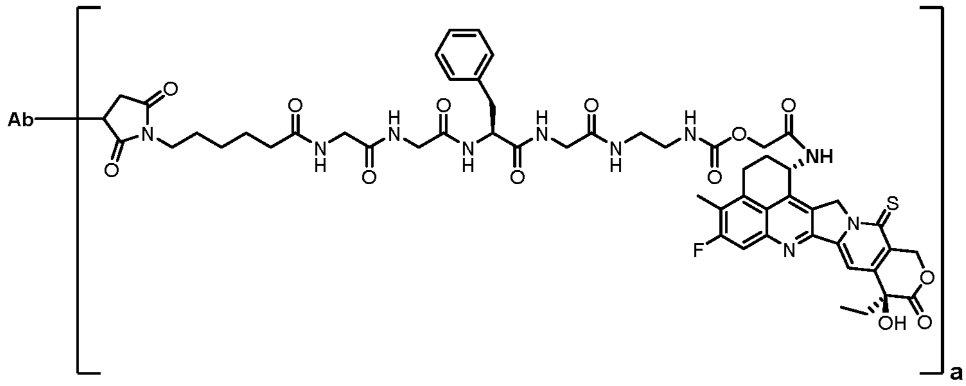

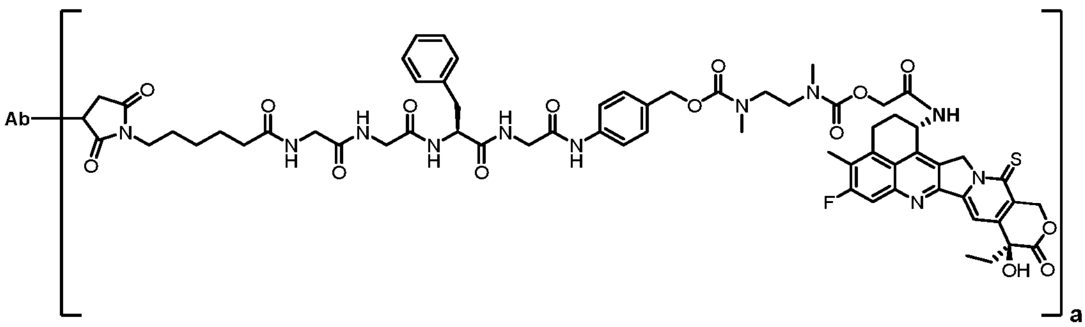

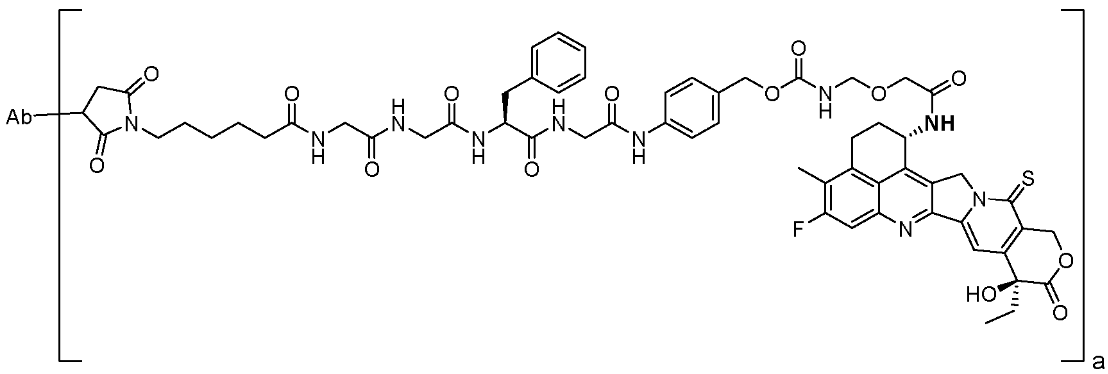

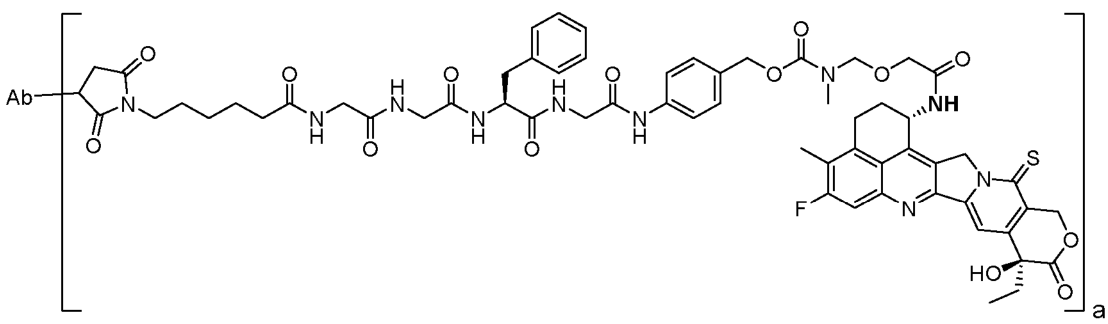

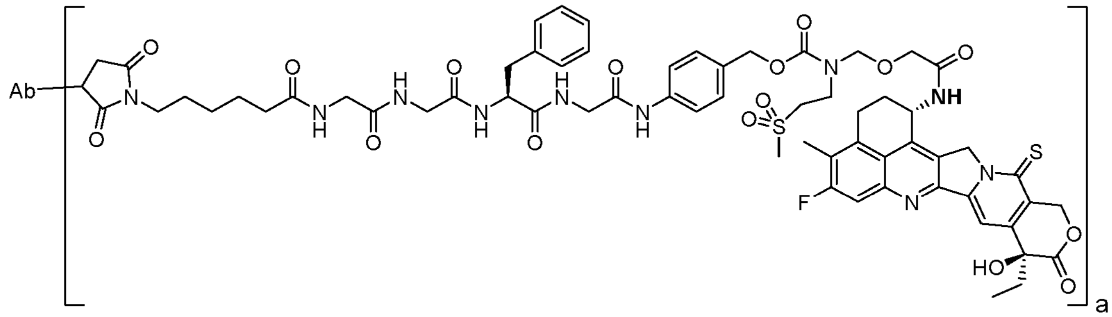

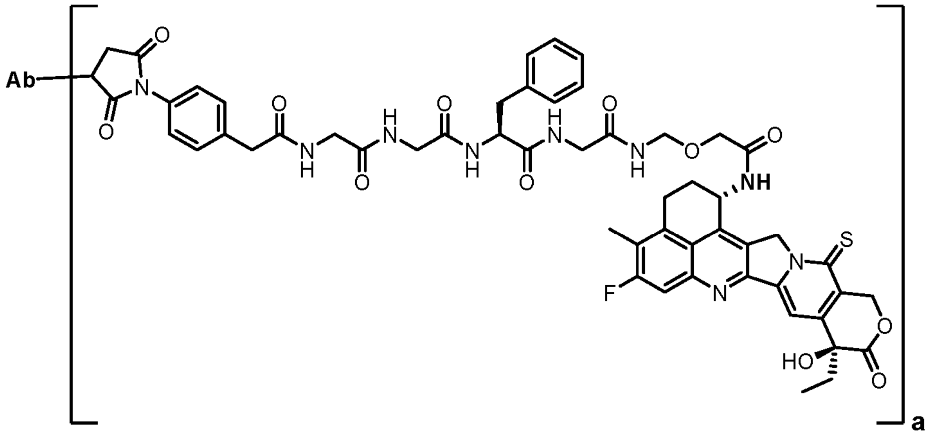

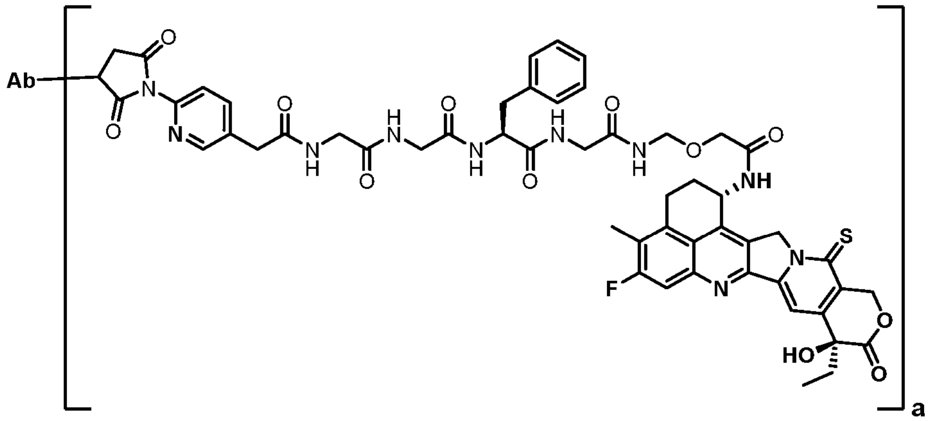

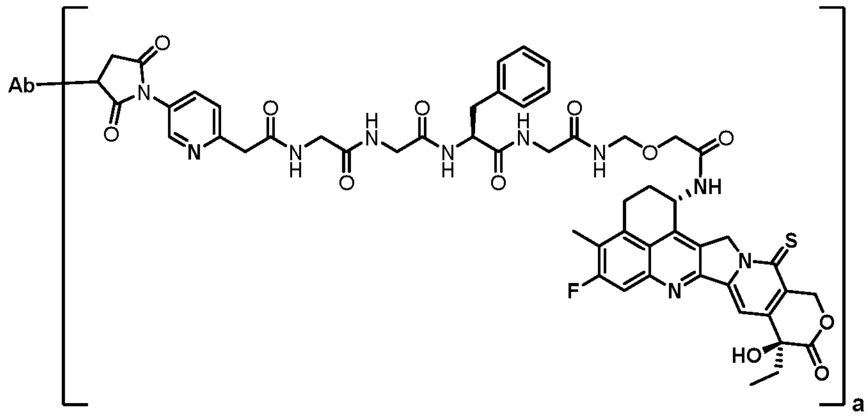

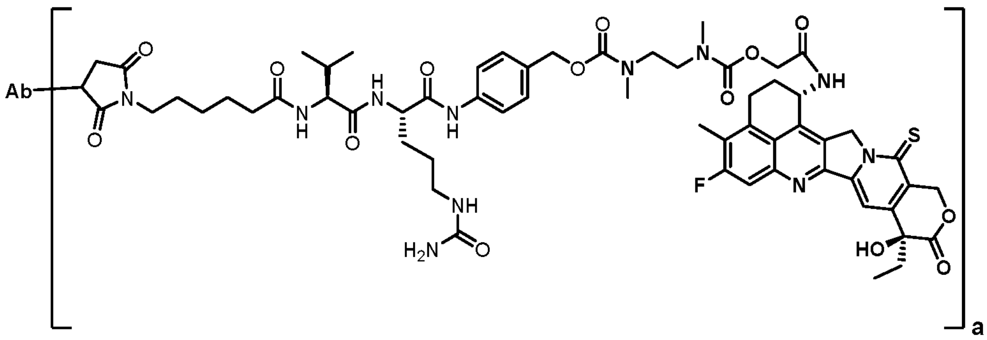

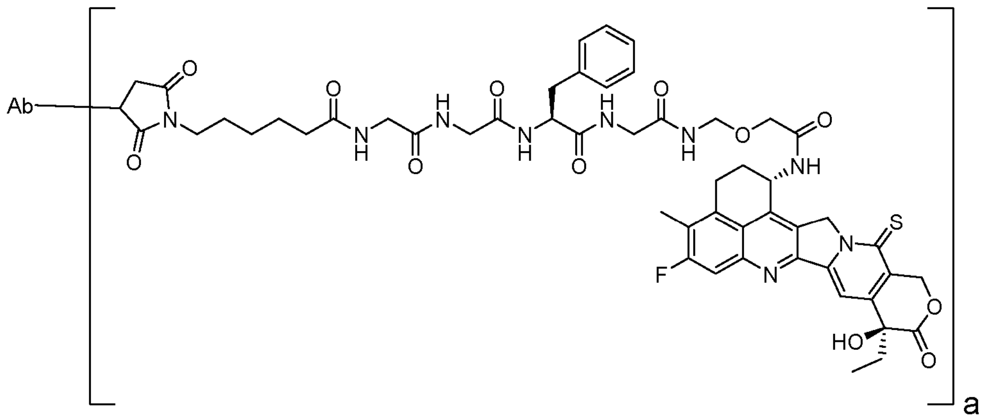

and

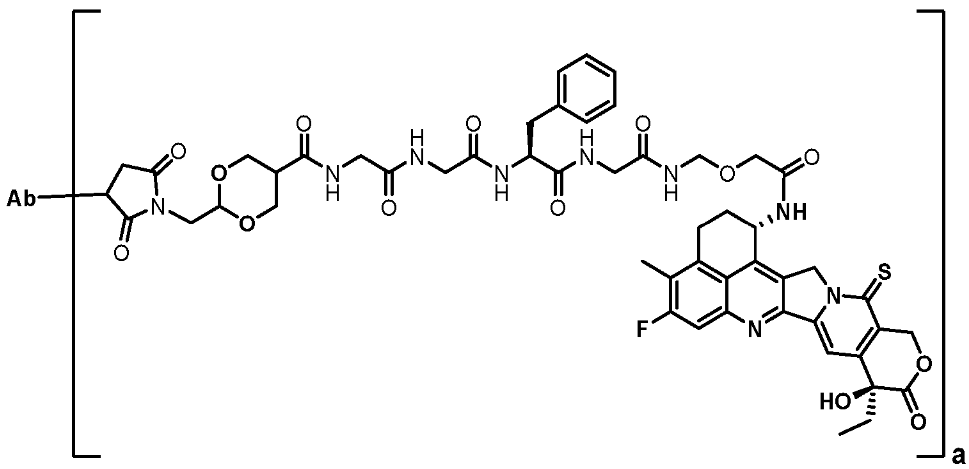,

wherein "a" is a number greater than 0, and "a" is a decimal or integer.

**35.** The ligand-drug conjugate, or a tautomer, a mesomer, a racemate, an enantiomer, a diastereomer, or a mixture thereof, or a pharmaceutically acceptable salt or hydrate thereof according to any one of claims 1-34, **characterized in that** the antibody is an IGF-1R specific antibody.

**36.** The ligand-drug conjugate according to claim 35, **characterized in that** the IGF-1R comprises IGF-1R derived from primates.

**37.** The ligand-drug conjugate according to claim 36, **characterized in that** the antibody comprises HCDR3, which comprises the amino acid sequence shown in SEQ ID NO: 3.

**38.** The ligand-drug conjugate according to claim 36, **characterized in that** the antibody comprises HCDR2, which comprises the amino acid sequence shown in SEQ ID NO: 2.

**39.** The ligand-drug conjugate according to claim 36, **characterized in that** the antibody comprises HCDR1, which comprises the amino acid sequence shown in SEQ ID NO: 1.

**40.** The ligand-drug conjugate according to claim 36, **characterized in that** the antibody comprises a heavy chain variable region VH, wherein the VH comprises the HCDR1, HCDR2, and HCDR3, and the HCDR3 comprises the amino acid sequence shown in SEQ ID NO: 3; The HCDR2 comprises the amino acid sequence shown in SEQ ID NO: 2; and the HCDR1 comprises the amino acid sequence shown in SEQ ID NO: 1.

**41.** The ligand-drug conjugate according to claim 36, **characterized in that** the antibody comprises a heavy chain variable region VH comprising the amino acid sequence shown in SEQ ID NO: 4.

**42.** The ligand-drug conjugate according to claim 36, **characterized in that** the antibody has a full-length sequence as shown in SEQ ID No. 5.

**43.** A pharmaceutical composition comprising a ligand-drug conjugate, or a tautomer, a mesomer, a racemate, an enantiomer, a diastereomer, or a mixture, or a pharmaceutically acceptable salt, prodrug, or solvate according to any one of claims 1-42.

**44.** The use of a ligand-drug conjugate, or a tautomer, a mesomer, a racemate, an enantiomer, a diastereomer, or a mixture thereof, or a pharmaceutically acceptable salt, prodrug, or solvate thereof according to any one of claims 1-42 and/or a pharmaceutical composition according to claim 43 in the preparation of a drug for the treatment and/or prevention of diseases or disorders associated with expression and/or abnormal expression of the target of the ligand.

**45.** The use according to claim 44, the disease or disorder related to the target expression and/or abnormal expression of the ligand is selected from the group consisting of tumor, cancer, autoimmune diseases or infectious diseases; preferably, the tumor/cancer is a tumor/cancer with high, medium, or low expression of the target of the ligand.

**46.** The ligand-drug conjugate precursor, or a tautomer, a mesomer, a racemate, an enantiomer, a diastereomer, or a pharmaceutically acceptable salt, or hydrate thereof, wherein the ligand-drug conjugate precursor comprises a structure represented by formula (IA):

$L_A$-P (IA),

wherein, $L_A$ is $L_{1A}$-$L_2$-$L_3$-$L_4$-$L_5$; Among them, $L_{1A}$ is

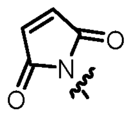 or 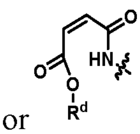 ;

$R^d$, $L_2$, $L_3$, $L_4$, $L_5$, and P are defined as in any one of claims 1-34.

**47.** The ligand-drug conjugate precursor, or a tautomer, a mesomer, a racemate, an enantiomer, a diastereomer, or a pharmaceutically acceptable salt, or hydrate thereof according to claim 46, wherein the ligand-drug conjugate precursor is selected from the group consisting of

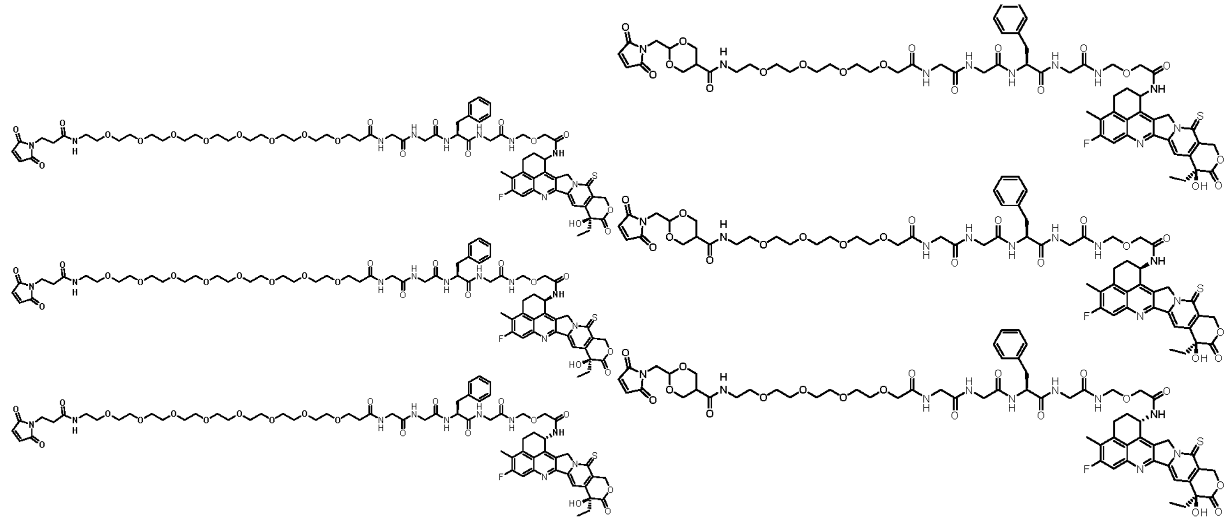

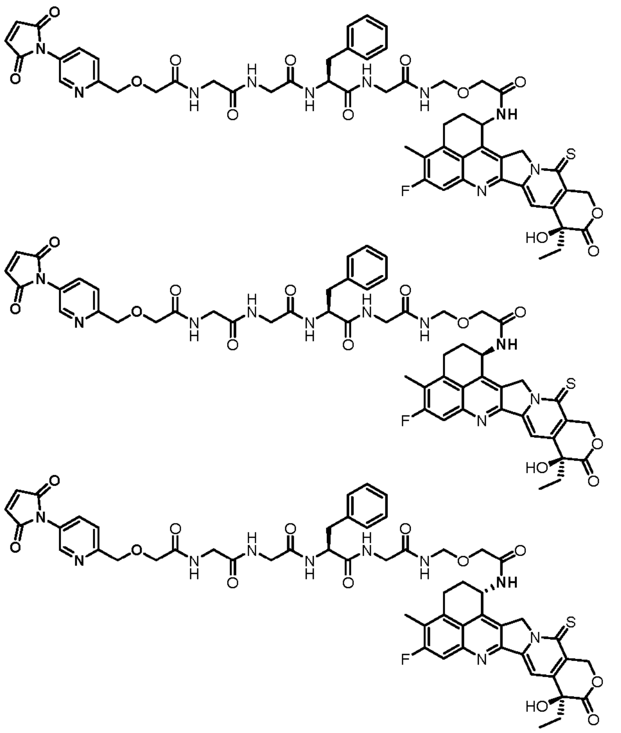

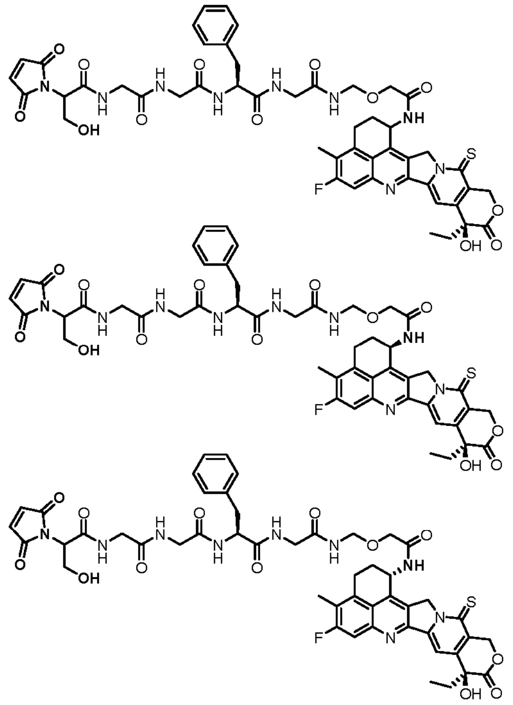

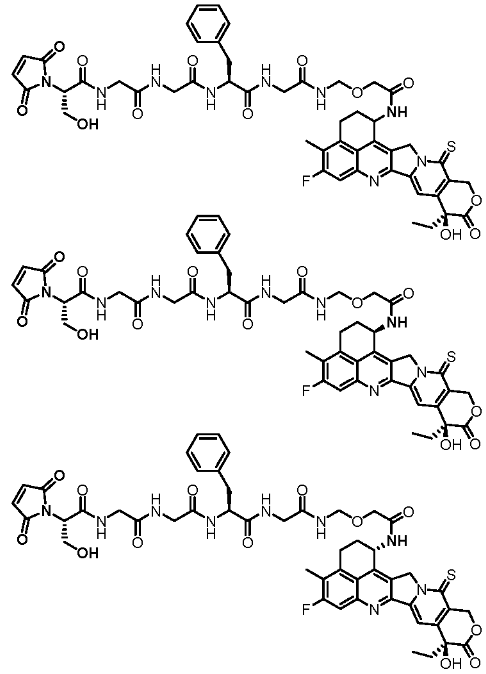
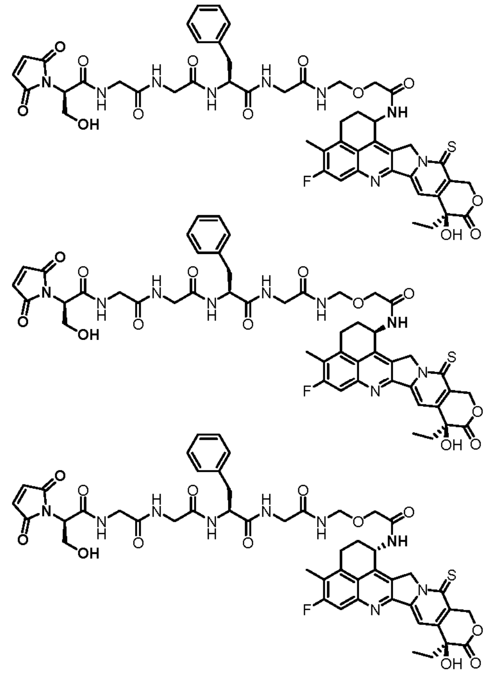
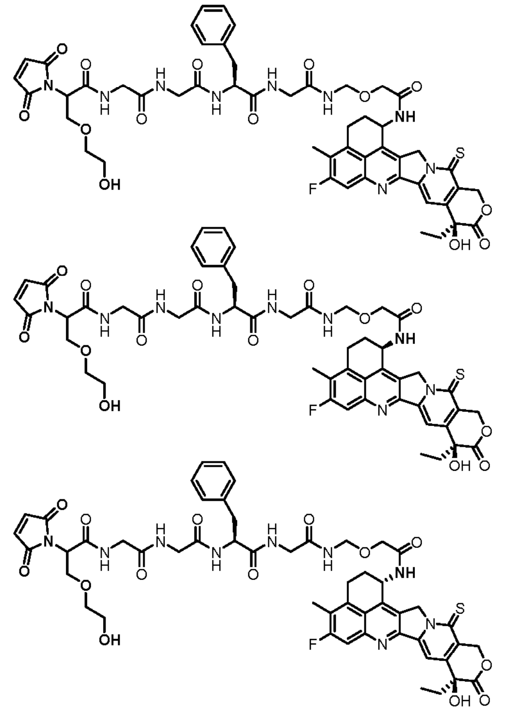
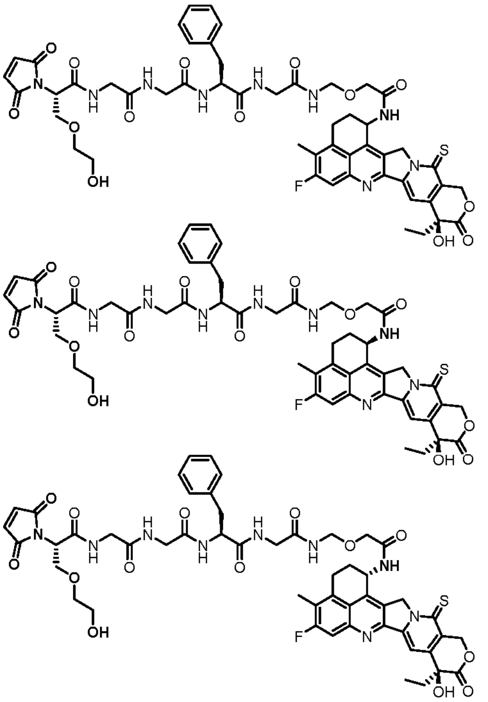

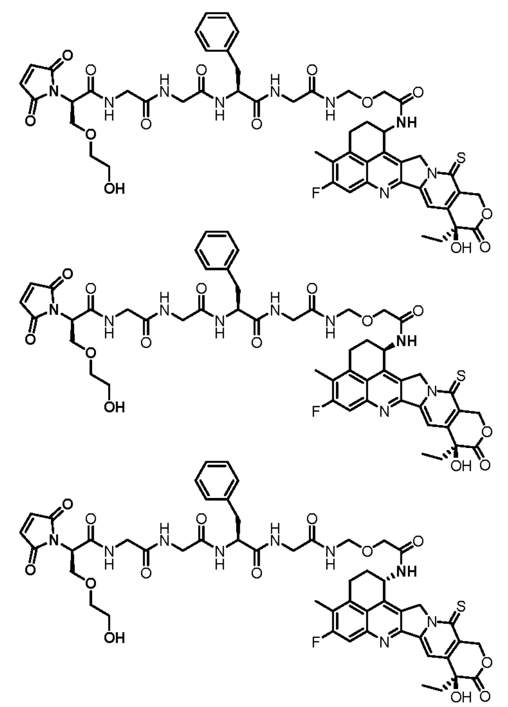

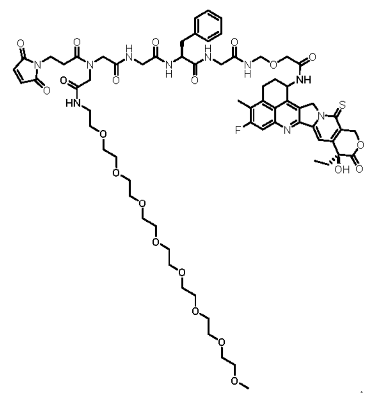

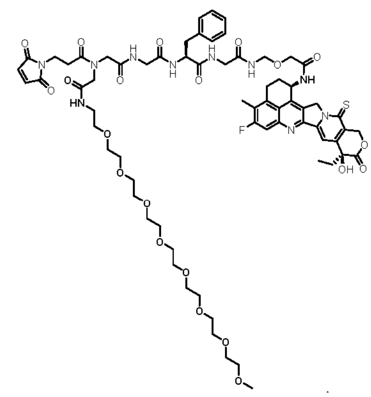

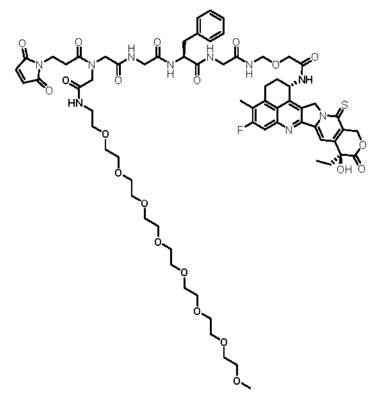

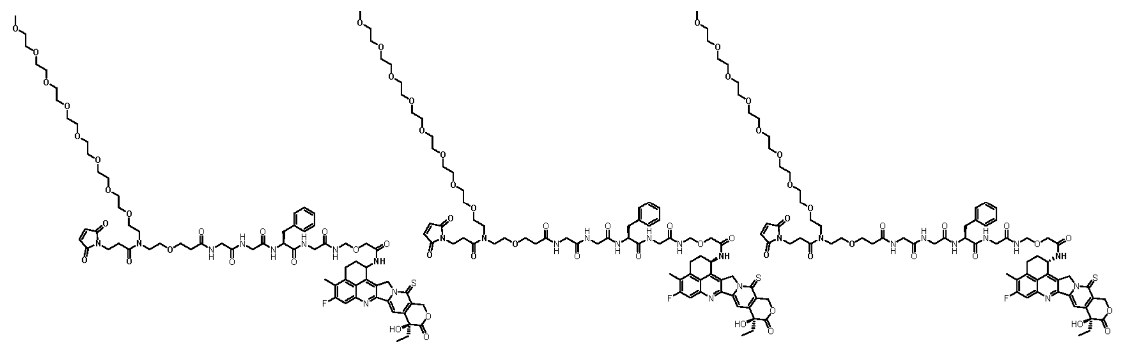

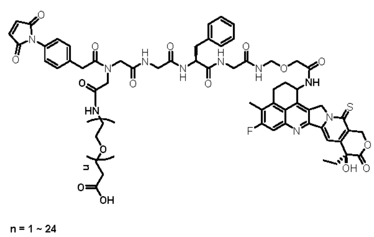

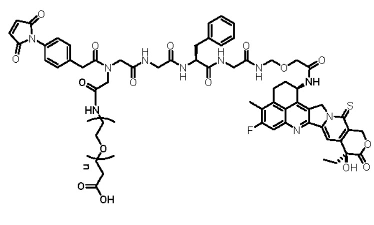

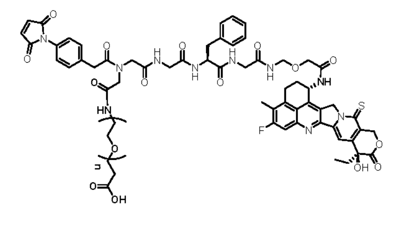

n = 1 ~ 24

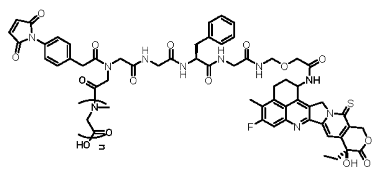
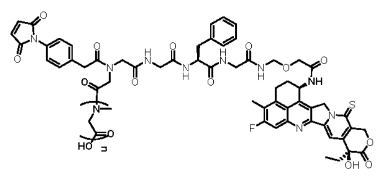
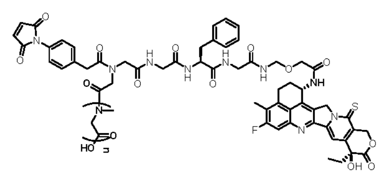

n = 1 ~ 24

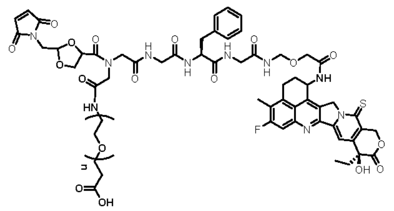
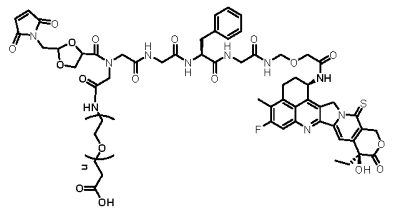
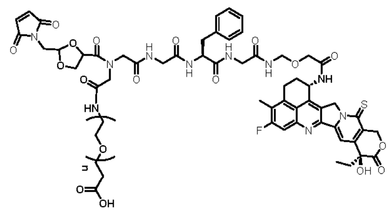

n = 1 ~ 24

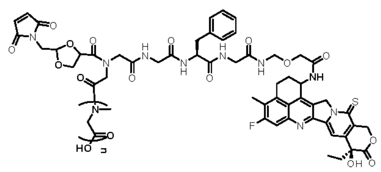
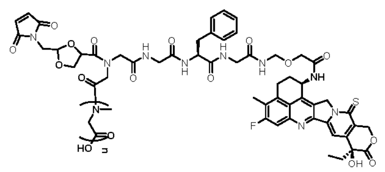
and
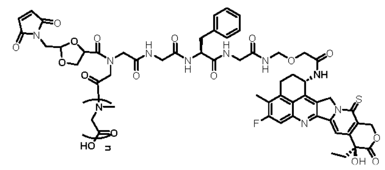

n = 1 ~ 24

**48.** A linker as shown in formula (L), which connects a drug unit to a ligand to form a ligand-drug conjugate:

$L_1$-$L_2$-$L_3$-$L_4$-$L_5$ (L),

wherein, $L_1$, $L_2$, $L_3$, $L_4$, and $L_5$ are defined as any one of claims 1-34.

**49.** The linker according to claim 48, **characterized in that** the linker is selected from the group consisting of

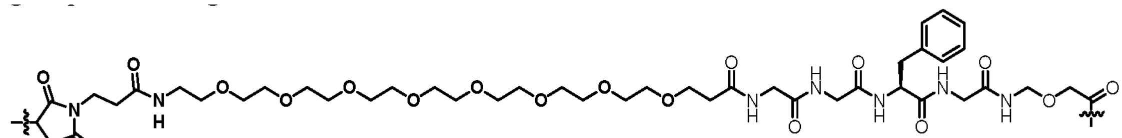

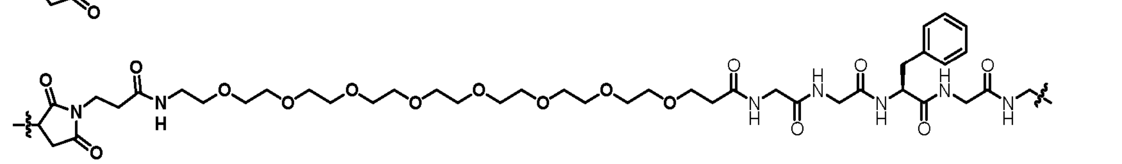

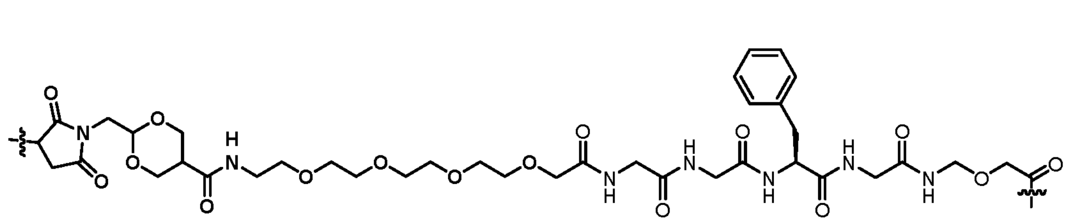

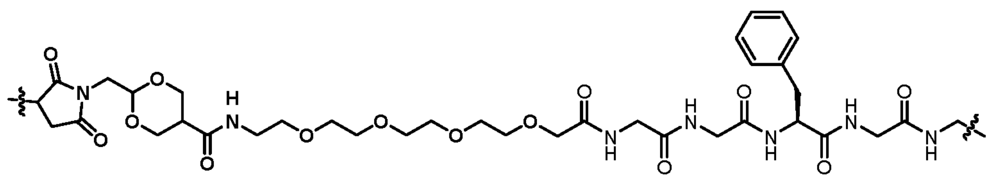

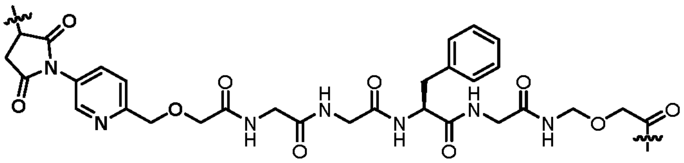

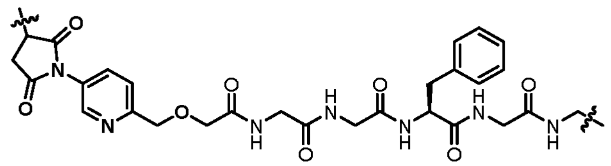

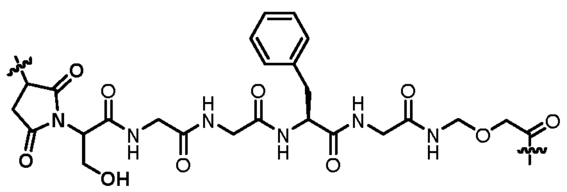

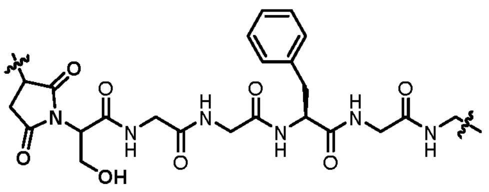

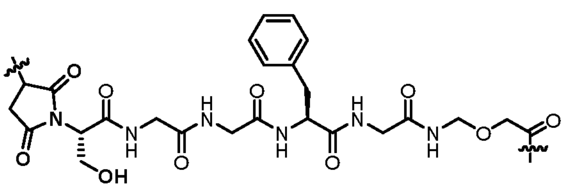

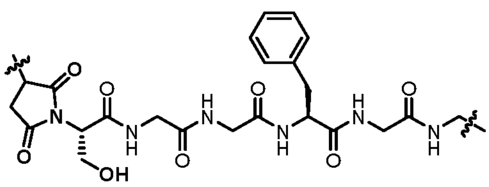

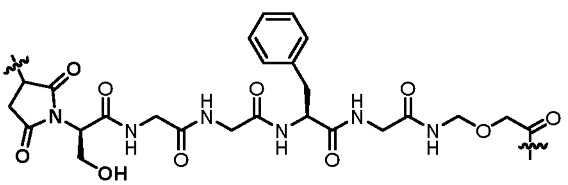

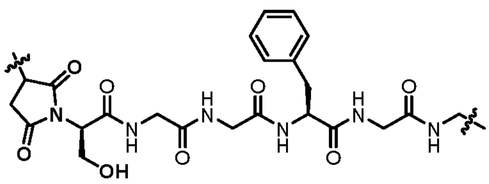

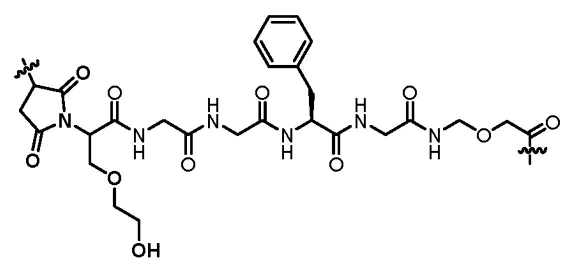

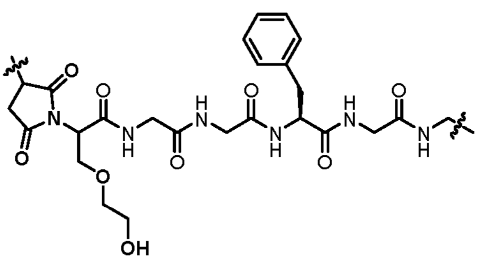

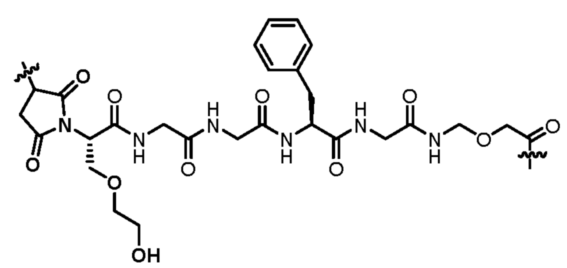

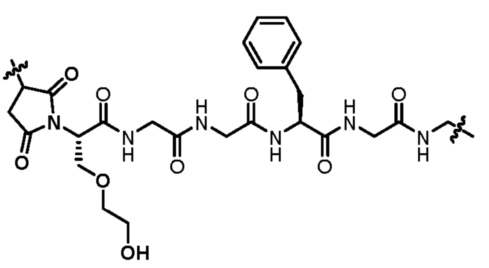

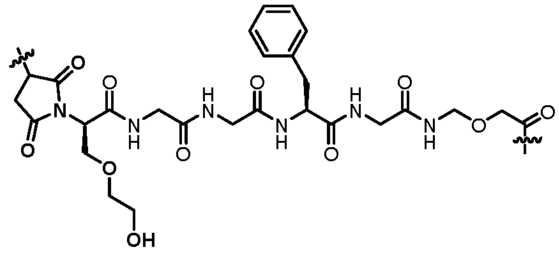

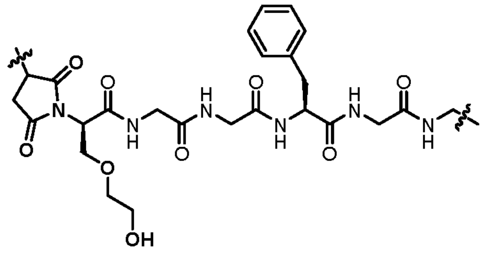

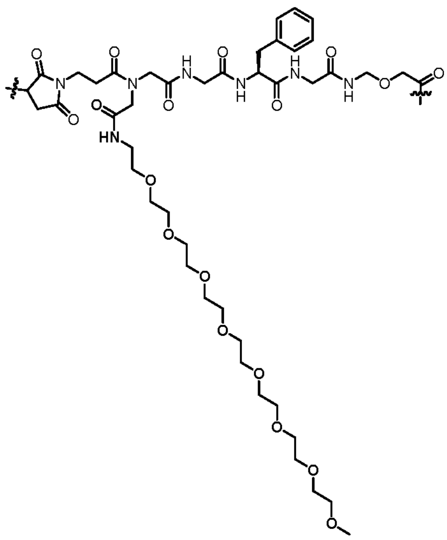

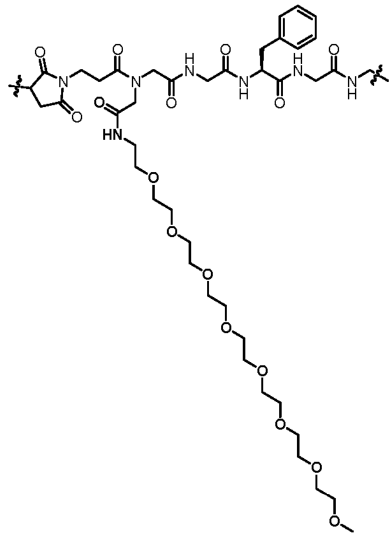

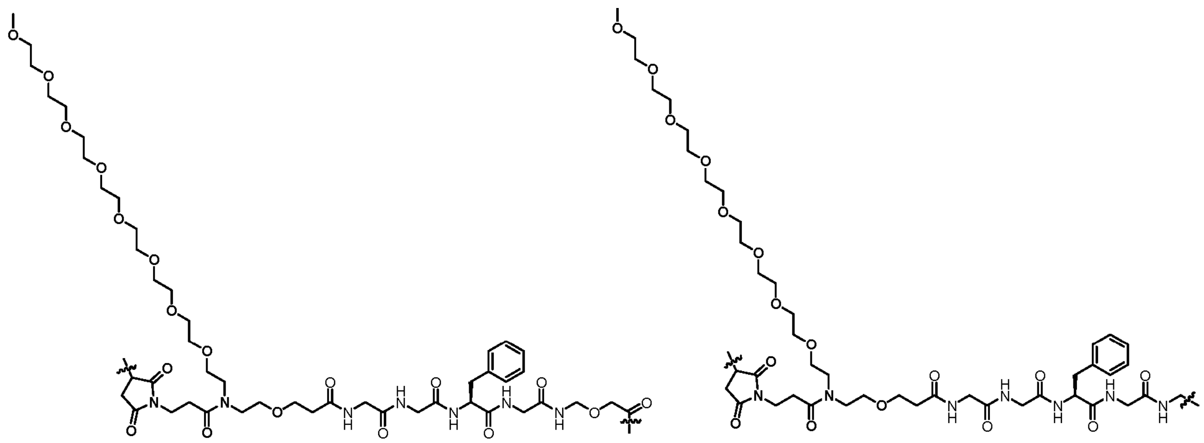

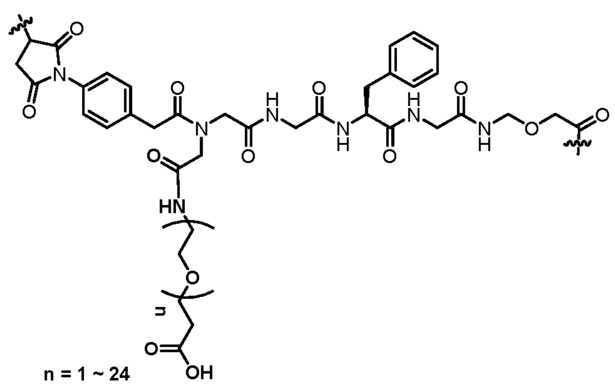

n = 1 ~ 24

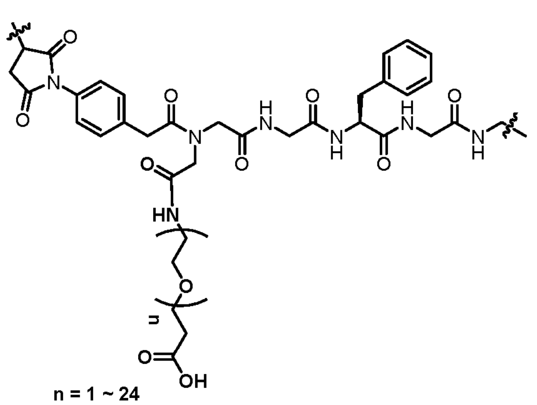

n = 1 ~ 24

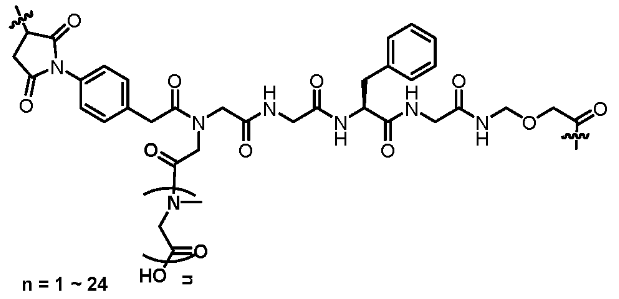

n = 1 ~ 24

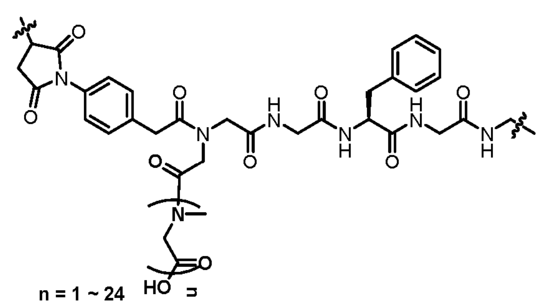

n = 1 ~ 24

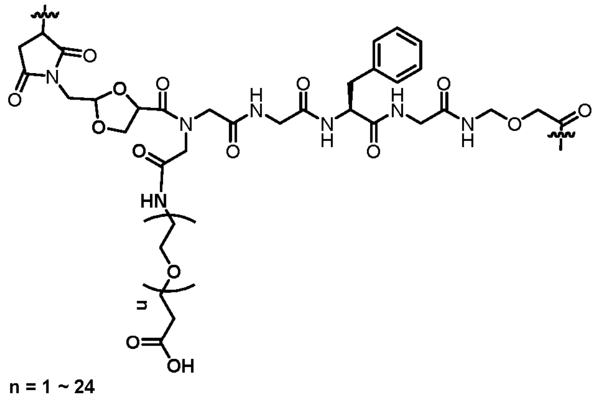

n = 1 ~ 24

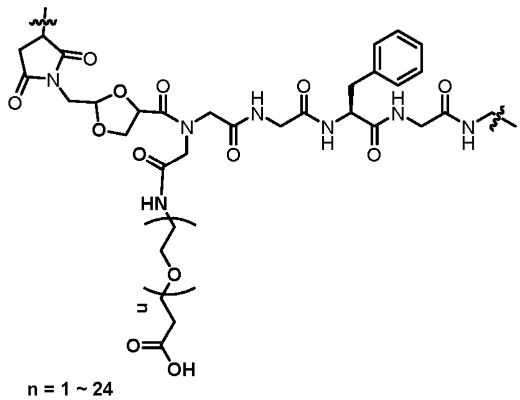

n = 1 ~ 24

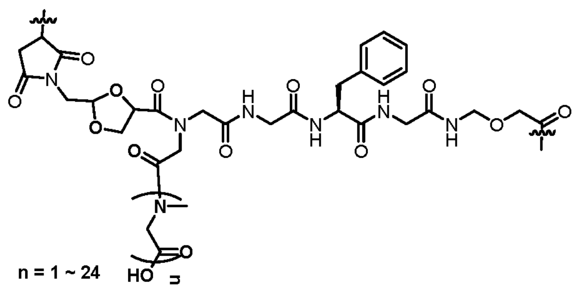

n = 1 ~ 24

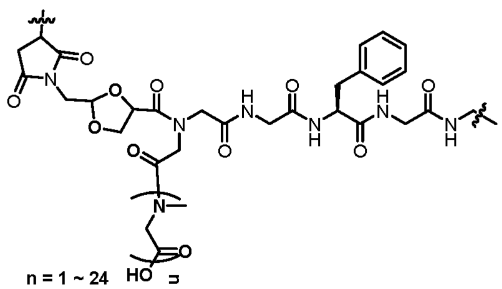

n = 1 ~ 24

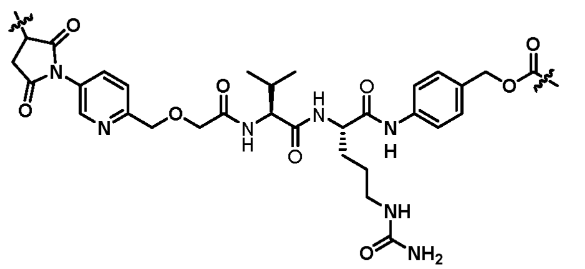

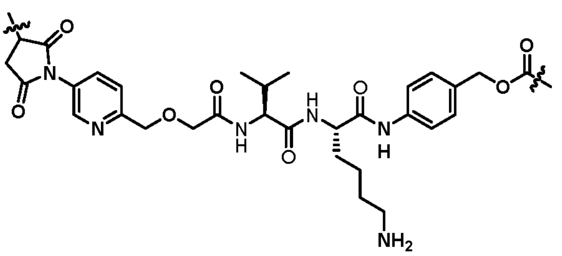

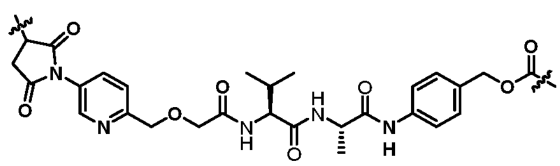

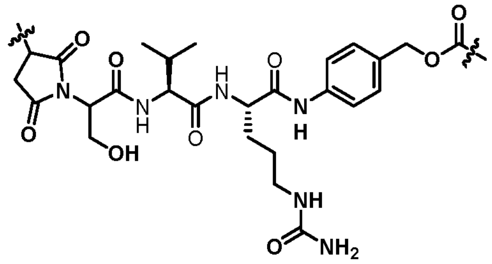
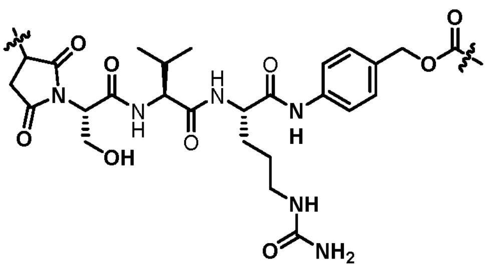

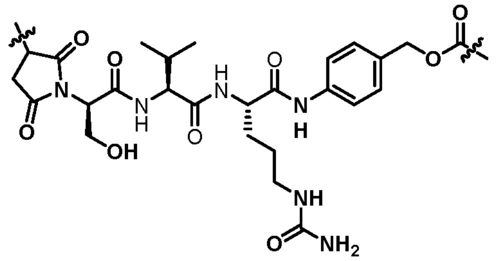

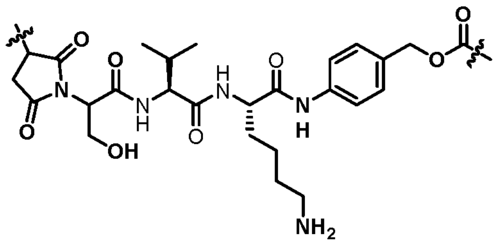
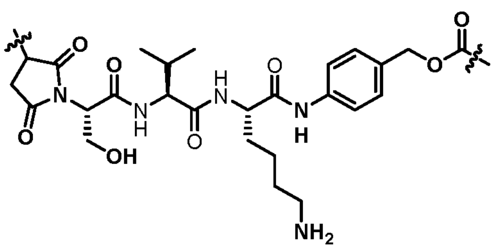

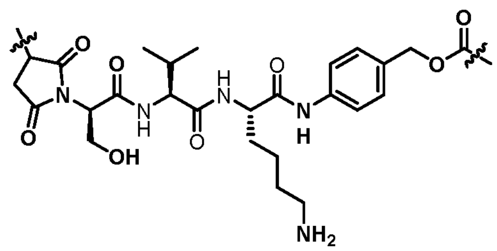

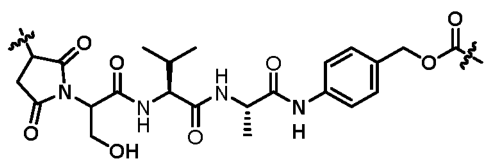
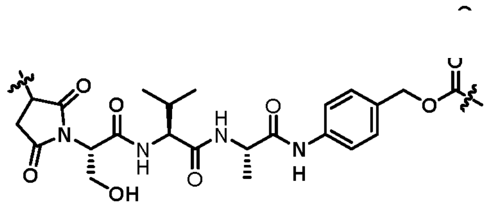

and

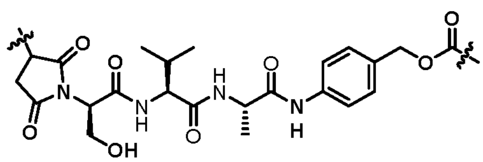

**50.** The linker according to any one of claims 48-49, **characterized in that** the linker is connected to the ligand via $L_1$ segment and to $P_1$ via $L_5$ segment to form a ligand-drug conjugate; and the P1 is selected from: glycopeptide antibiotics, such as bleomycin or pingyangmycin; DNA topoisomerase inhibitors, such as topoisomerase I inhibitors (such as camptothecin, hydroxycamptothecin, 9-aminocamptothecin, SN-38, irinotecan, exatecan, topotecan, belotecan or rubitecan, DXd, etc.), topoisomerase II inhibitors (such as actinomycin D, doxorubicin, daunorubicin,

mitoxantrone, podophyllotoxin or etoposide); DNA synthesis inhibitors, such as methotrexate, 5-fluorouracil, cytarabine, gemcitabine, mercaptopurine, pentostatin, fludarabine, cladribine, or nelarabine; structural protein-targeting agents, such as tubulin inhibitors, vinca alkaloids, vincristine, vinblastine, paclitaxel, docetaxel, or cabataside; tumor signaling pathway inhibitors, such as serine/threonine kinase inhibitors, tyrosine kinase inhibitors, aspartate kinase inhibitors, or histidine kinase inhibitors; proteasome inhibitors; histone deacetylase (HDAC) inhibitors; tumor angiogenesis inhibitor; cyclin inhibitors; maytansine derivative (such as DM1, DM4, etc.); calicheamicin derivatives; Auristatin derivatives (such as monomethyl auristatin E (MMAE), monomethyl auristatin F (MMAF), auristatin E, auristatin F, etc.); pyrrolobenzodiazepine dimers (PBD) derivatives; amatoxin derivatives (such as α-Amanitin, etc.); anthracene rings; duocarmycins; eribulin; melphalan; mitomycin C; chlorambucil; TLR agonist; STING agonist; glucocorticoids, and groups formed by the dehydrogenation of other active substances that inhibit tumor cell growth, promote tumor cell apoptosis or necrosis.

**51.** A linker precursor as shown in formula (L-1) for obtaining a ligand-drug conjugate formed by connecting a drug unit to a ligand:

$$L_A\text{-}R^g \quad (L\text{-}1),$$

wherein, $R^g$ is H, OH, O ($C_1$-$C_6$ alkyl); the $L_A$ is defined as in claim 46.

**52.** The precursor of the linker precursor according to claim 51, **characterized in that** the linker precursor is selected from the group consisting of

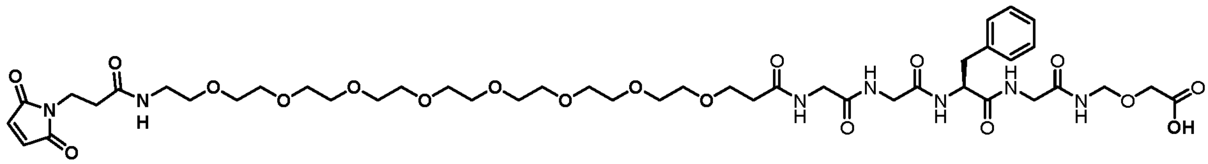

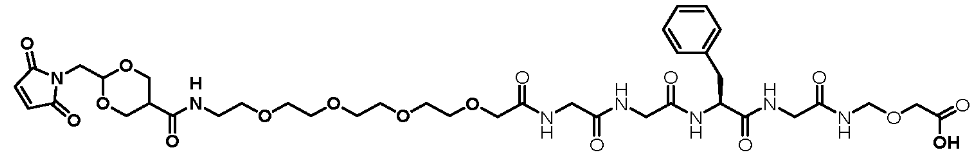

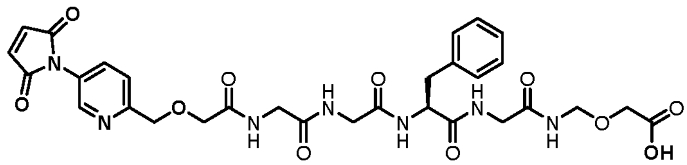

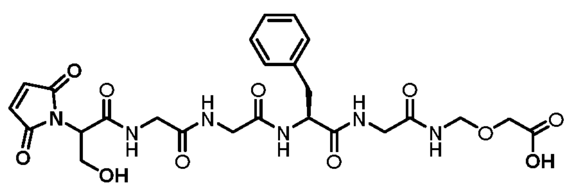

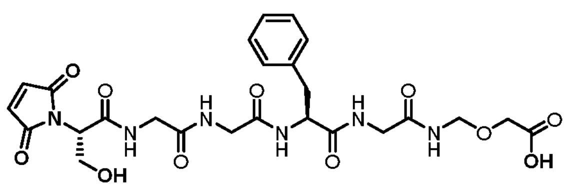

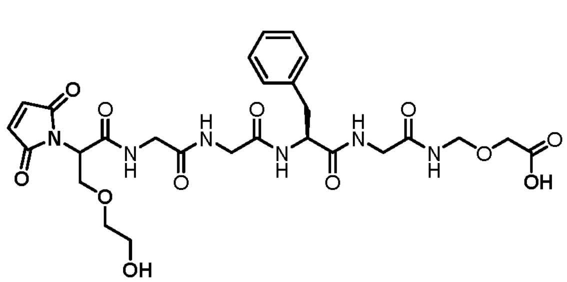

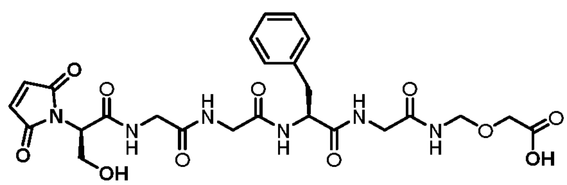

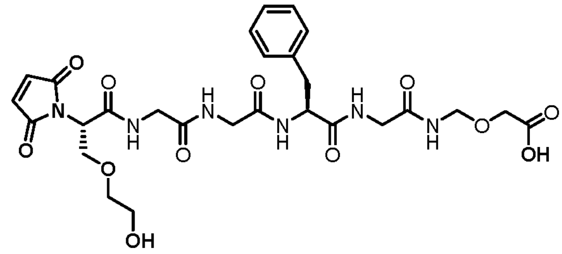

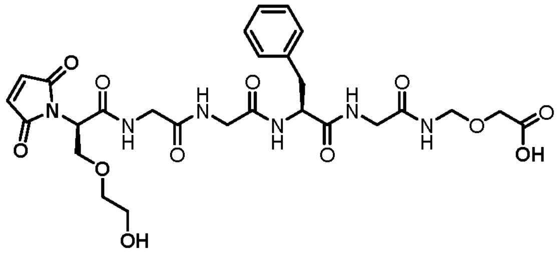

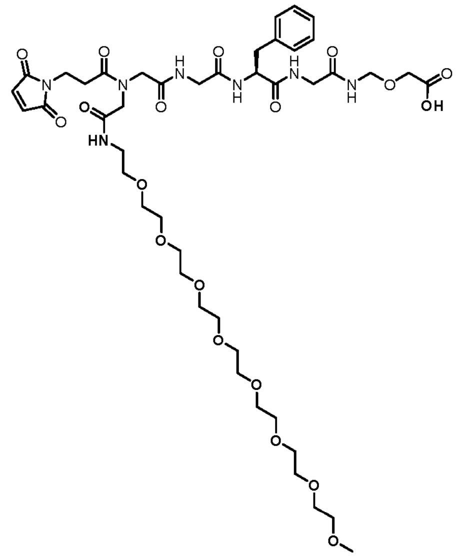

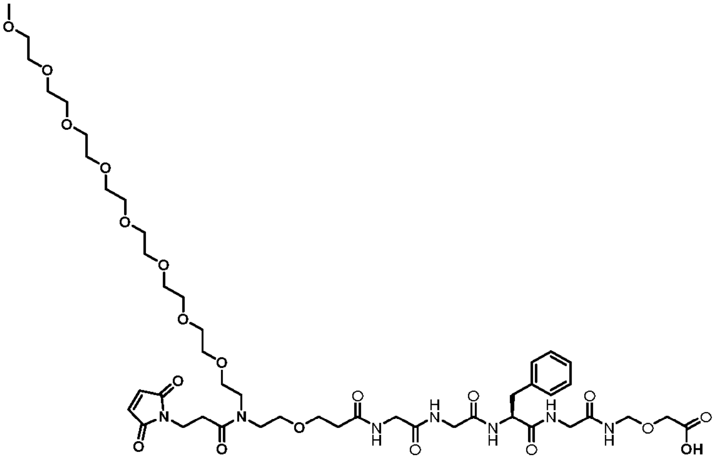

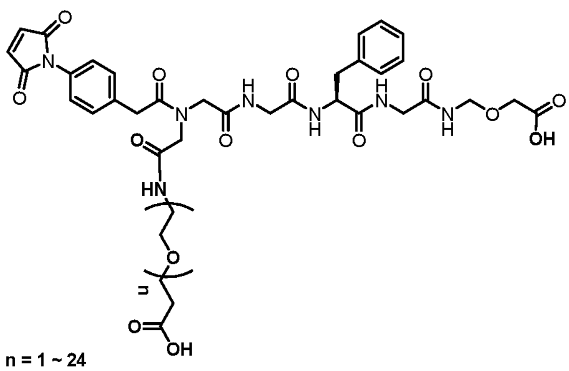

n = 1 ~ 24

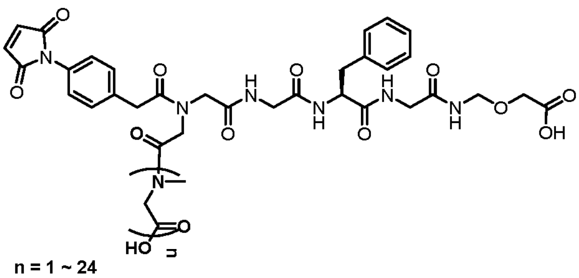

n = 1 ~ 24

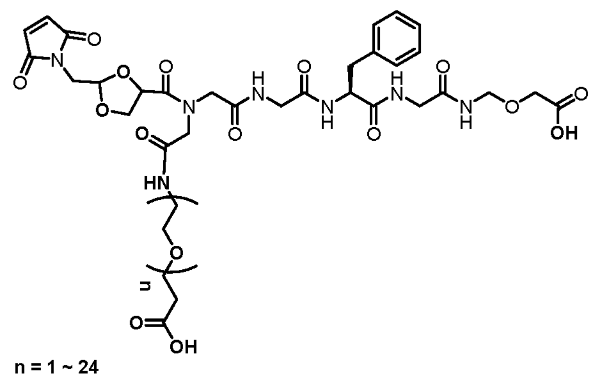

n = 1 ~ 24

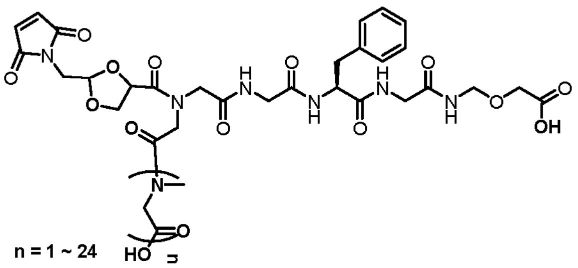

n = 1 ~ 24

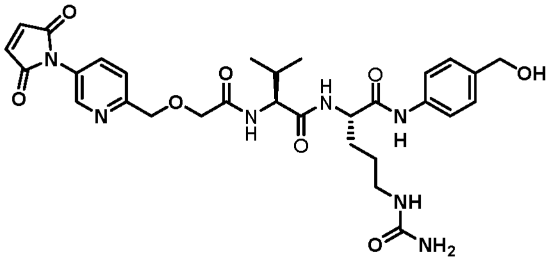
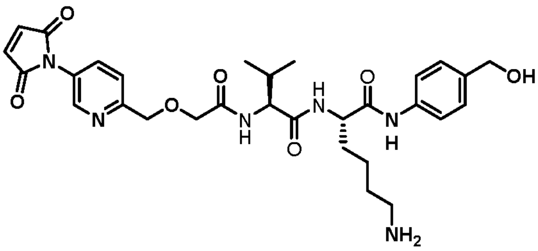
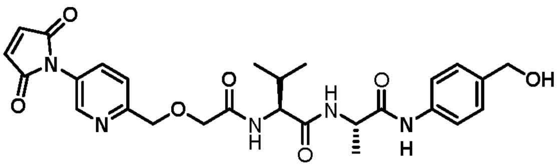
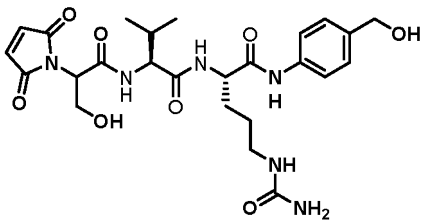
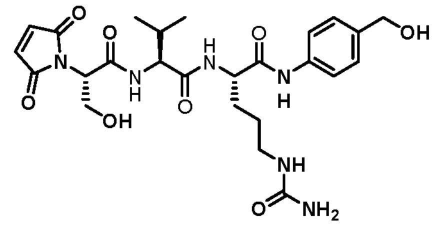
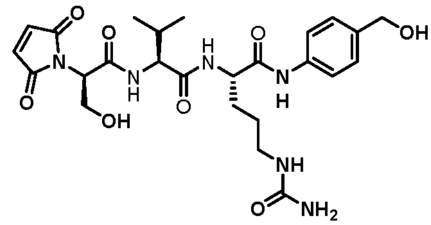
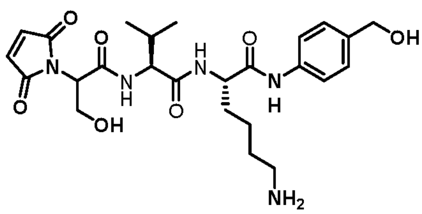
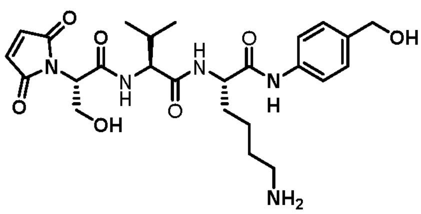
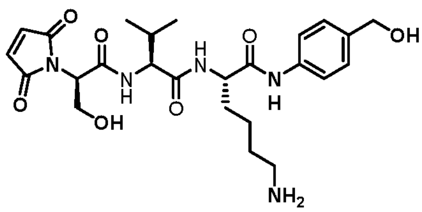
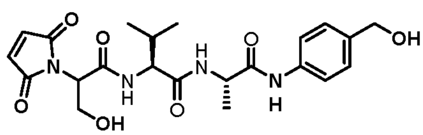
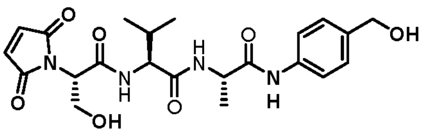

and

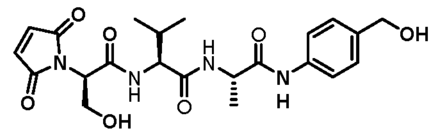

.

## INTERNATIONAL SEARCH REPORT

International application No. **PCT/CN2023/134190**

**A. CLASSIFICATION OF SUBJECT MATTER**

A61K47/68(2017.01)i; C07D491/22(2006.01)i; C07K16/28(2006.01)i; A61K39/395(2006.01)i; A61P35/00(2006.01)i

According to International Patent Classification (IPC) or to both national classification and IPC

**B. FIELDS SEARCHED**

Minimum documentation searched (classification system followed by classification symbols)

IPC:A61K C07D C07K A61P

Documentation searched other than minimum documentation to the extent that such documents are included in the fields searched

Electronic data base consulted during the international search (name of data base and, where practicable, search terms used)

CNABS, DWPI, WPABS, CNTXT, WOTXT, EPTXT, USTXT, 中国专利生物序列检索系统 (China Patents Biological Sequence Search System), CNKI, ISI Web of Science, PubMed, GenBank, EMBL, STNext: 明慧医药, 李傲, 喜树碱, 依沙替康, 依喜替康, 硫代, 偶联物, 缀合物, 抗体, IGF-1R, IGF1R, camptothecin, exatecan, conjugate, antibody, SEQ ID NOs: 1-5

**C. DOCUMENTS CONSIDERED TO BE RELEVANT**

| Category* | Citation of document, with indication, where appropriate, of the relevant passages | Relevant to claim No. |
|---|---|---|
| X | CN 113766933 A (SHANGHAI FUDAN-ZHANGJIANG BIO-PHARMACEUTICAL CO., LTD.) 07 December 2021 (2021-12-07) claims 1-15 | 1-36, 43-52 |
| PX | WO 2022262789 A1 (MINGHUI PHARMACEUTICAL (HANGZHOU) LTD. et al.) 22 December 2022 (2022-12-22) description, pages 1-2, 31-32, and 55-63, embodiments 1.1-1.4 | 1-36, 43-52 |
| X | CN 104755494 A (DAIICHI SANKYO COMPANY, LIMITED.) 01 July 2015 (2015-07-01) claims 1-49 | 1-36, 43-52 |
| X | CN 113766954 A (IMMUNOGEN, INC.) 07 December 2021 (2021-12-07) claims 1-119 | 1-36, 43-52 |
| A | WO 2022161479 A1 (MINGHUI PHARMACEUTICAL (HANGZHOU) LTD. et al.) 04 August 2022 (2022-08-04) claims 1-9 and 12 | 1, 28-31, 33, 43-45 |
| A | CN 101321784 A (ABLYNX NV) 10 December 2008 (2008-12-10) claims 2-37 | 35-42 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **15 March 2024** | **21 March 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2023/134190**

| Patent document cited in search report | | | Publication date (day/month/year) | Patent family member(s) | | | Publication date (day/month/year) |
|---|---|---|---|---|---|---|---|
| CN | 113766933 | A | 07 December 2021 | EP | 3991754 | A1 | 04 May 2022 |
| | | | | EP | 3991754 | A4 | 17 May 2023 |
| | | | | KR | 20220025861 | A | 03 March 2022 |
| | | | | AU | 2020301289 | A1 | 24 February 2022 |
| | | | | WO | 2020259258 | A1 | 30 December 2020 |
| | | | | BR | 112021026580 | A2 | 03 May 2022 |
| | | | | US | 2022233708 | A1 | 28 July 2022 |
| | | | | JP | 2022542222 | A | 30 September 2022 |
| | | | | JP | 7407845 | B2 | 04 January 2024 |
| | | | | CA | 3144790 | A1 | 30 December 2020 |
| WO | 2022262789 | A1 | 22 December 2022 | TW | 202313125 | A | 01 April 2023 |
| | | | | AU | 2022294861 | A1 | 01 February 2024 |
| | | | | IL | 309457 | A | 01 February 2024 |
| | | | | CA | 3223304 | A1 | 22 December 2022 |
| CN | 104755494 | A | 01 July 2015 | LT | 2907824 | T | 11 June 2018 |
| | | | | US | 2016279259 | A1 | 29 September 2016 |
| | | | | US | 9808537 | B2 | 07 November 2017 |
| | | | | CA | 3021435 | A1 | 17 April 2014 |
| | | | | CA | 3021435 | C | 12 October 2021 |
| | | | | BR | 122021014396 | B1 | 05 July 2022 |
| | | | | JP | 2022008581 | A | 13 January 2022 |
| | | | | JP | 7170812 | B2 | 14 November 2022 |
| | | | | JP | 6030267 | B1 | 24 November 2016 |
| | | | | JP | 2017036274 | A | 16 February 2017 |
| | | | | JP | 2023011799 | A | 24 January 2023 |
| | | | | BR | 112015006521 | A2 | 05 September 2017 |
| | | | | BR | 112015006521 | B1 | 03 March 2022 |
| | | | | SI | 2907824 | T1 | 29 June 2018 |
| | | | | SG | 10201804788 | XA | 30 July 2018 |
| | | | | PT | 2907824 | T | 07 June 2018 |
| | | | | PT | 3342785 | T | 25 February 2020 |
| | | | | CY | 1122789 | T1 | 05 May 2021 |
| | | | | NZ | 746440 | A | 29 November 2019 |
| | | | | KR | 20220100727 | A | 15 July 2022 |
| | | | | KR | 102456726 | B1 | 20 October 2022 |
| | | | | JP | 5953378 | B2 | 20 July 2016 |
| | | | | JPWO | 2014057687 | A1 | 05 September 2016 |
| | | | | US | 2020282073 | A1 | 10 September 2020 |
| | | | | US | 11633493 | B2 | 25 April 2023 |
| | | | | CA | 2885800 | A1 | 17 April 2014 |
| | | | | CA | 2885800 | C | 04 December 2018 |
| | | | | ES | 2773710 | T3 | 14 July 2020 |
| | | | | WO | 2014057687 | A1 | 17 April 2014 |
| | | | | IL | 302494 | A | 01 June 2023 |
| | | | | AU | 2020200548 | A1 | 13 February 2020 |
| | | | | AU | 2020200548 | B2 | 25 August 2022 |
| | | | | US | 2015297748 | A1 | 22 October 2015 |
| | | | | US | 10195288 | B2 | 05 February 2019 |
| | | | | TW | 202233186 | A | 01 September 2022 |
| | | | | TR | 201809636 | T4 | 23 July 2018 |

Form PCT/ISA/210 (patent family annex) (July 2022)

**INTERNATIONAL SEARCH REPORT**
**Information on patent family members**

International application No.
**PCT/CN2023/134190**

| Patent document cited in search report | Publication date (day/month/year) | Patent family member(s) | | | Publication date (day/month/year) |
|---|---|---|---|---|---|
| | | HRP | 20180870 | T1 | 13 July 2018 |
| | | MX | 2020010964 | A | 09 November 2020 |
| | | KR | 20180030734 | A | 23 March 2018 |
| | | KR | 101901558 | B1 | 21 September 2018 |
| | | KR | 20150067149 | A | 17 June 2015 |
| | | KR | 101841818 | B1 | 26 March 2018 |
| | | SG | 11201502887 | WA | 28 May 2015 |
| | | CY | 1120363 | T1 | 10 July 2019 |
| | | KR | 20180105271 | A | 27 September 2018 |
| | | KR | 102052319 | B1 | 05 December 2019 |
| | | KR | 20190135559 | A | 06 December 2019 |
| | | KR | 102087017 | B1 | 10 March 2020 |
| | | RS | 57278 | B1 | 31 August 2018 |
| | | TW | 202033499 | A | 16 September 2020 |
| | | TWI | 757740 | B | 11 March 2022 |
| | | TW | 201811746 | A | 01 April 2018 |
| | | TWI | 650312 | B | 11 February 2019 |
| | | MY | 170251 | A | 13 July 2019 |
| | | DK | 3342785 | T3 | 23 March 2020 |
| | | PL | 3342785 | T3 | 07 September 2020 |
| | | TW | 201420117 | A | 01 June 2014 |
| | | TWI | 615152 | B | 21 February 2018 |
| | | KR | 20220029776 | A | 08 March 2022 |
| | | KR | 102417310 | B1 | 05 July 2022 |
| | | EP | 3632471 | A1 | 08 April 2020 |
| | | ZA | 201501825 | B | 25 May 2022 |
| | | DK | 2907824 | T3 | 23 July 2018 |
| | | PH | 12018501433 | A1 | 27 February 2019 |
| | | PL | 2907824 | T3 | 31 August 2018 |
| | | KR | 20210132240 | A | 03 November 2021 |
| | | KR | 102369419 | B1 | 02 March 2022 |
| | | IL | 238143 | A0 | 31 May 2015 |
| | | IL | 238143 | B | 30 January 2020 |
| | | HK | 1256631 | A1 | 27 September 2019 |
| | | KR | 20210038728 | A | 07 April 2021 |
| | | KR | 102320907 | B1 | 02 November 2021 |
| | | SI | 3342785 | T1 | 28 February 2020 |
| | | KR | 20200026323 | A | 10 March 2020 |
| | | KR | 102237639 | B1 | 07 April 2021 |
| | | JP | 2016196484 | A | 24 November 2016 |
| | | JP | 6186045 | B2 | 23 August 2017 |
| | | KR | 20230142808 | A | 11 October 2023 |
| | | NZ | 705394 | A | 26 October 2018 |
| | | RU | 2015113767 | A | 10 December 2016 |
| | | RU | 2664465 | C2 | 17 August 2018 |
| | | IL | 271760 | A | 27 February 2020 |
| | | IL | 271760 | B | 01 August 2022 |
| | | RS | 60000 | B1 | 30 April 2020 |
| | | AU | 2013328111 | A1 | 12 March 2015 |
| | | AU | 2013328111 | B2 | 02 November 2017 |

Form PCT/ISA/210 (patent family annex) (July 2022)

**INTERNATIONAL SEARCH REPORT**
**Information on patent family members**

International application No.
**PCT/CN2023/134190**

| Patent document cited in search report | Publication date (day/month/year) | Patent family member(s) | Publication date (day/month/year) |
|---|---|---|---|
| | | EP 2907824 A1 | 19 August 2015 |
| | | EP 2907824 A4 | 08 June 2016 |
| | | EP 2907824 B1 | 11 April 2018 |
| | | ES 2671644 T3 | 07 June 2018 |
| | | TW 201920098 A | 01 June 2019 |
| | | TWI 696611 B | 21 June 2020 |
| | | MX 2015003903 A | 17 July 2015 |
| | | MX 364484 B | 29 April 2019 |
| | | NZ 740948 A | 29 November 2019 |
| | | NZ 746439 A | 29 November 2019 |
| | | HK 1210477 A1 | 22 April 2016 |
| | | EP 3342785 A1 | 04 July 2018 |
| | | EP 3342785 B1 | 25 December 2019 |
| | | JP 2020180124 A | 05 November 2020 |
| | | JP 6952835 B2 | 27 October 2021 |
| | | BR 122021014365 B1 | 05 July 2022 |
| | | KR 20230086800 A | 15 June 2023 |
| | | KR 102584005 B1 | 27 September 2023 |
| | | HUE 039000 T2 | 28 December 2018 |
| | | KR 20220146669 A | 01 November 2022 |
| | | KR 102498405 B1 | 09 February 2023 |
| | | JP 2018188455 A | 29 November 2018 |
| | | JP 6715888 B2 | 01 July 2020 |
| | | MX 2019004502 A | 21 August 2019 |
| | | PH 12015500667 A1 | 18 May 2015 |
| | | ZA 201900820 B | 31 August 2022 |
| | | LT 3342785 T | 10 February 2020 |
| | | US 2019008981 A1 | 10 January 2019 |
| | | US 10973924 B2 | 13 April 2021 |
| | | RU 2018128384 A | 02 October 2018 |
| | | RU 2018128384 A3 | 20 December 2021 |
| | | HRP 20200353 T1 | 12 June 2020 |
| | | HUE 048748 T2 | 28 August 2020 |
| | | IL 294622 A | 01 September 2022 |
| | | IL 294622 B1 | 01 June 2023 |
| | | IL 294622 B2 | 01 October 2023 |
| | | AU 2022203560 A1 | 16 June 2022 |
| | | KR 20230024433 A | 20 February 2023 |
| | | KR 102540419 B1 | 05 June 2023 |
| | | AU 2018200308 A1 | 01 February 2018 |
| | | AU 2018200308 B2 | 07 November 2019 |
| | | JP 2018008982 A | 18 January 2018 |
| | | JP 6371452 B2 | 08 August 2018 |
| CN 113766954 A | 07 December 2021 | EP 4316524 A2 | 07 February 2024 |
| | | TW 202106691 A | 16 February 2021 |
| | | DK 3958977 T3 | 11 December 2023 |
| | | AU 2020263231 A1 | 18 November 2021 |
| | | PT 3958977 T | 15 December 2023 |
| | | JP 2022529854 A | 24 June 2022 |
| | | FI 3958977 T3 | 12 December 2023 |

Form PCT/ISA/210 (patent family annex) (July 2022)

**INTERNATIONAL SEARCH REPORT**
**Information on patent family members**

International application No.
**PCT/CN2023/134190**

| Patent document cited in search report | Publication date (day/month/year) | Patent family member(s) | Publication date (day/month/year) |
|---|---|---|---|
| | | KR 20220027828 A | 08 March 2022 |
| | | US 2021077482 A1 | 18 March 2021 |
| | | US 11229639 B2 | 25 January 2022 |
| | | LT 3958977 T | 27 December 2023 |
| | | SG 11202110922 QA | 28 October 2021 |
| | | CA 3137125 A1 | 29 October 2020 |
| | | RS 64961 B1 | 31 January 2024 |
| | | WO 2020219287 A1 | 29 October 2020 |
| | | US 2022133711 A1 | 05 May 2022 |
| | | EP 3958977 A1 | 02 March 2022 |
| | | EP 3958977 B1 | 13 September 2023 |
| | | IL 286846 A | 31 October 2021 |
| WO 2022161479 A1 | 04 August 2022 | AU 2022212854 A1 | 14 September 2023 |
| | | IL 304779 A | 01 September 2023 |
| | | JP 2024504489 A | 31 January 2024 |
| | | KR 20230162595 A | 28 November 2023 |
| | | EP 4286389 A1 | 06 December 2023 |
| | | CA 3206519 A1 | 04 August 2022 |
| CN 101321784 A | 10 December 2008 | EP 1934259 A2 | 25 June 2008 |
| | | AU 2006301426 A1 | 19 April 2007 |
| | | CA 2624781 A1 | 19 April 2007 |
| | | WO 2007042289 A2 | 19 April 2007 |
| | | WO 2007042289 A3 | 04 October 2007 |
| | | JP 2009511032 A | 19 March 2009 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7238785 B2
- WO 2003034903 A2
- US 5952484 A
- WO 2021244590 A1
- WO 2022262789 A1